# EUROPEAN PATENT APPLICATION

(11) **EP 3 456 193 A1**
(43) Date of publication of application: **20.03.2019**
(21) Application number: 18184003.4
(22) Date of filing: 29.06.2009
(51) Int. Cl.: A01K 67/027, C12N 15/85, C07K 16/00, C12N 5/10

(54) **ANTIBODY PRODUCING NON-HUMAN MAMMALS**

(30) Priority: 27.06.2008 WO PCT/NL2008/050430
(62) Divisional of application: 12175544.1
(71) Applicant: Merus N.V., 3584 CM Utrecht (NL)
(72) Inventor: Houtzager, Erwin, 3702 SC Zeist (NL); Pinto, Rui Daniel, 3581 JS Utrecht (NL); Logtenberg, Ton, 3584 CM Utrecht (NL); Throsby, Mark, 3584 CM Utrecht (NL)
(74) Representative: Lahrtz, Fritz

(57) **Abstract**

The invention provides transgenic, non-human animals comprising a nucleic acid encoding an immunoglobulin light chain, whereby the immunoglobulin light chain is human, human-like, or humanized. The nucleic acid is provided with a means that renders it resistant to DNA rearrangements and/or somatic hypermutations. In one embodiment, the nucleic acid comprises an expression cassette for the expression of a desired molecule in cells during a certain stage of development in cells developing into mature B cells. The invention further provides methods for producing an immunoglobulin from the transgenic, non-human animal.

SEQUENCE LISTING

<110>  Merus N.V.

<120>  Antibody producing non-human mammals.

<130>  M70121PCEPT5

<140>  EP12175544.1
<141>  2009-06-29

<150>  PCT/NL2008/050430
<151>  2008-06-27

<160>  101

<170>  PatentIn version 3.3

<210>  1
<211>  20
<212>  DNA
<213>  Artificial

<220>
<223>  primer

<400>  1
ccctttccaa tctttatggg                                                    20


<210>  2
<211>  23
<212>  DNA
<213>  Artificial

<220>
<223>  primer

<400>  2
aggtggattg gtgtcttttt ctc                                                23


<210>  3
<211>  22
<212>  DNA
<213>  Artificial

<220>
<223>  primer

<400>  3
gtcatgtcgg cgaccctacg cc                                                 22


<210>  4
<211>  20
<212>  DNA
<213>  Artificial

<220>
<223>  primer

<400>  4

tgccaggggg aagaccgatg 20

<210> 5
<211> 38
<212> DNA
<213> Artificial

<220>
<223> primer

<400> 5
gccggccatg gccgaggtrm agcttcagga gtcaggac 38

<210> 6
<211> 38
<212> DNA
<213> Artificial

<220>
<223> primer

<400> 6
gccggccatg gccgaggtsc agctkcagca gtcaggac 38

<210> 7
<211> 36
<212> DNA
<213> Artificial

<220>
<223> primer

<400> 7
gccggccatg gcccaggtgc agctgaagsa stcagg 36

<210> 8
<211> 38
<212> DNA
<213> Artificial

<220>
<223> primer

<400> 8
gccggccatg gccgaggtgc agcttcagga gtcsggac 38

<210> 9
<211> 38
<212> DNA
<213> Artificial

<220>
<223> primer

<400> 9
gccggccatg gccgargtcc agctgcaaca gtcyggac 38

<210>  10
<211>  36
<212>  DNA
<213>  Artificial

<220>
<223>  primer

<400>  10
gccggccatg gcccaggtcc agctkcagca atctgg                                36


<210>  11
<211>  36
<212>  DNA
<213>  Artificial

<220>
<223>  primer

<400>  11
gccggccatg gcccagstbc agctgcagca gtctgg                                36


<210>  12
<211>  38
<212>  DNA
<213>  Artificial

<220>
<223>  primer

<400>  12
gccggccatg gcccaggtyc agctgcagca gtctggrc                              38


<210>  13
<211>  36
<212>  DNA
<213>  Artificial

<220>
<223>  primer

<400>  13
gccggccatg gcccaggtyc agctycagca gtctgg                                36


<210>  14
<211>  39
<212>  DNA
<213>  Artificial

<220>
<223>  primer

<400>  14
gccggccatg gccgaggtcc arctgcaaca atctggacc                             39


<210>  15
<211>  37
<212>  DNA

<213>  Artificial

<220>
<223>  primer

<400>  15
gccggccatg gcccaggtcc acgtgaagca gtctggg                                37


<210>  16
<211>  35
<212>  DNA
<213>  Artificial

<220>
<223>  primer

<400>  16
gccggccatg gccgaggtga asstggtgga atctg                                  35


<210>  17
<211>  35
<212>  DNA
<213>  Artificial

<220>
<223>  primer

<400>  17
gccggccatg gccgavgtga agytggtgga gtctg                                  35


<210>  18
<211>  38
<212>  DNA
<213>  Artificial

<220>
<223>  primer

<400>  18
gccggccatg gccgaggtgc agskggtgga gtctgggg                               38


<210>  19
<211>  37
<212>  DNA
<213>  Artificial

<220>
<223>  primer

<400>  19
gccggccatg gccgakgtgc amctggtgga gtctggg                                37


<210>  20
<211>  36
<212>  DNA
<213>  Artificial

<220>

<223>  primer

<400>  20
gccggccatg gccgaggtga agctgatgga rtctgg                                36


<210>  21
<211>  38
<212>  DNA
<213>  Artificial

<220>
<223>  primer

<400>  21
gccggccatg gccgaggtgc arcttgttga gtctggtg                              38


<210>  22
<211>  37
<212>  DNA
<213>  Artificial

<220>
<223>  primer

<400>  22
gccggccatg gccgargtra agcttctcga gtctgga                               37


<210>  23
<211>  36
<212>  DNA
<213>  Artificial

<220>
<223>  primer

<400>  23
gccggccatg gccgaagtga arsttgagga gtctgg                                36


<210>  24
<211>  37
<212>  DNA
<213>  Artificial

<220>
<223>  primer

<400>  24
gccggccatg gccgaagtga tgctggtgga gtctggg                               37


<210>  25
<211>  38
<212>  DNA
<213>  Artificial

<220>
<223>  primer

<400>  25

gccggccatg gcccaggtta ctctraaagw gtstggcc                                38

<210>  26
<211>  36
<212>  DNA
<213>  Artificial

<220>
<223>  primer

<400>  26
gccggccatg gcccaggtcc aactvcagca rcctgg                                  36

<210>  27
<211>  35
<212>  DNA
<213>  Artificial

<220>
<223>  primer

<400>  27
gccggccatg gcccaggtyc arctgcagca gtctg                                   35

<210>  28
<211>  36
<212>  DNA
<213>  Artificial

<220>
<223>  primer

<400>  28
gccggccatg gccgatgtga acttggaagt gtctgg                                  36

<210>  29
<211>  36
<212>  DNA
<213>  Artificial

<220>
<223>  primer

<400>  29
gccggccatg gccgaggtga aggtcatcga gtctgg                                  36

<210>  30
<211>  45
<212>  DNA
<213>  Artificial

<220>
<223>  primer

<220>
<221>  misc_feature
<222>  (44)..(44)

<223>  n is a, c, g, or t

<400>  30
cagtcacaga tcctcgcgaa ttggcccagc cggccatggc csang                45


<210>  31
<211>  45
<212>  DNA
<213>  Artificial

<220>
<223>  primer


<220>
<221>  misc_feature
<222>  (44)..(44)
<223>  n is a, c, g, or t

<400>  31
cagtcacaga tcctcgcgaa ttggcccagc cggccatggc csanc                45


<210>  32
<211>  37
<212>  DNA
<213>  Artificial

<220>
<223>  primer

<400>  32
gggggtgtcg ttttggctga ggagacggtg accgtgg                         37


<210>  33
<211>  37
<212>  DNA
<213>  Artificial

<220>
<223>  primer

<400>  33
gggggtgtcg ttttggctga ggagactgtg agagtgg                         37


<210>  34
<211>  37
<212>  DNA
<213>  Artificial

<220>
<223>  primer

<400>  34
gggggtgtcg ttttggctgc agagacagtg accagag                         37


<210>  35
<211>  37
<212>  DNA

<213>  Artificial

<220>
<223>  primer

<400>  35
gggggtgtcg ttttggctga ggagacggtg actgagg                                37


<210>  36
<211>  37
<212>  DNA
<213>  Artificial

<220>
<223>  primer

<400>  36
gggggtgtcg ttttggctga ggagacggtg accgtgg                                37


<210>  37
<211>  37
<212>  DNA
<213>  Artificial

<220>
<223>  primer

<400>  37
gggggtgtcg ttttggctga ggagacggtg acagtgg                                37


<210>  38
<211>  37
<212>  DNA
<213>  Artificial

<220>
<223>  primer

<400>  38
gggggtgtcg ttttggctga ggagacggtg accagag                                37


<210>  39
<211>  37
<212>  DNA
<213>  Artificial

<220>
<223>  primer

<400>  39
gggggtgtcg ttttggctga ggagacggtg accgagg                                37


<210>  40
<211>  15
<212>  PRT
<213>  Artificial

<220>

<223>  CDR3

<400>  40

His Gly Ala Tyr Tyr Thr Tyr Asp Glu Lys Ala Trp Phe Ala Tyr
1               5                   10                  15


<210>  41
<211>  15
<212>  PRT
<213>  Artificial

<220>
<223>  CDR3

<400>  41

His Gly Ala Phe Tyr Thr Tyr Asp Glu Lys Pro Trp Phe Ala Tyr
1               5                   10                  15


<210>  42
<211>  15
<212>  PRT
<213>  Artificial

<220>
<223>  CDR3

<400>  42

His Ile Ser Tyr Tyr Arg Tyr Asp Glu Glu Val Ser Phe Ala Tyr
1               5                   10                  15


<210>  43
<211>  7
<212>  PRT
<213>  Artificial

<220>
<223>  CDR3

<400>  43

Gly Trp Arg Ala Phe Ala Tyr
1               5


<210>  44
<211>  10
<212>  PRT
<213>  Artificial

<220>
<223>  CDR3

<400>  44

Asp Arg Gly Asn Tyr Tyr Gly Met Asp Tyr
1               5                   10

<210>  45
<211>  12
<212>  PRT
<213>  Artificial

<220>
<223>  CDR3

<400>  45

Leu Gly Asp Tyr Tyr Val Asp Trp Phe Phe Ala Val
1               5                   10


<210>  46
<211>  8
<212>  PRT
<213>  Artificial

<220>
<223>  CDR3

<400>  46

Asn Phe Pro Ala Trp Phe Ala Phe
1               5


<210>  47
<211>  17
<212>  PRT
<213>  Artificial

<220>
<223>  CDR3

<400>  47

Ser Phe Thr Pro Val Pro Phe Tyr Tyr Gly Tyr Asp Trp Tyr Phe Asp
1               5                   10                  15


Val



<210>  48
<211>  9
<212>  PRT
<213>  Artificial

<220>
<223>  CDR3

<400>  48

Ser Asp Tyr Asp Trp Tyr Phe Asp Val
1               5


<210>  49
<211>  10

<212>  PRT
<213>  Artificial

<220>
<223>  CDR3

<400>  49

Asp Ser Lys Trp Ala Tyr Tyr Phe Asp Tyr
1               5                   10


<210>  50
<211>  10
<212>  PRT
<213>  Artificial

<220>
<223>  CDR3

<400>  50

Gly Asp Tyr Thr Gly Tyr Gly Met Asp Tyr
1               5                   10


<210>  51
<211>  10
<212>  PRT
<213>  Artificial

<220>
<223>  CDR3

<400>  51

Gly Gly Tyr Asp Gly Tyr Trp Phe Pro Tyr
1               5                   10


<210>  52
<211>  15
<212>  PRT
<213>  Artificial

<220>
<223>  epitope IGKV1-39

<400>  52

Asp Ile Gln Met Thr Gln Ser Pro Ser Ser Leu Ser Ala Ser Val
1               5                   10                  15


<210>  53
<211>  15
<212>  PRT
<213>  Artificial

<220>
<223>  epitope IGKV1-39

<400>  53

Met Thr Gln Ser Pro Ser Ser Leu Ser Ala Ser Val Gly Asp Arg
1               5                   10                  15


<210>  54
<211>  15
<212>  PRT
<213>  Artificial

<220>
<223>  epitope IGKV1-39

<400>  54

Ser Pro Ser Ser Leu Ser Ala Ser Val Gly Asp Arg Val Thr Ile
1               5                   10                  15


<210>  55
<211>  15
<212>  PRT
<213>  Artificial

<220>
<223>  epitope IGKV1-39

<400>  55

Ser Leu Ser Ala Ser Val Gly Asp Arg Val Thr Ile Thr Cys Arg
1               5                   10                  15


<210>  56
<211>  15
<212>  PRT
<213>  Artificial

<220>
<223>  Epitope IGKV1-39

<400>  56

Ala Ser Val Gly Asp Arg Val Thr Ile Thr Cys Arg Ala Ser Gln
1               5                   10                  15


<210>  57
<211>  15
<212>  PRT
<213>  Artificial

<220>
<223>  Epitope IGKV1-39

<400>  57

Gly Asp Arg Val Thr Ile Thr Cys Arg Ala Ser Gln Ser Ile Ser
1               5                   10                  15


<210>  58
<211>  15

<212>  PRT
<213>  Artificial

<220>
<223>  Epitope IGKV1-39

<400>  58

Val Thr Ile Thr Cys Arg Ala Ser Gln Ser Ile Ser Ser Tyr Leu
1               5                   10                  15


<210>  59
<211>  15
<212>  PRT
<213>  Artificial

<220>
<223>  Epitope IGKV1-39

<400>  59

Thr Cys Arg Ala Ser Gln Ser Ile Ser Ser Tyr Leu Asn Trp Tyr
1               5                   10                  15


<210>  60
<211>  15
<212>  PRT
<213>  Artificial

<220>
<223>  Epitope IGKV1-39

<400>  60

Ala Ser Gln Ser Ile Ser Ser Tyr Leu Asn Trp Tyr Gln Gln Lys
1               5                   10                  15


<210>  61
<211>  15
<212>  PRT
<213>  Artificial

<220>
<223>  Epitope IGKV1-39

<400>  61

Ser Ile Ser Ser Tyr Leu Asn Trp Tyr Gln Gln Lys Pro Gly Lys
1               5                   10                  15


<210>  62
<211>  15
<212>  PRT
<213>  Artificial

<220>
<223>  Epitope IGKV1-39

<400>  62

Ser Tyr Leu Asn Trp Tyr Gln Gln Lys Pro Gly Lys Ala Pro Lys
1               5                   10                  15

<210>  63
<211>  15
<212>  PRT
<213>  Artificial

<220>
<223>  Epitope IGKV1-39

<400>  63

Asn Trp Tyr Gln Gln Lys Pro Gly Lys Ala Pro Lys Leu Leu Ile
1               5                   10                  15

<210>  64
<211>  15
<212>  PRT
<213>  Artificial

<220>
<223>  Epitope IGKV1-39

<400>  64

Gln Gln Lys Pro Gly Lys Ala Pro Lys Leu Leu Ile Tyr Ala Ala
1               5                   10                  15

<210>  65
<211>  15
<212>  PRT
<213>  Artificial

<220>
<223>  Epitope IGKV1-39

<400>  65

Pro Gly Lys Ala Pro Lys Leu Leu Ile Tyr Ala Ala Ser Ser Leu
1               5                   10                  15

<210>  66
<211>  15
<212>  PRT
<213>  Artificial

<220>
<223>  Epitope IGKV1-39

<400>  66

Ala Pro Lys Leu Leu Ile Tyr Ala Ala Ser Ser Leu Gln Ser Gly
1               5                   10                  15

<210>  67
<211>  15

<212>  PRT
<213>  Artificial

<220>
<223>  Epitope IGKV1-39

<400>  67

Leu Leu Ile Tyr Ala Ala Ser Ser Leu Gln Ser Gly Val Pro Ser
1               5                   10                  15


<210>  68
<211>  15
<212>  PRT
<213>  Artificial

<220>
<223>  Epitope IGKV1-39

<400>  68

Tyr Ala Ala Ser Ser Leu Gln Ser Gly Val Pro Ser Arg Phe Ser
1               5                   10                  15


<210>  69
<211>  15
<212>  PRT
<213>  Artificial

<220>
<223>  Epitope IGKV1-39

<400>  69

Ser Ser Leu Gln Ser Gly Val Pro Ser Arg Phe Ser Gly Ser Gly
1               5                   10                  15


<210>  70
<211>  15
<212>  PRT
<213>  Artificial

<220>
<223>  Epitope IGKV1-39

<400>  70

Gln Ser Gly Val Pro Ser Arg Phe Ser Gly Ser Gly Ser Gly Thr
1               5                   10                  15


<210>  71
<211>  15
<212>  PRT
<213>  Artificial

<220>
<223>  Epitope IGKV1-39

<400>  71

Val Pro Ser Arg Phe Ser Gly Ser Gly Ser Gly Thr Asp Phe Thr
1               5                   10                  15


<210>  72
<211>  15
<212>  PRT
<213>  Artificial

<220>
<223>  Epitope IGKV1-39

<400>  72

Arg Phe Ser Gly Ser Gly Ser Gly Thr Asp Phe Thr Leu Thr Ile
1               5                   10                  15


<210>  73
<211>  15
<212>  PRT
<213>  Artificial

<220>
<223>  Epitope IGKV1-39

<400>  73

Gly Ser Gly Ser Gly Thr Asp Phe Thr Leu Thr Ile Ser Ser Leu
1               5                   10                  15


<210>  74
<211>  15
<212>  PRT
<213>  Artificial

<220>
<223>  Epitope IGKV1-39

<400>  74

Ser Gly Thr Asp Phe Thr Leu Thr Ile Ser Ser Leu Gln Pro Glu
1               5                   10                  15


<210>  75
<211>  15
<212>  PRT
<213>  Artificial

<220>
<223>  Epitope IGKV1-39

<400>  75

Asp Phe Thr Leu Thr Ile Ser Ser Leu Gln Pro Glu Asp Phe Ala
1               5                   10                  15


<210>  76
<211>  15

<212>  PRT
<213>  Artificial

<220>
<223>  Epitope IGKV1-39

<400>  76

Leu Thr Ile Ser Ser Leu Gln Pro Glu Asp Phe Ala Thr Tyr Tyr
1               5                   10                  15


<210>  77
<211>  15
<212>  PRT
<213>  Artificial

<220>
<223>  Epitope IGKV1-39

<400>  77

Ser Ser Leu Gln Pro Glu Asp Phe Ala Thr Tyr Tyr Cys Gln Gln
1               5                   10                  15


<210>  78
<211>  15
<212>  PRT
<213>  Artificial

<220>
<223>  Epitope IGKV1-39

<400>  78

Gln Pro Glu Asp Phe Ala Thr Tyr Tyr Cys Gln Gln Ser Tyr Ser
1               5                   10                  15


<210>  79
<211>  15
<212>  PRT
<213>  Artificial

<220>
<223>  Epitope IGKV1-39

<400>  79

Asp Phe Ala Thr Tyr Tyr Cys Gln Gln Ser Tyr Ser Thr Pro Pro
1               5                   10                  15


<210>  80
<211>  15
<212>  PRT
<213>  Artificial

<220>
<223>  Epitope IGKV1-39

<400>  80

Thr Tyr Tyr Cys Gln Gln Ser Tyr Ser Thr Pro Pro Thr Phe Gly
1               5                   10                  15

<210>  81
<211>  15
<212>  PRT
<213>  Artificial

<220>
<223>  Epitope IGKV1-39

<400>  81

Cys Gln Gln Ser Tyr Ser Thr Pro Pro Thr Phe Gly Gln Gly Thr
1               5                   10                  15

<210>  82
<211>  15
<212>  PRT
<213>  Artificial

<220>
<223>  Epitope IGKV1-39

<400>  82

Ser Tyr Ser Thr Pro Pro Thr Phe Gly Gln Gly Thr Lys Val Glu
1               5                   10                  15

<210>  83
<211>  14
<212>  PRT
<213>  Artificial

<220>
<223>  Epitope IGKV1-39

<400>  83

Thr Pro Pro Thr Phe Gly Gln Gly Thr Lys Val Glu Ile Lys
1               5                   10

<210>  84
<211>  321
<212>  DNA
<213>  Homo sapiens

<220>
<221>  CDS
<222>  (1)..(321)

<400>  84
gac atc cag atg acc cag agc ccc agc agc ctg agc gcc agc gtg ggc        48
Asp Ile Gln Met Thr Gln Ser Pro Ser Ser Leu Ser Ala Ser Val Gly
1               5                   10                  15

gac aga gtg acc atc acc tgc aga gcc agc cag agc atc agc agc tac        96

Asp Arg Val Thr Ile Thr Cys Arg Ala Ser Gln Ser Ile Ser Ser Tyr
            20                  25                  30

ctg aac tgg tat cag cag aag ccc ggc aag gcc ccc aag ctg ctg atc      144
Leu Asn Trp Tyr Gln Gln Lys Pro Gly Lys Ala Pro Lys Leu Leu Ile
        35                  40                  45

tac gcc gcc agc tcc ctg cag agc ggc gtg ccc agc aga ttc agc ggc      192
Tyr Ala Ala Ser Ser Leu Gln Ser Gly Val Pro Ser Arg Phe Ser Gly
    50                  55                  60

agc ggc tcc ggc acc gac ttc acc ctg acc atc agc agc ctg cag ccc      240
Ser Gly Ser Gly Thr Asp Phe Thr Leu Thr Ile Ser Ser Leu Gln Pro
65                  70                  75                  80

gag gac ttc gcc acc tac tac tgc cag cag agc tac agc acc ccc ccc      288
Glu Asp Phe Ala Thr Tyr Tyr Cys Gln Gln Ser Tyr Ser Thr Pro Pro
                85                  90                  95

acc ttc ggc cag ggc acc aag gtg gag atc aag                          321
Thr Phe Gly Gln Gly Thr Lys Val Glu Ile Lys
            100                 105


<210>  85
<211>  107
<212>  PRT
<213>  Homo sapiens

<400>  85

Asp Ile Gln Met Thr Gln Ser Pro Ser Ser Leu Ser Ala Ser Val Gly
1               5                   10                  15


Asp Arg Val Thr Ile Thr Cys Arg Ala Ser Gln Ser Ile Ser Ser Tyr
            20                  25                  30


Leu Asn Trp Tyr Gln Gln Lys Pro Gly Lys Ala Pro Lys Leu Leu Ile
        35                  40                  45


Tyr Ala Ala Ser Ser Leu Gln Ser Gly Val Pro Ser Arg Phe Ser Gly
    50                  55                  60


Ser Gly Ser Gly Thr Asp Phe Thr Leu Thr Ile Ser Ser Leu Gln Pro
65                  70                  75                  80


Glu Asp Phe Ala Thr Tyr Tyr Cys Gln Gln Ser Tyr Ser Thr Pro Pro
                85                  90                  95


Thr Phe Gly Gln Gly Thr Lys Val Glu Ile Lys
            100                 105


<210>  86
<211>  330
<212>  DNA
<213>  Homo sapiens

<220>
<221>  CDS
<222>  (1)..(330)

<400>  86
cag tct gcc ctg acc cag ccc gcc tct gtg tct ggc agc cct ggc cag      48
Gln Ser Ala Leu Thr Gln Pro Ala Ser Val Ser Gly Ser Pro Gly Gln
1               5                   10                  15

agc atc acc atc agc tgc acc ggc acc agc agc gac gtg ggc ggc tac      96
Ser Ile Thr Ile Ser Cys Thr Gly Thr Ser Ser Asp Val Gly Gly Tyr
            20                  25                  30

aac tac gtg tcc tgg tat cag cag cac ccc ggc aag gcc ccc aag ctg      144
Asn Tyr Val Ser Trp Tyr Gln Gln His Pro Gly Lys Ala Pro Lys Leu
        35                  40                  45

atg atc tac gag gtg tcc aac aga ccc agc ggc gtg agc aac aga ttc      192
Met Ile Tyr Glu Val Ser Asn Arg Pro Ser Gly Val Ser Asn Arg Phe
    50                  55                  60

agc ggc agc aag agc ggc aac acc gcc agc ctg acc atc agc ggc ctc      240
Ser Gly Ser Lys Ser Gly Asn Thr Ala Ser Leu Thr Ile Ser Gly Leu
65                  70                  75                  80

cag gct gag gac gag gcc gac tac tac tgc agc agc tac acc agc agc      288
Gln Ala Glu Asp Glu Ala Asp Tyr Tyr Cys Ser Ser Tyr Thr Ser Ser
                85                  90                  95

tcc acc ctg gtg ttt ggc ggc gga aca aag ctg acc gtg ctg              330
Ser Thr Leu Val Phe Gly Gly Gly Thr Lys Leu Thr Val Leu
            100                 105                 110


<210>  87
<211>  110
<212>  PRT
<213>  Homo sapiens

<400>  87

Gln Ser Ala Leu Thr Gln Pro Ala Ser Val Ser Gly Ser Pro Gly Gln
1               5                   10                  15


Ser Ile Thr Ile Ser Cys Thr Gly Thr Ser Ser Asp Val Gly Gly Tyr
            20                  25                  30


Asn Tyr Val Ser Trp Tyr Gln Gln His Pro Gly Lys Ala Pro Lys Leu
        35                  40                  45


Met Ile Tyr Glu Val Ser Asn Arg Pro Ser Gly Val Ser Asn Arg Phe
    50                  55                  60


Ser Gly Ser Lys Ser Gly Asn Thr Ala Ser Leu Thr Ile Ser Gly Leu
65                  70                  75                  80

Gln Ala Glu Asp Glu Ala Asp Tyr Tyr Cys Ser Ser Tyr Thr Ser Ser
                85                  90                  95

Ser Thr Leu Val Phe Gly Gly Gly Thr Lys Leu Thr Val Leu
            100                 105                 110

<210>  88
<211>  321
<212>  DNA
<213>  Rattus norvegicus

<220>
<221>  CDS
<222>  (1)..(321)

<400>  88
aga gcc gac gcc gct ccc acc gtg tcc atc ttc ccc ccc agc atg gaa       48
Arg Ala Asp Ala Ala Pro Thr Val Ser Ile Phe Pro Pro Ser Met Glu
1               5                   10                  15

cag ctg acc tct ggc gga gcc acc gtg gtc tgc ttc gtg aac aac ttc       96
Gln Leu Thr Ser Gly Gly Ala Thr Val Val Cys Phe Val Asn Asn Phe
            20                  25                  30

tac ccc aga gac atc agc gtg aag tgg aag atc gac ggc agc gag cag      144
Tyr Pro Arg Asp Ile Ser Val Lys Trp Lys Ile Asp Gly Ser Glu Gln
        35                  40                  45

agg gac ggc gtg ctg gac agc gtg acc gac cag gac agc aag gac tcc      192
Arg Asp Gly Val Leu Asp Ser Val Thr Asp Gln Asp Ser Lys Asp Ser
    50                  55                  60

acc tac agc atg agc agc acc ctg agc ctg acc aag gtg gag tac gag      240
Thr Tyr Ser Met Ser Ser Thr Leu Ser Leu Thr Lys Val Glu Tyr Glu
65                  70                  75                  80

agg cac aac ctg tac acc tgc gag gtg gtg cac aag acc agc tcc agc      288
Arg His Asn Leu Tyr Thr Cys Glu Val Val His Lys Thr Ser Ser Ser
                85                  90                  95

ccc gtg gtc aag tcc ttc aac cgg aac gag tgt                          321
Pro Val Val Lys Ser Phe Asn Arg Asn Glu Cys
            100                 105

<210>  89
<211>  107
<212>  PRT
<213>  Rattus norvegicus

<400>  89

Arg Ala Asp Ala Ala Pro Thr Val Ser Ile Phe Pro Pro Ser Met Glu
1               5                   10                  15

Gln Leu Thr Ser Gly Gly Ala Thr Val Val Cys Phe Val Asn Asn Phe
            20                  25                  30

Tyr Pro Arg Asp Ile Ser Val Lys Trp Lys Ile Asp Gly Ser Glu Gln
        35                  40                  45

Arg Asp Gly Val Leu Asp Ser Val Thr Asp Gln Asp Ser Lys Asp Ser
    50                  55                  60

Thr Tyr Ser Met Ser Ser Thr Leu Ser Leu Thr Lys Val Glu Tyr Glu
65                  70                  75                  80

Arg His Asn Leu Tyr Thr Cys Glu Val Val His Lys Thr Ser Ser Ser
                85                  90                  95

Pro Val Val Lys Ser Phe Asn Arg Asn Glu Cys
            100                 105

<210>  90
<211>  865
<212>  DNA
<213>  Artificial

<220>
<223>  IGKV1-39/J-Ck

<400>  90
ggtaccgcgg ccgccaccat ggacatgaga gtgcccgccc agctcctggg gctcctgcta      60

ctctggctcc gaggtaagga tggagaacac taggaattta ctcagccagt gtgctcagta     120

ctgactggaa cttcagggaa gttctctgat aacatgatta atagtaagaa tatttgtttt     180

tatgtttcca atctcaggtg ccagatgtga catccagatg acccagagcc ccagcagcct     240

gagcgccagc gtgggcgaca gagtgaccat cacctgcaga gccagccaga gcatcagcag     300

ctacctgaac tggtatcagc agaagcccgg caaggccccc aagctgctga tctacgccgc     360

cagctccctg cagagcggcg tgcccagcag attcagcggc agcggctccg gcaccgactt     420

caccctgacc atcagcagcc tgcagcccga ggacttcgcc acctactact gccagcagag     480

ctacagcacc ccccccacct tcggccaggg caccaaggtg gagatcaaga gagccgacgc     540

cgctcccacc gtgtccatct tcccccccag catggaacag ctgacctctg gcggagccac     600

cgtggtctgc ttcgtgaaca acttctaccc cagagacatc agcgtgaagt ggaagatcga     660

cggcagcgag cagagggacg gcgtgctgga cagcgtgacc gaccaggaca gcaaggactc     720

cacctacagc atgagcagca ccctgagcct gaccaaggtg gagtacgaga ggcacaacct     780

gtacacctgc gaggtggtgc acaagaccag ctccagcccc gtggtcaagt ccttcaaccg     840

gaacgagtgt tgagctagcg agctc                                           865

<210>  91
<211>  874
<212>  DNA

<213>  Artificial

<220>
<223>  IGLV2-14/J-Ck

<400>  91
ggtaccgcgg ccgccaccat ggacatgaga gtgcccgccc agctcctggg gctcctgcta      60

ctctggctcc gaggtaagga tggagaacac taggaattta ctcagccagt gtgctcagta     120

ctgactggaa cttcagggaa gttctctgat aacatgatta atagtaagaa tatttgtttt     180

tatgtttcca atctcaggtg ccagatgtca gtctgccctg acccagcccg cctctgtgtc     240

tggcagccct ggccagagca tcaccatcag ctgcaccggc accagcagcg acgtgggcgg     300

ctacaactac gtgtcctggt atcagcagca ccccggcaag gcccccaagc tgatgatcta     360

cgaggtgtcc aacagaccca gcggcgtgag caacagattc agcggcagca agagcggcaa     420

caccgccagc ctgaccatca gcggcctcca ggctgaggac gaggccgact actactgcag     480

cagctacacc agcagctcca ccctggtgtt tggcggcgga acaaagctga ccgtgctgag     540

agccgacgcc gctcccaccg tgtccatctt cccccccagc atggaacagc tgacctctgg     600

cggagccacc gtggtctgct tcgtgaacaa cttctacccc agagacatca gcgtgaagtg     660

gaagatcgac ggcagcgagc agagggacgg cgtgctggac agcgtgaccg accaggacag     720

caaggactcc acctacagca tgagcagcac cctgagcctg accaaggtgg agtacgagag     780

gcacaacctg tacacctgcg aggtggtgca caagaccagc tccagccccg tggtcaagtc     840

cttcaaccgg aacgagtgtt gagctagcga gctc                                 874


<210>  92
<211>  13373
<212>  DNA
<213>  Artificial

<220>
<223>  VkP-IGKV1-39/J-Ck

<400>  92
ggccggccca catgaaacaa tgggaaccat gtgacaatca cagaggtgtt gttactatag      60

caaaagggat tgttactctc cacatccctt taagtaactt gaaggcctga tagacccacc     120

ctctaagact tcattagaca ttccctacga atggttatac tctcctgtat actcccaata     180

caactctaaa atatattatt ccatatagtc cttaggtttg tattaaagtt tgactttttt     240

ccttcaaaat atctcttgtc acaacagcgg ctctagagag aaatacattc cctccaggca     300

aatctatgct gcgctggtct gacctgggac cctggggaca ttgcccctgt gctgagttac     360

taagatgagc cagccctgca gctgtgctca gcctgcccca tgccctgctg attgatttgc     420

atgttccaga gcacagcccc ctgccctgaa gactttttta tgggctggtc gcaccctgtg     480

caggagtcag tctcagtcag gagccaccat ggacatgaga gtgcccgccc agctcctggg     540

gctcctgcta ctctggctcc gaggtaagga tggagaacac taggaattta ctcagccagt 600

gtgctcagta ctgactggaa cttcagggaa gttctctgat aacatgatta atagtaagaa 660

tatttgtttt tatgtttcca atctcaggtg ccagatgtga catccagatg acccagagcc 720

ccagcagcct gagcgccagc gtgggcgaca gagtgaccat cacctgcaga gccagccaga 780

gcatcagcag ctacctgaac tggtatcagc agaagcccgg caaggccccc aagctgctga 840

tctacgccgc cagctccctg cagagcggcg tgcccagcag attcagcggc agcggctccg 900

gcaccgactt caccctgacc atcagcagcc tgcagcccga ggacttcgcc acctactact 960

gccagcagag ctacagcacc ccccccacct tcggccaggg caccaaggtg gagatcaaac 1020

gtaagtacac ttttctcatc tttttttatg tgtaagacac aggttttcat gttaggagtt 1080

aaagtcagtt cagaaaatct tgagaaaatg gagagggctc attatcagtt gacgtggcat 1140

acagtgtcag attttctgtt tatcaagcta gtgagattag gggcaaaaag aggctttagt 1200

tgagaggaaa gtaattaata ctatggtcac catccaagag attggatcgg agaataagca 1260

tgagtagtta ttgagatctg ggtctgactg caggtagcgt ggtcttctag acgtttaagt 1320

gggagatttg gaggggatga ggaatgaagg aacttcagga tagaaaaggg ctgaagtcaa 1380

gttcagctcc taaaatggat gtgggagcaa actttgaaga taaactgaat gacccagagg 1440

atgaaacagc gcagatcaaa gaggggcctg gagctctgag aagagaagga gactcatccg 1500

tgttgagttt ccacaagtac tgtcttgagt tttgcaataa aagtgggata gcagagttga 1560

gtgagccgta ggctgagttc tctcttttgt ctcctaagtt tttatgacta caaaaatcag 1620

tagtatgtcc tgaaataatc attaagctgt ttgaaagtat gactgcttgc catgtagata 1680

ccatggcttg ctgaataatc agaagaggtg tgactcttat tctaaaattt gtcacaaaat 1740

gtcaaaatga gagactctgt aggaacgagt ccttgacaga cagctcaagg ggtttttttc 1800

ctttgtctca tttctacatg aaagtaaatt tgaaatgatc ttttttatta taagagtaga 1860

aatacagttg ggtttgaact atatgtttta atggccacgg ttttgtaaga catttggtcc 1920

tttgttttcc cagttattac tcgattgtaa ttttatatcg ccagcaatgg actgaaacgg 1980

tccgcaacct cttctttaca actgggtgac ctcgcggctg tgccagccat ttggcgttca 2040

ccctgccgct aagggccatg tgaacccccg cggtagcatc ccttgctccg cgtggaccac 2100

tttcctgagg cacagtgata ggaacagagc cactaatctg aagagaacag agatgtgaca 2160

gactacacta atgtgagaaa aacaaggaaa gggtgactta ttggagattt cagaaataaa 2220

atgcatttat tattatattc ccttatttta attttctatt agggaattag aaaggcataa 2280

aactgcttta tccagtgtta tattaaaagc ttaatgtata taatctttta gaggtaaaat 2340

ctacagccag caaaagtcat ggtaaatatt ctttgactga actctcacta aactcctcta 2400

aattatatgt catattaact ggttaaatta atataaattt gtgacatgac cttaactggt 2460
taggtaggat atttttcttc atgcaaaaat atgactaata ataatttagc acaaaaatat 2520
ttcccaatac tttaattctg tgatagaaaa atgtttaact cagctactat aatcccataa 2580
ttttgaaaac tatttattag cttttgtgtt tgacccttcc ctagccaaag gcaactattt 2640
aaggaccctt taaaactctt gaaactactt tagagtcatt aagttattta accactttta 2700
attactttaa aatgatgtca attccctttt aactattaat ttattttaag gggggaaagg 2760
ctgctcataa ttctattgtt tttcttggta aagaactctc agttttcgtt tttactacct 2820
ctgtcaccca agagttggca tctcaacaga ggggactttc cgagaggcca tctggcagtt 2880
gcttaagatc agaagtgaag tctgccagtt cctcccaggc aggtggccca gattacagtt 2940
gacctgttct ggtgtggcta aaaattgtcc catgtggtta caaaccatta gaccagggtc 3000
tgatgaattg ctcagaatat ttctggacac ccaaatacag accctggctt aaggccctgt 3060
ccatacagta ggtttagctt ggctacacca aaggaagcca tacagaggct aatatcagag 3120
tattcttgga agagacagga gaaaatgaaa gccagtttct gctcttacct tatgtgcttg 3180
tgttcagact cccaaacatc aggagtgtca gataaactgg tctgaatctc tgtctgaagc 3240
atggaactga aaagaatgta gtttcaggga agaaaggcaa tagaaggaag cctgagaata 3300
tcttcaaagg gtcagactca atttactttc taaagaagta gctaggaact agggaataac 3360
ttagaaacaa caagattgta tatatgtgca tcctggcccc attgttcctt atctgtaggg 3420
ataagcgtgc ttttttgtgt gtctgtatat aacataactg tttacacata atacactgaa 3480
atggagccct tccttgttac ttcataccat cctctgtgct tccttcctca ggggccgacg 3540
ccgctcccac cgtgtccatc ttccccccca gcatggaaca gctgacctct ggcggagcca 3600
ccgtggtctg cttcgtgaac aacttctacc ccagagacat cagcgtgaag tggaagatcg 3660
acggcagcga gcagagggac ggcgtgctgg acagcgtgac cgaccaggac agcaaggact 3720
ccacctacag catgagcagc accctgagcc tgaccaaggt ggagtacgag aggcacaacc 3780
tgtacacctg cgaggtggtg cacaagacca gctccagccc cgtggtcaag tccttcaacc 3840
ggaacgagtg ttgaagacaa aggtcctgag acgccaccac cagctcccca gctccatcct 3900
atcttccctt ctaaggtctt ggaggcttcc ccacaagcga cctaccactg ttgcggtgct 3960
ccaaacctcc tccccacctc cttctcctcc tcctcccttt ccttggcttt tatcatgcta 4020
atatttgcag aaaatattca ataaagtgag tctttgcact tgagatctct gtctttctta 4080
ctaaatggta gtaatcagtt gtttttccag ttacctgggt ttctcttcta aagaagttaa 4140
atgtttagtt gccctgaaat ccaccacact taaaggataa ataaaaccct ccacttgccc 4200
tggttggctg tccactacat ggcagtcctt tctaaggttc acgagtacta ttcatggctt 4260
atttctctgg gccatggtag gtttgaggag gcatacttcc tagttttctt cccctaagtc 4320

gtcaaagtcc tgaaggggga cagtctttac aagcacatgt tctgtaatct gattcaacct 4380
acccagtaaa cttggcgaag caaagtagaa tcattatcac aggaagcaaa ggcaacctaa 4440
atgtgcaagc aataggaaaa tgtggaagcc catcatagta cttggacttc atctgctttt 4500
gtgccttcac taagttttta aacatgagct ggctcctatc tgccattggc aaggctgggc 4560
actacccaca acctacttca aggacctcta taccgtgaga ttacacacat acatcaaaat 4620
ttgggaaaag ttctaccaag ctgagagctg atcaccccac tcttaggtgc ttatctctgt 4680
acaccagaaa ccttaagaag caaccagtat tgagagactc atttatgaaa gtctaaaact 4740
ggatacaacc aaaatgtcca ccaacagtta aattatgaca tgttcacaat tgagctatta 4800
cttaataagg agaattaata aaataaaact taagagcata gtttaatctc ataaacaaga 4860
taataagcaa aacaaaacat tttttcatcc atgtaagttt aaaagcaggt aaaatttaaa 4920
attaagagag acataagttt tgaggtagca agatggaaac tctggggctt ggggaatgtt 4980
ctgtctctct gtatgggatg tgaaagttac tattgtggaa ttgggatcta tgttcttcct 5040
gtatatattg tatacttcat aataacttca cctaaagaaa tatctaatac ccagtgcata 5100
cataaaagag gatacaagga atgaatcata cgtcaaggcc agaaagacaa taaagtaggg 5160
gatccaggat caaatctccc acaaccttga gccttctact attctgcctt ccagagctca 5220
aagtacaaaa cacataattc aaacacatga tccctccttg gggtctcttc cttcatgcat 5280
cgaattagaa atagccatgt ataaaatgag atagaagaga ccttcatcaa caggtcaaag 5340
aatataggta attttgtctg ggtatgaaga gcccacgtat caaaggttac attagggaag 5400
gaagaggaca ctaacagtga ctttcattct ccccctcttc ctggaggccc ctgcatttag 5460
tccctcgtgg gctcatccac tcagcacaca tttactaagc atcttctcag cctacactct 5520
gaaggcagtg cagaataatg ttagtgtccc ttcccccagt taatatgcag tccagtttcc 5580
ctgctccttc cctttctcag tccacataag gatgatggga aaggacagtc accaaatagg 5640
agagggcaac cctttgcctt cctacctctt gagaatgtac attattatcc actttttgaa 5700
acttctttta attgcttttt tttaatttgt cttttcaaat agcataacct tgttcatcca 5760
tttctgggaa ccaaatttat caatcaacag tgcctctaat ctggctatta atacaaaaat 5820
gcctcctcaa aatatatatg ttcgagtctt atctaaaaca gaacccacaa taaaaaagaa 5880
gaaagaatac atataagcat ttatataatt ctgagcaacc ttgtgctttg tgaaaaaaat 5940
ataatctaat gtcacatgct gtattctttt tatttaacac tggtgaaatt ataccattag 6000
agagaaagag gacagatcac tgatcctagg atctagggat gttacagata agaaaacaaa 6060
tgtgacaaag agctgtcaca aggaggatct tcaaggtcac agaatcactg tcttgatttc 6120
agtggtggtt acatacattt aaatatgtga taaaatgttg ttgaactata ttcatatatt 6180

gtaccaatgt caaatgctta attttggctc tatagtataa ttatgcacta aataactatt 6240
tggacaaaga aaatgatgtt tacatcaaag gtgaggccat atttgttagg aacataactt 6300
aaaaaccatt ttggataact aatgaaaagc cattttgtgt gccttggcat atcatgccta 6360
agctgtcacc agatagatct aataagacct aagcctcaga agcaagcccc tgcccagcaa 6420
gcaggcagca cagataagag ctaaacccag gacaggccat gatatgctaa tgaactacct 6480
tcaaggtggt gttgctgacc tagtgaacca gccccaagct gtgagcccca atagcacaaa 6540
gctactgccc aaagaaatta tacaaaaatt ggaactttgg gaatggtgtg caggatcgct 6600
ctgctgtatg cctggaacac agcttctcta tgttttgtat tgataccagt ctagaagctt 6660
ccaaaacttt ctcactgaag aagattcccc atgtgggacc cctacagact cttttgccca 6720
aacaactgct tccctcctgg tgtgatatct gttttgcttt tatgttagca taatattata 6780
aggaatgttt gtgtgaataa accaaacata ttttaaaagc aaatattgta tgcacatcct 6840
aattgctaaa aagtttacag ctaatagtcc catgctctcc acaatactgg atccaaataa 6900
gtcctaattt caatgttggg catctttaca gagagaaaga cattaaaaat gaagagacat 6960
gcagagagtg caccatgcca tcgtggagac agactgaagt gacacaactg ttagtcaaag 7020
aggattaagg acttccagaa gccaccaaag gaaggaggta tgaagtggtt tctccctcag 7080
agtatccaga ggagactaaa ccaaccaaca cctttttgct taagacttct tgccttcagg 7140
actgtgagaa ggtagcttcc tattgttcta agccccagta tgtggcattt tgttaaggta 7200
gagtcaagaa accaataaaa tgcagacaga caaaaggata gctgagtttt ccaggccctt 7260
ccttcttatt tttggttttg ttggtggtgg tggtggtggt ggtgatggtg gtggttttgt 7320
ttatgttttg tttggggagt tttttggggt ttttttgggt tttgtttttg ttgttgtttt 7380
gggggttttt gttgttgttg ttgtttgctt ttttgttttt tgttttttgt ttttttgaga 7440
cagtgtttct ctgtatagcc ctggctgtcc tggagttcct tctatctcta atgtctacat 7500
ctcagagggg atcctctaat ttcaaatgag cagtagctct ccatttttag ctcttattta 7560
ttcatttatt tacttactta cttattgtct gtagatgaaa gaattttgga gtgggaaagg 7620
gttcatgagc ccccagcaac taatgaggag ctacagacaa ttgatgtttc tggggaaagg 7680
agactcagtt tctttgagag tatagcttct gatgggtcaa ccatgttcct gtggctgatg 7740
tcacacccag gagtatgcag acaacagaaa ctggagttaa tgagttgttt taaaaataaa 7800
aaagggcatg aagcttggga tagaaattaa ggataaatac aattaaatac aggaaattct 7860
gaaagaatta ataaaaacat ttcttttttt aaaaaaaaat ccagaattag ctatgcttct 7920
tcaaaattgc ttctggagaa ctttacaagt taaataagtt atattgtaga aaaggtagag 7980
aggagaatag tggaagagag agataaggag acttcaaaag gagtggaggg agatagagga 8040
ggagaaagca gaagcaatgg ctgatagaca caggataaga gggaacagaa aggagaaaga 8100

ggaagccagg atgggtattt ctttgcctat ctgtgacttg cacatggtct tggcaattat 8160
tgatgagttc aaggcttaat tcttcacttg tgccaactca acagagtctt tctttcttat 8220
aaccaggccc ccagtatgct catgtatgta tcaggtcctc ttatctcctt atagcaatcc 8280
tgtttataac tgggtaactt tgtgaaggga aggaagtgca cactgagatg tgctacaact 8340
ttttaataca aaattttgaa gagtttgtac aatgtatgta taattaataa ttaatattat 8400
gcactttaga ttttgatttc aactcaagat actaattcta tatatatggg ttaaatcaat 8460
atattaataa gtttaatttc acatgcttat ttttattgtg gttttcgaga cagggtttct 8520
ctgtatagcc ctggctgtcc tggaacccac tttgtagacc aggctggcct caaactcaga 8580
aacctacctg cctctgcctc tgcctctgcc tctgcctctg cctctgcctc tgcctctgcc 8640
tctgcctctg cctctgcctc tgcctctgcc tctgcctctg cctctgcctc tgcctctgcc 8700
tctgcctctg cctctgcctc tgcctctgcc tctgcctctg cctctgcctc tgcctctgcc 8760
tctgcctagt gctggaatta aaggtttgcg ccaccacgcc cggtgaaatt tttaaacttt 8820
atatatgtct cattctattt ctatcagata ggactgtgta gactgtgcta aactaataaa 8880
tgtgccctca aaagtaatcg caagttgtat tgttgttgtt ttgctttgct ttgctttgct 8940
ttgctttgct ttgctttgct ttgctttgct ttgctttgct ttgctttgct ttgctttgct 9000
ttgctttgct ttgctttgct ttgctttttt gttttgggtt tttttccggg ggagggaggg 9060
tggagaaaga atcttactat gaagctctga ctgtcctggg aactcactat atagatcagg 9120
cttgattcaa ctcatagaga tctgccttct tctgcctccc aagtgctggg aataaaggca 9180
tacacctcca tgcccagata gtgatcccaa gttttagcaa aagtttctag acttgacatt 9240
aatcgatgga gatagacatg aattacacaa agaactaatg tggagtttac ctgaatcata 9300
ctctatactt tatcagagat taaattaaca tttaataatc cagtgccagg ctagaggcac 9360
cattcaatgg cagtgtttgc catcatgcat aggcttagtc ttcagtgctg aaaggcattg 9420
ggggcaatat tactcattat acagatgaga aactgggaaa gacttgcctc agattctcta 9480
ctgaaaggct gagtttgtgg cttctagaaa atcttttact ttcaatattt ttaatgtata 9540
atttttttat ttccactgat tttatttttt atttttaaca tttataagaa ataaatgcaa 9600
taaaccaaat acatggacaa aaaaatacaa gaatcatatg atcacctcaa tggaaggaaa 9660
aaaaaagaaa gaaaaagtct ttgataagat tcaacattca ttcttttttt attagatatt 9720
ttcttcattt acatttcaaa tgctatcccc aaagccccct ataccttccc ctgccctgct 9780
ccccaaccca cccactcctg ctttctggcc ctggcattcc tctgtactga ggcatatgat 9840
cttcaaaaaa ccaagggcct ctcctctcat tggtggccga ctattaggcc atcttttgct 9900
acatatgcaa ctagagacac agctctgggg gttactggtt agttcatatt gttagtcctc 9960

ctatagagtt gcagacccct ttagctcctt ggatactttc tctagttcct tcattagggg 10020

ccctgtgtcc catccaatag atgactgtga gcatccactt ctgtatttgc caggcactgg 10080

catagcctca cgagaaagag agagctatgt caggatcctg tcagtaaaat ctttctggca 10140

tatgcaatag tatctgggtt tggtggttgt atatgggatg gatccccaag tggagcagtc 10200

tctgaatggt ccttccttcc atctcagctc caaactttgt ctctataact ccttccatgg 10260

gtattttgtt ccccattcta agaaggagtg aagaatccac actttggtct tccttcttct 10320

tgagtttcat atgttgcatc ttggatattc taagtttctg ggttaatatc cacgtatcag 10380

tgagtgcata tcatgcgtgt tattttgtga ttagtttacc tcactcagga tgatatcctc 10440

cagatgcatc catttgccta agaatttcat taattcactg tttttaattg ctgaatagta 10500

ctccattgtg taaatgtacc acattttctg tatccattcc tctgttgagg ggcatctggg 10560

ttctttccag cttctggcta ttataaataa ggctgctatg agcatagcgg agcatgtgtc 10620

cttatcaagt tggaacatct tctaggtata tgcccaggag aggaattgct ggatcttccg 10680

gtagtaccat caacatgcat tcttaataaa agccctagaa caaggaggac tgtaggaaac 10740

atattccaac ataataaagg ttatgtatga caaactcatg accaatatca tcctaaatga 10800

atgaaaccat taataagctc cattaaaatc agaggactgc ccactatccc tacttctcat 10860

ccataatgag attgaagcat tagctggagc aataaggcaa gagaagggat acaaatggga 10920

aaatattaag tcaaattgtt ttcaattgaa gattatatta tcttataccc aatgacctca 10980

aattttgact agaaaaattg tagaaattat caataatttc agcaaagtgt tatgatgcac 11040

cacatcctta ttcttctccc cagcttctgc ttgcttctct cttcttgctc ttcatccttt 11100

ctgtccttcc atctgcctgc actcttgtct caagactgag tgcagcgtgt aactctcctg 11160

tgactgagta tctcacaaaa cgttctacct gccaaacctg gatgagccct ttgtctttct 11220

gaagctatga ggctctctac atagactcaa gaaggaaatg acaggaggag ggtaataatg 11280

aagtggggaa ggctgacatt agcattgctc ctgtgtggct ccttaatttc tcatacttca 11340

cactgagatg ttattaactg tgactcatag gtgaagaagc cagagctaag gttctcatat 11400

ttgagtgtta tagaatgagt agagcagtag ttctcaaact atgggtcatg actcctttat 11460

gggtcaaact accctttcac acaggttgca tatcagatat cctaatttta tatacatata 11520

tatatgcata tgtatatata tatatttcac aacagtagga aaattattta gtaatcattt 11580

tatagttgtg ggtcatggca acatgaggaa ctgtattaaa gggttgcagc attaggaatg 11640

ttgagaccca ctgtaataga gaatgaggct taaggcaggg ctataaagcc caatggacca 11700

tgtgcctttt ccaacatttg ccacatggta agctctgtat agacttttta aagaacattg 11760

gtttgtaatt ttaaatggat aagggtcttc actgtctatc acccatctat ataataaata 11820

cataagtttt gattccacca tggattcaaa tgcaaaaatc ctcaacctaa gacatagcag 11880

tgaaacattg atgaccaaat aggaaatcca tgtagagacc ttctatcttc tgatggctcc 11940

acaggcacca tcttgcaaca gagttctact ttgctaccag taatgaatac agtgtctcaa 12000

ctcctgccat tgaatcttca ggaagcccct gaaatgactt gtactacacc atttcttaaa 12060

gacagaaaag ctaagactta gagggaataa atgtcatgcc tgagatcatg caaccaatta 12120

agtccaactt ggcctgatca agaggcacaa ttcaaaagca atgttgttcc ttcactagct 12180

cttgtgtatg gttgctgatt ccggaagcaa agtatcagtg aatatcccta gtgggaaaag 12240

acttggaaat caaatgtctc atttaacaga ttaggagatg aaacggtaga ctctgtgtag 12300

ttgtacaccc ctgtgatccc atcgctagga agactgaggc aggaagtcct cgagctcaaa 12360

ccagcttagg ctacacagag aaactatcta aaaaataatt actaactact taataggaga 12420

ttggatgtta agatctggtc actaagaggc agaattgaga ttcgaagcca gtattttcta 12480

cctggtatgt tttaaattgc agtaaggatc taagtgtaga tatataataa taagattcta 12540

ttgatctctg caacaacaga gagtgttaga tttgtttgga aaaaaatatt atcagccaac 12600

atcttctacc atttcagtat agcacagagt acccacccat atctccccac ccatccccca 12660

taccagactg gttattgatt ttcatggtga ctggcctgag aagattaaaa aaagtaatgc 12720

taccttattg ggagtgtccc atggaccaag atagcaactg tcatagctac cgtcacactg 12780

ctttgatcaa gaagaccctt tgaggaactg aaaacagaac cttaggcaca tctgttgctt 12840

tcgctcccat cctcctccaa cagcctgggt ggtgcactcc acaccctttc aagtttccaa 12900

agcctcatac acctgctccc taccccagca cctggccaag gctgtatcca gcactgggat 12960

gaaaatgata ccccacctcc atcttgtttg atattactct atctcaagcc ccaggttagt 13020

ccccagtccc aatgcttttg cacagtcaaa actcaacttg gaataatcag tatccttgaa 13080

gagttctgat atggtcactg ggcccatata ccatgtaaga catgtggaaa agatgtttca 13140

tggggcccag acacgttcta gaagtacctg agagtggcaa aaaatagttg tgctaaatag 13200

tttggccatc tttaggctga gagactagga aatacagcga tggactatat cagcattgca 13260

ggatagttgt cagtaaacac cccacaaccc ataacagaag tattctcttc tttctatatc 13320

ccttttccat ccatgtagat ggctgtcttc atatttgttc tagacggccg gcc 13373

<210> 93
<211> 12892
<212> DNA
<213> Artificial

<220>
<223> VkP-IGKV1-39/J-Ck-delta1

<400> 93
ggccggccca catgaaacaa tgggaaccat gtgacaatca cagaggtgtt gttactatag 60

caaaagggat tgttactctc cacatccctt taagtaactt gaaggcctga tagacccacc 120

ctctaagact tcattagaca ttccctacga atggttatac tctcctgtat actcccaata 180

caactctaaa atatattatt ccatatagtc cttaggtttg tattaaagtt tgactttttt 240

ccttcaaaat atctcttgtc acaacagcgg ctctagagag aaatacattc cctccaggca 300

aatctatgct gcgctggtct gacctgggac cctggggaca ttgcccctgt gctgagttac 360

taagatgagc cagccctgca gctgtgctca gcctgcccca tgccctgctg attgatttgc 420

atgttccaga gcacagcccc ctgccctgaa gactttttta tgggctggtc gcaccctgtg 480

caggagtcag tctcagtcag gagccaccat ggacatgaga gtgcccgccc agctcctggg 540

gctcctgcta ctctggctcc gaggtaagga tggagaacac taggaattta ctcagccagt 600

gtgctcagta ctgactggaa cttcagggaa gttctctgat aacatgatta atagtaagaa 660

tatttgtttt tatgtttcca atctcaggtg ccagatgtga catccagatg acccagagcc 720

ccagcagcct gagcgccagc gtgggcgaca gagtgaccat cacctgcaga gccagccaga 780

gcatcagcag ctacctgaac tggtatcagc agaagcccgg caaggccccc aagctgctga 840

tctacgccgc cagctccctg cagagcggcg tgcccagcag attcagcggc agcggctccg 900

gcaccgactt caccctgacc atcagcagcc tgcagcccga ggacttcgcc acctactact 960

gccagcagag ctacagcacc ccccccacct tcggccaggg caccaaggtg gagatcaaac 1020

gtaagtacac ttttctcatc tttttttatg tgtaagacac aggttttcat gttaggagtt 1080

aaagtcagtt cagaaaatct tgagaaaatg gagagggctc attatcagtt gacgtggcat 1140

acagtgtcag attttctgtt tatcaagcta gtgagattag gggcaaaaag aggctttagt 1200

tgagaggaaa gtaattaata ctatggtcac catccaagag attggatcgg agaataagca 1260

tgagtagtta ttgagatctg ggtctgactg caggtagcgt ggtcttctag acgtttaagt 1320

gggagatttg gaggggatga ggaatgaagg aacttcagga tagaaaaggg ctgaagtcaa 1380

gttcagctcc taaaatggat gtgggagcaa actttgaaga taaactgaat gacccagagg 1440

atgaaacagc gcagatcaaa gaggggcctg gagctctgag aagagaagga gactcatccg 1500

tgttgagttt ccacaagtac tgtcttgagt tttgcaataa aagtgggata gcagagttga 1560

gtgagccgta ggctgagttc tctcttttgt ctcctaagtt tttatgacta caaaaatcag 1620

tagtatgtcc tgaaataatc attaagctgt ttgaaagtat gactgcttgc catgtagata 1680

ccatggcttg ctgaataatc agaagaggtg tgactcttat tctaaaattt gtcacaaaat 1740

gtcaaaatga gagactctgt aggaacgagt ccttgacaga cagctcaagg ggtttttttc 1800

ctttgtctca tttctacatg aaagtaaatt tgaaatgatc ttttttatta taagagtaga 1860

aatacagttg ggtttgaact atatgtttta atggccacgg ttttgtaaga catttggtcc 1920

tttgttttcc cagttattac tcgattgtaa ttttatatcg ccagcaatgg actgaaacgg 1980

tccgcaacct cttctttaca actgggtgac ctcgcggctg tgccagccat ttggcgttca 2040

ccctgccgct aagggccatg tgaacccccg cggtagcatc ccttgctccg cgtggaccac 2100

tttcctgagg cacagtgata ggaacagagc cactaatctg aagagaacag agatgtgaca 2160

gactacacta atgtgagaaa aacaaggaaa gggtgactta ttggagattt cagaaataaa 2220

atgcatttat tattatattc ccttatttta attttctatt agggaattag aaagggcata 2280

aactgcttta tccagtgtta tattaaaagc ttaatgtata taatctttta gaggtaaaat 2340

ctacagccag caaaagtcat ggtaaatatt ctttgactga actctcacta aactcctcta 2400

aattatatgt catattaact ggttaaatta atataaattt gtgacatgac cttaactggt 2460

taggtaggat atttttcttc atgcaaaaat atgactaata ataatttagc acaaaaatat 2520

ttcccaatac tttaattctg tgatagaaaa atgtttaact cagctactat aatcccataa 2580

ttttgaaaac tatttatttg gctacaccaa aggaagccat acagaggcta atatcagagt 2640

attcttggaa gagacaggag aaaatgaaag ccagtttctg ctcttacctt atgtgcttgt 2700

gttcagactc ccaaacatca ggagtgtcag ataaactggt ctgaatctct gtctgaagca 2760

tggaactgaa aagaatgtag tttcagggaa gaaaggcaat agaaggaagc ctgagaatat 2820

cttcaaaggg tcagactcaa tttactttct aaagaagtag ctaggaacta gggaataact 2880

tagaaacaac aagattgtat atatgtgcat cctggcccca ttgttcctta tctgtaggga 2940

taagcgtgct tttttgtgtg tctgtatata acataactgt ttacacataa tacactgaaa 3000

tggagccctt ccttgttact tcataccatc ctctgtgctt ccttcctcag gggccgacgc 3060

cgctcccacc gtgtccatct tcccccccag catggaacag ctgacctctg gcggagccac 3120

cgtggtctgc ttcgtgaaca acttctaccc cagagacatc agcgtgaagt ggaagatcga 3180

cggcagcgag cagagggacg gcgtgctgga cagcgtgacc gaccaggaca gcaaggactc 3240

cacctacagc atgagcagca ccctgagcct gaccaaggtg gagtacgaga ggcacaacct 3300

gtacacctgc gaggtggtgc acaagaccag ctccagcccc gtggtcaagt ccttcaaccg 3360

gaacgagtgt tgaagacaaa ggtcctgaga cgccaccacc agctccccag ctccatccta 3420

tcttcccttc taaggtcttg gaggcttccc cacaagcgac ctaccactgt tgcggtgctc 3480

caaacctcct ccccacctcc ttctcctcct cctccctttc cttggctttt atcatgctaa 3540

tatttgcaga aaatattcaa taaagtgagt ctttgcactt gagatctctg tctttcttac 3600

taaatggtag taatcagttg tttttccagt tacctgggtt tctcttctaa agaagttaaa 3660

tgtttagttg ccctgaaatc caccacactt aaaggataaa taaaaccctc cacttgccct 3720

ggttggctgt ccactacatg gcagtccttt ctaaggttca cgagtactat tcatggctta 3780

tttctctggg ccatggtagg tttgaggagg catacttcct agttttcttc ccctaagtcg 3840

tcaaagtcct gaagggggac agtctttaca agcacatgtt ctgtaatctg attcaaccta 3900

cccagtaaac ttggcgaagc aaagtagaat cattatcaca ggaagcaaag gcaacctaaa 3960

tgtgcaagca ataggaaaat gtggaagccc atcatagtac ttggacttca tctgcttttg 4020

tgccttcact aagtttttaa acatgagctg gctcctatct gccattggca aggctgggca 4080

ctacccacaa cctacttcaa ggacctctat accgtgagat tacacacata catcaaaatt 4140

tgggaaaagt tctaccaagc tgagagctga tcaccccact cttaggtgct tatctctgta 4200

caccagaaac cttaagaagc aaccagtatt gagagactca tttatgaaag tctaaaactg 4260

gatacaacca aaatgtccac caacagttaa attatgacat gttcacaatt gagctattac 4320

ttaataagga gaattaataa aataaaactt aagagcatag tttaatctca taaacaagat 4380

aataagcaaa acaaaacatt ttttcatcca tgtaagttta aaagcaggta aaatttaaaa 4440

ttaagagaga cataagtttt gaggtagcaa gatggaaact ctggggcttg gggaatgttc 4500

tgtctctctg tatgggatgt gaaagttact attgtggaat tgggatctat gttcttcctg 4560

tatatattgt atacttcata ataacttcac ctaaagaaat atctaatacc cagtgcatac 4620

ataaaagagg atacaaggaa tgaatcatac gtcaaggcca gaaagacaat aaagtagggg 4680

atccaggatc aaatctccca caaccttgag ccttctacta ttctgccttc cagagctcaa 4740

agtacaaaac acataattca aacacatgat ccctccttgg ggtctcttcc ttcatgcatc 4800

gaattagaaa tagccatgta taaaatgaga tagaagagac cttcatcaac aggtcaaaga 4860

atataggtaa ttttgtctgg gtatgaagag cccacgtatc aaaggttaca ttagggaagg 4920

aagaggacac taacagtgac tttcattctc cccctcttcc tggaggcccc tgcatttagt 4980

ccctcgtggg ctcatccact cagcacacat ttactaagca tcttctcagc ctacactctg 5040

aaggcagtgc agaataatgt tagtgtccct tcccccagtt aatatgcagt ccagtttccc 5100

tgctccttcc ctttctcagt ccacataagg atgatgggaa aggacagtca ccaaatagga 5160

gagggcaacc ctttgccttc ctacctcttg agaatgtaca ttattatcca ctttttgaaa 5220

cttcttttaa ttgctttttt ttaatttgtc ttttcaaata gcataacctt gttcatccat 5280

ttctgggaac caaatttatc aatcaacagt gcctctaatc tggctattaa tacaaaaatg 5340

cctcctcaaa atatatatgt tcgagtctta tctaaaacag aacccacaat aaaaaagaag 5400

aaagaataca tataagcatt tatataattc tgagcaacct tgtgctttgt gaaaaaaata 5460

taatctaatg tcacatgctg tattcttttt atttaacact ggtgaaatta taccattaga 5520

gagaaagagg acagatcact gatcctagga tctagggatg ttacagataa gaaaacaaat 5580

gtgacaaaga gctgtcacaa ggaggatctt caaggtcaca gaatcactgt cttgatttca 5640

gtggtggtta catacattta aatatgtgat aaaatgttgt tgaactatat tcatatattg 5700

taccaatgtc aaatgcttaa ttttggctct atagtataat tatgcactaa ataactattt 5760

ggacaaagaa aatgatgttt acatcaaagg tgaggccata tttgttagga acataactta 5820

aaaaccattt tggataacta atgaaaagcc attttgtgtg ccttggcata tcatgcctaa 5880

gctgtcacca gatagatcta ataagaccta agcctcagaa gcaagcccct gcccagcaag 5940

caggcagcac agataagagc taaacccagg acaggccatg atatgctaat gaactacctt 6000

caaggtggtg ttgctgacct agtgaaccag ccccaagctg tgagccccaa tagcacaaag 6060

ctactgccca aagaaattat acaaaaattg gaactttggg aatggtgtgc aggatcgctc 6120

tgctgtatgc ctggaacaca gcttctctat gttttgtatt gataccagtc tagaagcttc 6180

caaaactttc tcactgaaga agattcccca tgtgggaccc ctacagactc ttttgcccaa 6240

acaactgctt ccctcctggt gtgatatctg ttttgctttt atgttagcat aatattataa 6300

ggaatgtttg tgtgaataaa ccaaacatat tttaaaagca aatattgtat gcacatccta 6360

attgctaaaa agtttacagc taatagtccc atgctctcca caatactgga tccaaataag 6420

tcctaatttc aatgttgggc atctttacag agagaaagac attaaaaatg aagagacatg 6480

cagagagtgc accatgccat cgtggagaca gactgaagtg acacaactgt tagtcaaaga 6540

ggattaagga cttccagaag ccaccaaagg aaggaggtat gaagtggttt ctccctcaga 6600

gtatccagag gagactaaac caaccaacac ctttttgctt aagacttctt gccttcagga 6660

ctgtgagaag gtagcttcct attgttctaa gccccagtat gtggcatttt gttaaggtag 6720

agtcaagaaa ccaataaaat gcagacagac aaaaggatag ctgagttttc caggcccttc 6780

cttcttattt ttggttttgt tggtggtggt ggtggtggtg gtgatggtgg tggttttgtt 6840

tatgttttgt ttggggagtt ttttggggtt tttttgggtt ttgtttttgt tgttgttttg 6900

ggggtttttg ttgttgttgt tgtttgcttt tttgtttttt gttttttgtt tttttgagac 6960

agtgtttctc tgtatagccc tggctgtcct ggagttcctt ctatctctaa tgtctacatc 7020

tcagagggga tcctctaatt tcaaatgagc agtagctctc catttttagc tcttatttat 7080

tcatttattt acttacttac ttattgtctg tagatgaaag aattttggag tgggaaaggg 7140

ttcatgagcc cccagcaact aatgaggagc tacagacaat tgatgtttct ggggaaagga 7200

gactcagttt ctttgagagt atagcttctg atgggtcaac catgttcctg tggctgatgt 7260

cacacccagg agtatgcaga caacagaaac tggagttaat gagttgtttt aaaaataaaa 7320

aagggcatga agcttgggat agaaattaag gataaataca attaaataca ggaaattctg 7380

aaagaattaa taaaaacatt tcttttttta aaaaaaaatc cagaattagc tatgcttctt 7440

caaaattgct tctggagaac tttacaagtt aaataagtta tattgtagaa aaggtagaga 7500

ggagaatagt ggaagagaga gataaggaga cttcaaaagg agtggaggga gatagaggag 7560

gagaaagcag aagcaatggc tgatagacac aggataagag ggaacagaaa ggagaaagag 7620

gaagccagga tgggtatttc tttgcctatc tgtgacttgc acatggtctt ggcaattatt 7680

gatgagttca aggcttaatt cttcacttgt gccaactcaa cagagtcttt ctttcttata 7740

accaggcccc cagtatgctc atgtatgtat caggtcctct tatctcctta tagcaatcct 7800

gtttataact gggtaacttt gtgaagggaa ggaagtgcac actgagatgt gctacaactt 7860

tttaatacaa aattttgaag agtttgtaca atgtatgtat aattaataat taatattatg 7920

cactttagat tttgatttca actcaagata ctaattctat atatatgggt taaatcaata 7980

tattaataag tttaatttca catgcttatt tttattgtgg ttttcgagac agggtttctc 8040

tgtatagccc tggctgtcct ggaacccact ttgtagacca ggctggcctc aaactcagaa 8100

acctacctgc ctctgcctct gcctctgcct ctgcctctgc ctctgcctct gcctctgcct 8160

ctgcctctgc ctctgcctct gcctctgcct ctgcctctgc ctctgcctct gcctctgcct 8220

ctgcctctgc ctctgcctct gcctctgcct ctgcctctgc ctctgcctct gcctctgcct 8280

ctgcctagtg ctggaattaa aggtttgcgc caccacgccc ggtgaaattt ttaaacttta 8340

tatatgtctc attctatttc tatcagatag gactgtgtag actgtgctaa actaataaat 8400

gtgccctcaa aagtaatcgc aagttgtatt gttgttgttt tgctttgctt tgctttgctt 8460

tgctttgctt tgctttgctt tgctttgctt tgctttgctt tgctttgctt tgctttgctt 8520

tgctttgctt tgctttgctt tgcttttttg ttttgggttt ttttccgggg gagggagggt 8580

ggagaaagaa tcttactatg aagctctgac tgtcctggga actcactata tagatcaggc 8640

ttgattcaac tcatagagat ctgccttctt ctgcctccca agtgctggga ataaaggcat 8700

acacctccat gcccagatag tgatcccaag ttttagcaaa agtttctaga cttgacatta 8760

atcgatggag atagacatga attacacaaa gaactaatgt ggagtttacc tgaatcatac 8820

tctatacttt atcagagatt aaattaacat ttaataatcc agtgccaggc tagaggcacc 8880

attcaatggc agtgtttgcc atcatgcata ggcttagtct tcagtgctga aaggcattgg 8940

gggcaatatt actcattata cagatgagaa actgggaaag acttgcctca gattctctac 9000

tgaaaggctg agtttgtggc ttctagaaaa tcttttactt tcaatatttt taatgtataa 9060

tttttttatt tccactgatt ttatttttta tttttaacat ttataagaaa taaatgcaat 9120

aaaccaaata catggacaaa aaaatacaag aatcatatga tcacctcaat ggaaggaaaa 9180

aaaaagaaag aaaaagtctt tgataagatt caacattcat tcttttttta ttagatattt 9240

tcttcattta catttcaaat gctatcccca aagcccccta taccttcccc tgccctgctc 9300

cccaacccac ccactcctgc tttctggccc tggcattcct ctgtactgag gcatatgatc 9360

ttcaaaaaac caagggcctc tcctctcatt ggtggccgac tattaggcca tcttttgcta 9420

catatgcaac tagagacaca gctctggggg ttactggtta gttcatattg ttagtcctcc 9480

tatagagttg cagacccctt tagctccttg gatactttct ctagttcctt cattaggggc 9540

cctgtgtccc atccaataga tgactgtgag catccacttc tgtatttgcc aggcactggc 9600
atagcctcac gagaaagaga gagctatgtc aggatcctgt cagtaaaatc tttctggcat 9660
atgcaatagt atctgggttt ggtggttgta tatgggatgg atccccaagt ggagcagtct 9720
ctgaatggtc cttccttcca tctcagctcc aaactttgtc tctataactc cttccatggg 9780
tattttgttc cccattctaa gaaggagtga agaatccaca ctttggtctt ccttcttctt 9840
gagtttcata tgttgcatct tggatattct aagtttctgg gttaatatcc acgtatcagt 9900
gagtgcatat catgcgtgtt attttgtgat tagtttacct cactcaggat gatatcctcc 9960
agatgcatcc atttgcctaa gaatttcatt aattcactgt ttttaattgc tgaatagtac 10020
tccattgtgt aaatgtacca cattttctgt atccattcct ctgttgaggg gcatctgggt 10080
tctttccagc ttctggctat tataaataag gctgctatga gcatagcgga gcatgtgtcc 10140
ttatcaagtt ggaacatctt ctaggtatat gcccaggaga ggaattgctg gatcttccgg 10200
tagtaccatc aacatgcatt cttaataaaa gccctagaac aaggaggact gtaggaaaca 10260
tattccaaca taataaaggt tatgtatgac aaactcatga ccaatatcat cctaaatgaa 10320
tgaaaccatt aataagctcc attaaaatca gaggactgcc cactatccct acttctcatc 10380
cataatgaga ttgaagcatt agctggagca ataaggcaag agaagggata caaatgggaa 10440
aatattaagt caaattgttt tcaattgaag attatattat cttataccca atgacctcaa 10500
attttgacta gaaaaattgt agaaattatc aataatttca gcaaagtgtt atgatgcacc 10560
acatccttat tcttctcccc agcttctgct tgcttctctc ttcttgctct tcatcctttc 10620
tgtccttcca tctgcctgca ctcttgtctc aagactgagt gcagcgtgta actctcctgt 10680
gactgagtat ctcacaaaac gttctacctg ccaaacctgg atgagccctt tgtctttctg 10740
aagctatgag gctctctaca tagactcaag aaggaaatga cagggaggag gtaataatga 10800
agtggggaag gctgacatta gcattgctcc tgtgtggctc cttaatttct catacttcac 10860
actgagatgt tattaactgt gactcatagg tgaagaagcc agagctaagg ttctcatatt 10920
tgagtgttat agaatgagta gagcagtagt tctcaaacta tgggtcatga ctcctttatg 10980
ggtcaaacta ccctttcaca caggttgcat atcagatatc ctaattttat atacatatat 11040
atatgcatat gtatatatat atatttcaca acagtaggaa aattatttag taatcatttt 11100
atagttgtgg gtcatggcaa catgaggaac tgtattaaag ggttgcagca ttaggaatgt 11160
tgagacccac tgtaatagag aatgaggctt aaggcagggc tataaagccc aatggaccat 11220
gtgccttttc caacatttgc cacatggtaa gctctgtata gactttttaa agaacattgg 11280
tttgtaattt taaatggata agggtcttca ctgtctatca cccatctata taataaatac 11340
ataagttttg attccaccat ggattcaaat gcaaaaatcc tcaacctaag acatagcagt 11400

gaaacattga tgaccaaata ggaaatccat gtagagacct tctatcttct gatggctcca 11460

caggcaccat cttgcaacag agttctactt tgctaccagt aatgaataca gtgtctcaac 11520

tcctgccatt gaatcttcag gaagcccctg aaatgacttg tactacacca tttcttaaag 11580

acagaaaagc taagacttag agggaataaa tgtcatgcct gagatcatgc aaccaattaa 11640

gtccaacttg gcctgatcaa gaggcacaat tcaaaagcaa tgttgttcct tcactagctc 11700

ttgtgtatgg ttgctgattc cggaagcaaa gtatcagtga atatccctag tgggaaaaga 11760

cttggaaatc aaatgtctca tttaacagat taggagatga aacggtagac tctgtgtagt 11820

tgtacacccc tgtgatccca tcgctaggaa gactgaggca ggaagtcctc gagctcaaac 11880

cagcttaggc tacacagaga aactatctaa aaaataatta ctaactactt aataggagat 11940

tggatgttaa gatctggtca ctaagaggca gaattgagat tcgaagccag tattttctac 12000

ctggtatgtt ttaaattgca gtaaggatct aagtgtagat atataataat aagattctat 12060

tgatctctgc aacaacagag agtgttagat ttgtttggaa aaaaatatta tcagccaaca 12120

tcttctacca tttcagtata gcacagagta cccacccata tctccccacc catcccccat 12180

accagactgg ttattgattt tcatggtgac tggcctgaga agattaaaaa aagtaatgct 12240

accttattgg gagtgtccca tggaccaaga tagcaactgt catagctacc gtcacactgc 12300

tttgatcaag aagacccttt gaggaactga aaacagaacc ttaggcacat ctgttgcttt 12360

cgctcccatc ctcctccaac agcctgggtg gtgcactcca caccctttca agtttccaaa 12420

gcctcataca cctgctccct accccagcac ctggccaagg ctgtatccag cactgggatg 12480

aaaatgatac cccacctcca tcttgtttga tattactcta tctcaagccc caggttagtc 12540

cccagtccca atgcttttgc acagtcaaaa ctcaacttgg aataatcagt atccttgaag 12600

agttctgata tggtcactgg gcccatatac catgtaagac atgtggaaaa gatgtttcat 12660

ggggcccaga cacgttctag aagtacctga gagtggcaaa aaatagttgt gctaaatagt 12720

ttggccatct ttaggctgag agactaggaa atacagcgat ggactatatc agcattgcag 12780

gatagttgtc agtaaacacc ccacaaccca taacagaagt attctcttct ttctatatcc 12840

cttttccatc catgtagatg gctgtcttca tatttgttct agacggccgg cc 12892

<210> 94
<211> 6425
<212> DNA
<213> VkP-IGKV1-39/J-Ck-delta2

<400> 94
ggccggccca catgaaacaa tgggaaccat gtgacaatca cagaggtgtt gttactatag 60

caaaagggat tgttactctc cacatccctt taagtaactt gaaggcctga tagacccacc 120

ctctaagact tcattagaca ttccctacga atggttatac tctcctgtat actcccaata 180

caactctaaa atatattatt ccatatagtc cttaggtttg tattaaagtt tgactttttt 240
ccttcaaaat atctcttgtc acaacagcgg ctctagagag aaatacattc cctccaggca 300
aatctatgct gcgctggtct gacctgggac cctggggaca ttgcccctgt gctgagttac 360
taagatgagc cagccctgca gctgtgctca gcctgcccca tgccctgctg attgatttgc 420
atgttccaga gcacagcccc ctgccctgaa gactttttta tgggctggtc gcaccctgtg 480
caggagtcag tctcagtcag gagccaccat ggacatgaga gtgcccgccc agctcctggg 540
gctcctgcta ctctggctcc gaggtaagga tggagaacac taggaattta ctcagccagt 600
gtgctcagta ctgactggaa cttcagggaa gttctctgat aacatgatta atagtaagaa 660
tatttgtttt tatgtttcca atctcaggtg ccagatgtga catccagatg acccagagcc 720
ccagcagcct gagcgccagc gtgggcgaca gagtgaccat cacctgcaga gccagccaga 780
gcatcagcag ctacctgaac tggtatcagc agaagcccgg caaggccccc aagctgctga 840
tctacgccgc cagctccctg cagagcggcg tgcccagcag attcagcggc agcggctccg 900
gcaccgactt caccctgacc atcagcagcc tgcagcccga ggacttcgcc acctactact 960
gccagcagag ctacagcacc ccccccacct tcggccaggg caccaaggtg gagatcaaac 1020
gtaagtacac ttttctcatc tttttttatg tgtaagacac aggttttcat gttaggagtt 1080
aaagtcagtt cagaaaatct tgagaaaatg gagagggctc attatcagtt gacgtggcat 1140
acagtgtcag attttctgtt tatcaagcta gtgagattag gggcaaaaag aggctttagt 1200
tgagaggaaa gtaattaata ctatggtcac catccaagag attggatcgg agaataagca 1260
tgagtagtta ttgagatctg ggtctgactg caggtagcgt ggtcttctag acgtttaagt 1320
gggagatttg gaggggatga ggaatgaagg aacttcagga tagaaaaggg ctgaagtcaa 1380
gttcagctcc taaaatggat gtgggagcaa actttgaaga taaactgaat gacccagagg 1440
atgaaacagc gcagatcaaa gaggggcctg gagctctgag aagagaagga gactcatccg 1500
tgttgagttt ccacaagtac tgtcttgagt tttgcaataa aagtgggata gcagagttga 1560
gtgagccgta ggctgagttc tctcttttgt ctcctaagtt tttatgacta caaaaatcag 1620
tagtatgtcc tgaaataatc attaagctgt ttgaaagtat gactgcttgc catgtagata 1680
ccatggcttg ctgaataatc agaagaggtg tgactcttat tctaaaattt gtcacaaaat 1740
gtcaaaatga gagactctgt aggaacgagt ccttgacaga cagctcaagg ggtttttttc 1800
ctttgtctca tttctacatg aaagtaaatt tgaaatgatc ttttttatta taagagtaga 1860
aatacagttg ggtttgaact atatgtttta atggccacgg ttttgtaaga catttggtcc 1920
tttgttttcc cagttattac tcgattgtaa ttttatatcg ccagcaatgg actgaaacgg 1980
tccgcaacct cttctttaca actgggtgac ctcgcggctg tgccagccat ttggcgttca 2040
ccctgccgct aagggccatg tgaacccccg cggtagcatc ccttgctccg cgtggaccac 2100

tttcctgagg cacagtgata ggaacagagc cactaatctg aagagaacag agatgtgaca 2160

gactacacta atgtgagaaa aacaaggaaa gggtgactta ttggagattt cagaaataaa 2220

atgcatttat tattatattc ccttatttta attttctatt agggaattag aaagggcata 2280

aactgcttta tccagtgtta tattaaaagc ttaatgtata taatctttta gaggtaaaat 2340

ctacagccag caaaagtcat ggtaaatatt ctttgactga actctcacta aactcctcta 2400

aattatatgt catattaact ggttaaatta atataaattt gtgacatgac cttaactggt 2460

taggtaggat atttttcttc atgcaaaaat atgactaata ataatttagc acaaaaatat 2520

ttcccaatac tttaattctg tgatagaaaa atgtttaact cagctactat aatcccataa 2580

ttttgaaaac tatttatttg gctacaccaa aggaagccat acagaggcta atatcagagt 2640

attcttggaa gagacaggag aaaatgaaag ccagtttctg ctcttacctt atgtgcttgt 2700

gttcagactc ccaaacatca ggagtgtcag ataaactggt ctgaatctct gtctgaagca 2760

tggaactgaa aagaatgtag tttcagggaa gaaaggcaat agaaggaagc ctgagaatat 2820

cttcaaaggg tcagactcaa tttactttct aaagaagtag ctaggaacta gggaataact 2880

tagaaacaac aagattgtat atatgtgcat cctggcccca ttgttcctta tctgtaggga 2940

taagcgtgct tttttgtgtg tctgtatata acataactgt ttacacataa tacactgaaa 3000

tggagccctt ccttgttact tcataccatc ctctgtgctt ccttcctcag gggccgacgc 3060

cgctcccacc gtgtccatct tcccccccag catggaacag ctgacctctg gcggagccac 3120

cgtggtctgc ttcgtgaaca acttctaccc cagagacatc agcgtgaagt ggaagatcga 3180

cggcagcgag cagagggacg gcgtgctgga cagcgtgacc gaccaggaca gcaaggactc 3240

cacctacagc atgagcagca ccctgagcct gaccaaggtg gagtacgaga ggcacaacct 3300

gtacacctgc gaggtggtgc acaagaccag ctccagcccc gtggtcaagt ccttcaaccg 3360

gaacgagtgt tgaagacaaa ggtcctgaga cgccaccacc agctccccag ctccatccta 3420

tcttcccttc taaggtcttg gaggcttccc cacaagcgac ctaccactgt tgcggtgctc 3480

caaacctcct ccccacctcc ttctcctcct cctccctttc cttggctttt atcatgctaa 3540

tatttgcaga aaatattcaa taaagtgagt ctttgcactt gagatctctg tctttcttac 3600

taaatggtag taatcagttg tttttccagt tacctgggtt tctcttctaa agaagttaaa 3660

tgtttagttg ccctgaaatc caccacactt aaaggataaa taaaaccctc cacttgccct 3720

ggttggctgt ccactacatg gcagtccttt ctaaggttca cgagtactat tcatggctta 3780

tttctctggg ccatggtagg tttgaggagg catacttcct agttttcttc ccctaagtcg 3840

tcaaagtcct gaagggggac agtctttaca agcacatgtt ctgtaatctg attcaaccta 3900

cccagtaaac ttggcgaagc aaagtagaat cattatcaca ggaagcaaag gcaacctaaa 3960

tgtgcaagca ataggaaaat gtggaagccc atcatagtac ttggacttca tctgcttttg 4020

tgccttcact aagtttttaa acatgagctg gctcctatct gccattggca aggctgggca 4080

ctacccacaa cctacttcaa ggacctctat accgtgagat tacacacata catcaaaatt 4140

tgggaaaagt tctaccaagc tgagagctga tcaccccact cttaggtgct tatctctgta 4200

caccagaaac cttaagaagc aaccagtatt gagagactca tttatgaaag tctaaaactg 4260

gatacaacca aaatgtccac caacagttaa attatgacat gttcacaatt gagctattac 4320

ttaataagga gaattaataa aataaaactt aagagcatag tttaatctca taaacaagat 4380

aataagcaaa acaaaacatt ttttcatcca tgtaagttta aaagcaggta aaatttaaaa 4440

ttaagagaga cataagtttt gaggtagcaa gatggaaact ctggggcttg gggaatgttc 4500

tgtctctctg tatgggatgt gaaagttact attgtggaat tgggatctat gttcttcctg 4560

tatatattgt atacttcata ataacttcac ctaaagaaat atctaatacc cagtgcatac 4620

ataaaagagg atacaaggaa tgaatcatac gtcaaggcca gaaagacaat aaagtagggg 4680

atccaggatc aaatctccca caaccttgag ccttctacta ttctgccttc cagagctcaa 4740

agtacaaaac acataattca aacacatgat ccctccttgg ggtctcttcc ttcatgcatc 4800

gaattagaaa tagccatgta taaaatgaga tagaagagac cttcatcaac aggtcaaaga 4860

atataggtaa ttttgtctgg gtatgaagag cccacgtatc aaaggttaca ttagggaagg 4920

aagaggacac taacagtgac tttcattctc cccctcttcc tggaggcccc tgcatttagt 4980

ccctcgtggg ctcatccact cagcacacat ttactaagca tcttctcagc ctacactctg 5040

aaggcagtgc agaataatgt tagtgtccct tcccccagtt aatatgcagt ccagtttccc 5100

tgctccttcc ctttctcagt ccacataagg atgatgggaa aggacagtca ccaaatagga 5160

gagggcaacc ctttgccttc ctacctcttg agaatgtaca ttattatcca ctttttgaaa 5220

cttcttttaa ttgctttttt ttaatttgtc ttttcaaata gcataacctt gttcatccat 5280

ttctgggaac caaatttatc aatcaacagt gcctctaatc tggctattaa tacaaaaatg 5340

cctcctcaaa atatatatgt tcgagtctta tctaaaacag aacccacaat aaaaaagaag 5400

aaagaataca tataagcatt tatataattc tgagcaacct tgtgctttgt gaaaaaaata 5460

taatctaatg tcacatgctg tattcttttt atttaacact ggtgaaatta taccattaga 5520

gagaaagagg acagatcact gatcctagga tctagggatg ttacagataa gaaaacaaat 5580

gtgacaaaga gctgtcacaa ggaggatctt caaggtcaca gaatcactgt cttgatttca 5640

gtggtggtta catacattta aatatgtgat aaaatgttgt tgaactatat tcatatattg 5700

taccaatgtc aaatgcttaa ttttggctct atagtataat tatgcactaa ataactattt 5760

ggacaaagaa aatgatgttt acatcaaagg tgaggccata tttgttagga acataactta 5820

aaaaccattt tggataacta atgaaaagcc attttgtgtg ccttggcata tcatgcctaa 5880

gctgtcacca gatagatcta ataagaccta agcctcagaa gcaagcccct gcccagcaag 5940

caggcagcac agataagagc taaacccagg acaggccatg atatgctaat gaactacctt 6000

caaggtggtg ttgctgacct agtgaaccag ccccaagctg tgagccccaa tagcacaaag 6060

ctactgccca aagaaattat acaaaaattg gaactttggg aatggtgtgc aggatcgctc 6120

tgctgtatgc ctggaacaca gcttctctat gttttgtatt gataccagtc tagaagcttc 6180

caaaactttc tcactgaaga agattcccca tgtgggaccc ctacagactc ttttgcccaa 6240

acaactgctt ccctcctggt gtgatcatgg accaagatag caactgtcat agctaccgtc 6300

acactgcttt gatcaagaag accctttgag gaactgaaaa cagaacctta ggcacatctg 6360

ttgctttcgc tcccatcctc ctccaacagc atggctgtct tcatatttgt tctagacggc 6420

cggcc 6425

<210> 95
<211> 13382
<212> DNA
<213> Artificial

<220>
<223> VkP-IGLV2-14/J-Ck

<400> 95
ggccggccca catgaaacaa tgggaaccat gtgacaatca cagaggtgtt gttactatag 60

caaaagggat tgttactctc cacatccctt taagtaactt gaaggcctga tagacccacc 120

ctctaagact tcattagaca ttccctacga atggttatac tctcctgtat actcccaata 180

caactctaaa atatattatt ccatatagtc cttaggtttg tattaaagtt tgactttttt 240

ccttcaaaat atctcttgtc acaacagcgg ctctagagag aaatacattc cctccaggca 300

aatctatgct gcgctggtct gacctgggac cctggggaca ttgcccctgt gctgagttac 360

taagatgagc cagccctgca gctgtgctca gcctgcccca tgccctgctg attgatttgc 420

atgttccaga gcacagcccc ctgccctgaa gactttttta tgggctggtc gcaccctgtg 480

caggagtcag tctcagtcag gagccaccat ggacatgaga gtgcccgccc agctcctggg 540

gctcctgcta ctctggctcc gaggtaagga tggagaacac taggaattta ctcagccagt 600

gtgctcagta ctgactggaa cttcagggaa gttctctgat aacatgatta atagtaagaa 660

tatttgtttt tatgtttcca atctcaggtg ccagatgtca gtctgccctg acccagcccg 720

cctctgtgtc tggcagccct ggccagagca tcaccatcag ctgcaccggc accagcagcg 780

acgtgggcgg ctacaactac gtgtcctggt atcagcagca ccccggcaag gcccccaagc 840

tgatgatcta cgaggtgtcc aacagaccca gcggcgtgag caacagattc agcggcagca 900

agagcggcaa caccgccagc ctgaccatca gcggcctcca ggctgaggac gaggccgact 960

actactgcag cagctacacc agcagctcca ccctggtgtt tggcggcgga acaaagctga 1020

ccgtgctgcg taagtacact tttctcatct ttttttatgt gtaagacaca ggttttcatg 1080

ttaggagtta aagtcagttc agaaaatctt gagaaaatgg agagggctca ttatcagttg 1140

acgtggcata cagtgtcaga ttttctgttt atcaagctag tgagattagg ggcaaaaaga 1200

ggctttagtt gagaggaaag taattaatac tatggtcacc atccaagaga ttggatcgga 1260

gaataagcat gagtagttat tgagatctgg gtctgactgc aggtagcgtg gtcttctaga 1320

cgtttaagtg ggagatttgg aggggatgag gaatgaagga acttcaggat agaaaagggc 1380

tgaagtcaag ttcagctcct aaaatggatg tgggagcaaa ctttgaagat aaactgaatg 1440

acccagagga tgaaacagcg cagatcaaag aggggcctgg agctctgaga agagaaggag 1500

actcatccgt gttgagtttc cacaagtact gtcttgagtt ttgcaataaa agtgggatag 1560

cagagttgag tgagccgtag gctgagttct ctcttttgtc tcctaagttt ttatgactac 1620

aaaaatcagt agtatgtcct gaaataatca ttaagctgtt tgaaagtatg actgcttgcc 1680

atgtagatac catggcttgc tgaataatca gaagaggtgt gactcttatt ctaaaatttg 1740

tcacaaaatg tcaaaatgag agactctgta ggaacgagtc cttgacagac agctcaaggg 1800

gtttttttcc tttgtctcat ttctacatga aagtaaattt gaaatgatct tttttattat 1860

aagagtagaa atacagttgg gtttgaacta tatgttttaa tggccacggt tttgtaagac 1920

atttggtcct ttgttttccc agttattact cgattgtaat tttatatcgc cagcaatgga 1980

ctgaaacggt ccgcaacctc ttctttacaa ctgggtgacc tcgcggctgt gccagccatt 2040

tggcgttcac cctgccgcta agggccatgt gaacccccgc ggtagcatcc cttgctccgc 2100

gtggaccact ttcctgaggc acagtgatag gaacagagcc actaatctga agagaacaga 2160

gatgtgacag actacactaa tgtgagaaaa acaaggaaag ggtgacttat tggagatttc 2220

agaaataaaa tgcatttatt attatattcc cttattttaa ttttctatta gggaattaga 2280

aagggcataa actgctttat ccagtgttat attaaaagct taatgtatat aatcttttag 2340

aggtaaaatc tacagccagc aaaagtcatg gtaaatattc tttgactgaa ctctcactaa 2400

actcctctaa attatatgtc atattaactg gttaaattaa tataaatttg tgacatgacc 2460

ttaactggtt aggtaggata tttttcttca tgcaaaaata tgactaataa taatttagca 2520

caaaaatatt tcccaatact ttaattctgt gatagaaaaa tgtttaactc agctactata 2580

atcccataat tttgaaaact atttattagc ttttgtgttt gacccttccc tagccaaagg 2640

caactattta aggacccttt aaaactcttg aaactacttt agagtcatta agttatttaa 2700

ccacttttaa ttactttaaa atgatgtcaa ttccctttta actattaatt tattttaagg 2760

ggggaaaggc tgctcataat tctattgttt ttcttggtaa agaactctca gttttcgttt 2820

ttactacctc tgtcacccaa gagttggcat ctcaacagag gggactttcc gagaggccat 2880

ctggcagttg cttaagatca gaagtgaagt ctgccagttc ctcccaggca ggtggcccag 2940

attacagttg acctgttctg gtgtggctaa aaattgtccc atgtggttac aaaccattag 3000

accagggtct gatgaattgc tcagaatatt tctggacacc caaatacaga ccctggctta 3060

aggccctgtc catacagtag gtttagcttg gctacaccaa aggaagccat acagaggcta 3120

atatcagagt attcttggaa gagacaggag aaaatgaaag ccagtttctg ctcttacctt 3180

atgtgcttgt gttcagactc ccaaacatca ggagtgtcag ataaactggt ctgaatctct 3240

gtctgaagca tggaactgaa aagaatgtag tttcagggaa gaaaggcaat agaaggaagc 3300

ctgagaatat cttcaaaggg tcagactcaa tttactttct aaagaagtag ctaggaacta 3360

gggaataact tagaaacaac aagattgtat atatgtgcat cctggcccca ttgttcctta 3420

tctgtaggga taagcgtgct tttttgtgtg tctgtatata acataactgt ttacacataa 3480

tacactgaaa tggagccctt ccttgttact tcataccatc ctctgtgctt ccttcctcag 3540

gggccgacgc cgctcccacc gtgtccatct tcccccccag catggaacag ctgacctctg 3600

gcggagccac cgtggtctgc ttcgtgaaca acttctaccc cagagacatc agcgtgaagt 3660

ggaagatcga cggcagcgag cagagggacg gcgtgctgga cagcgtgacc gaccaggaca 3720

gcaaggactc cacctacagc atgagcagca ccctgagcct gaccaaggtg gagtacgaga 3780

ggcacaacct gtacacctgc gaggtggtgc acaagaccag ctccagcccc gtggtcaagt 3840

ccttcaaccg gaacgagtgt tgaagacaaa ggtcctgaga cgccaccacc agctccccag 3900

ctccatccta tcttcccttc taaggtcttg gaggcttccc cacaagcgac ctaccactgt 3960

tgcggtgctc caaacctcct ccccacctcc ttctcctcct cctccctttc cttggctttt 4020

atcatgctaa tatttgcaga aaatattcaa taaagtgagt ctttgcactt gagatctctg 4080

tctttcttac taaatggtag taatcagttg tttttccagt tacctgggtt tctcttctaa 4140

agaagttaaa tgtttagttg ccctgaaatc caccacactt aaaggataaa taaaaccctc 4200

cacttgccct ggttggctgt ccactacatg gcagtccttt ctaaggttca cgagtactat 4260

tcatggctta tttctctggg ccatggtagg tttgaggagg catacttcct agttttcttc 4320

ccctaagtcg tcaaagtcct gaagggggac agtctttaca agcacatgtt ctgtaatctg 4380

attcaaccta cccagtaaac ttggcgaagc aaagtagaat cattatcaca ggaagcaaag 4440

gcaacctaaa tgtgcaagca ataggaaaat gtggaagccc atcatagtac ttggacttca 4500

tctgcttttg tgccttcact aagtttttaa acatgagctg gctcctatct gccattggca 4560

aggctgggca ctacccacaa cctacttcaa ggacctctat accgtgagat tacacacata 4620

catcaaaatt tgggaaaagt tctaccaagc tgagagctga tcaccccact cttaggtgct 4680

tatctctgta caccagaaac cttaagaagc aaccagtatt gagagactca tttatgaaag 4740

tctaaaactg gatacaacca aaatgtccac caacagttaa attatgacat gttcacaatt 4800

gagctattac ttaataagga gaattaataa aataaaactt aagagcatag tttaatctca 4860

taaacaagat aataagcaaa acaaaacatt ttttcatcca tgtaagttta aaagcaggta 4920

aaatttaaaa ttaagagaga cataagtttt gaggtagcaa gatggaaact ctggggcttg 4980

gggaatgttc tgtctctctg tatgggatgt gaaagttact attgtggaat tgggatctat 5040

gttcttcctg tatatattgt atacttcata ataacttcac ctaaagaaat atctaatacc 5100

cagtgcatac ataaaagagg atacaaggaa tgaatcatac gtcaaggcca gaaagacaat 5160

aaagtagggg atccaggatc aaatctccca caaccttgag ccttctacta ttctgccttc 5220

cagagctcaa agtacaaaac acataattca aacacatgat ccctccttgg ggtctcttcc 5280

ttcatgcatc gaattagaaa tagccatgta taaaatgaga tagaagagac cttcatcaac 5340

aggtcaaaga atataggtaa ttttgtctgg gtatgaagag cccacgtatc aaaggttaca 5400

ttagggaagg aagaggacac taacagtgac tttcattctc cccctcttcc tggaggcccc 5460

tgcatttagt ccctcgtggg ctcatccact cagcacacat ttactaagca tcttctcagc 5520

ctacactctg aaggcagtgc agaataatgt tagtgtccct tcccccagtt aatatgcagt 5580

ccagtttccc tgctccttcc ctttctcagt ccacataagg atgatgggaa aggacagtca 5640

ccaaatagga gagggcaacc ctttgccttc ctacctcttg agaatgtaca ttattatcca 5700

ctttttgaaa cttcttttaa ttgctttttt ttaatttgtc ttttcaaata gcataacctt 5760

gttcatccat ttctgggaac caaatttatc aatcaacagt gcctctaatc tggctattaa 5820

tacaaaaatg cctcctcaaa atatatatgt tcgagtctta tctaaaacag aacccacaat 5880

aaaaaagaag aaagaataca tataagcatt tatataattc tgagcaacct tgtgctttgt 5940

gaaaaaaata taatctaatg tcacatgctg tattcttttt atttaacact ggtgaaatta 6000

taccattaga gagaaagagg acagatcact gatcctagga tctagggatg ttacagataa 6060

gaaaacaaat gtgacaaaga gctgtcacaa ggaggatctt caaggtcaca gaatcactgt 6120

cttgatttca gtggtggtta catacattta aatatgtgat aaaatgttgt tgaactatat 6180

tcatatattg taccaatgtc aaatgcttaa ttttggctct atagtataat tatgcactaa 6240

ataactattt ggacaaagaa aatgatgttt acatcaaagg tgaggccata tttgttagga 6300

acataactta aaaaccattt tggataacta atgaaaagcc attttgtgtg ccttggcata 6360

tcatgcctaa gctgtcacca gatagatcta ataagaccta agcctcagaa gcaagcccct 6420

gcccagcaag caggcagcac agataagagc taaacccagg acaggccatg atatgctaat 6480

gaactacctt caaggtggtg ttgctgacct agtgaaccag ccccaagctg tgagccccaa 6540

tagcacaaag ctactgccca aagaaattat acaaaaattg gaactttggg aatggtgtgc 6600

aggatcgctc tgctgtatgc ctggaacaca gcttctctat gttttgtatt gataccagtc 6660

tagaagcttc caaaactttc tcactgaaga agattcccca tgtgggaccc ctacagactc 6720

ttttgcccaa acaactgctt ccctcctggt gtgatatctg ttttgctttt atgttagcat 6780

aatattataa ggaatgtttg tgtgaataaa ccaaacatat tttaaaagca aatattgtat 6840

gcacatccta attgctaaaa agtttacagc taatagtccc atgctctcca caatactgga 6900

tccaaataag tcctaatttc aatgttgggc atctttacag agagaaagac attaaaaatg 6960

aagagacatg cagagagtgc accatgccat cgtggagaca gactgaagtg acacaactgt 7020

tagtcaaaga ggattaagga cttccagaag ccaccaaagg aaggaggtat gaagtggttt 7080

ctccctcaga gtatccagag gagactaaac caaccaacac ctttttgctt aagacttctt 7140

gccttcagga ctgtgagaag gtagcttcct attgttctaa gccccagtat gtggcatttt 7200

gttaaggtag agtcaagaaa ccaataaaat gcagacagac aaaaggatag ctgagttttc 7260

caggcccttc cttcttattt ttggttttgt tggtggtggt ggtggtggtg gtgatggtgg 7320

tggttttgtt tatgttttgt ttggggagtt ttttggggtt tttttgggtt ttgtttttgt 7380

tgttgttttg ggggtttttg ttgttgttgt tgtttgcttt tttgtttttt gttttttgtt 7440

tttttgagac agtgtttctc tgtatagccc tggctgtcct ggagttcctt ctatctctaa 7500

tgtctacatc tcagagggga tcctctaatt tcaaatgagc agtagctctc catttttagc 7560

tcttatttat tcatttattt acttacttac ttattgtctg tagatgaaag aattttggag 7620

tgggaaaggg ttcatgagcc cccagcaact aatgaggagc tacagacaat tgatgtttct 7680

ggggaaagga gactcagttt ctttgagagt atagcttctg atgggtcaac catgttcctg 7740

tggctgatgt cacacccagg agtatgcaga caacagaaac tggagttaat gagttgtttt 7800

aaaaataaaa aagggcatga agcttgggat agaaattaag gataaataca attaaataca 7860

ggaaattctg aaagaattaa taaaaacatt tcttttttta aaaaaaaatc cagaattagc 7920

tatgcttctt caaaattgct tctggagaac tttacaagtt aaataagtta tattgtagaa 7980

aaggtagaga ggagaatagt ggaagagaga gataaggaga cttcaaaagg agtggaggga 8040

gatagaggag gagaaagcag aagcaatggc tgatagacac aggataagag ggaacagaaa 8100

ggagaaagag gaagccagga tgggtatttc tttgcctatc tgtgacttgc acatggtctt 8160

ggcaattatt gatgagttca aggcttaatt cttcacttgt gccaactcaa cagagtcttt 8220

ctttcttata accaggcccc cagtatgctc atgtatgtat caggtcctct tatctcctta 8280

tagcaatcct gtttataact gggtaacttt gtgaagggaa ggaagtgcac actgagatgt 8340

gctacaactt tttaatacaa aattttgaag agtttgtaca atgtatgtat aattaataat 8400

taatattatg cactttagat tttgatttca actcaagata ctaattctat atatatgggt 8460

taaatcaata tattaataag tttaatttca catgcttatt tttattgtgg ttttcgagac 8520

agggtttctc tgtatagccc tggctgtcct ggaacccact ttgtagacca ggctggcctc 8580
aaactcagaa acctacctgc ctctgcctct gcctctgcct ctgcctctgc ctctgcctct 8640
gcctctgcct ctgcctctgc ctctgcctct gcctctgcct ctgcctctgc ctctgcctct 8700
gcctctgcct ctgcctctgc ctctgcctct gcctctgcct ctgcctctgc ctctgcctct 8760
gcctctgcct ctgcctagtg ctggaattaa aggtttgcgc caccacgccc ggtgaaattt 8820
ttaaacttta tatatgtctc attctatttc tatcagatag gactgtgtag actgtgctaa 8880
actaataaat gtgccctcaa aagtaatcgc aagttgtatt gttgttgttt tgctttgctt 8940
tgctttgctt tgctttgctt tgctttgctt tgctttgctt tgctttgctt tgctttgctt 9000
tgctttgctt tgctttgctt tgctttgctt tgcttttttg ttttgggttt ttttccgggg 9060
gagggagggt ggagaaagaa tcttactatg aagctctgac tgtcctggga actcactata 9120
tagatcaggc ttgattcaac tcatagagat ctgccttctt ctgcctccca agtgctggga 9180
ataaaggcat acacctccat gcccagatag tgatcccaag ttttagcaaa agtttctaga 9240
cttgacatta atcgatggag atagacatga attacacaaa gaactaatgt ggagtttacc 9300
tgaatcatac tctatacttt atcagagatt aaattaacat ttaataatcc agtgccaggc 9360
tagaggcacc attcaatggc agtgtttgcc atcatgcata ggcttagtct tcagtgctga 9420
aaggcattgg gggcaatatt actcattata cagatgagaa actgggaaag acttgcctca 9480
gattctctac tgaaaggctg agtttgtggc ttctagaaaa tcttttactt tcaatatttt 9540
taatgtataa tttttttatt tccactgatt ttatttttta tttttaacat ttataagaaa 9600
taaatgcaat aaaccaaata catggacaaa aaaatacaag aatcatatga tcacctcaat 9660
ggaaggaaaa aaaaagaaag aaaaagtctt tgataagatt caacattcat tcttttttta 9720
ttagatattt tcttcattta catttcaaat gctatcccca aagcccccta taccttcccc 9780
tgccctgctc cccaacccac ccactcctgc tttctggccc tggcattcct ctgtactgag 9840
gcatatgatc ttcaaaaaac caagggcctc tcctctcatt ggtggccgac tattaggcca 9900
tcttttgcta catatgcaac tagagacaca gctctggggg ttactggtta gttcatattg 9960
ttagtcctcc tatagagttg cagacccctt tagctccttg gatactttct ctagttcctt 10020
cattaggggc cctgtgtccc atccaataga tgactgtgag catccacttc tgtatttgcc 10080
aggcactggc atagcctcac gagaaagaga gagctatgtc aggatcctgt cagtaaaatc 10140
tttctggcat atgcaatagt atctgggttt ggtggttgta tatgggatgg atccccaagt 10200
ggagcagtct ctgaatggtc cttccttcca tctcagctcc aaactttgtc tctataactc 10260
cttccatggg tattttgttc cccattctaa gaaggagtga agaatccaca ctttggtctt 10320
ccttcttctt gagtttcata tgttgcatct tggatattct aagtttctgg gttaatatcc 10380
acgtatcagt gagtgcatat catgcgtgtt attttgtgat tagtttacct cactcaggat 10440

gatatcctcc agatgcatcc atttgcctaa gaatttcatt aattcactgt ttttaattgc 10500

tgaatagtac tccattgtgt aaatgtacca cattttctgt atccattcct ctgttgaggg 10560

gcatctgggt tctttccagc ttctggctat tataaataag gctgctatga gcatagcgga 10620

gcatgtgtcc ttatcaagtt ggaacatctt ctaggtatat gcccaggaga ggaattgctg 10680

gatcttccgg tagtaccatc aacatgcatt cttaataaaa gccctagaac aaggaggact 10740

gtaggaaaca tattccaaca taataaaggt tatgtatgac aaactcatga ccaatatcat 10800

cctaaatgaa tgaaaccatt aataagctcc attaaaatca gaggactgcc cactatccct 10860

acttctcatc cataatgaga ttgaagcatt agctggagca ataaggcaag agaagggata 10920

caaatgggaa aatattaagt caaattgttt tcaattgaag attatattat cttataccca 10980

atgacctcaa attttgacta gaaaaattgt agaaattatc aataatttca gcaaagtgtt 11040

atgatgcacc acatccttat tcttctcccc agcttctgct tgcttctctc ttcttgctct 11100

tcatcctttc tgtccttcca tctgcctgca ctcttgtctc aagactgagt gcagcgtgta 11160

actctcctgt gactgagtat ctcacaaaac gttctacctg ccaaacctgg atgagccctt 11220

tgtctttctg aagctatgag gctctctaca tagactcaag aaggaaatga cagggaggag 11280

gtaataatga agtggggaag gctgacatta gcattgctcc tgtgtggctc cttaatttct 11340

catacttcac actgagatgt tattaactgt gactcatagg tgaagaagcc agagctaagg 11400

ttctcatatt tgagtgttat agaatgagta gagcagtagt tctcaaacta tgggtcatga 11460

ctcctttatg ggtcaaacta ccctttcaca caggttgcat atcagatatc ctaattttat 11520

atacatatat atatgcatat gtatatatat atatttcaca acagtaggaa aattatttag 11580

taatcatttt atagttgtgg gtcatggcaa catgaggaac tgtattaaag ggttgcagca 11640

ttaggaatgt tgagacccac tgtaatagag aatgaggctt aaggcagggc tataaagccc 11700

aatggaccat gtgccttttc caacatttgc cacatggtaa gctctgtata gactttttaa 11760

agaacattgg tttgtaattt taaatggata agggtcttca ctgtctatca cccatctata 11820

taataaatac ataagttttg attccaccat ggattcaaat gcaaaaatcc tcaacctaag 11880

acatagcagt gaaacattga tgaccaaata ggaaatccat gtagagacct tctatcttct 11940

gatggctcca caggcaccat cttgcaacag agttctactt tgctaccagt aatgaataca 12000

gtgtctcaac tcctgccatt gaatcttcag gaagcccctg aaatgacttg tactacacca 12060

tttcttaaag acagaaaagc taagacttag agggaataaa tgtcatgcct gagatcatgc 12120

aaccaattaa gtccaacttg gcctgatcaa gaggcacaat tcaaaagcaa tgttgttcct 12180

tcactagctc ttgtgtatgg ttgctgattc cggaagcaaa gtatcagtga atatccctag 12240

tgggaaaaga cttggaaatc aaatgtctca tttaacagat taggagatga aacggtagac 12300

tctgtgtagt tgtacacccc tgtgatccca tcgctaggaa gactgaggca ggaagtcctc 12360

gagctcaaac cagcttaggc tacacagaga aactatctaa aaaataatta ctaactactt 12420

aataggagat tggatgttaa gatctggtca ctaagaggca gaattgagat tcgaagccag 12480

tattttctac ctggtatgtt ttaaattgca gtaaggatct aagtgtagat atataataat 12540

aagattctat tgatctctgc aacaacagag agtgttagat ttgtttggaa aaaaatatta 12600

tcagccaaca tcttctacca tttcagtata gcacagagta cccacccata tctccccacc 12660

catcccccat accagactgg ttattgattt tcatggtgac tggcctgaga agattaaaaa 12720

aagtaatgct accttattgg gagtgtccca tggaccaaga tagcaactgt catagctacc 12780

gtcacactgc tttgatcaag aagacccttt gaggaactga aaacagaacc ttaggcacat 12840

ctgttgcttt cgctcccatc ctcctccaac agcctgggtg gtgcactcca caccctttca 12900

agtttccaaa gcctcataca cctgctccct accccagcac ctggccaagg ctgtatccag 12960

cactgggatg aaaatgatac cccacctcca tcttgtttga tattactcta tctcaagccc 13020

caggttagtc cccagtccca atgcttttgc acagtcaaaa ctcaacttgg aataatcagt 13080

atccttgaag agttctgata tggtcactgg gcccatatac catgtaagac atgtggaaaa 13140

gatgtttcat ggggcccaga cacgttctag aagtacctga gagtggcaaa aaatagttgt 13200

gctaaatagt ttggccatct ttaggctgag agactaggaa atacagcgat ggactatatc 13260

agcattgcag gatagttgtc agtaaacacc ccacaaccca taacagaagt attctcttct 13320

ttctatatcc cttttccatc catgtagatg gctgtcttca tatttgttct agacggccgg 13380

cc 13382

<210> 96
<211> 4638
<212> DNA
<213> Artificial

<220>
<223> pSELECT-IGKV1-39/J-Ck

<400> 96
gcggccgcaa taaaatatct ttattttcat tacatctgtg tgttggtttt ttgtgtgaat 60

cgtaactaac atacgctctc catcaaaaca aaacgaaaca aaacaaacta gcaaaatagg 120

ctgtccccag tgcaagtgca ggtgccagaa catttctcta tcgaaggatc tgcgatcgct 180

ccggtgcccg tcagtgggca gagcgcacat cgcccacagt ccccgagaag ttggggggag 240

gggtcggcaa ttgaacgggt gcctagagaa ggtggcgcgg ggtaaactgg gaaagtgatg 300

tcgtgtactg gctccgcctt tttcccgagg gtgggggaga accgtatata agtgcagtag 360

tcgccgtgaa cgttcttttt cgcaacgggt ttgccgccag aacacagctg aagcttcgag 420

gggctcgcat ctctccttca cgcgcccgcc gccctacctg aggccgccat ccacgccggt 480

tgagtcgcgt tctgccgcct cccgcctgtg gtgcctcctg aactgcgtcc gccgtctagg 540

taagtttaaa gctcaggtcg agaccgggcc tttgtccggc gctcccttgg agcctaccta 600

gactcagccg gctctccacg ctttgcctga ccctgcttgc tcaactctac gtctttgttt 660

cgttttctgt tctgcgccgt tacagatcca agctgtgacc ggcgcctacc tgagatcacc 720

ggcgtgtcga cgccaccatg gacatgagag tgcccgccca gctcctgggg ctcctgctac 780

tctggctccg aggtaaggat ggagaacact aggaatttac tcagccagtg tgctcagtac 840

tgactggaac ttcagggaag ttctctgata acatgattaa tagtaagaat atttgttttt 900

atgtttccaa tctcaggtgc cagatgtgac atccagatga cccagagccc cagcagcctg 960

agcgccagcg tgggcgacag agtgaccatc acctgcagag ccagccagag catcagcagc 1020

tacctgaact ggtatcagca gaagcccggc aaggccccca agctgctgat ctacgccgcc 1080

agctccctgc agagcggcgt gcccagcaga ttcagcggca gcggctccgg caccgacttc 1140

accctgacca tcagcagcct gcagcccgag gacttcgcca cctactactg ccagcagagc 1200

tacagcaccc cccccacctt cggccagggc accaaggtgg agatcaagag agccgacgcc 1260

gctcccaccg tgtccatctt cccccccagc atggaacagc tgacctctgg cggagccacc 1320

gtggtctgct tcgtgaacaa cttctacccc agagacatca gcgtgaagtg gaagatcgac 1380

ggcagcgagc agagggacgg cgtgctggac agcgtgaccg accaggacag caaggactcc 1440

acctacagca tgagcagcac cctgagcctg accaaggtgg agtacgagag gcacaacctg 1500

tacacctgcg aggtggtgca caagaccagc tccagccccg tggtcaagtc cttcaaccgg 1560

aacgagtgtt gagctagctg gccagacatg ataagataca ttgatgagtt tggacaaacc 1620

acaactagaa tgcagtgaaa aaaatgcttt atttgtgaaa tttgtgatgc tattgcttta 1680

tttgtaacca ttataagctg caataaacaa gttaacaaca acaattgcat tcattttatg 1740

tttcaggttc agggggaggt gtgggaggtt ttttaaagca agtaaaacct ctacaaatgt 1800

ggtatggaat tctaaaatac agcatagcaa aactttaacc tccaaatcaa gcctctactt 1860

gaatcctttt ctgagggatg aataaggcat aggcatcagg ggctgttgcc aatgtgcatt 1920

agctgtttgc agcctcacct tctttcatgg agtttaagat atagtgtatt ttcccaaggt 1980

ttgaactagc tcttcatttc tttatgtttt aaatgcactg acctcccaca ttcccttttt 2040

agtaaaatat tcagaaataa tttaaataca tcattgcaat gaaaataaat gttttttatt 2100

aggcagaatc cagatgctca aggcccttca taatatcccc cagtttagta gttggactta 2160

gggaacaaag gaacctttaa tagaaattgg acagcaagaa agcgagcttc tagcgaattc 2220

tcgactcatt cctttgccct cggacgagtg ctggggcgtc ggtttccact atcggcgagt 2280

acttctacac agccatcggt ccagacggcc gcgcttctgc gggcgatttg tgtacgcccg 2340

acagtcccgg ctccggatcg gacgattgcg tcgcatcgac cctgcgccca agctgcatca 2400

tcgaaattgc cgtcaaccaa gctctgatag agttggtcaa gaccaatgcg gagcatatac 2460

gcccggagcc gcggcgatcc tgcaagctcc ggatgcctcc gctcgaagta gcgcgtctgc 2520

tgctccatac aagccaacca cggcctccag aagaagatgt tggcgacctc gtattgggaa 2580

tccccgaaca tcgcctcgct ccagtcaatg accgctgtta tgcggccatt gtccgtcagg 2640

acattgttgg agccgaaatc cgcgtgcacg aggtgccgga cttcggggca gtcctcggcc 2700

caaagcatca gctcatcgag agcctgcgcg acggacgcac tgacggtgtc gtccatcaca 2760

gtttgccagt gatacacatg gggatcagca atcgcgcata tgaaatcacg ccatgtagtg 2820

tattgaccga ttccttgcgg tccgaatggg ccgaacccgc tcgtctggct aagatcggcc 2880

gcagcgatcg catccatgag ctccgcgacg ggttgcagaa cagcgggcag ttcggtttca 2940

ggcaggtctt gcaacgtgac accctgtgca cggcgggaga tgcaataggt caggctctcg 3000

ctgaattccc caatgtcaag cacttccgga atcgggagcg cggccgatgc aaagtgccga 3060

taaacataac gatctttgta gaaaccatcg gcgcagctat ttacccgcag gacatatcca 3120

cgccctccta catcgaagct gaaagcacga gattcttcgc cctccgagag ctgcatcagg 3180

tcggagacgc tgtcgaactt ttcgatcaga aacttctcga cagacgtcgc ggtgagttca 3240

ggctttttca tgatggccct cctatagtga gtcgtattat actatgccga tatactatgc 3300

cgatgattaa ttgtcaaaac agcgtggatg gcgtctccag cttatctgac ggttcactaa 3360

acgagctctg cttatataga cctcccaccg tacacgccta ccgcccattt gcgtcaatgg 3420

ggcggagttg ttacgacatt ttggaaagtc ccgttgattt actagtcaaa acaaactccc 3480

attgacgtca atggggtgga gacttggaaa tccccgtgag tcaaaccgct atccacgccc 3540

attgatgtac tgccaaaacc gcatcatcat ggtaatagcg atgactaata cgtagatgta 3600

ctgccaagta ggaaagtccc ataaggtcat gtactgggca taatgccagg cgggccattt 3660

accgtcattg acgtcaatag ggggcgtact tggcatatga tacacttgat gtactgccaa 3720

gtgggcagtt taccgtaaat actccaccca ttgacgtcaa tggaaagtcc ctattggcgt 3780

tactatggga acatacgtca ttattgacgt caatgggcgg gggtcgttgg gcggtcagcc 3840

aggcgggcca tttaccgtaa gttatgtaac gcctgcaggt taattaagaa catgtgagca 3900

aaaggccagc aaaaggccag gaaccgtaaa aaggccgcgt tgctggcgtt tttccatagg 3960

ctccgccccc ctgacgagca tcacaaaaat cgacgctcaa gtcagaggtg gcgaaacccg 4020

acaggactat aaagatacca ggcgtttccc cctggaagct ccctcgtgcg ctctcctgtt 4080

ccgaccctgc cgcttaccgg atacctgtcc gcctttctcc cttcgggaag cgtggcgctt 4140

tctcatagct cacgctgtag gtatctcagt tcggtgtagg tcgttcgctc caagctgggc 4200

tgtgtgcacg aaccccccgt tcagcccgac cgctgcgcct tatccggtaa ctatcgtctt 4260

gagtccaacc cggtaagaca cgacttatcg ccactggcag cagccactgg taacaggatt 4320

agcagagcga ggtatgtagg cggtgctaca gagttcttga agtggtggcc taactacggc 4380

tacactagaa gaacagtatt tggtatctgc gctctgctga agccagttac cttcggaaaa 4440

agagttggta gctcttgatc cggcaaacaa accaccgctg gtagcggtgg tttttttgtt 4500

tgcaagcagc agattacgcg cagaaaaaaa ggatctcaag aagatccttt gatcttttct 4560

acggggtctg acgctcagtg gaacgaaaac tcacgttaag ggattttggt catggctagt 4620

taattaacat ttaaatca 4638

<210> 97
<211> 5349
<212> DNA
<213> Artificial

<220>
<223> pSelect-IGVL2-14/J-Ck

<400> 97
gcggccgcaa taaaatatct ttattttcat tacatctgtg tgttggtttt ttgtgtgaat 60

cgtaactaac atacgctctc catcaaaaca aaacgaaaca aaacaaacta gcaaaatagg 120

ctgtccccag tgcaagtgca ggtgccagaa catttctcta tcgaaggatc tgcgatcgct 180

ccggtgcccg tcagtgggca gagcgcacat cgcccacagt ccccgagaag ttggggggag 240

gggtcggcaa ttgaacgggt gcctagagaa ggtggcgcgg ggtaaactgg gaaagtgatg 300

tcgtgtactg gctccgcctt tttcccgagg gtgggggaga accgtatata agtgcagtag 360

tcgccgtgaa cgttcttttt cgcaacgggt ttgccgccag aacacagctg aagcttcgag 420

gggctcgcat ctctccttca cgcgcccgcc gccctacctg aggccgccat ccacgccggt 480

tgagtcgcgt tctgccgcct cccgcctgtg gtgcctcctg aactgcgtcc gccgtctagg 540

taagtttaaa gctcaggtcg agaccgggcc tttgtccggc gctcccttgg agcctaccta 600

gactcagccg gctctccacg ctttgcctga ccctgcttgc tcaactctac gtctttgttt 660

cgttttctgt tctgcgccgt tacagatcca agctgtgacc ggcgcctacc tgagatcacc 720

ggcgtgtcga cgccaccatg gacatgagag tgcccgccca gctcctgggg ctcctgctac 780

tctggctccg aggtaaggat ggagaacact aggaatttac tcagccagtg tgctcagtac 840

tgactggaac ttcagggaag ttctctgata acatgattaa tagtaagaat atttgttttt 900

atgtttccaa tctcaggtgc cagatgtcag tctgccctga cccagcccgc ctctgtgtct 960

ggcagccctg gccagagcat caccatcagc tgcaccggca ccagcagcga cgtgggcggc 1020

tacaactacg tgtcctggta tcagcagcac cccggcaagg cccccaagct gatgatctac 1080

gaggtgtcca acagacccag cggcgtgagc aacagattca gcggcagcaa gagcggcaac 1140

accgccagcc tgaccatcag cggcctccag gctgaggacg aggccgacta ctactgcagc 1200
agctacacca gcagctccac cctggtgttt ggcggcggaa caaagctgac cgtgctgaga 1260
gccgacgccg ctcccaccgt gtccatcttc ccccccagca tggaacagct gacctctggc 1320
ggagccaccg tggtctgctt cgtgaacaac ttctacccca gagacatcag cgtgaagtgg 1380
aagatcgacg gcagcgagca gagggacggc gtgctggaca gcgtgaccga ccaggacagc 1440
aaggactcca cctacagcat gagcagcacc ctgagcctga ccaaggtgga gtacgagagg 1500
cacaacctgt acacctgcga ggtggtgcac aagaccagct ccagccccgt ggtcaagtcc 1560
ttcaaccgga acgagtgttg agctagctgg ccagacatga taagatacat tgatgagttt 1620
ggacaaacca caactagact gactcagcct gcctccgtgt ctgggtctcc tggacagtcg 1680
atcaccatct cctgcactgg aaccagcagt gacgttggtg gttataacta tgtctcctgg 1740
taccaacagc acccaggcaa agcccccaaa ctcatgattt atgaggtcag taatcggccc 1800
tcaggggttt ctaatcgctt ctctggctcc aagtctggca acacggcctc cctgaccatc 1860
tctgggctcc aggctgagga cgaggctgat tattactgca gctcatatac aagcagcagc 1920
actctcgtat tcggcggagg gaccaagctg accgtcctac gggctgatgc tgcaccaact 1980
gtatccatct tcccaccatc catggaacag ttaacatctg gaggtgccac agtcgtgtgc 2040
ttcgtgaaca acttctatcc cagagacatc agtgtcaagt ggaagattga tggcagtgaa 2100
caacgagatg gtgtcctgga cagtgttact gatcaggaca gcaaagacag cacgtacagc 2160
atgagcagca ccctctcgtt gaccaaggtt gaatatgaaa ggcataacct ctatacctgt 2220
gaggttgttc ataagacatc atcctcaccc gtcgtcaaga gcttcaacag gaatgagtgt 2280
taggctagct ggccagacat gataagatac attgatgagt ttggacaaac cacaactaga 2340
atgcagtgaa aaaaatgctt tatttgtgaa atttgtgatg ctattgcttt atttgtaacc 2400
attataagct gcaataaaca agttaacaac aacaattgca ttcattttat gtttcaggtt 2460
cagggggagg tgtgggaggt tttttaaagc aagtaaaacc tctacaaatg tggtatggaa 2520
ttctaaaata cagcatagca aaactttaac ctccaaatca agcctctact tgaatccttt 2580
tctgagggat gaataaggca taggcatcag gggctgttgc caatgtgcat tagctgtttg 2640
cagcctcacc ttctttcatg gagtttaaga tatagtgtat tttcccaagg tttgaactag 2700
ctcttcattt ctttatgttt taaatgcact gacctcccac attccctttt tagtaaaata 2760
ttcagaaata atttaaatac atcattgcaa tgaaaataaa tgttttttat taggcagaat 2820
ccagatgctc aaggcccttc ataatatccc ccagtttagt agttggactt agggaacaaa 2880
ggaaccttta atagaaattg gacagcaaga aagcgagctt ctagcgaatt ctcgactcat 2940
tcctttgccc tcggacgagt gctggggcgt cggtttccac tatcggcgag tacttctaca 3000
cagccatcgg tccagacggc cgcgcttctg cgggcgattt gtgtacgccc gacagtcccg 3060

gctccggatc ggacgattgc gtcgcatcga ccctgcgccc aagctgcatc atcgaaattg 3120

ccgtcaacca agctctgata gagttggtca agaccaatgc ggagcatata cgcccggagc 3180

cgcggcgatc ctgcaagctc cggatgcctc cgctcgaagt agcgcgtctg ctgctccata 3240

caagccaacc acggcctcca gaagaagatg ttggcgacct cgtattggga atccccgaac 3300

atcgcctcgc tccagtcaat gaccgctgtt atgcggccat tgtccgtcag gacattgttg 3360

gagccgaaat ccgcgtgcac gaggtgccgg acttcggggc agtcctcggc ccaaagcatc 3420

agctcatcga gagcctgcgc gacggacgca ctgacggtgt cgtccatcac agtttgccag 3480

tgatacacat ggggatcagc aatcgcgcat atgaaatcac gccatgtagt gtattgaccg 3540

attccttgcg gtccgaatgg gccgaacccg ctcgtctggc taagatcggc cgcagcgatc 3600

gcatccatga gctccgcgac gggttgcaga acagcgggca gttcggtttc aggcaggtct 3660

tgcaacgtga caccctgtgc acggcgggag atgcaatagg tcaggctctc gctgaattcc 3720

ccaatgtcaa gcacttccgg aatcgggagc gcggccgatg caaagtgccg ataaacataa 3780

cgatctttgt agaaaccatc ggcgcagcta tttacccgca ggacatatcc acgccctcct 3840

acatcgaagc tgaaagcacg agattcttcg ccctccgaga gctgcatcag gtcggagacg 3900

ctgtcgaact tttcgatcag aaacttctcg acagacgtcg cggtgagttc aggctttttc 3960

atgatggccc tcctatagtg agtcgtatta tactatgccg atatactatg ccgatgatta 4020

attgtcaaaa cagcgtggat ggcgtctcca gcttatctga cggttcacta aacgagctct 4080

gcttatatag acctcccacc gtacacgcct accgcccatt tgcgtcaatg gggcggagtt 4140

gttacgacat tttggaaagt cccgttgatt tactagtcaa aacaaactcc cattgacgtc 4200

aatggggtgg agacttggaa atccccgtga gtcaaaccgc tatccacgcc cattgatgta 4260

ctgccaaaac cgcatcatca tggtaatagc gatgactaat acgtagatgt actgccaagt 4320

aggaaagtcc cataaggtca tgtactgggc ataatgccag gcgggccatt taccgtcatt 4380

gacgtcaata gggggcgtac ttggcatatg atacacttga tgtactgcca agtgggcagt 4440

ttaccgtaaa tactccaccc attgacgtca atggaaagtc cctattggcg ttactatggg 4500

aacatacgtc attattgacg tcaatgggcg ggggtcgttg ggcggtcagc caggcgggcc 4560

atttaccgta agttatgtaa cgcctgcagg ttaattaaga acatgtgagc aaaaggccag 4620

caaaaggcca ggaaccgtaa aaaggccgcg ttgctggcgt ttttccatag gctccgcccc 4680

cctgacgagc atcacaaaaa tcgacgctca agtcagaggt ggcgaaaccc gacaggacta 4740

taaagatacc aggcgtttcc ccctggaagc tccctcgtgc gctctcctgt tccgaccctg 4800

ccgcttaccg gatacctgtc cgcctttctc ccttcgggaa gcgtggcgct ttctcatagc 4860

tcacgctgta ggtatctcag ttcggtgtag gtcgttcgct ccaagctggg ctgtgtgcac 4920

gaaccccccg ttcagcccga ccgctgcgcc ttatccggta actatcgtct tgagtccaac 4980

ccggtaagac acgacttatc gccactggca gcagccactg gtaacaggat tagcagagcg 5040

aggtatgtag gcggtgctac agagttcttg aagtggtggc ctaactacgg ctacactaga 5100

agaacagtat ttggtatctg cgctctgctg aagccagtta ccttcggaaa aagagttggt 5160

agctcttgat ccggcaaaca aaccaccgct ggtagcggtg gtttttttgt ttgcaagcag 5220

cagattacgc gcagaaaaaa aggatctcaa gaagatcctt tgatcttttc tacggggtct 5280

gacgctcagt ggaacgaaaa ctcacgttaa gggattttgg tcatggctag ttaattaaca 5340

tttaaatca 5349

<210> 98
<211> 6772
<212> DNA
<213> Artificial

<220>
<223> MV1043

<400> 98
cttgatttgg gtgatggttc acgtagtggg ccatcgccct gatagacggt ttttcgccct 60

ttgacgttgg agtccacgtt ctttaatagt ggactcttgt tccaaactgg aacaacactc 120

aactctatct cgggctattc ttttgattta taagggattt tgccgatttc ggtctattgg 180

ttaaaaaatg agctgattta acaaaaattt aacgcgaatt ttaacaaaat attaacgttt 240

acaattttat ggtgcagtct cagtacaatc tgctctgatg ccgcatagtt aagccagccc 300

cgacacccgc caacacccgc tgacgcgccc tgacgggctt gtctgctccc ggcatccgct 360

tacagacaag ctgtgaccgt ctccgggagc tgcatgtgtc agaggttttc accgtcatca 420

ccgaaacgcg cgagacgaaa gggcctcgtg atacgcctat ttttataggt taatgtcatg 480

ataataatgg tttcttagac gtcaggtggc acttttcggg gaaatgtgcg cggaacccct 540

atttgtttat ttttctaaat acattcaaat atgtatccgc tcatgagaca ataaccctga 600

taaatgcttc aataatattg aaaaaggaag agtatgagta ttcaacattt ccgtgtcgcc 660

cttattccct tttttgcggc attttgcctt cctgtttttg ctcacccaga aacgctggtg 720

aaagtaaaag atgctgaaga tcagttgggt gcccgagtgg gttacatcga actggatctc 780

aacagcggta agatccttga gagttttcgc cccgaagaac gttttccaat gatgagcact 840

tttaaagttc tgctatgtgg cgcggtatta tcccgtattg acgccgggca agagcaactc 900

ggtcgccgca tacactattc tcagaatgac ttggttgagt actcaccagt cacagaaaag 960

catcttacgg atggcatgac agtaagagaa ttatgcagtg ctgccataac catgagtgat 1020

aacactgcgg ccaacttact tctgacaacg atcggaggac cgaaggagct aaccgctttt 1080

ttgcacaaca tgggggatca tgtaactcgc cttgatcgtt gggaaccgga gctgaatgaa 1140

gccataccaa acgacgagcg tgacaccacg atgcctgtag caatggcaac aacgttgcgc 1200

aaactattaa ctggcgaact acttactcta gcttcccggc aacaattaat agactggatg 1260

gaggcggata aagttgcagg accacttctg cgctcggccc ttccggctgg ctggtttatt 1320

gctgataaat ctggagccgg tgagcgtggg tctcgcggta tcattgcagc actggggcca 1380

gatggtaagc cctcccgtat cgtagttatc tacacgacgg ggagtcaggc aactatggat 1440

gaacgaaata gacagatcgc tgagataggt gcctcactga ttaagcattg gtaactgtca 1500

gaccaagttt actcatatat actttagatt gatttaaaac ttcattttta atttaaaagg 1560

atctaggtga agatcctttt tgataatctc atgaccaaaa tcccttaacg tgagttttcg 1620

ttccactgag cgtcagaccc cgtagaaaag atcaaaggat cttcttgaga tccttttttt 1680

ctgcgcgtaa tctgctgctt gcaaacaaaa aaaccaccgc taccagcggt ggtttgtttg 1740

ccggatcaag agctaccaac tctttttccg aaggtaactg gcttcagcag agcgcagata 1800

ccaaatactg ttcttctagt gtagccgtag ttaggccacc acttcaagaa ctctgtagca 1860

ccgcctacat acctcgctct gctaatcctg ttaccagtgg ctgctgccag tggcgataag 1920

tcgtgtctta ccgggttgga ctcaagacga tagttaccgg ataaggcgca gcggtcgggc 1980

tgaacggggg gttcgtgcat acagcccagc ttggagcgaa cgacctacac cgaactgaga 2040

tacctacagc gtgagctatg agaaagcgcc acgcttcccg aagggagaaa ggcggacagg 2100

tatccggtaa gcggcagggt cggaacagga gagcgcacga gggagcttcc agggggaaac 2160

gcctggtatc tttatagtcc tgtcgggttt cgccacctct gacttgagcg tcgatttttg 2220

tgatgctcgt caggggggcg gagcctatgg aaaaacgcca gcaacgcggc ctttttacgg 2280

ttcctggcct tttgctggcc ttttgctcac atgttctttc ctgcgttatc ccctgattct 2340

gtggataacc gtattaccgc ctttgagtga gctgataccg ctcgccgcag ccgaacgacc 2400

gagcgcagcg agtcagtgag cgaggaagcg gaagagcgcc caatacgcaa accgcctctc 2460

cccgcgcgtt ggccgattca ttaatgcagc tggcacgaca ggtttcccga ctggaaagcg 2520

ggcagtgagc gcaacgcaat taatgtgagt tagctcactc attaggcacc ccaggcttta 2580

cactttatgc ttccggctcg tatgttgtgt ggaattgtga gcggataaca atttcacaca 2640

ggaaacagct atgaccatga ttacgccaag ctttggagcc ttttttttgg agattttcaa 2700

cgtgaaaaaa ttattattcg caattccttt agttgttcct ttctattctc acagtgcaca 2760

gatccaaatg acccagtctc catcctccct gtctgcatct gtaggagaca gagtcaccat 2820

cacttgccgg gcaagtcaga gcattagcag ctacttaaat tggtatcagc agaaaccagg 2880

gaaagcccct aagctcctga tctatgctgc atccagtttg caaagtgggg tcccatcaag 2940

gttcagtggc agtggatctg ggacagattt cactctcacc atcagcagtc tgcaacctga 3000

agattttgca acttactact gtcaacagag ttacagtacc cctccaacgt tcggccaagg 3060

gaccaagctc gagatcaaac gtactgtggc tgcaccatct gtcttcatct tcccgccatc 3120

tgatgagcag ttgaaatctg gaactgcctc tgttgtgtgc ctgctgaata acttctatcc 3180

cagagaggcc aaagtacagt ggaaggtgga taacgccctc caatcgggta actcccagga 3240

gagtgtcaca gagcaggaca gcaaggacag cacctacagc ctcagcagca ccctgacgct 3300

gagcaaagca gactacgaga aacacaaagt ctacgcctgc gaagtcaccc atcagggcct 3360

gagctcgccc gtcacaaaga gcttcaacag gggagagtgt tagtaaggcg cgccaattct 3420

atttcaagga gacagtcata atgaaatacc tattgcctac ggcagccgct ggattgttat 3480

tactcgcggc ccagccggcc atggcgatgc ctgcttgccg aatatcatgg tggaaaatgg 3540

ccgcttttct ggattcatcg actgtggccg gctgggtgtg gcggaccgct atcaggacat 3600

agcgttggct acccgtgata ttgctgaaga gcttggcggc gaatgggctg accgcttcct 3660

cgtgctttac ggtatcgccg ctcccgattc gcagcgcatc gccttctatc gccttcttga 3720

cgagttcttc tgagcgggac tctggggttc ggtgctacga gatttcgatt ccaccgccgc 3780

cttctatgaa aggttgggct tcggaatcgt tttccgggac gccggctgga tgatcctcca 3840

gcgcggggat ctcatgctgg agttcttcgc ccaccccaac ttgtttattg cagcttataa 3900

tggttacaaa taaagcaata gcatcacaaa tttcacaaat aaagcatttt tttcactgca 3960

ttctagttgt ggtttgtcca aactcatcaa tgtatcttat catgtctgta taccgtcgac 4020

ctctagctag agcttggcgt aatcatggtc atagctgttt cctgtgtgaa attgttatcc 4080

gctcacaatt ccacacaaca tacgagccgg aagcataaag tgtaaagcct ggggtgccta 4140

atgagtgagc taactcacat taattgcgtt gcgctcactg cccgctttcc agtcgggaaa 4200

cctgtcgtgc cagaattgca tgaagaatct gcttagggtt aggcgttttg cgctgcttcg 4260

ctaggtggtc aatattggcc attagccata ttattcattg gttatatagc ataaatcaat 4320

attggctatt ggccattgca tacgttgtat ccatatcata atatgtacat ttatattggc 4380

tcatgtccaa cattaccgcc atgttgacat tgattattga ctagttatta atagtaatca 4440

attacggggt cattagttca tagcccatat atggagttcc gcgttacata acttacggta 4500

aatggcccgc ctggctgacc gcccaacgac ccccgcccat tgacgtcaat aatgacgtat 4560

gttcccatag taacgccaat agggactttc cattgacgtc aatgggtgga gtatttacgg 4620

taaactgccc acttggcagt acatcaagtg tatcatatgc caagtacgcc ccctattgac 4680

gtcaatgacg gtcaccgtct caagcgcctc caccaagggc ccatcggtct tccccctggc 4740

accctcctcc aagagcacct ctgggggcac agcggccctg ggctgcctgg tcaaggacta 4800

cttccccgaa ccggtgacgg tgtcgtggaa ctcaggcgcc ctgaccagcg gcgtccacac 4860

cttcccggct gtcctacagt cctcaggact ctactccctc agcagcgtag tgaccgtgcc 4920

ctccagcagc ttgggcaccc agacctacat ctgcaacgtg aatcacaagc ccagcaacac 4980

caaggtggac aagaaagttg agcccaaatc ttgtgcggcc gcacatcatc atcaccatca 5040

cggggccgca gaacaaaaac tcatctcaga agaggatctg aatggggccg catagactgt 5100

tgaaagttgt ttagcaaaac ctcatacaga aaattcattt actaacgtct ggaaagacga 5160

caaaacttta gatcgttacg ctaactatga gggctgtctg tggaatgcta caggcgttgt 5220

ggtttgtact ggtgacgaaa ctcagtgtta cggtacatgg gttcctattg ggcttgctat 5280

ccctgaaaat gagggtggtg gctctgaggg tggcggttct gagggtggcg gttctgaggg 5340

tggcggtact aaacctcctg agtacggtga tacacctatt ccgggctata cttatatcaa 5400

ccctctcgac ggcacttatc cgcctggtac tgagcaaaac cccgctaatc ctaatccttc 5460

tcttgaggag tctcagcctc ttaatacttt catgtttcag aataataggt tccgaaatag 5520

gcagggtgca ttaactgttt atacgggcac tgttactcaa ggcactgacc ccgttaaaac 5580

ttattaccag tacactcctg tatcatcaaa agccatgtat gacgcttact ggaacggtaa 5640

attcagagac tgcgctttcc attctggctt taatgaggat ccattcgttt gtgaatatca 5700

aggccaatcg tctgacctgc ctcaacctcc tgtcaatgct ggcggcggct ctggtggtgg 5760

ttctggtggc ggctctgagg gtggcggctc tgagggtggc ggctctgagg gtggcggttc 5820

tgagggtggc ggctctgagg gtggcggttc cggtggcggc tccggttccg gtgattttga 5880

ttatgaaaaa atggcaaacg ctaataaggg ggctatgacc gaaaatgccg atgaaaacgc 5940

gctacagtct gacgctaaag gcaaacttga ttctgtcgct actgattacg gtgctgctat 6000

cgatggtttc attggtgacg tttccggcct tgctaatggt aatggtgcta ctggtgattt 6060

tgctggctct aattcccaaa tggctcaagt cggtgacggt gataattcac ctttaatgaa 6120

taatttccgt caatatttac cttctttgcc tcagtcggtt gaatgtcgcc cttatgtctt 6180

tggcgctggt aaaccatatg aattttctat tgattgtgac aaaataaact tattccgtgg 6240

tgtctttgcg tttcttttat atgttgccac ctttatgtat gtattttcga cgtttgctaa 6300

catactgcgt aataaggagt cttaataaga attcactggc cgtcgtttta caacgtcgtg 6360

actgggaaaa ccctggcgtt acccaactta atcgccttgc agcacatccc cctttcgcca 6420

gctggcgtaa tagcgaagag gcccgcaccg atcgcccttc ccaacagttg cgcagcctga 6480

atggcgaatg gcgcctgatg cggtattttc tccttacgca tctgtgcggt atttcacacc 6540

gcatacgtca aagcaaccat agtacgcgcc ctgtagcggc gcattaagcg cggcgggtgt 6600

ggtggttacg cgcagcgtga ccgctacact tgccagcgcc ttagcgcccg ctcctttcgc 6660

tttcttccct tcctttctcg ccacgttcgc cggctttccc cgtcaagctc taaatcgggg 6720

gctcccttta gggttccgat ttagtgcttt acggcacctc gaccccaaaa aa 6772

<210>  99
<211>  10293
<212>  DNA
<213>  Artificial

<220>
<223>  MV1057

<400>  99
   tactcttcct ttttcaatat tattgaagca tttatcaggg ttattgtctc atgagcggat      60
   acatatttga atgtatttag aaaaataaac aaataggggt tccgcgcaca tttccccgaa     120
   aagtgccacc tgacgtcgac ggatcgggag atctcccgat cccctatggt gcactctcag     180
   tacaatctgc tctgatgccg catagttaag ccagtatctg ctccctgctt gtgtgttgga     240
   ggtcgctgag tagtgcgcga gcaaaattta agctacaaca aggcaaggct tgaccgacaa     300
   ttgcatgaag aatctgctta gggttaggcg ttttgcgctg cttcgctagg tggtcaatat     360
   tggccattag ccatattatt cattggttat atagcataaa tcaatattgg ctattggcca     420
   ttgcatacgt tgtatccata tcataatatg tacatttata ttggctcatg tccaacatta     480
   ccgccatgtt gacattgatt attgactagt tattaatagt aatcaattac ggggtcatta     540
   gttcatagcc catatatgga gttccgcgtt acataactta cggtaaatgg cccgcctggc     600
   tgaccgccca acgacccccg cccattgacg tcaataatga cgtatgttcc catagtaacg     660
   ccaataggga ctttccattg acgtcaatgg gtggagtatt tacggtaaac tgcccacttg     720
   gcagtacatc aagtgtatca tatgccaagt acgcccccta ttgacgtcaa tgacggtaaa     780
   tggcccgcct ggcattatgc ccagtacatg accttatggg actttcctac ttggcagtac     840
   atctacgtat tagtcatcgc tattaccatg gtgatgcggt tttggcagta catcaatggg     900
   cgtggatagc ggtttgactc acggggattt ccaagtctcc accccattga cgtcaatggg     960
   agtttgtttt ggcaccaaaa tcaacgggac tttccaaaat gtcgtaacaa ctccgcccca    1020
   ttgacgcaaa tgggcggtag gcgtgtacgg tgggaggtct atataagcag agctcgttta    1080
   gtgaaccgtc agatcgcctg gagacgccat ccacgctgtt ttgacctcca tagaagacac    1140
   cgggaccgat ccagcctccg cggccgggaa cggtgcattg gaagcttggt accggtgaat    1200
   tggccggccc gcgccgtcga ggttatcgat ccgaccgacg cgttcgcgag aggccgcaat    1260
   tccctagcca ccatgggatg gagctgtatc atcctcttct tggtactgct gctggcccag    1320
   ccggccatgg ggcggagaat gggcggaact gggcggagtt aggggcggga tgggcggagt    1380
   taggggcggg actatggttg ctgactaatt gagatgcgga tccgctggca cgacaggttt    1440
   cccgactgga aagcgggcag tgagcgcaac gcaattaatg tgagttagct cactcattag    1500
   gcaccccagg ctttacactt tatgcttccg gctcgtatgt tgtgtggaat tgtgagcgga    1560
   taacaatttc acacaggaaa cagctatgac catgattacg ccaagcttgg gctgcaggtt    1620
   ctttccgcct cagaagccat agagcccacc gcatccccag catgcctgct attgtcttcc 1680
   caatcctccc ccttgctgtc ctgccccacc ccacccccca gaatagaatg acacctactc 1740
   agacaatgcg atgcaatttc ctcattttat taggaaagga cagtgggagt ggcaccttcc 1800
   agggtcaagg aaggcacggg ggaggggcaa acaacagatg gctggcaact agaaggcaca 1860
   gtcgaggctg atcagcgagc tctagatcat cgatgcatgg ggtcgtgcgc tcctttcggt 1920
   cgggcgctgc gggtcgtggg gcgggcgtca ggcaccgggc ttgcgggtca tgcaccaggt 1980
   gcgcggtcct tcgggcacct cgacgtcggc ggtgacggtg aagccgagcc gctcgtagaa 2040
   ggggaggttg cggggcgcgg aggtctccag gaaggcgggc accccggcgc gctcggccgc 2100
   ctccactccg gggagcacga cggcgctgcc cagacccttg ccctggtggt cgggcgagac 2160
   gccgacggtg gccaggaacc acgcgggctc cttgggccgg tgcggcgcca ggaggccttc 2220
   catctgttgc tgcgcggcca gccgggaacc gctcaactcg gccatgcgcg ggccgatctc 2280
   ggcgaacacc gcccccgctt cgacgctctc cggcgtggtc cagaccgcca ccgcggcgcc 2340
   gtcgtccgcg acccacacct tgccgatgtc gagcccgacg cgcgtgagga agagttcttg 2400
   cagctcggtc accgtctcca gtgctagcac caagggccca tcggtcttcc ccctggcacc 2460
   ctcctccaag agcacctctg ggggcacagc ggccctgggc tgcctggtca aggactactt 2520
   ccccgaaccg gtgacggtgt cgtggaactc aggcgccctg accagcggcg tgcacacctt 2580
   cccggctgtc ctacagtcct caggactcta ctccctcagc agcgtcgtga ccgtgccctc 2640
   cagcagcttg ggcacccaga cctacatctg caacgtgaat cacaagccca gcaacaccaa 2700
   ggtggacaag agagttggtg agaggccagc acagggaggg agggtgtctg ctggaagcca 2760
   ggctcagcgc tcctgcctgg acgcatcccg gctatgcagt cccagtccag ggcagcaagg 2820
   caggccccgt ctgcctcttc acccggaggc ctctgcccgc cccactcatg ctcagggaga 2880
   gggtcttctg gctttttccc caggctctgg gcaggcacag gctaggtgcc cctaacccag 2940
   gccctgcaca caaaggggca ggtgctgggc tcagacctgc caagagccat atccgggagg 3000
   accctgcccc tgacctaagc ccaccccaaa ggccaaactc tccactccct cagctcggac 3060
   accttctctc ctcccagatt ccagtaactc ccaatcttct ctctgcagag cccaaatctt 3120
   gtgacaaaac tcacacatgc ccaccgtgcc cagcacctga actcctgggg ggaccgtcag 3180
   tcttcctctt ccccccaaaa cccaaggaca ccctcatgat ctcccggacc cctgaggtca 3240
   catgcgtggt ggtggacgtg agccacgaag accctgaggt caagttcaac tggtacgtgg 3300
   acggcgtgga ggtgcataat gccaagacaa agccgcggga ggagcagtac aacagcacgt 3360
   accgtgtggt cagcgtcctc accgtcctgc accaggactg gctgaatggc aaggagtaca 3420
   agtgcaaggt ctccaacaaa gccctcccag cccccatcga gaaaaccatc tccaaagcca 3480
   aagggcagcc ccgagaacca caggtgtaca ccctgccccc atcccgggag gagatgacca 3540
   agaaccaggt cagcctgacc tgcctggtca aaggcttcta tcccagcgac atcgccgtgg 3600
   agtgggagag caatgggcag ccggagaaca actacaagac cacgcctccc gtgctggact 3660
   ccgacggctc cttcttcctc tatagcaagc tcaccgtgga caagagcagg tggcagcagg 3720
   ggaacgtctt ctcatgctcc gtgatgcatg aggctctgca caaccactac acgcagaaga 3780
   gcctctccct gtctccgggt aaatgagttt aacggatctt aattaatccg agctcggtac 3840
   caagcttaag tttaaaccgc tgatcagcct cgactgtgcc ttctagttgc cagccatctg 3900
   ttgtttgccc ctcccccgtg ccttccttga ccctggaagg tgccactccc actgtccttt 3960
   cctaataaaa tgaggaaatt gcatcgcatt gtctgagtag gtgtcattct attctggggg 4020
   gtggggtggg gcaggacagc aagggggagg attgggaaga caatagcagg catgctgggg 4080
   atgcggtggg ctctatggct tctgaggcgg aaagaaccag ctggggctct agggggtatc 4140
   cccacgcgcc ctgtagcggc gcattaagcg cggcgggtgt ggtggttacg cgcagcgtga 4200
   ccgctacact tgccagcgcc ctagcgcccg tcctttcgct ttcttccctt cctttctcgc 4260
   cacgttcgcc ggctttcccc gtcaagctct aaatcggggg ctccctttag ggttccgatt 4320
   tagtgcttta cggcacctcg accccaaaaa acttgattag ggtgatggtt cacgtagtgg 4380
   gccatcgccc tgatagacgg tttttcgccc tttgacgttg gagtccacgt tctttaatag 4440
   tggactcttg ttccaaactg gaacaacact caaccctatc tcggtctatt cttttgattt 4500
   ataagggatt ttgccgattt cggcctattg gttaaaaaat gagctgattt aacaaaaatt 4560
   taacgcgaat taattctgtg gaatgtgtgt cagttagggt gtggaaagtc cccaggctcc 4620
   ccagcaggca gaagtatgca aagcatgcat ctcaattagt cagcaaccag gtgtggaaag 4680
   tccccaggct ccccagcagg cagaagtatg caaagcatgc atctcaatta gtcagcaacc 4740
   atagtcccgc ccctaactcc gcccatcccg cccctaactc cgcccagttc cgcccattct 4800
   ccgccccatg gctgactaat tttttttatt tatgcagagg ccgaggccgc ctctgcctct 4860
   gagctattcc agaagtagtg aggaggcttt tttggaggcc taggcttttg caaaaagctc 4920
   ccgggagctt ggatatccat tttcggatct gatcaagaga caggatgagg atcgtttcgc 4980
   atgattgaac aagatggatt gcacgcaggt tctccggccg cttgggtgga gaggctattc 5040
   ggctatgact gggcacaaca gacaatcggc tgctctgatg ccgccgtgtt ccggctgtca 5100
   gcgcaggggc gcccggttct ttttgtcaag accgacctgt ccggtgccct gaatgaactg 5160
   caggacgagg cagcgcggct atcgtggctg gccacgacgg gcgttccttg cgcagctgtg 5220
   ctcgacgttg tcactgaagc gggaagggac tggctgctat tgggcgaagt gccggggcag 5280
   gatctcctgt catctcacct tgctcctgcc gagaaagtat ccatcatggc tgatgcaatg 5340
   cggcggctgc atacgcttga tccggctacc tgcccattcg accaccaagc gaaacatcgc 5400
   atcgagcgag cacgtactcg gatggaagcc ggtcttgtcg atcaggatga tctggacgaa 5460
   gagcatcagg ggctcgcgcc agccgaactg ttcgccaggc tcaaggcgcg catgcccgac 5520
   ggcgaggatc tcgtcgtgac ccatggcgat gcctgcttgc cgaatatcat ggtggaaaat 5580
   ggccgctttt ctggattcat cgactgtggc cggctgggtg tggcggaccg ctatcaggac 5640
   atagcgttgg ctacccgtga tattgctgaa gagcttggcg gcgaatgggc tgaccgcttc 5700
   ctcgtgcttt acggtatcgc cgctcccgat tcgcagcgca tcgccttcta tcgccttctt 5760
   gacgagttct tctgagcggg actctggggt tcggtgctac gagatttcga ttccaccgcc 5820
   gccttctatg aaaggttggg cttcggaatc gttttccggg acgccggctg gatgatcctc 5880
   cagcgcgggg atctcatgct ggagttcttc gcccacccca acttgtttat tgcagcttat 5940
   aatggttaca aataaagcaa tagcatcaca aatttcacaa ataaagcatt tttttcactg 6000
   cattctagtt gtggtttgtc caaactcatc aatgtatctt atcatgtctg tataccgtcg 6060
   acctctagct agagcttggc gtaatcatgg tcatagctgt ttcctgtgtg aaattgttat 6120
   ccgctcacaa ttccacacaa catacgagcc ggaagcataa agtgtaaagc ctggggtgcc 6180
   taatgagtga gctaactcac attaattgcg ttgcgctcac tgcccgcttt ccagtcggga 6240
   aacctgtcgt gccagaattg catgaagaat ctgcttaggg ttaggcgttt tgcgctgctt 6300
   cgctaggtgg tcaatattgg ccattagcca tattattcat tggttatata gcataaatca 6360
   atattggcta ttggccattg catacgttgt atccatatca taatatgtac atttatattg 6420
   gctcatgtcc aacattaccg ccatgttgac attgattatt gactagttat taatagtaat 6480
   caattacggg gtcattagtt catagcccat atatggagtt ccgcgttaca taacttacgg 6540
   taaatggccc gcctggctga ccgcccaacg acccccgccc attgacgtca ataatgacgt 6600
   atgttcccat agtaacgcca atagggactt tccattgacg tcaatgggtg gagtatttac 6660
   ggtaaactgc ccacttggca gtacatcaag tgtatcatat gccaagtacg ccccctattg 6720
   acgtcaatga cggtaaatgg cccgcctggc attatgccca gtacatgacc ttatgggact 6780
   ttcctacttg gcagtacatc tacgtattag tcatcgctat taccatggtg atgcggtttt 6840
   ggcagtacat caatgggcgt ggatagcggt ttgactcacg gggatttcca agtctccacc 6900
   ccattgacgt caatgggagt ttgttttggc accaaaatca acgggacttt ccaaaatgtc 6960
   gtaacaactc cgccccattg acgcaaatgg gcggtaggcg tgtacggtgg gaggtctata 7020
   taagcagagc tcgtttagtg aaccgtcaga tcgcctggag acgccatcca cgctgttttg 7080
   acctccatag aagacaccgg gaccgatcca gcctccgcgg ccgggaacgg tgcattggaa 7140
   gcttggtacc ggtgaattag gcgcgccgtc gaggttatcg atccgaccga cgcgttcgcg 7200
   agaggccgca attccctagc caccatggca tgccctggct tcctgtgggc acttgtgatc 7260
   tccacctgtc ttgaattctc catggctgac atccagatga cccagtctcc atcctccctg 7320
   tctgcatctg taggagacag agtcaccatc acttgccggg caagtcagag cattagcagc 7380
   tacttaaatt ggtatcagca gaaaccaggg aaagccccta agctcctgat ctatgctgca 7440
   tccagtttgc aaagtggggt cccatcaagg ttcagtggca gtggatctgg gacagatttc 7500
   actctcacca tcagcagtct gcaacctgaa gattttgcaa cttactactg tcaacagagt 7560
   tacagtaccc ctccaacgtt cggccaaggg accaaggtgg agatcaaacg taagtgcact 7620
   ttgcggccgc taggaagaaa ctcaaaacat caagatttta aatacgcttc ttggtctcct 7680
   tgctataatt atctgggata agcatgctgt tttctgtctg tccctaacat gccctgtgat 7740
   tatccgcaaa caacacaccc aagggcagaa ctttgttact taaacaccat cctgtttgct 7800
   tctttcctca ggaactgtgg ctgcaccatc tgtcttcatc ttcccgccat ctgatgagca 7860
   gttgaaatct ggaactgcct ctgttgtgtg cctgctgaat aacttctatc ccagagaggc 7920
   caaagtacag tggaaggtgg ataacgccct ccaatcgggt aactcccagg agagtgtcac 7980
   agagcaggac agcaaggaca gcacctacag cctcagcagc accctgacgc tgagcaaagc 8040
   agactacgag aaacacaaag tctacgcctg cgaagtcacc catcagggcc tgagctcgcc 8100
   cgtcacaaag agcttcaaca ggggagagtg ttaggtttaa cggatccgag ctcggtacca 8160
   agctcaagtt taaaccgctg atcagcctcg actgtgcctt ctagttgcca gccatctgtt 8220
   gtttgcccct cccccgtgcc ttccttgacc ctggaaggtg ccactcccac tgtcctttcc 8280
   taataaaatg aggaaattgc atcgcattgt ctgagtaggt gtcattctat tctggggggt 8340
   ggggtggggc aggacagcaa gggggaggat tgggaagaca atagcaggca tgctggggat 8400
   gcggtgggct ctatggcttc tgaggcggaa agaaccagct gcattaatga atcggccaac 8460
   gcgcggggag aggcggtttg cgtattgggc gctcttccgc ttcctcgctc actgactcgc 8520
   tgcgctcggt cgttcggctg cggcgagcgg tatcagctca ctcaaaggcg gtaatacggt 8580
   tatccacaga atcaggggat aacgcaggaa agaacatgtg agcaaaaggc cagcaaaagg 8640
   ccaggaaccg taaaaaggcc gcgttgctgg cgtttttcca taggctccgc ccccctgacg 8700
   agcatcacaa aaatcgacgc tcaagtcaga ggtggcgaaa cccgacagga ctataaagat 8760
   accaggcgtt tccccctgga agctccctcg tgcgctctcc tgttccgacc ctgccgctta 8820
   ccggatacct gtccgccttt ctcccttcgg gaagcgtggc gctttctcat agctcacgct 8880
   gtaggtatct cagttcggtg taggtcgttc gctccaagct gggctgtgtg cacgaacccc 8940
   ccgttcagcc cgaccgctgc gccttatccg gtaactatcg tcttgagtcc aacccggtaa 9000
   gacacgactt atcgccactg gcagcagcca ctggtaacag gattagcaga gcgaggtatg 9060
   taggcggtgc tacagagttc ttgaagtggt ggcctaacta cggctacact agaagaacag 9120
   tatttggtat ctgcgctctg ctgaagccag ttaccttcgg aaaaagagtt ggtagctctt 9180
   gatccggcaa acaaaccacc gctggtagcg gtggtttttt tgtttgcaag cagcagatta 9240
   cgcgcagaaa aaaaggatct caagaagatc ctttgatctt ttctacgggg tctgacgctc 9300
   agtggaacga aaactcacgt taagggattt tggtcatgag attatcaaaa aggatcttca 9360
   cctagatcct tttaaattaa aaatgaagtt ttaaatcaat ctaaagtata tatgagtaaa 9420
   cttggtctga cagttaccaa tgcttaatca gtgaggcacc tatctcagcg atctgtctat 9480
   ttcgttcatc catagttgcc tgactccccg tcgtgtagat aactacgata cgggagggct 9540
   taccatctgg ccccagtgct gcaatgatac cgcgagaccc acgctcaccg gctccagatt 9600
   tatcagcaat aaaccagcca gccggaaggg ccgagcgcag aagtggtcct gcaactttat 9660
   ccgcctccat ccagtctatt aattgttgcc gggaagctag agtaagtagt tcgccagtta 9720
   atagtttgcg caacgttgtt gccattgcta caggcatcgt ggtgtcacgc tcgtcgtttg 9780
   gtatggcttc attcagctcc ggttcccaac gatcaaggcg agttacatga tcccccatgt 9840
   tgtgcaaaaa agcggttagc tccttcggtc ctccgatcgt tgtcagaagt aagttggccg 9900
   cagtgttatc actcatggtt atggcagcac tgcataattc tcttactgtc atgccatccg 9960
   taagatgctt ttctgtgact ggtgagtact caaccaagtc attctgagaa tagtgtatgc 10020
   ggcgaccgag ttgctcttgc ccggcgtcaa tacgggataa taccgcgcca catagcagaa 10080
   ctttaaaagt gctcatcatt ggaaaacgtt cttcggggcg aaaactctca aggatcttac 10140
   cgctgttgag atccagttcg atgtaaccca ctcgtgcacc caactgatct tcagcatctt 10200
   ttactttcac cagcgtttct gggtgagcaa aaacaggaag gcaaaatgcc gcaaaaaagg 10260
   gaataagggc gacacggaaa tgttgaatac tca 10293

<210> 100
<211> 8179
<212> DNA
<213> Artificial

<220>
<223> pCAGGS-IgVK1-39 targeting vector

<400> 100
atccaggcgc ggatcaataa aagatcatta ttttcaatag atctgtgtgt tggttttttg 60

tgtgccttgg gggaggggga ggccagaatg aggcgcggcc aagggggagg gggaggccag 120

aatgaccttg gggggagggg aggccagaat gaccttgggg gagggggagg ccagaatgag 180

gcgcggatcc ggagaagttc ctattccgaa gttcctattc ttcaaatagt ataggaactt 240

cgctcgaggg atcggccatt gaacaagatg gattgcacgc aggttctccg gccgcttggg 300

tggagaggct attcggctat gactgggcac aacagacaat cggctgctct gatgccgccg 360

tgttccggct gtcagcgcag gggcgcccgg ttctttttgt caagaccgac ctgtccggtg 420

ccctgaatga actgcaggac gaggcagcgc ggctatcgtg gctggccacg acgggcgttc 480
cttgcgcagc tgtgctcgac gttgtcactg aagcgggaag ggactggctg ctattgggcg 540
aagtgccggg gcaggatctc ctgtcatctc accttgctcc tgccgagaaa gtatccatca 600
tggctgatgc aatgcggcgg ctgcatacgc ttgatccggc tacctgccca ttcgaccacc 660
aagcgaaaca tcgcatcgag cgagcacgta ctcggatgga agccggtctt gtcgatcagg 720
atgatctgga cgaagagcat caggggctcg cgccagccga actgttcgcc aggctcaagg 780
cgcgcatgcc cgacggcgag gatctcgtcg tgacccatgg cgatgcctgc ttgccgaata 840
tcatggtgga aaatggccgc ttttctggat tcatcgactg tggccggctg ggtgtggcgg 900
accgctatca ggacatagcg ttggctaccc gtgatattgc tgaagagctt ggcggcgaat 960
gggctgaccg cttcctcgtg ctttacggta tcgccgctcc cgattcgcag cgcatcgcct 1020
tctatcgcct tcttgacgag ttcttctgag gggatcgatc cgctgtaagt ctgcagaaat 1080
tgatgatcta ttaaacaata aagatgtcca ctaaaatgga agtttttcct gtcatacttt 1140
gttaagaagg gtgagaacag agtacctaca ttttgaatgg aaggattgga gctacggggg 1200
tgggggtggg gtgggattag ataaatgcct gctctttact gaaggctctt tactattgct 1260
ttatgataat gtttcatagt tggatatcat aatttaaaca agcaaaacca aattaagggc 1320
cagctcattc ctcccactca tgatctatag atctatagat ctctcgtggg atcattgttt 1380
ttctcttgat tcccactttg tggttctaag tactgtggtt tccaaatgtg tcagtttcat 1440
agcctgaaga acgagatcag cagcctctgt tccacataca cttcattctc agtattgttt 1500
tgccaagttc taattccatc agaagctgac tctagatggc gcgtatgcag gttttcgaca 1560
ttgattattg actagttatt aatagtaatc aattacgggg tcattagttc atagcccata 1620
tatggagttc cgcgttacat aacttacggt aaatggcccg cctggctgac cgcccaacga 1680
cccccgccca ttgacgtcaa taatgacgta tgttcccata gtaacgccaa tagggacttt 1740
ccattgacgt caatgggtgg agtatttacg gtaaactgcc cacttggcag tacatcaagt 1800
gtatcatatg ccaagtacgc cccctattga cgtcaatgac ggtaaatggc ccgcctggca 1860
ttatgcccag tacatgacct tatgggactt tcctacttgg cagtacatct acgtattagt 1920
catcgctatt accatggtcg aggtgagccc cacgttctgc ttcactctcc ccatctcccc 1980
cccctcccca cccccaattt tgtatttatt tattttttaa ttattttgtg cagcgatggg 2040
ggcggggggg gggggggcgc gcgccaggcg gggcggggcg gggcgagggg cggggcgggg 2100
cgaggcggag aggtgcggcg gcagccaatc agagcggcgc gctccgaaag tttcctttta 2160
tggcgaggcg gcggcggcgg cggccctata aaaagcgaag cgcgcggcgg gcgggagtcg 2220
ctgcgttgcc ttcgccccgt gccccgctcc gcgccgcctc gcgccgcccg ccccggctct 2280
gactgaccgc gttactccca caggtgagcg ggcgggacgg cccttctcct ccgggctgta 2340

attagcgctt ggtttaatga cggctcgttt cttttctgtg gctgcgtgaa agccttaaag 2400

ggctccggga gggccctttg tgcggggggg agcggctcgg ggggtgcgtg cgtgtgtgtg 2460

tgcgtgggga gcgccgcgtg cggcccgcgc tgcccggcgg ctgtgagcgc tgcgggcgcg 2520

gcgcggggct ttgtgcgctc cgcgtgtgcg cgaggggagc gcggccgggg gcggtgcccc 2580

gcggtgcggg ggggctgcga ggggaacaaa ggctgcgtgc ggggtgtgtg cgtggggggg 2640

tgagcagggg gtgtgggcgc ggcggtcggg ctgtaacccc cccctgcacc cccctccccg 2700

agttgctgag cacggcccgg cttcgggtgc ggggctccgt gcggggcgtg gcgcggggct 2760

cgccgtgccg ggcggggggt ggcggcaggt gggggtgccg ggcggggcgg ggccgcctcg 2820

ggccggggag ggctcggggg aggggcgcgg cggccccgga gcgccggcgg ctgtcgaggc 2880

gcggcgagcc gcagccattg ccttttatgg taatcgtgcg agagggcgca gggacttcct 2940

ttgtcccaaa tctgtgcgga gccgaaatct gggaggcgcc gccgcacccc ctctagcggg 3000

cgcggggcga agcggtgcgg cgccggcagg aaggaaatgg gcggggaggg ccttcgtgcg 3060

tcgccgcgcc gccgtcccct tctccctctc cagcctcggg gctgtccgcg gggggacggc 3120

tgccttcggg ggggacgggg cagggcgggg ttcggcttct ggcgtgtgac cggcggctct 3180

agaagcgttg gggtgagtac tccctctcaa aagcgggcat gacttctgcg ctaagattgt 3240

cagtttccaa aaacgaggag gatttgatat tcacctggcc cgcggtgatg cctttgaggg 3300

tggccgcgtc catctggtca gaaaagacaa tctttttgtt gtcaagcttg aggtgtggca 3360

ggcttgagat ctggccatac acttgagtga cattgacatc cactttgcct ttctctccac 3420

aggtgtccac tcccagggcg gcctccggag cgatcgccga tccgcctagg caattgttta 3480

aatcggccgg ccataacttc gtataatgta tgctatacga agttatggat cctcacagta 3540

ggtggcatcg ttcctttctg actgcccgcc ccccgcatgc cgtcccgcga tattgagctc 3600

cgaacctctc gccctgccgc cgccggtgct ccgtcgccgc cgcgccgcca tggaatcgaa 3660

gccaccatgg atcttaccgg aaaactcgac gcaagaaaaa tcagagagat cctcataaag 3720

gtcaagaagg gcggaaagat cgccgtgtaa ttctagaccg gttcgagatc caggcgcgga 3780

tcaataaaag atcattattt tcaatagatc tgtgtgttgg ttttttgtgt gccttggggg 3840

agggggaggc cagaatgagg cgcggccaag ggggaggggg aggccagaat gaccttgggg 3900

gagggggagg ccagaatgac cttgggggag ggggaggcca gaatgaggcg cgccctccgt 3960

cgacctataa cttcgtataa tgtatgctat acgaagttat ggcggccgcc accatggaca 4020

tgagagtgcc cgcccagctc ctggggctcc tgctactctg gctccgaggt aaggatggag 4080

aacactagga atttactcag ccagtgtgct cagtactgac tggaacttca gggaagttct 4140

ctgataacat gattaatagt aagaatattt gtttttatgt ttccaatctc aggtgccaga 4200

tgtgacatcc agatgaccca gagccccagc agcctgagcg ccagcgtggg cgacagagtg 4260

accatcacct gcagagccag ccagagcatc agcagctacc tgaactggta tcagcagaag 4320

cccggcaagg cccccaagct gctgatctac gccgccagct ccctgcagag cggcgtgccc 4380

agcagattca gcggcagcgg ctccggcacc gacttcaccc tgaccatcag cagcctgcag 4440

cccgaggact tcgccaccta ctactgccag cagagctaca gcaccccccc caccttcggc 4500

cagggcacca aggtggagat caagagagcc gacgccgctc ccaccgtgtc catcttcccc 4560

cccagcatgg aacagctgac ctctggcgga gccaccgtgg tctgcttcgt gaacaacttc 4620

taccccagag acatcagcgt gaagtggaag atcgacggca gcgagcagag ggacggcgtg 4680

ctggacagcg tgaccgacca ggacagcaag gactccacct acagcatgag cagcaccctg 4740

agcctgacca aggtggagta cgagaggcac aacctgtaca cctgcgaggt ggtgcacaag 4800

accagctcca gccccgtggt caagtccttc aaccggaacg agtgttgagc tagcttaaga 4860

tttaaatagg ccggccgcgt cgacctcgag atccaggcgc ggatcaataa aagatcatta 4920

ttttcaatag atctgtgtgt tggttttttg tgtgccttgg gggaggggga ggccagaatg 4980

aggcgcggcc aagggggagg gggaggccag aatgaccttg ggggaggggg aggccagaat 5040

gaccttgggg gagggggagg ccagaatgag gcgcgccccc gggtaccgag ctcgaattag 5100

tggatcctca cagtaggtgg catcgttcct ttctgactgc ccgccccccg catgccgtcc 5160

cgcgatattg agctccgaac ctctcgccct gccgccgccg gtgctccgtc gccgccgcgc 5220

cgccatggaa tcgcgccggt aaccgaagtt cctatacttt ctagagaata ggaacttcgg 5280

aataggaact tcaagccggt acccagcttt tgttcccttt agtgagggtt aatttcgagc 5340

ttggcgtaat catggtcata gctgtttcct gtgtgaaatt gttatccgct cacaattcca 5400

cacaacatac gagccggaag cataaagtgt aaagcctggg gtgcctaatg agtgagctaa 5460

ctcacattaa ttgcgttgcg ctcactgccc gctttccagt cgggaaacct gtcgtgccag 5520

ctgcattaat gaatcggcca acgcgcgggg agaggcggtt tgcgtattgg gcgctcttcc 5580

gcttcctcgc tcactgactc gctgcgctcg gtcgttcggc tgcggcgagc ggtatcagct 5640

cactcaaagg cggtaatacg gttatccaca gaatcagggg ataacgcagg aaagaacatg 5700

tgagcaaaag gccagcaaaa ggccaggaac cgtaaaaagg ccgcgttgct ggcgtttttc 5760

cataggctcc gcccccctga cgagcatcac aaaaatcgac gctcaagtca gaggtggcga 5820

aacccgacag gactataaag ataccaggcg tttccccctg gaagctccct cgtgcgctct 5880

cctgttccga ccctgccgct taccggatac ctgtccgcct ttctcccttc gggaagcgtg 5940

gcgctttctc atagctcacg ctgtaggtat ctcagttcgg tgtaggtcgt tcgctccaag 6000

ctgggctgtg tgcacgaacc ccccgttcag cccgaccgct gcgccttatc cggtaactat 6060

cgtcttgagt ccaacccggt aagacacgac ttatcgccac tggcagcagc cactggtaac 6120

aggattagca gagcgaggta tgtaggcggt gctacagagt tcttgaagtg gtggcctaac 6180

tacggctaca ctagaaggac agtatttggt atctgcgctc tgctgaagcc agttaccttc 6240

ggaaaaagag ttggtagctc ttgatccggc aaacaaacca ccgctggtag cggtggtttt 6300

tttgtttgca agcagcagat tacgcgcaga aaaaaaggat ctcaagaaga tcctttgatc 6360

ttttctacgg ggtctgacgc tcagtggaac gaaaactcac gttaagggat tttggtcatg 6420

agattatcaa aaaggatctt cacctagatc cttttaaatt aaaaatgaag ttttaaatca 6480

atctaaagta tatatgagta aacttggtct gacagttacc aatgcttaat cagtgaggca 6540

cctatctcag cgatctgtct atttcgttca tccatagttg cctgactccc cgtcgtgtag 6600

ataactacga tacgggaggg cttaccatct ggccccagtg ctgcaatgat accgcgagac 6660

ccacgctcac cggctccaga tttatcagca ataaaccagc cagccggaag ggccgagcgc 6720

agaagtggtc ctgcaacttt atccgcctcc atccagtcta ttaattgttg ccgggaagct 6780

agagtaagta gttcgccagt taatagtttg cgcaacgttg ttgccattgc tacaggcatc 6840

gtggtgtcac gctcgtcgtt tggtatggct tcattcagct ccggttccca acgatcaagg 6900

cgagttacat gatcccccat gttgtgcaaa aaagcggtta gctccttcgg tcctccgatc 6960

gttgtcagaa gtaagttggc cgcagtgtta tcactcatgg ttatggcagc actgcataat 7020

tctcttactg tcatgccatc cgtaagatgc ttttctgtga ctggtgagta ctcaaccaag 7080

tcattctgag aatagtgtat gcggcgaccg agttgctctt gcccggcgtc aatacgggat 7140

aataccgcgc cacatagcag aactttaaaa gtgctcatca ttggaaaacg ttcttcgggg 7200

cgaaaactct caaggatctt accgctgttg agatccagtt cgatgtaacc cactcgtgca 7260

cccaactgat cttcagcatc ttttactttc accagcgttt ctgggtgagc aaaaacagga 7320

aggcaaaatg ccgcaaaaaa gggaataagg gcgacacgga aatgttgaat actcatactc 7380

ttcctttttc aatattattg aagcatttat cagggttatt gtctcatgag cggatacata 7440

tttgaatgta tttagaaaaa taaacaaata ggggttccgc gcacatttcc ccgaaaagtg 7500

ccacctaaat tgtaagcgtt aatattttgt taaaattcgc gttaaatttt tgttaaatca 7560

gctcattttt taaccaatag gccgaaatcg gcaaaatccc ttataaatca aaagaataga 7620

ccgagatagg gttgagtgtt gttccagttt ggaacaagag tccactatta aagaacgtgg 7680

actccaacgt caaagggcga aaaaccgtct atcagggcga tggcccacta cgtgaaccat 7740

caccctaatc aagttttttg gggtcgaggt gccgtaaagc actaaatcgg aaccctaaag 7800

ggagcccccg atttagagct tgacggggaa agccggcgaa cgtggcgaga aaggaaggga 7860

agaaagcgaa aggagcgggc gctagggcgc tggcaagtgt agcggtcacg ctgcgcgtaa 7920

ccaccacacc cgccgcgctt aatgcgccgc tacagggcgc gtcccattcg ccattcaggc 7980

tgcgcaactg ttgggaaggg cgatcggtgc gggcctcttc gctattacgc cagctggcga 8040

aagggggatg tgctgcaagg cgattaagtt gggtaacgcc agggttttcc cagtcacgac 8100

gttgtaaaac gacggccagt gagcgcgcgt aatacgactc actatagggc gaattggggg 8160

taactaagta aggatcgag 8179

<210> 101
<211> 8188
<212> DNA
<213> pCAGGS-IgVL2-14 targeting vector

<400> 101
atccaggcgc ggatcaataa aagatcatta ttttcaatag atctgtgtgt tggttttttg 60

tgtgccttgg gggaggggga ggccagaatg aggcgcggcc aagggggagg gggaggccag 120

aatgaccttg gggggagggg aggccagaat gaccttgggg gagggggagg ccagaatgag 180

gcgcggatcc ggagaagttc ctattccgaa gttcctattc ttcaaatagt ataggaactt 240

cgctcgaggg atcggccatt gaacaagatg gattgcacgc aggttctccg gccgcttggg 300

tggagaggct attcggctat gactgggcac aacagacaat cggctgctct gatgccgccg 360

tgttccggct gtcagcgcag gggcgcccgg ttctttttgt caagaccgac ctgtccggtg 420

ccctgaatga actgcaggac gaggcagcgc ggctatcgtg gctggccacg acgggcgttc 480

cttgcgcagc tgtgctcgac gttgtcactg aagcgggaag ggactggctg ctattgggcg 540

aagtgccggg gcaggatctc ctgtcatctc accttgctcc tgccgagaaa gtatccatca 600

tggctgatgc aatgcggcgg ctgcatacgc ttgatccggc tacctgccca ttcgaccacc 660

aagcgaaaca tcgcatcgag cgagcacgta ctcggatgga agccggtctt gtcgatcagg 720

atgatctgga cgaagagcat caggggctcg cgccagccga actgttcgcc aggctcaagg 780

cgcgcatgcc cgacggcgag gatctcgtcg tgacccatgg cgatgcctgc ttgccgaata 840

tcatggtgga aaatggccgc ttttctggat tcatcgactg tggccggctg ggtgtggcgg 900

accgctatca ggacatagcg ttggctaccc gtgatattgc tgaagagctt ggcggcgaat 960

gggctgaccg cttcctcgtg ctttacggta tcgccgctcc cgattcgcag cgcatcgcct 1020

tctatcgcct tcttgacgag ttcttctgag gggatcgatc cgctgtaagt ctgcagaaat 1080

tgatgatcta ttaaacaata aagatgtcca ctaaaatgga agtttttcct gtcatacttt 1140

gttaagaagg gtgagaacag agtacctaca ttttgaatgg aaggattgga gctacggggg 1200

tgggggtggg gtgggattag ataaatgcct gctctttact gaaggctctt tactattgct 1260

ttatgataat gtttcatagt tggatatcat aatttaaaca agcaaaacca aattaagggc 1320

cagctcattc ctcccactca tgatctatag atctatagat ctctcgtggg atcattgttt 1380

ttctcttgat tcccactttg tggttctaag tactgtggtt tccaaatgtg tcagtttcat 1440

agcctgaaga acgagatcag cagcctctgt tccacataca cttcattctc agtattgttt 1500

tgccaagttc taattccatc agaagctgac tctagatggc gcgtatgcag gttttcgaca 1560

ttgattattg actagttatt aatagtaatc aattacgggg tcattagttc atagcccata 1620

tatggagttc cgcgttacat aacttacggt aaatggcccg cctggctgac cgcccaacga 1680

cccccgccca ttgacgtcaa taatgacgta tgttcccata gtaacgccaa tagggacttt 1740

ccattgacgt caatgggtgg agtatttacg gtaaactgcc cacttggcag tacatcaagt 1800

gtatcatatg ccaagtacgc cccctattga cgtcaatgac ggtaaatggc ccgcctggca 1860

ttatgcccag tacatgacct tatgggactt tcctacttgg cagtacatct acgtattagt 1920

catcgctatt accatggtcg aggtgagccc cacgttctgc ttcactctcc ccatctcccc 1980

cccctcccca cccccaattt tgtatttatt tattttttaa ttattttgtg cagcgatggg 2040

ggcggggggg gggggggcgc gcgccaggcg gggcggggcg gggcgagggg cggggcgggg 2100

cgaggcggag aggtgcggcg gcagccaatc agagcggcgc gctccgaaag tttcctttta 2160

tggcgaggcg gcggcggcgg cggccctata aaaagcgaag cgcgcggcgg gcgggagtcg 2220

ctgcgttgcc ttcgccccgt gccccgctcc gcgccgcctc gcgccgcccg ccccggctct 2280

gactgaccgc gttactccca caggtgagcg ggcgggacgg cccttctcct ccgggctgta 2340

attagcgctt ggtttaatga cggctcgttt cttttctgtg gctgcgtgaa agccttaaag 2400

ggctccggga gggccctttg tgcggggggg agcggctcgg ggggtgcgtg cgtgtgtgtg 2460

tgcgtgggga gcgccgcgtg cggcccgcgc tgcccggcgg ctgtgagcgc tgcgggcgcg 2520

gcgcggggct ttgtgcgctc cgcgtgtgcg cgaggggagc gcggccgggg gcggtgcccc 2580

gcggtgcggg ggggctgcga ggggaacaaa ggctgcgtgc ggggtgtgtg cgtggggggg 2640

tgagcagggg gtgtgggcgc ggcggtcggg ctgtaacccc cccctgcacc cccctccccg 2700

agttgctgag cacggcccgg cttcgggtgc ggggctccgt gcggggcgtg gcgcggggct 2760

cgccgtgccg ggcggggggt ggcggcaggt gggggtgccg ggcggggcgg ggccgcctcg 2820

ggccggggag ggctcggggg aggggcgcgg cggccccgga gcgccggcgg ctgtcgaggc 2880

gcggcgagcc gcagccattg ccttttatgg taatcgtgcg agagggcgca gggacttcct 2940

ttgtcccaaa tctgtgcgga gccgaaatct gggaggcgcc gccgcacccc ctctagcggg 3000

cgcggggcga agcggtgcgg cgccggcagg aaggaaatgg gcggggaggg ccttcgtgcg 3060

tcgccgcgcc gccgtcccct tctccctctc cagcctcggg gctgtccgcg gggggacggc 3120

tgccttcggg ggggacgggg caggcggggg ttcggcttct ggcgtgtgac cggcggctct 3180

agaagcgttg gggtgagtac tccctctcaa aagcgggcat gacttctgcg ctaagattgt 3240

cagtttccaa aaacgaggag gatttgatat tcacctggcc cgcggtgatg cctttgaggg 3300

tggccgcgtc catctggtca gaaaagacaa tctttttgtt gtcaagcttg aggtgtggca 3360

ggcttgagat ctggccatac acttgagtga cattgacatc cactttgcct ttctctccac 3420

aggtgtccac tcccagggcg gcctccggag cgatcgccga tccgcctagg caattgttta 3480

aatcggccgg ccataacttc gtataatgta tgctatacga agttatggat cctcacagta 3540

ggtggcatcg ttcctttctg actgcccgcc ccccgcatgc cgtcccgcga tattgagctc 3600

cgaacctctc gccctgccgc cgccggtgct ccgtcgccgc cgcgccgcca tggaatcgaa 3660

gccaccatgg atcttaccgg aaaactcgac gcaagaaaaa tcagagagat cctcataaag 3720

gtcaagaagg gcggaaagat cgccgtgtaa ttctagaccg gttcgagatc caggcgcgga 3780

tcaataaaag atcattattt tcaatagatc tgtgtgttgg ttttttgtgt gccttggggg 3840

agggggaggc cagaatgagg cgcggccaag gggagggggg aggccagaat gaccttgggg 3900

gagggggagg ccagaatgac cttgggggag ggggaggcca gaatgaggcg cgccctccgt 3960

cgacctataa cttcgtataa tgtatgctat acgaagttat ggcggccgcc accatggaca 4020

tgagagtgcc cgcccagctc ctggggctcc tgctactctg gctccgaggt aaggatggag 4080

aacactagga atttactcag ccagtgtgct cagtactgac tggaacttca gggaagttct 4140

ctgataacat gattaatagt aagaatattt gtttttatgt ttccaatctc aggtgccaga 4200

tgtcagtctg ccctgaccca gcccgcctct gtgtctggca gccctggcca gagcatcacc 4260

atcagctgca ccggcaccag cagcgacgtg ggcggctaca actacgtgtc ctggtatcag 4320

cagcaccccg gcaaggcccc caagctgatg atctacgagg tgtccaacag acccagcggc 4380

gtgagcaaca gattcagcgg cagcaagagc ggcaacaccg ccagcctgac catcagcggc 4440

ctccaggctg aggacgaggc cgactactac tgcagcagct acaccagcag ctccaccctg 4500

gtgtttggcg gcggaacaaa gctgaccgtg ctgagagccg acgccgctcc caccgtgtcc 4560

atcttccccc ccagcatgga acagctgacc tctggcggag ccaccgtggt ctgcttcgtg 4620

aacaacttct accccagaga catcagcgtg aagtggaaga tcgacggcag cgagcagagg 4680

gacggcgtgc tggacagcgt gaccgaccag gacagcaagg actccaccta cagcatgagc 4740

agcaccctga gcctgaccaa ggtggagtac gagaggcaca acctgtacac ctgcgaggtg 4800

gtgcacaaga ccagctccag ccccgtggtc aagtccttca accggaacga gtgttgagct 4860

agcttaagat ttaaataggc cggccgcgtc gacctcgaga tccaggcgcg gatcaataaa 4920

agatcattat tttcaataga tctgtgtgtt ggttttttgt gtgccttggg ggagggggag 4980

gccagaatga ggcgcggcca aggggagggg ggaggccaga atgaccttgg gggaggggga 5040

ggccagaatg accttggggg agggggaggc cagaatgagg cgcgcccccg ggtaccgagc 5100

tcgaattagt ggatcctcac agtaggtggc atcgttcctt tctgactgcc cgccccccgc 5160

atgccgtccc gcgatattga gctccgaacc tctcgccctg ccgccgccgg tgctccgtcg 5220

ccgccgcgcc gccatggaat cgcgccggta accgaagttc ctatactttc tagagaatag 5280

gaacttcgga ataggaactt caagccggta cccagctttt gttcccttta gtgagggtta 5340

atttcgagct tggcgtaatc atggtcatag ctgtttcctg tgtgaaattg ttatccgctc 5400

acaattccac acaacatacg agccgggagc ataaagtgta aagcctgggg tgcctaatga 5460

gtgagctaac tcacattaat tgcgttgcgc tcactgcccg ctttccagtc gggaaacctg 5520

tcgtgccagc tgcattaatg aatcggccaa cgcgcgggga gaggcggttt gcgtattggg 5580

cgctcttccg cttcctcgct cactgactcg ctgcgctcgg tcgttcggct gcggcgagcg 5640

gtatcagctc actcaaaggc ggtaatacgg ttatccacag aatcagggga taacgcagga 5700

aagaacatgt gagcaaaagg ccagcaaaag gccaggaacc gtaaaaaggc cgcgttgctg 5760

gcgtttttcc ataggctccg cccccctgac gagcatcaca aaaatcgacg ctcaagtcag 5820

aggtggcgaa acccgacagg actataaaga taccaggcgt ttccccctgg aagctccctc 5880

gtgcgctctc ctgttccgac cctgccgctt accggatacc tgtccgcctt tctcccttcg 5940

ggaagcgtgg cgctttctca tagctcacgc tgtaggtatc tcagttcggt gtaggtcgtt 6000

cgctccaagc tgggctgtgt gcacgaaccc cccgttcagc ccgaccgctg cgccttatcc 6060

ggtaactatc gtcttgagtc caacccggta agacacgact tatcgccact ggcagcagcc 6120

actggtaaca ggattagcag agcgaggtat gtaggcggtg ctacagagtt cttgaagtgg 6180

tggcctaact acggctacac tagaaggaca gtatttggta tctgcgctct gctgaagcca 6240

gttaccttcg gaaaaagagt tggtagctct tgatccggca aacaaaccac cgctggtagc 6300

ggtggttttt ttgtttgcaa gcagcagatt acgcgcagaa aaaaaggatc tcaagaagat 6360

cctttgatct tttctacggg gtctgacgct cagtggaacg aaaactcacg ttaagggatt 6420

ttggtcatga gattatcaaa aaggatcttc acctagatcc ttttaaatta aaaatgaagt 6480

tttaaatcaa tctaaagtat atatgagtaa acttggtctg acagttacca atgcttaatc 6540

agtgaggcac ctatctcagc gatctgtcta tttcgttcat ccatagttgc ctgactcccc 6600

gtcgtgtaga taactacgat acgggagggc ttaccatctg gccccagtgc tgcaatgata 6660

ccgcgagacc cacgctcacc ggctccagat ttatcagcaa taaaccagcc agccggaagg 6720

gccgagcgca gaagtggtcc tgcaacttta tccgcctcca tccagtctat taattgttgc 6780

cgggaagcta gagtaagtag ttcgccagtt aatagtttgc gcaacgttgt tgccattgct 6840

acaggcatcg tggtgtcacg ctcgtcgttt ggtatggctt cattcagctc cggttcccaa 6900

cgatcaaggc gagttacatg atcccccatg ttgtgcaaaa aagcggttag ctccttcggt 6960

cctccgatcg ttgtcagaag taagttggcc gcagtgttat cactcatggt tatggcagca 7020

ctgcataatt ctcttactgt catgccatcc gtaagatgct tttctgtgac tggtgagtac 7080

tcaaccaagt cattctgaga atagtgtatg cggcgaccga gttgctcttg cccggcgtca 7140

atacgggata ataccgcgcc acatagcaga actttaaaag tgctcatcat tggaaaacgt 7200

tcttcggggc gaaaactctc aaggatctta ccgctgttga gatccagttc gatgtaaccc 7260

actcgtgcac ccaactgatc ttcagcatct tttactttca ccagcgtttc tgggtgagca 7320

aaaacaggaa ggcaaaatgc cgcaaaaaag ggaataaggg cgacacggaa atgttgaata 7380

ctcatactct tcctttttca atattattga agcatttatc agggttattg tctcatgagc 7440

ggatacatat ttgaatgtat ttagaaaaat aaacaaatag gggttccgcg cacatttccc 7500

cgaaaagtgc cacctaaatt gtaagcgtta atattttgtt aaaattcgcg ttaaattttt 7560

gttaaatcag ctcatttttt aaccaatagg ccgaaatcgg caaaatccct tataaatcaa 7620

aagaatagac cgagataggg ttgagtgttg ttccagtttg gaacaagagt ccactattaa 7680

agaacgtgga ctccaacgtc aaagggcgaa aaaccgtcta tcagggcgat ggcccactac 7740

gtgaaccatc accctaatca agttttttgg ggtcgaggtg ccgtaaagca ctaaatcgga 7800

accctaaagg gagcccccga tttagagctt gacggggaaa gccggcgaac gtggcgagaa 7860

aggaagggaa gaaagcgaaa ggagcgggcg ctagggcgct ggcaagtgta gcggtcacgc 7920

tgcgcgtaac caccacaccc gccgcgctta atgcgccgct acagggcgcg tcccattcgc 7980

cattcaggct gcgcaactgt tgggaagggc gatcggtgcg ggcctcttcg ctattacgcc 8040

agctggcgaa agggggatgt gctgcaaggc gattaagttg ggtaacgcca gggttttccc 8100

agtcacgacg ttgtaaaacg acggccagtg agcgcgcgta atacgactca ctatagggcg 8160

aattggggt aactaagtaa ggatcgag 8188

2

74

## Description

### TECHNICAL FIELD

The invention relates to the production and use of non-human animals capable of producing antibodies or derivatives thereof, which are expressed from at least partially exogenous nucleic acids (transgenes). Transgenes to produce such transgenic animals and methods to produce such heterologous antibodies; methods and vectors for producing such transgenic animals are disclosed.

### BACKGROUND OF THE INVENTION

B cells mediate humoral immunity by producing specific antibodies. The basic structural subunit of an antibody (Ab) is an immunoglobulin (Ig) molecule. Ig molecules consist of a complex of two identical heavy (H) and two identical light (L) polypeptide chains. At the amino terminus of each H chain and L chain is a region that varies in amino acid sequence named the variable (V) region. The remaining portion of the H and L chains is relatively constant in amino acid sequence and is named the constant (C) region. In an Ig molecule, the H and L chain V regions (VH and VL) are juxtaposed to form the potential antigen-binding site. The genes that encode H and L chain V regions are assembled somatically from segments of germline DNA during precursor B (pre-B) cell differentiation: V, D and J gene segments for the H chain and V and J gene segments for the L chain. Within Ig V regions are three regions of greatest amino acid sequence variability that interact to form the antigen-recognition site and are thus referred to as complementarity determining regions (CDRs).

The V gene segment encodes the bulk of the V region domain, including CDR1 and CDR2. Diversity in CDR1 and CDR2 derives from sequence heterogeneity among multiple different germline-encoded V segments. CDR3 is encoded by sequences that are formed by the joining of H chain V, D, and J gene segments and L chain V and J segments and by mechanisms that create nucleotide sequence heterogeneity where these segments are combined. Additional diversity may be derived from pairing of different H and L chain V regions. Collectively these processes yield a primary repertoire of antibodies encoded by germline gene segments and expressed by newly-formed B cells.

An additional source of antibody diversity is imposed on top of the diversity generated by recombination of Ig gene segments. B cells are able to introduce mutations into the antibody V regions that they express, a process called somatic hypermutation. Thus, when an animal first encounters an antigen, the antigen binds to a specific B cell which happens to carry antibodies which have a V domain which binds the antigen. This primary response may activate this B cell to go on to secrete the cognate antibody. These activated B cells can also now target a somatic mutation process to their rearranged antibody gene segments and thus allow the production of daughter cells which make variants of the antibodies of the primary response, A selection process amplifies those variant B cell descendants which make an antibody of improved affinity of the antigen. In B cells, somatic hypermutations are targeted to a restricted genomic region including both the rearranged VH and VL genes. Thus somatic mutation allows affinity maturation - the production and selection of high affinity antibodies. Therefore, somatic mutation is important for the generation of high affinity antibodies.

The exquisite specificity and high affinity of antibodies and the discovery of hybridoma technology allowing the generation of monoclonal antibodies (mAbs) has generated great expectations for their utilization as targeted therapeutics for human diseases. MAbs are identical because they are produced by a single B cell and its progeny. MAbs are made by fusing the spleen cells from a mouse that has been immunized with the desired antigen with myeloma cells to generate immortalized hybridomas. One of the major impediments facing the development of in vivo applications for mAbs in humans is the intrinsic immunogenicity of non-human Igs. Patients respond to therapeutic doses of mouse mAbs by making antibodies against the mouse Ig sequences (Human Anti Mouse Antibodies; HAMA), causing acute toxicity, alter their biodistribution and accelerate clearance, thus reducing the efficacy of subsequent administrations (Mirick, et al., (2004) Q. Nucl. Med. Mol. Imaging 48, 251-257).

To circumvent the generation of HAMA, antibody humanization methods have been developed in an attempt to produce mAbs with decreased immunogenicity when applied to humans. These endeavors have yielded various recombinant DNA-based approaches aimed at increasing the content of human amino acid sequences in mAbs while retaining the specificity and affinity of the parental non-human antibody. Humanization began with the construction of mouse-human chimeric mAbs (Morrison, S. L., et al., (1984). Proc. Natl. Acad. Sci. USA., 81, 6851-5), in which the Ig C regions in murine mAbs were replaced by human C regions. Chimeric mAbs contain 60-70% of human amino acid sequences and are considerably less immunogenic than their murine counterparts when injected into humans, albeit that a human anti-chimeric antibody response was still observed (Hwang, W. Y., et al. (2005). Methods, 36, 3-10).

In attempts to further humanize murine mAbs, CDR grafting was developed. In CDR grafting, murine antibodies are humanized by grafting their CDRs onto the VL and VH frameworks of human Ig molecules, while retaining those murine framework residues deemed essential for specificity and affinity (Jones, P.T., et al., (1986). Nature, 321, 522). Overall, CDR-grafted antibodies consist of more than 80% human amino acid sequences (Queen, C. et al. (1989) Proc. Natl. Acad. Sci. U. S. A. 86, 10029; Carter, P. et al. (1992) Proc. Natl. Acad. Sci. U. S. A. 89, 4285). Despite these efforts, CDR-grafted, humanized antibodies were shown to still evoke an antibody response against the grafted V region (Hwang, W Y., et al. (2005). Methods, 36, 3).

Subsequently to CDR grafting, humanization methods based on different paradigms such as resurfacing (Padlan, E. A., et al., (1991). Mol. Immunol., 28, 489), superhumanization (Tan, P., D. A., et al., (2002) J. Immunol., 169, 1119), human string content optimization (Lazar, G. A., et al., (2007). Mol. Immunol., 44, 1986) and humaneering have been developed in an attempt to further decrease the content of non-human sequences in therapeutic mAbs (Almagro, J. C., et al., (2008). Frontiers in Bioscience 13, 1619). As in CDR grafting approaches, these methods rely on analyses of the antibody structure and sequence comparison of the non-human and human mAbs in order to evaluate the impact of the humanization process into immunogenicity of the final product. When comparing the immunogenicity of chimeric and humanized antibodies, humanization of variable regions appears to decrease immunogenicity further (Hwang, W. Y., et al. (2005). Methods, 36, 3-10).

De-immunization is another approach developed to reduce the immunogenicity of chimeric or mouse antibodies. It involves the identification of linear T-cell epitopes in the antibody of interest, using bioinformatics, and their subsequent replacement by site-directed mutagenesis to human or non-immunogenic sequences (WO09852976A1). Although de-immunized antibodies exhibited reduced immunogenicity in primates, compared with their chimeric counterparts, some loss of binding affinity was observed (Jain, M., et al., (2007). Trends in Biotechnol. 25, 307).

The development of phage display technology complemented and extended humanization approaches in attempts to obtain less immunogenic mAbs for therapy in humans. In phage display, large collections ('libraries') of human antibody VH and VL regions are expressed on the surface of filamentous bacteriophage particles. From these libraries, rare phages are selected through binding interaction with antigen; soluble antibody fragments are expressed from infected bacteria and the affinity of binding of selected antibodies is improved by mutation (Winter, G., et al. (1994). Annu. Rev. Immunol. 12, 433). The process mimics immune selection, and antibodies with many different bindings specificities have been isolated using this approach (Hoogenboom, H. R., et al. (2005). Nat. Biotechnol., 23, 1105). Various sources of H and L chain V regions have been used to construct phage display libraries including those isolated from non-immune or immune donors. In addition, phage display libraries have been constructed of V regions that contain artificially randomized synthetic CDR regions in order to create additional diversity. Often, antibodies obtained from phage display libraries are subjected to in vitro affinity maturation to obtain high affinity antibodies (Hoogenboom, H. R., et al. (2005). Nat. Biotechnol., 23, 1105).

The creation of transgenic mouse strains producing human antibodies in the absence of mouse antibodies has provided another technology platform for the generation of specific and high affinity human mAbs for application in humans. In these transgenic animals, the endogenous mouse antibody machinery is inactivated and replaced by human Ig loci to substantially reproduce the human humoral immune system in mice (Jakobovits, A., et al. (2007). Nat. Biotechnol. 25, 1134. Lonberg, N. (2005). Nat. Biotechnol. 23, 1117). B cell development as well as Ig diversification by recombination of gene segments is faithfully reproduced in these mice, leading to a diverse repertoire of murine B cells expressing human Igs. By immunizing these mice with antigens, it was further demonstrated that these transgenic animals accumulated somatic mutations in the V regions of both heavy and light chains to produce a wide diversity of high-affinity human mAbs (Lonberg, N. (2005). Nat. Biotechnol, 23, 1117).

The question, whether "fully human" mAbs such as derived from phage display libraries or transgenic mice are less immunogenic than humanized mAbs cannot be answered yet, because full immunogenicity data are available for just two human mAbs. An anti-tumor necrosis factor mAb, developed from phage-displayed human libraries induced antibody responses in 12% of patients - at the higher end of the incidence of anti-antibody responses of the humanized antibodies (Hwang, W. Y., et al. (2005). Methods, 36, 3-10).

Evaluation of the immunogenicity of the first registered human mAb generated by the transgenic approach demonstrated that mAb treatment resulted in the generation of antibodies in approximately 5.5% of treated cancer patients (Jakobovits, A., et al. (2007). Nat. Biotechnol. 25, 1134., Lofgren, J. A., et aL (2007). J. Immunol. 178, 7467).

A need therefore remains for a method and means for producing antibodies that are specific for their targets, but are less immunogenic. According to the invention the reduction of immunogenicity is at least partially achieved by providing a transgenic non-human mammal comprising, at least in its B cell lineage, a nucleic acid encoding at least an immunoglobulin light chain or heavy chain, wherein the heavy- or light chain encoding sequence is provided with a means that renders it resistant to DNA rearrangements and/or somatic hypermutations, preferably such a non-human animal is a rodent, more specifically a mouse. The nucleic acid preferably encodes a human, human-like or humanized immunoglobulin chain.

In the remainder of the specification mice are typically used as examples of the non-human mammals. The transgenic non-human mammalian hosts are capable of mounting an immune response to an antigen, where the response produces antibodies having primate, particularly human, variable regions. Various transgenic hosts may be employed, particularly murine, lagomorpha, ovine, avine, porcine, equine, canine, feline, or the like. Mice have been used for the production of B-lymphocytes for immortalization for the production of antibodies. Since mice are easy to handle, can be bred in large numbers, and are known to have an extensive immune repertoire, mice will usually be the animal of choice. Therefore, in the following discussion, the discussion will refer to mice, but it should be understood that other animals, particularly non-primate mammals, may be readily substituted for the mice, following the same procedures.

The reason for preventing rearrangements and hypermutation is that in this manner a non-immunogenic polypeptide can be chosen beforehand knowing that this polypeptide chain will remain non-immunogenic. At least one of the chains of the resulting immunoglobulin is thus less immunogenic. The resulting antibody needs to have (usually) both a light- and a heavy chain. The non-immunogenic chain must therefore be capable of pairing with the other chain. The other chain may be an endogenous chain, an exogenous chain or a hybrid of both. For human therapy, the non-immunogenic chain should be as close to human as possible.

A means for rendering a gene encoding an immunoglobulin chain (or chains) resistant to DNA rearrangement and/or mutation is of course removal of all genetic elements responsible for said rearrangement and/or mutation. The drawback thereof is that the variability of the two chains is eliminated, whereas the invention preferably retains the variability in one chain (preferably the heavy chain) and inhibits and/or prevents the rearrangement-mutation of the other chain (preferably the light chain).

The elements for rearrangement and/or hypermutation characterized so far are located within the loci for immunoglobulins. Therefore the means for rendering the immunoglobulin encoding sequence resistant to DNA rearrangement and/or mutation is inserting the gene in a locus outside the immunoglobulin loci.

Thus according to the invention a transgenic non-human mammal is provided wherein the light/heavy chain encoding sequence is integrated in the genome of the non-human mammal in a locus outside the immunoglobulin loci. Preferably the insertion is in a locus that is resistant to gene silencing. According to the invention, the integration is in the Rosa-locus or a comparable locus.

It is preferred to provide an expression cassette which can be inserted in a Rosa locus or comparable locus with a means that allows expression of the immunoglobulin chain(s) essentially limited to cells of B cell lineage, preferably with a means that allows expression of the light chain encoding nucleic acid during a certain stage of the development of B cells. The term "essentially limited expression" indicates that expression is predominantly in cells of the B-cell lineage, but that lower levels of expression in other cells, as compared to the level of expression in B-cells, is possible. In a preferred embodiment, the term "essentially limited expression" indicates that the expression is exclusively present in cells of the B-cell lineage. Such means typically and preferably include B cell (developmental stage) specific promoters such as CD 19, CD20, µHC (all V-genes), VpreB1, VpreB2, VpreB3, λ5, Igα, Igβ, κLC (all genes), λLC (all genes), BSAP (Pax5). Although it is very well possible to direct the expression of the DNA rearrangement and/or mutation resistant chain by such promoters, they are relatively weak. A strong promoter will typically be required to ensure adequate surface expression of the B cell receptor (made up of the membrane attached Ig H and L chain) and to compete with the expression and pairing of endogenous chains (if present) through allelic exclusion. Such a promoter, however is usually not tissue specific. To confer tissue specificity, an indirect system employing Cre/lox or the like is preferred. The desired chain is put under control of a strong promoter inhibited by an element that can be removed by the action of a Cre-protein, leading to activation of the desired immunoglobulin encoding gene. This system is described in detail in Wunderlich F. T. (2004), "Generation of inducible Cre systems for conditional gene inactivation in mice", Inauguraldissertation zur Erlangung des Doktorgrades der Mathematisch-Naturwissenschaftlichen Fakultät der Universität zu Köln; http://deposit.ddb.de/cgi-bin/dokserv?idn= 97557230x&dok_var=d1&dok_ext=pdf&filename=97557230x.pdf.

Preferably the immunoglobulin chain produced in a manner resistant to rearrangements and hypermutation is a light chain capable of pairing with different heavy chains encoded by the non-human mammal. The light chain will then be the same (and less immunogenic) in all antibodies, but variety in specificity is retained through rearrangements and hypermutations in the heavy chains. It may in that case be preferable to silence at least one of the endogenous loci encoding a light chain, although allelic exclusion may render this unnecessary.

According to this embodiment, preferably the endogenous kappa (κ) light chain locus is functionally silenced.

If the endogenous κ light chain locus is silenced, but also for other reasons, it is preferred that the resistant light chain is a κ light chain, preferably a light chain that has a germline-like sequence. According to the invention such a light chain would lead to an antibody with reduced immunogenicity. The preferred germline sequence is based on the human IGKV1-39 (012) as this light chain is very frequently observed in the human repertoire (de Wildt et al. 1999. J. Mol. Biol. 285(3):895) and has superior thermodynamic stability, yield and solubility (Ewert et al. 2003. J. Mol. Biol. 325(3):531).

The following gives more specific embodiments of the expression cassette with which the non-human animal can be provided according to the invention. Although this is typically advantageous for immunoglobulins, other genes of interest are also contemplated.

Thus the invention provides in a specific embodiment a transgenic non-human mammal wherein the light chain encoding nucleic acid comprises in 5'-3' direction: a B cell specific promoter, a leader, a rearranged human V gene, optionally a MοEκi enhancer, a constant region (κ) and optionally a (truncated) MoEκ3' enhancer. Neuberger identified and examined a novel B-cell specific enhancer located downstream of the kappa constant region (EP004690251). This enhancer has been shown to play a crucial role in the expression of kappa genes as removal of the 808bp enhancer strongly reduced expression. Deletion of the 3' kappa enhancer also strongly reduced the level of somatic hypermutations (SHM). In transgenic and cell expression studies it has been revealed that reduced, mutated or deleted 3' kappa enhancers not only lowered expression levels but also decreased the level of somatic hypermutations. Currently, it can not be determined whether the 3' kappa enhancer is involved in SHM processes, expression regulation or both (review Odegard, V. H., et al. (2006). Nat. Rev. Immunol. 6, 573; Inlay, M., et al. (2002). Nat. Immunol. 3, 463.).

Detailed expression studies using engineered variants of the 3' kappa enhancer indicated that a 50 nucleotide region is sufficient to drive expression. However for proper expression a reduced sequence of 145 nucleotides is preferred (EP04690251; Meyer, K. B., et al. (1990) Nucleic Acids Res. 18(19):5609-15)

Thus the invention in one aspect provides a nucleic acid for insertion into the genome of a non human animal that is an expression cassette for the expression of a desired proteinaceous molecule in cells developing into mature B cells during a certain stage of development, said cassette comprising means for preventing silencing of expression of the desired proteinaceous molecule after introduction into a host cell, and means for timing expression of the desired proteinaceous molecule with the desired developmental stage of the host cell.

An expression cassette is defined as a nucleic acid that has been provided with means for introduction into the genome of a host cell, such as sequences which allow for homologous recombination with a certain site in the genome. Usually the nucleic acid will be DNA, typically double stranded. Typically the expression cassette will be provided to the cell in a vector from which it is transferred to the genome of the cell. The expression cassette further comprises all elements necessary for expression of the gene in a host cell, although in certain embodiments some of such elements may be present on a second nucleic acid to be introduced, whereby these elements act in trans. Elements necessary for expression in a host cell include promoters, enhancers and other regulatory elements. Only those elements are necessary that are not provided by the host cell.

According to the invention it is important that the expression of the gene of interest is not silenced in the genome of the host cell, especially not in the development stage where expression is required. This can be done by various means, such as insertion into the endogenous locus or by providing the cassette with nucleic acid elements that prevent silencing (Kwaks et al. (2006) Trends Biotechnol. 24(3), p. 137-142; which is incorporated herein by reference). It is preferred that the expression cassette is inserted in a locus that is not silenced in the host cells (EP 01439234; which is incorporated herein by reference).

Said means for prevention of silencing comprise STabilizing Anti-Repression-sequences (STAR®-sequences) and Matrix Attachment Regions (MARs). A STAR sequence is a nucleic acid sequence that comprises a capacity to influence transcription of genes in cis. Typically, although not necessarily, a STAR sequence does not code by itself for a functional protein element. In one embodiment one STAR element is used. Preferably, however, more than one STAR element is used. In a particularly preferred embodiment an expression cassette according to the invention is provided with two STAR sequences; one STAR sequence at the 5' side of the coding sequence of the immunoglobulin gene and one STAR sequence at the 3' side of the coding sequence of the immunoglobulin gene. MARs are DNA sequences that are involved in anchoring DNA/chromatin to the nuclear matrix and they have been described in both mammalian and plant species. MARs possess a number of features that facilitate the opening and maintenance of euchromatin. MARs can increase transgene expression and limit position-effects.

According to the invention it is important that expression from the cassette only occurs during a certain period in the development of a cell, in particular a developing B cell, more in particular a B cell in a transgenic non-human animal, in particular a mouse. In this particular case the developmental period is chosen such that the expression of the gene from the cassette (typically a light- or heavy chain-like polypeptide) does not significantly interfere with the normal differentiation and/or maturation of the cell and when applicable, allows for pairing of the polypeptide chain produced with its counterpart.

According to the invention this may, in one embodiment, be achieved by providing a nucleic acid according to the invention, wherein said means for timing expression is a promoter of which the activity is essentially limited to the certain stage of development. In a developing B cell, which, e.g. after immunization is maturing and/or differentiating, the expression of the gene of interest, when it is one of the polypeptide chains of an immunoglobulin, must not interfere (significantly) with said maturation and/or differentiation and it needs to be timed such that the resulting polypeptide can pair with its counterparts. Therefore the invention provides a nucleic acid according to the invention wherein said certain stage starts at a stage immediately preceding or coinciding with the onset of the expression of light chain molecules by said cells at a certain stage of development into a mature B cell.

This may be achieved by selecting a promoter which is active only during said suitable period. Such a promoter may be a CD 19 promoter, the Ig-α promoter, the Ig-β promoter, the µhc (all genes) promoter, the Vk promoter or analogues or homologues thereof.

In a specific embodiment of the present invention the promoter as disclosed above does not drive the expression of the gene of interest directly. Instead it drives the expression of a gene of which the product activates in trans the expression of the gene of interest. Such an activating gene may be a gene encoding a so-called Cre recombinase or Cre-like protein. The expression cassette for the gene of interest may e.g. be provided with a sequence that inhibits expression of the gene of interest. Said sequence can be removed by the action of the Cre recombinase, which is under control of the desired promoter (active during the proper stage of development). In this embodiment a set of expression cassettes is required.

Therefore the invention provides a set of nucleic acids that are expression cassettes, wherein one nucleic acid comprises an expression cassette encoding a Cre-like protein under control of a promoter active during the desired stage of development of the host cell and the second nucleic acid comprises a sequence encoding a desired proteinaceous molecule under control of a constitutive promoter which can be activated by the action of a Cre-like protein. Said activation is preferably achieved by removal of a stop sequence flanked by loxP sites. The Cre/lox system is described in detail in Rajewsky et al. (1996) J. Clin. Invest. 98, p.600-603, which is incorporated herein by reference. Such systems are reviewed in Wunderlich F. T. (2004), "Generation of inducible Cre systems for conditional gene inactivation in mice", Inauguraldissertation zur Erlangung des Doktorgrades der Mathematisch-Naturwissenschaftlichen Fakultät der Universität zu Köln; http://deposit.ddb.de/cgi-bin/dokserv?idn=97557230x&dok_var=d1&dok_ext=pdf&filename=9755723 0x.pd, which is incorporated herein by reference.

The invention further provides a transgenic non-human animal which has been provided with an expression cassette according to invention, wherein the desired proteinaceous molecule is a polypeptide chain of an immunoglobulin. A preferred polypeptide chain is a light chain. A more preferred polypeptide is a germline or germline-like light chain. A most preferred polypeptide is 012, preferably the rearranged germline kappa light chain IGKV1-39*01/ IGKJ1*01 (nomenclature according to the IMGT database, http://www.imgt.org).

It is further preferred that the polypeptide chain is rendered essentially incapable of rearrangement and/or of excluded of any sequence modification such as normally operating on Ig during the process of B cell affinity maturation. Therefore, the invention provides a transgenic non-human animal which has been provided with an expression cassette according to the invention, wherein said re-arrrangement and/or sequence modifications are prevented by the absence of elements at least partially responsible for somatic hypermutation such as, for example, the MοEκi enhancer.

A preferred expression cassette according to the invention comprises means for prevention of silencing. In one embodiment, said means for prevention of silencing are means for insertion into a locus in the genome of the host cell that is resistant to silencing. Said means for insertion are preferably means for homologous recombination into said site resistant to silencing. A preferred locus when the non-human animal is a mouse is the rosa-locus.

A further preferred expression cassette according to the invention comprises in 5'-3' direction: a Vκ promoter, a mouse leader, a human V gene, optionally a MoEκi enhancer, a rat constant region (Cκ) and optionally a (truncated) MoEκ3' enhancer.

Yet a further preferred expression cassette according to the invention comprises in 5'-3' direction: a Vκ promoter, a human leader, a human V gene, optionally a MoEκi enhancer, a rat constant region (Cκ) and optionally a (truncated) MoEκ3' enhancer.

Of course the ultimate goal of the invention is to produce antibodies to be used in human therapeutics. Thus the invention provides a method for producing a desired antibody comprising exposing a non-human mammal according to the invention to an antigen such that an antibody response is induced and isolating the antibodies specific for the antigen.

In an alternative embodiment, the invention provides a method for producing a desired antibody comprising exposing a non-human mammal according to the invention to an antigen such that an antibody response is induced and isolating cells producing such antibodies, culturing and optionally immortalizing said cells and harvesting said antibodies.

In a further embodiment, the invention provides a method for producing a desired antibody comprising exposing a non-human mammal according to the invention to an antigen such that an antibody response is induced and isolating a nucleic acid encoding at least part of such an antibody, inserting said nucleic acid or a copy or a derivative thereof in an expression cassette and expressing said antibody in a host cell.

The methods for producing antibodies from transgenic mice are known to a person skilled in the art. Particularly preferred are methods for production of mixtures of antibodies from one cell, whereby the nucleic acids encoding these antibodies have been derived from mice according to the invention.

These so-called oligoclonics are disclosed in WO04106375 and WO05068622, which are incorporated herein by reference.

The present invention provides transgenic non-human mammals, preferably mice, capable of generating specific and high affinity hybrid mouse-human antibodies with preferably human immunoglobulin light chain variable (VL) regions in or near germline configuration and preferably murine immunoglobulin heavy chain variable (VH) regions that may have accumulated somatic mutations during the process of antigen-driven affinity maturation. It is envisaged that the murine VH regions of the hybrid antibodies may be subjected to humanization procedures to yield mAbs that have reduced immunogenicity when applied in humans based on germline or near-germline VL regions and murine VH regions that have been humanized.

In particular, it has been shown in the present invention that transgenic mice that harbor a DNA expression construct that encodes a rearranged human VL region under the control of cis-acting genetic elements that provide timely and regulated expression of the transgene on a significant proportion of B cells during B cell development, yet lack elements that direct the somatic hypermutation machinery to the transgene, are capable of generating specific and high affinity mouse-human hybrid antibodies with essentially unmutated L chains. It is shown that the rearranged human transgene is capable of pairing with a diversity of endogenous murine immunoglobulin H chains to form mouse-human hybrid immunoglobulins expressed on the surface of B cells and to sufficiently facilitate murine B cell development to obtain a sizeable and diverse peripheral B cell compartment.

In a preferred embodiment, the transgene expression construct harbors the coding sequences of a human rearranged L chain V region under the control of a human VL promoter to direct B-cell specific expression. In addition, the construct harbors the murine 3' Ck enhancer sequence for B cell specific and inducible and high level expression of the tranegene. Furthermore, the construct is designed to lack regulatory elements that facilitate the recruitment of the somatic hypermutation machinery to the transgene, such as the intron enhancer and the 3' C-kappa enhancer.

In a related embodiment, the rearranged human VL gene is inserted in the murine Rosa26 locus by site-specific integration. The Rosa26 locus is useful in the context of the "targeted transgenesis" approach for efficient generation of transgenic organisms (such as mice) with a predictable transgene expression pattern.

In a preferred embodiment, the rearranged human VL region is selected for its capacity to pair with many different murine VH genes so as to ensure the generation of a population of B cells with a diverse VH gene repertoire. A method of obtaining such VL regions comprises amplifying a repertoire of rearranged VH genes from the B cells of mice and a repertoire of human rearranged germline VL regions from the B cells of humans and cloning them into phagemid display vectors to prepare diverse libraries of hybrid immunoglobulins in bacteria. By nucleotide sequence analysis of collections of unselected and antigen-selected VH/VL pairs, human germline VL genes that pair with many different murine VH genes are identified. A collection of human germline VL genes with this capacity is described.

In one embodiment, it is shown that upon immunization with antigen, the B cells are capable of mounting an immune response, leading to the generation of B cells that secrete hybrid antibodies with high specificity and affinity. The V regions encoding these antibodies are characterized by the human transgenic light chain that harbors no or very few mutations and a murine heavy chain that harbors a variable number of mutations introduced by the somatic hypermutation machinery.

In a related embodiment, strategies to obtain high affinity hybrid monoclonal antibodies from the transgenic mice by hybridoma and display technologies are contemplated as well as procedures to humanize the murine VH regions to obtain less immunogenic antibodies for application in humans.

In one embodiment, the invention provides an immunoglobulin L chain transgene construct comprising DNA sequences that encode a human immunoglobulin VL region in combination with a light chain constant region (CL) of an animal immunoglobulin protein, which sequences are operably linked to transcription regulatory sequences that, when integrated in a non-human transgenic animal, produce an Ig VL-CL polypeptide with a human VL region that is not or marginally subject to somatic hypermutation. The Ig VL is capable of pairing with rearranged VH-CH polypeptides that are generated during B cell development in the non-human transgenic animal, with said VH-CH polypeptides retaining the capacity to undergo somatic hypermutation upon stimulation. The CL region may be of any animal species and is generally capable of pairing with the CH regions of the non-human transgenic animal.

The invention also includes the use of a transgene construct as above in producing a transgenic non-human animal capable of the production of hybrid antibodies consisting of VL-CL polypeptides and VH-CH polypeptides in which the VL region is of human origin and the CL, VH and CH may be of any animal species, including human. Upon immunization, these transgenic animals are capable of generating high affinity antibodies encoded by somatically hypermutated VH genes and essentially non-mutated VL genes encoded by the transgene.

In another aspect, the invention provides a process for the production of a transgenic non-human animal capable of the production of hybrid antibodies in response to antigenic challenge, comprising functionally disrupting the endogenous immunoglobulin light chain locus and inserting into the animal genome a transgene construct of the invention.

The invention includes the use of animals obtainable by this process in the production of B cells that produce immunoglobulin having human VL light chain. In another aspect of the invention there is provided a process for the production of B cells that produce immunoglobulin having human VL and binding to a selected antigen, comprising challenging an animal obtainable by a process as above with said antigen and screening for B cells from said animal that bind said antigen. The invention further includes B cells obtainable by this process and hybridomas obtainable by immortalizing such B cells, e.g. hybridomas obtained by fusing B cells as above with myeloma cells. The invention also includes a process for producing monoclonal antibody comprising cultivating such a hybridoma. In yet a further aspect, the invention provides the use of the above B cells in producing a hybridoma or corresponding monoclonal antibody.

Yet a further aspect of the invention is a process for the production of immunoglobulin having human VL chain and binding to a selected antigen, comprising challenging an animal obtainable as above with said antigen and obtaining immunoglobulin there from.

In one strategy, as an individual step, a rearranged VL region encoded by human germline V and J gene segments and a light chain constant region of any animal species but preferably a murine constant region is introduced into the mouse germ line. The transgene DNA may be introduced into the pronuclei of fertilized oocytes or embryonic stem cells. The integration may be random or homologous depending on the particular strategy to be employed. For example, the VL transgene may be introduced by random insertion, resulting in mice that bear one or multiple copies of the transgene in the genome. Alternatively, the human VL transgene may be targeted to a specific genomic locus using site-specific recombination as described in the art.

In one preferred embodiment, the VL transgene is targeted to the murine ROSA26 locus which is a suitable integration site allowing strong and predictable expression of inserted transgenes (EP 1439234). The targeting vector allows insertion of a single copy of a gene expression cassette, thus avoiding modulation of transgene expression by the arrangement of multiple copies. By choosing the autosomal Rosa26 locus as insertion site, the expression pattern of the inserted transgene in the non-human animal is predictable. Furthermore, random X inactivation and/or modulation by chromosomal position effects are avoided. This also eliminates the need to generate and analyse multiple transgenic strains for any given transgene. Finally, the Rosa26 targeting vector for the site-specific integration can be used for multiple gene expression cassettes. Thus, it may be envisaged that 2 or more different rearranged germline human VL regions are inserted into the Rosa26 locus to further increase the diversity of the repertoire of hybrid or human antibodies.

In another embodiment, a rearranged human VL region may be targeted to the murine Ig kappa or lambda light chain locus so as to functionally inactivate the endogenous locus or mice containing the rearranged human VL region may be bred with mice that lack functional kappa or lambda Ig loci or both. Thus, by using transformation, using repetitive steps or in combination with breeding, transgenic animals may be obtained which are able to produce antibodies harboring the human VL transgene in the substantial absence of endogenous host immunoglobulin light chains.
In one embodiment, a human VL transgene is selected for its capacity to pair with a substantial portion of murine VH regions to form a diverse repertoire of functional mouse-human hybrid antibodies expressed on the surface of B cells. By a substantial portion of murine VH regions is meant that the human VL pairs with at least with 0.1% of the murine VH regions generated during B cell development, more preferably with at least 1% and most preferably with at least 10%. Methods to identify human VL genes with this characteristic include randomly pairing a repertoire of human VL regions with a repertoire of murine VH regions, co-expression of VH and VL regions in appropriate eukaryotic or prokaryotic expression vectors and screening for human VL regions that pair with a substantial portion of murine VH regions. In one embodiment, phagemid vectors may be used to direct expression of mouse-human antibody fragments in bacterial cells or to the surface of filamentous phage and analysis of binding capacity of antibody fragments by methods known in the art.

In another embodiment, a human VL transgene is selected for its capacity to pair with a substantial portion of human VH regions to form a diverse repertoire of human antibodies expressed on the surface of B cells. By a substantial portion of human VH regions is meant that the human VL pairs with at least with 0.1% of the human VH regions generated during B cell development, more preferably with at least 1% and most preferably with at least 10%.

In the latter embodiment, the human VL transgenic mice are crossed with mice that harbor functional rearranged or non-rearranged human H chain immunoglobulin loci and functionally inactivated endogenous H chain Ig loci as described in the art. The functional inactivation of the two copies of each of the three host Ig loci (heavy chain, kappa and lambda light chain), where the host contains the human IgH and the rearranged human VL transgene would allow for the production of purely human antibody molecules without the production of host or host human chimeric antibodies. Such a host strain, by immunization with specific antigens, would respond by the production of mouse B-cells producing specific human antibodies, which B-cells are subsequently fused with mouse myeloma cells or are immortalized in any other manner for the continuous stable production of human monoclonal antibodies. Alternatively, said population of B cells is used as a source of VH regions that can be obtained by constructing cDNA libraries or by PCR amplification using primers for human VH regions as is known in the art.

A human rearranged VL gene is reconstructed in an appropriate eukaryotic or prokaryotic microorganism and the resulting DNA fragments can be introduced into pronuclei of fertilized mouse oocytes or embryonic stem cells. Various constructs that direct B cell specific expression of VL transgenes have been described in the art and have the following general format: a leader sequence and relevant upstream sequences to direct B cell specific expression of the transgene, a coding sequence of a human VL transgene, an enhancer sequence that directs B cell specific and high level expression of the transgene and a murine constant region gene. In a preferred format, the enhancer is the C-kappa 3' enhancer because it directs high level expression in B-lineage cells, but does not recruit somatic hypermutation when used in transgene constructs.

In one embodiment, animals, preferably mice, comprising one or multiple copies of the transgene in the genome are isolated and analyzed for stable expression. Animals are selected that show stable expression of the transgene over longer periods of time, preferably in B-cells. If required, different animal lines comprising independent insertions of one or multiple copies of the transgene, preferably on different chromosomes, are crossed to obtain animals with different insertions of one or multiple copies of the transgene to increase expression of the transgene in animals, preferably in B-cells.

The invention further provides progeny of a transgenic non-human animal according to the invention, the progeny comprising, at least in its B-cell lineage, a heavy- or light chain encoding sequence together with a means that renders the sequence resistant to DNA rearrangements and/or somatic hypermutations.

The invention further provides progeny of a transgenic non-human animal according to the invention, the progeny comprising an expression cassette for the expression of a desired proteinaceous molecule in cells during a certain stage of development in cells developing into mature B cells.

The invention in addition provides a cell that is isolated from a transgenic non-human animal according to the invention, the cell comprising a heavy- or light chain encoding sequence together with a means that renders the sequence resistant to DNA rearrangements and/or somatic hypermutations. The invention in addition provides a cell that is isolated from a transgenic non-human animal according to the invention, the cell comprising an expression cassette for the expression of a desired proteinaceous molecule in cells during a certain stage of development in cells developing into mature B cells, A cell according to the invention, preferably an antibody-producing B-cell or a cell that is capable of differentiating or maturating into an antibody-producing B-cell, can be used for in vitro production of antibodies, as is known to the skilled person, for example from Gascan et al. 1991. J. Exp. Med. 173: 747-750. Methods for immortalization of a cell according to the invention are known in the art and include the generation of hybridomas, for example by fusion with a myeloma cell, transformation with Epstein Barr Virus; expression of the signal transducer of activation and transcription (STAT), activation via CD40 and IL4 receptor signaling, and/or expression of Bcl6 (Shvarts et al. 2002. Genes Dev 16: 681-686).

In a separate step the mouse endogenous Kappa and Lambda light chain loci are rendered essentially non-functional such that at least the majority of B cells in the transgenic mice bear Ig receptors that contain the transgenic human VL region. Inactivation of the endogenous mouse immunoglobulin loci is achieved by targeted disruption of the appropriate loci by homologous recombination in mouse embryonic stem cells. Said targeted disruption comprises alteration of the genomic sequence such that substantially no functional endogenous mouse immunoglobulin Kappa and/or Lambda light chain is produced. The term "substantially no functional endogenous mouse immunoglobulin" indicates that the endogenous Kappa and/or Lambda light chain loci are functionally silenced such that the level of functional protein expression of the endogenous Kappa and/or Lambda light chain loci, preferably the endogenous Kappa light chain locus, is reduced to about 20% of the level of expression in a reference mouse, more preferred to about 10%, more preferred to about 5%, more preferred to about 2% and more preferred to about 1%. In a most preferred embodiment, the level of functional protein expression of the endogenous Kappa and/or Lambda light chain loci is reduced to 0%. The level of functional protein expression can be determined by means known to the skilled person, including western blotting and pairing with a mouse heavy chain. Said reference mouse is a mouse in which the endogenous Kappa and/or Lambda light chain loci is not disrupted. Said alteration comprises mutation and/or deletion of gene sequences that are required for functional expression of the endogenous immunoglobulin genes. Alternatively, said alteration comprises insertion of a nucleic acid into the endogenous mouse immunoglobulin Kappa and/or Lambda light chain loci such that the functional expression of the endogenous immunoglobulin genes is reduced. In one embodiment, said nucleic acid comprises a silencing element resulting in transcriptional silencing of the endogenous immunoglobulin gene. In a further embodiment, or in addition, said nucleic acid comprises a sequence that disrupts splicing and/or translation of the endogenous immunoglobulin gene, for example by introducing an exon that renders a frame shift in the coding sequence, or that comprises a premature stop codon. In each case chimeric animals are generated which are derived in part from the modified embryonic stem cells and are capable of transmitting the genetic modifications through the germ line. The mating of mouse strains with human immunoglobulin loci to strains with inactivated mouse loci yields animals which produce antibodies comprising essentially only human light chains.

A construct for homologous recombination is prepared by means known in the art and any undesirable sequences are removed, e.g., procaryotic sequences. Any convenient technique for introducing a construct for homologous recombination into a target cell may be employed. These techniques include spheroplast fusion, lipofection, electroporation, calcium phosphate-mediated DNA transfer or direct microinjection. After transformation or transfection of the target cells, target cells are selected by means of positive and/or negative markers, for example by neomycin resistance and/or acyclovir and/or gancyclovir resistance. Those cells which show the desired phenotype may then be further analyzed by restriction analysis, electrophoresis, Southern analysis, PCR, or the like. By identifying fragments which show the presence of the lesion(s) at the target locus, cells in which homologous recombination has occurred to inactivate a copy of the target locus are identified.

Furthermore, it is shown that upon immunization, the murine and human VH regions in the afore-mentioned transgenic mice but not the VL regions are capable of undergoing somatic hypermutations to generate high affinity antibodies. Advantageously, these antibodies encoded by germline VL regions are predicted to contribute to lower immunogenicity when applied in humans and result in more stable antibodies that are less prone to aggregation and thus safer for therapeutic use in humans.

MAbs derived from the afore-mentioned non-human transgenic animals or cells all share the same identical human VL regions. It has been described that mAbs that share the same identical VL region may be co-expressed in a single clonal cell for the production of mixtures of recombinant antibodies with functional binding sites (see WO04106375 and WO05068622). Thus, the invention provides a platform for the generation of specific and high affinity mAbs that constitute the basis for mixtures of mAbs produced by clonal cells.

It is preferred that mAbs derived from the afore-mentioned non-human transgenic animals or cells are directed against cellular targets. Preferred targets are human surface-expressed or soluble proteins or carbohydrate molecules. Further preferred targets are surface-expressed proteins or carbohydrate molecules that are expressed on the surface of bacteria, viruses, and other pathogens, especially of humans.

More specifically, preferred targets include cytokines and chemokines, including but not limited to InterLeukin 1beta (IL1beta), IL2, IL4, IL5, IL7, IL8, IL12, IL13, IL15, IL18, IL21, IL23 and chemokines such as, for example, CXC chemokines, CC chemokines, C chemokines (or γ chemokines) such as XCL1 (lymphotactin-α) and XCL2 (lymphotactin-β), and CX3C chemokines. Further included as preferred targets are receptor molecules of the cytokines and chemokines, including type I cytokine receptors such as, for example, the IL-2 receptor, type II cytokine receptors such as, for example interferon receptors, immunoglobulin (Ig) superfamily receptors, tumor necrosis factor receptor family including receptors for CD40, CD27 and CD30, serine/threonine-protein kinase receptors such as TGF beta receptors, G-protein coupled receptors such as CXCR1-CXCR7, and tyrosine kinase receptors such as fibroblast growth factor receptor (FGFR) family members, EGF receptor family members including erbB1 (EGF-R; HER1), erbB2, (HER2), erbB3 (HER3), and erbB4 (HER4), insulin receptor family members including IGF-R1 and IGF-RII, PDGF receptor family members, Hepatocyte growth factor receptor family members including c-Met (HGF-R), Trk receptor family members, AXL receptor family members, LTK receptor family members, TIE receptor family members, ROR receptor family members, DDR receptor family members, KLG receptor family members, RYK receptor family members, MuSK receptor family members, and vascular endothelial growth factor receptor (VEGFR) family members.

Further preferred targets are targets that are over-expressed or selectively expressed in tumors such as, for example, VEGF, CD20, CD38, CD33, CEA, EpCAM, PSMA, CD54, Lewis Y, CD52, CD40, CD22, CD51/CD61, CD74, MUC-1, CD38, CD19, CD262 (TRAIL-R2), RANKL, CTLA4, and CD30; targets that are involved in chronic inflammation such as, for example, CD25, CD11a, TNF, CD4, CD80, CD23, CD3, CD14, IFNgamma, CD40L, CD50, CD122, TGFbeta and TGFalpha.

Preferred surface-expressed proteins or carbohydrate molecules that are expressed on the surface of bacteria, viruses, and other parasitic pathogens, especially of humans, include surface markers of influenza A and B viruses such as hemagglutinin (HA) and neuraminidase (NA), filoviruses such as Ebola virus, rabies, measles, rubella, mumps, flaviviruses such as Dengue virus types 1.4, tick-borne encephalitis virus, West Nile virus, Japanese encephalitis virus, and Yellow fever virus, Paramyxoviruses including Paramyxovirus such as Parainfluenza 1, 3, Rubulavirus such as Mumpsvirus and Parainfluenza 2, 4, Morbillivirus, and Pneumovirus such as Respiratory syncytial virus, Vaccinia, small pox, coronaviruses, including Severe Acute Respiratory Syndrome (SARS) virus, hepatitis virus A, B and C, Human Immunodeficiency Virus, Herpes viruses, including cytomegalovirus, Epstein Barr virus, Herpes simplex virus, and Varicella zoster virus, parvoviruses such as, for example, B19; Legionella pneumophila; Listeria monocytogenes; Campylobacter jejuni; Staphylococcus aureus; E. coli O157:H7; Borrelia burgdorferi; Helicobacter pylori; Ehrlichia chaffeensis; Clostridium difficile; Vibrio cholera; Salmonella enterica Serotype Typhimurium; Bartonella henselae; Streptococcus pyogenes (Group A Strep); Streptococcus agalactiae (Group B Strep); Multiple drug resistant S. aureus (e.g. MRSA); Chlamydia pneumoniae; Clostridium botulinum; Vibrio vulnificus; Parachlamydia pneumonia; Corynebacterium amycolatum; Klebsiella pneumonia; Linezolid-resistant enterococci (E. faecalis and E. faecium); and Multiple drug resistant Acinetobacter baumannii.

Most preferred targets are IL-6 and its receptor, IL-6Ralpha, glycoprotein-denominated gp130, RSV, especially the surface proteins F, G and SH and non-structural proteins such as N and M, and receptor tyrosine kinases, in particular erbB1. (EGF-R; HER1), erbB2, (HER2), erbB3 (HER3), erbB4 (HER4), IGF-R1 and IGF-RII, c-Met (HGF-R).

Therefore, the invention provides a platform for the generation of specific and high affinity mAbs against the above mentioned targets that constitute the basis for mixtures of mAbs produced by clonal cells. In a preferred embodiment, said specific and high affinity mAbs comprise mAbs that are directed against different epitopes on at least one of the targets. In a further preferred embodiment, said specific and high affinity mAbs comprise mAbs that are directed against different targets, such as, for example, one or more members of the EGF-receptor family, including erbB1 (EGF-R; HER1), erbB2, (HER2), erbB3 (HER3) and erbB4 (HER4).

Unless otherwise defined, scientific and technical terms used in connection with the present invention shall have the meanings that are commonly understood by those of ordinary skill in the art. Further, unless otherwise required by context, singular terms shall include pluralities and plural terms shall include the singular. Generally, nomenclatures utilized in connection with, and techniques of, cell and tissue culture, molecular biology, and protein and oligo- or polynucleotide chemistry and hybridization described herein are those well known and commonly used in the art. Standard techniques are used for recombinant DNA, oligonucleotide synthesis, and tissue culture and transformation (e.g., electroporation, lipofection). Enzymatic reactions and purification techniques are performed according to manufacturer's specifications or as commonly accomplished in the art or as described herein. The foregoing techniques and procedures are generally performed according to conventional methods well known in the art and as described in various general and more specific references that are cited and discussed throughout the present specification. See e.g., Sambrook et al. Molecular Cloning: A Laboratory Manual (3rd edition, Cold Spring Harbor Laboratory Press, Cold Spring Harbor, N.Y. (2001)), which is incorporated herein by reference. The nomenclatures utilized in connection with, and the laboratory procedures and techniques of, analytical chemistry, synthetic organic chemistry, and medicinal and pharmaceutical chemistry described herein are those well known and commonly used in the art. Standard techniques are used for chemical syntheses, chemical analyses, pharmaceutical preparation, formulation, and delivery, and treatment of patients.

### Figure legends

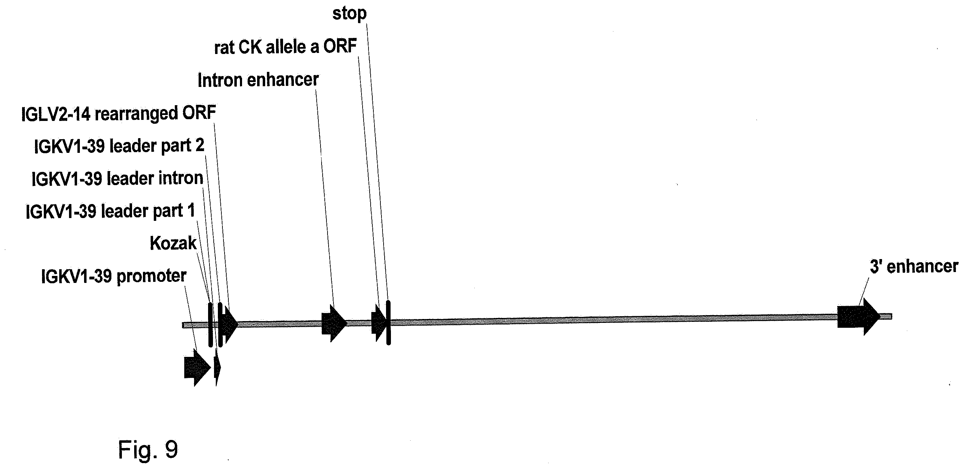

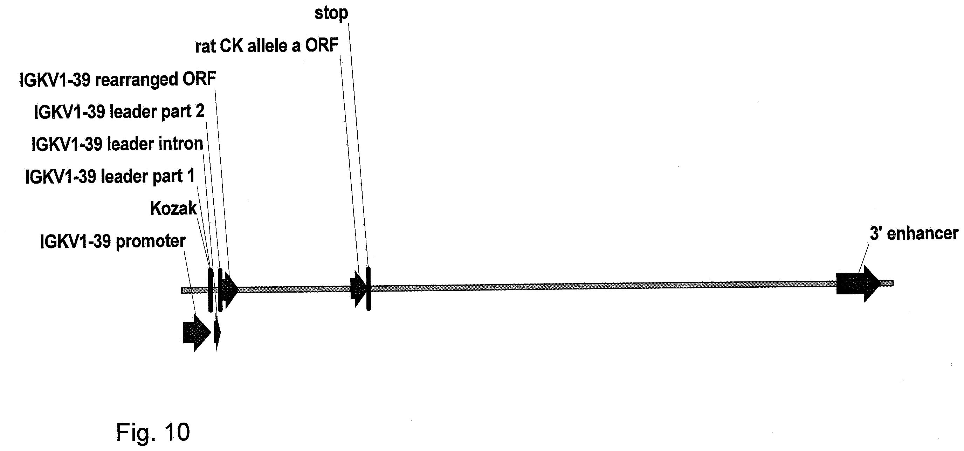

Figure 1
   A topology map of the annealing locations of mouse specific VH primers and the position of required restriction sites that are introduced by overhanging sequences at the 3' end of primers.
Figure 2
   PCR amplification steps (Amplification, Intermediate and Site introduction). The location and names of the mouse VH amplification primers (and mixtures of primers) are indicated per step.
Figure 3
   Topology of the MV1043 vector. This vector is used for the cloning of human or murine VH fragments. 012 (IGKV1-39) is indicated as the VL gene. Products of this vector in combination with helper phages in E. coli cells allow the generation of phages that display Fab fragments on the surface of the phage particles as a fusion product to the g3 protein and presence of the vector in the phage as the genetic content (F1 ORI).
Figure 4
   The topology of the mouse Ckappa locus downstream of the J-segments. Both enhancers and Ckappa region are indicated. The lower arrow indicates the region that is removed in order to silence the locus.
Figure 5
   The topology of the mouse C-lambda locus. All three active V-regions are indicated (Igl-V1, V2 and V3) as are the J-segments (Igl-J1, Igl-J2, Igl-J3, Igl-J4 and the pseudo segment Igl-J3p) and constant regions (Igl-Cl, Igl-C2, Igl-C3 and Igl-C4). The regions that are deleted in order to silence the locus are indicated by deletion markers. These deletions include all active V genes (1, 2 and 3) and the intergenic segment between V2 and V3.
Figure 6
   Construct topology of IGKV1-39/J-Ck with an intron located in the leader open reading frame (ORF).
Figure 7
   Construct topology of IGLV2-14/J-Ck with an intron located in the leader open reading frame (ORF).
Figure 8
   Construct topology of VkP-IGKV1-39/J-Ck (VkP-O12). The promoter originates from the IGKV1-39 gene and is placed directly in front of the required elements for efficient transcription and translation. Intergenic sequences (including the enhancers) are derived from mice and obtained from BAC clones. The C-kappa sequence codes for the kappa constant region of rat.
Figure 9
   Construct topology of VkP-IGLV2-14/J-Ck (VkP-2a2). The promoter originates from the IGKV1.39 gene and is placed directly in front of the required elements for efficient transcription and translation. Intergenic sequences (including the enhancers) are derived from mice and obtained from BAC clones. The C-kappa sequence codes for the kappa constant region of rat.
Figure 10
   Construct topology of VkP-IGKV1-39/J-Ck-Δ1 (VkP-O12-del1) is identical to VkP-IGKV1-39/J-Ck from figure 9 except that the intron enhancer region is removed.
Figure 11
   Construct topology of VkP-IGKV1-39/J-Ck-Δ2 VkP-O12-del2) is identical to VkP-IGKV1-39/J-Ck-Δ1 from figure 10 except that a large piece of the intergenic region between the Ck gene and 3'enhancer is deleted. In addition, the 3' enhancer is reduced in size from 809 bp to 125 bp.
Figure 12
   Overview of the sequences used or referred to in this application.
Figure 13
   Generation of Rosa26-IgVk1-39 KI allele. (A) Schematic drawing of the pCAGGS-IgVK1-39 targeting vector. (B) Nucleotide sequence of the pCAGGS-IgVK1-39 targeting vector. (C) Targeting strategy.
Figure 14
   (A) Southern blot analysis of genomic DNA of ES clones comprising an insertion of the pCAGGS-IgVK1-39 targeting vector. Genomic DNA of 4 independent clones was digested with AseI and probed with 5e1 indicating the 5'-border of the targeting vector. All clones comprise a correct insertion of the targeting vector at the 5' end.
   (B) Southern blot analysis of genomic DNA of ES clones comprising an insertion of the pCAGGS-IgVK1-39 targeting vector. Genomic DNA of 4 independent clones was digested with MscI and probed with 3e1 indicating the 3'-border of the targeting vector. All clones comprise a correct insertion of the targeting vector at the 3' end.
   (C) Southern blot analysis of genomic DNA of ES clones comprising an insertion of the pCAGGS-IgVK1-39 targeting vector. Genomic DNA of 4 independent clones was digested with BamHI and probed with an internal Neo probe indicating the 5'-border of the targeting vector. All clones comprise a correct, single insertion of the targeting vector.
Figure 15
   Generation of Rosa26-IgV12-14 KI allele. (A) Schematic drawing of the pCAGGS-IgVL2-14 targeting vector. (B) Nucleotide sequence of the pCAGGS-IgVL2-14 targeting vector containing the CAGGS expression insert based on the rearranged germline IGLV2-14/J V lambda region (IGLV2-14/J-Ck). (C) Targeting strategy.
Figure 16
   Epibase® profile of IGKV1-39 residues 1-107. Sub-figure A displays the binding strength for DRB1 allotypes, while C displays the binding strength for DRB3/4/5, DQ and DP allotypes. The values in the figure represent dissociation constants (Kd's) and are plotted on a logarithmic scale in the range 0.01 µM - 0.1 µM (very strong binders may have run off the plot). For medium binding peptides, qualitative values are given only, and weak and non-binders are not shown. Values are plotted on the first residue of the peptide in the target sequence (the peptide itself extends by another 9 residues). Importantly, only the strongest binding receptor for each peptide is shown: cross-reacting allotypes with lower affinity are not visible in this plot. The strongest binding receptor is indicated by its serotypic name. Finally, any germline-filtered peptides are plotted with a lighter color in the epitope map (in this case, no non-self epitopes were found). Sub-figure B shows the HLA binding promiscuity for every decameric peptide (Y-axis: the number of HLA allotypes recognizing critical epitopes in each of the peptides starting at the indicated residue shown on the X-axis). The promiscuity is measured as the number of allotypes out of the total of 47 for which the peptide is a critical binder. White columns refer to self-peptides, and black columns (absent here) to non-self peptides.
Figure 17
   Epitope map of IGKV1-39 showing the presence of peptide binders predicted in the sequence of IGKV1-39 by serotype in the 15-mer format. Each 15-mer is numbered as indicated in the top of the figure. The full sequence of the corresponding 15-mer is listed in Table 7. Black boxes indicate the presence of one or more critical self-epitopes in the 15-mer for the serotype listed on the left. Critical epitopes are operationally defined as strong or medium DRB1 binders and strong DRB3/4/5 or DP or DQ binders.
Figure 18
   Constitutive knock-out (KO) of the Ig kappa locus. (A) Targeting strategy. (B) Schematic drawing of the pIgKappa targeting vector,
Figure 19
   Constitutive KO of the Ig lambda locus, (A) First step of the targeting strategy. (B) Second step of the targeting strategy.
Figure 20
   Schematic drawing of targeting vectors. (A) pVkP-O12 (VkP-IGKV1-39/J-Ck); (B) pVkP-O12-del1 (VkP-IGKV1-39/J-Ck-Δ1); (C) pVkP-O12-del2 (VkP-IGKV1-39/J-Ck-Δ2).
Figure 21
   Targeting strategies for insertion of transgene into the Rosa26 locus by targeted transgenesis using RMCE. (A) VkP-O12 (VkP-IGKV1-39/J-Ck); (B) VkP-O12-del1 (VkP-IGKV1-39/J-Ck-Δ1); (C) VkP-O12-del2 (VkP-IGKV1-39/J-Ck-Δ2).
Figure 22
   Topology of the MV1057 vector. Replacing the indicated stuffer fragment with a VH fragment yields an expression vector that can be transfected to eukaryotic cells for the production of IgG1 antibodies with light chains containing an 012 (IGKV1-39) VL gene.
Figure 23
   Lack of transgenic human Vk1 light chain expression in non-B cell populations of the spleen.
Figure 24
   Transgenic human Vk1 light chain is expressed in all B cell populations of the spleen.
Figure 25
   Transgenic human Vk1 light chain is expressed in B1 cells of the peritoneal cavity.
Figure 26
   Transgenic human Vk1 light chain is not expressed in pro- and pre-B cells but in the immature and recirculating populations B cells in the bone marrow, (A) Gating of bone marrow cells. (B) Histograms of transgene expression with overlay from one WT control.
Figure 27
   Transgenic human Vk1 light chain is directly correlated with endogenous light chain and IgM expression in circulating B cells in the blood.

### Examples.

### Example 1

### Human light chain V-gene clones

This example describes the rationale behind the choice of two human light chain V- genes, one gene of the kappa type and one gene of the lambda type, that are used as a proof of concept for light chain expressing transgenic mice. De Wildt et al. 1999 (de Wildt et al. (1999) J. Mol. Biol. 285(3):895) analysed the expression of human light chains in peripheral IgG-positive B-cells. Based on these data, IGKV1-39 (O12) and IGLV2-14 (2a2) were chosen as light chains as they were well represented in the B-cell repertoire. The J-segment sequence of the light chains has been chosen based upon sequences as presented in GenBank ABA26122 for IGKV1-39 (Rabquer,B.J., Smithson,S.L., Shriner,A.K. and Westerink,M.A.J.) and GenBank AAF20450 for IGLV2-14 (Ignatovich,O., Tomlinson,I.M., Popov,A.V., Bruggemann,M. and Winter,G. J. Mol. Biol. 294 (2), 457-465 (1999)).

All framework segments are converted into germline amino acid sequences to provide the lowest immunogenicity possible in potential clinical applications.

### Example 2

### Obtaining mouse heavy chain V-genes that pair with human IGKV1-39 gene segment to form functional antibody binding sites

This example describes the identification of mouse heavy chain V-genes that are capable of pairing with a single, rearranged human germline IGKV1-39/J region. A spleen VH repertoire from mice that were immunized with tetanus toxoid was cloned in a phage display Fab vector with a single human IGKV1-39-C kappa light chain and subjected to panning against tetanus toxoid. Clones obtained after a single round of panning were analyzed for their binding specificity. The murine VH genes encoding tetanus toxoid-specific Fab fragments were subjected to sequence analysis to identify unique clones and assign VH, DH and JH utilization.

Many of the protocols described here are standard protocols for the construction of phage display libraries and the panning of phages for binding to an antigen of interest and described in Antibody Phage Display: Methods and Protocols (editor(s): Philippa M. O'Brien, Robert Aitken).

### Immunizations

BALB/c mice received one immunization with tetanus toxoid and were boosted after 6 weeks with tetanus toxoid.

### Splenocyte isolation

Preparation of spleen cell suspension. After dissection, the spleen was washed with PBS and transferred to a 60 mm petridish with 20 ml PBS. A syringe capped with 20 ml PBS and a G20 needle was used to repeatedly flush the spleen. After washing the flushed cells with PBS, the cells were carefully brought into suspension using 20 ml PBS and left on a bench for 5 minutes to separate the splenocytes from the debris and cell clusters. The splenocytes suspension was transferred on top of a Ficoll-Paque™ PLUS-filled tube and processed according to the manufacturer's procedures for lymphocyte isolation (Amersham Biosciences).

### RNA isolation and cDNA synthesis

After isolation and pelleting of lymphocytes, the cells were suspended in TRIzol LS Reagent (Invitrogen) for the isolation of total RNA according to the accompanying manufacturer's protocol and subjected to reverse transcription reaction using 1 microgram of RNA, Superscript III RT in combination with dT20 according to manufacturer's procedures (Invitrogen).

### PCR amplification of cDNA

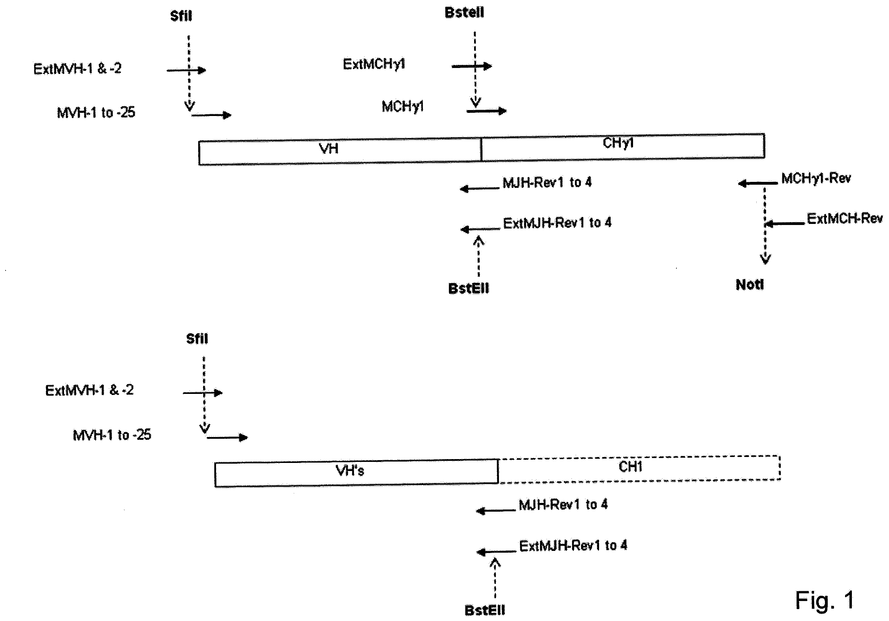

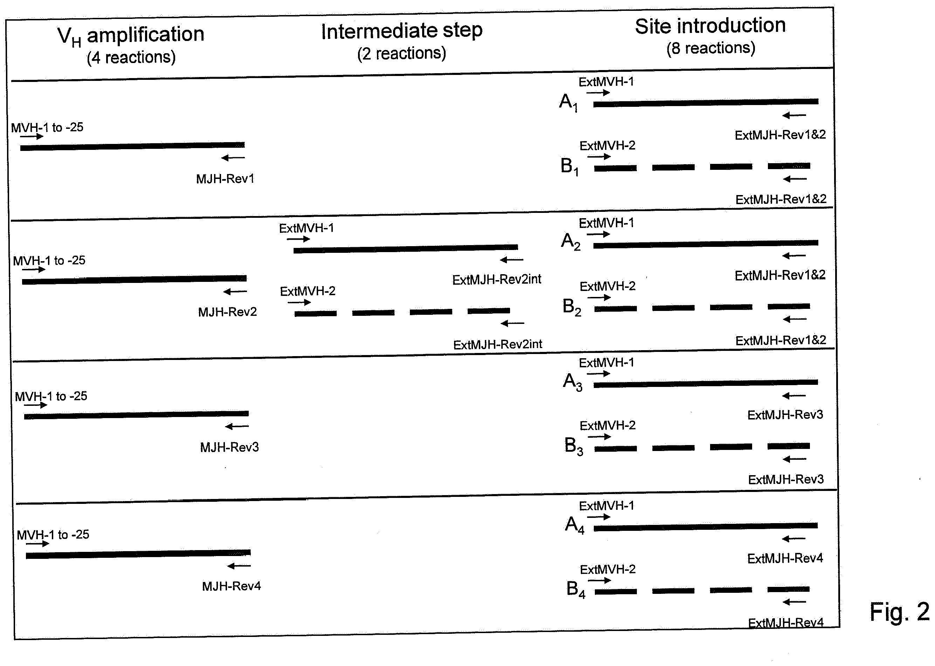

The cDNA was amplified in a PCR reaction using primer combinations that allow the amplification of approximately 110 different murine V-genes belonging to 15 VH families (table 1; RefSeq NG_005838; Thiebe et al., 1999. European Journal of Immunology 29: 2072 - 2081). In the first round, primer combinations that bind to the 5' end of the V-genes and 3' end of the J regions were used. In the second round, PCR products that were generated with the MJH-Rev2 primer were amplified in order to introduce modifications in the 3' region to enable efficient cloning of the products. In the last round of amplification, all PCR products were amplified using primers that introduce a SfiI restriction site at the 5' end and a BstEII restriction site at the 3' end (see figures 1 and 2, and table 1).

Reaction conditions for 1st round PCR: 4 different reactions combining all 25 forward primers (MVH1 to MVH25, table 1 and figure 2) and 1 reverse primer per reaction (MJH-Rev1, MJH-Rev2, MJH-Rev3 or MJH-Rev4; see table 1 and figure 2). 50 microliter PCR volumes were composed of 2 microliter cDNA (from RT reactions), 10 microliter 5* Phusion polymerase HF buffer, 40 nM of each of the 25 forward primers (total concentration of 1 micromolar), 1 micromolar reverse primer, 1 microliter 10 mM dNTP stock, 1.25 unit Phusion polymerase and sterile MQ water. The thermocycler program consisted of a touch down program: 1 cycle 98°C for 30 seconds, 30 cycles 98°C for 10 seconds, 58°C decreasing 0.2°C per cycle 10 seconds, 72°C 20 seconds and 1 cycle 72°C for 3 minutes. The second round PCR program was set up only for the products of the 1st PCR that contain the MJH-Rev2 primer: 2 different reactions combining either the ExtMVH-1 or ExtMVH-2 primers (table 1 and figure 2) in combination with the reverse primer ExtMJH-Rev2int (table 1 and figure 2). 50 microliter PCR volumes were composed of 50 ng PCR product (from first PCR round), 10 microliter 5* Phusion polymerase HF buffer, 500 nM of each forward primer, 1 micromolar reverse primer, 1 microliter 10 mM dNTP stock, 1.25 unit Phusion polymerase and sterile MQ water. The thermocycler program consisted of a touch down program followed by a regular amplification step: 1 cycle 98°C for 30 seconds, 10 cycles 98°C for 10 seconds, 65°C decreasing 1.5°C per cycle 10 seconds, 72°C 20 seconds, 10 cycles 98°C for 10 seconds, 55°C 10 seconds, 72°C 20 seconds and 1 cycle 72°C for 3 minutes. The third round PCR program was setup as described in figure 2. 50 microliter PCR volumes were composed of 50 ng PCR product (from earlier PCR rounds, figure 2), 10 microliter 5* Phusion polymerase HF buffer, 1 micromolar forward primer (table 1 and figure 2), 1 micromolar reverse primer, 1 microliter 10 mM dNTP stock, 1,25 unit Phusion polymerase and sterile MQ water. The program consists of a touch down program followed by a regular amplification step: 1 cycle 98°C for 30 seconds, 10 cycles 98°C for 10 seconds, 65°C decreasing 1.5°C per cycle 10 seconds, 72°C 20 seconds, 10 cycles 98°C for 10 seconds, 55°C 10 seconds, 72°C 20 seconds and 1 cycle 72°C for 3 minutes. After PCR amplifications, all PCR products were gel purified using Qiaex II according to the manufacturer's protocols.

### Restriction enzyme digestions

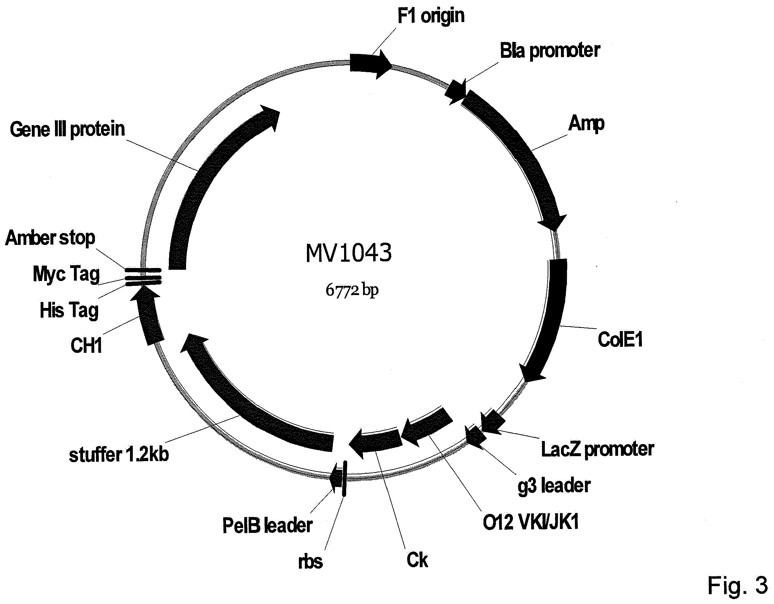

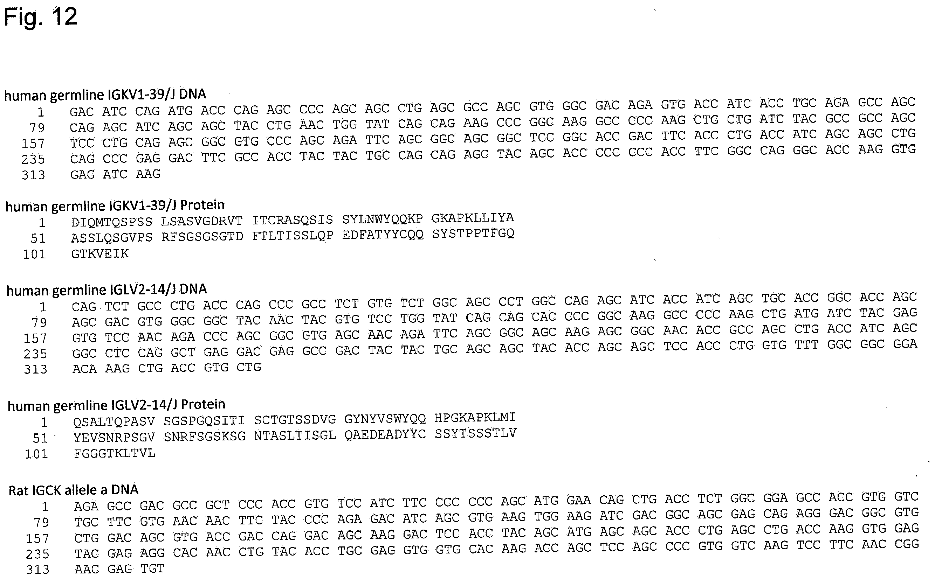

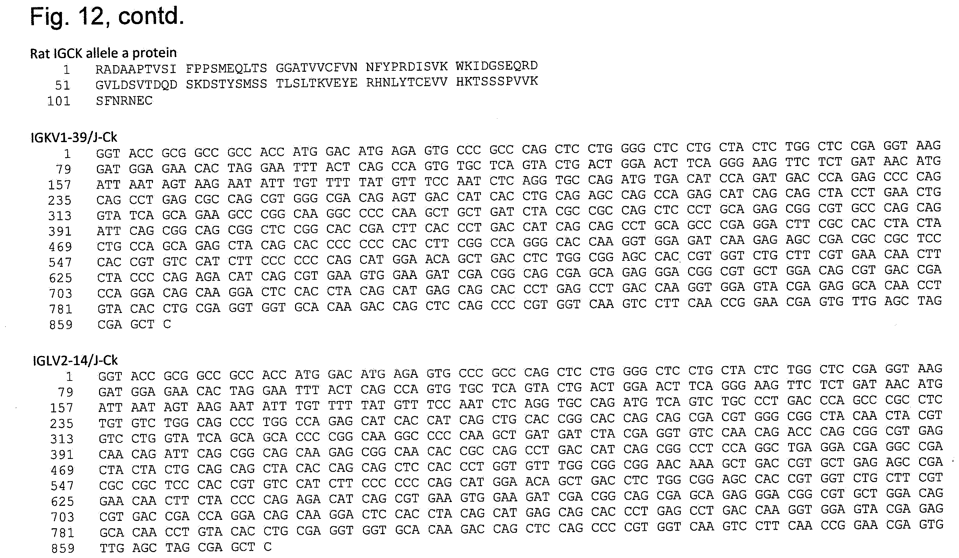

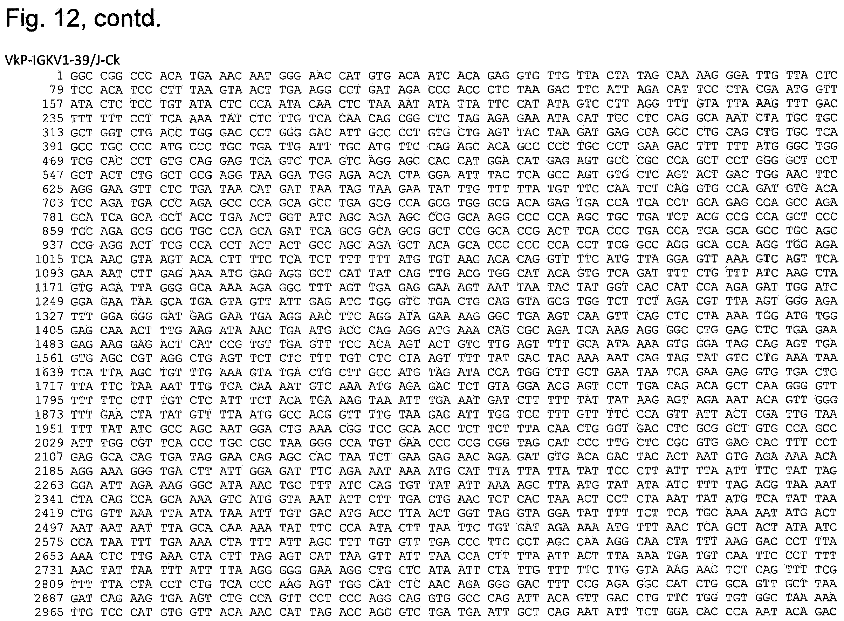

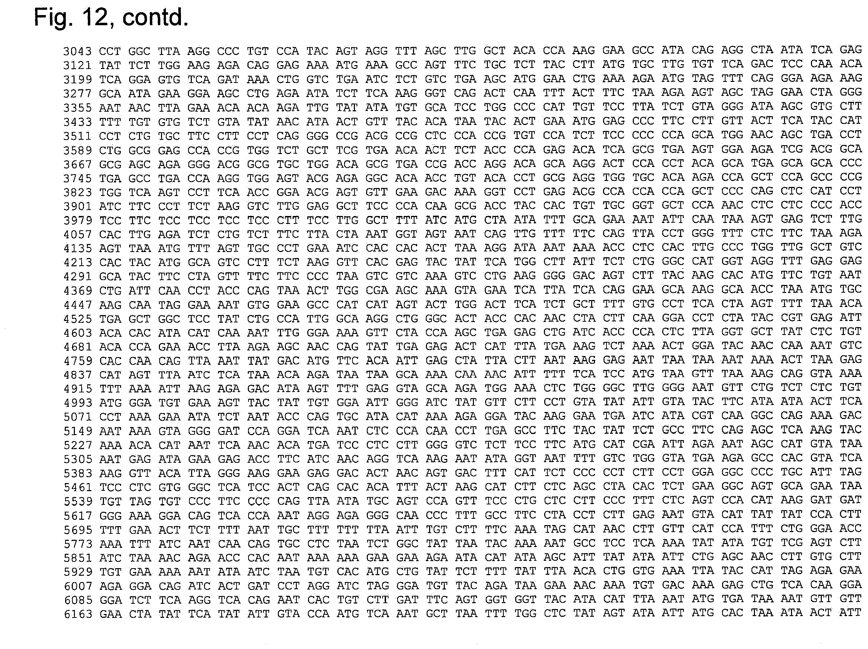

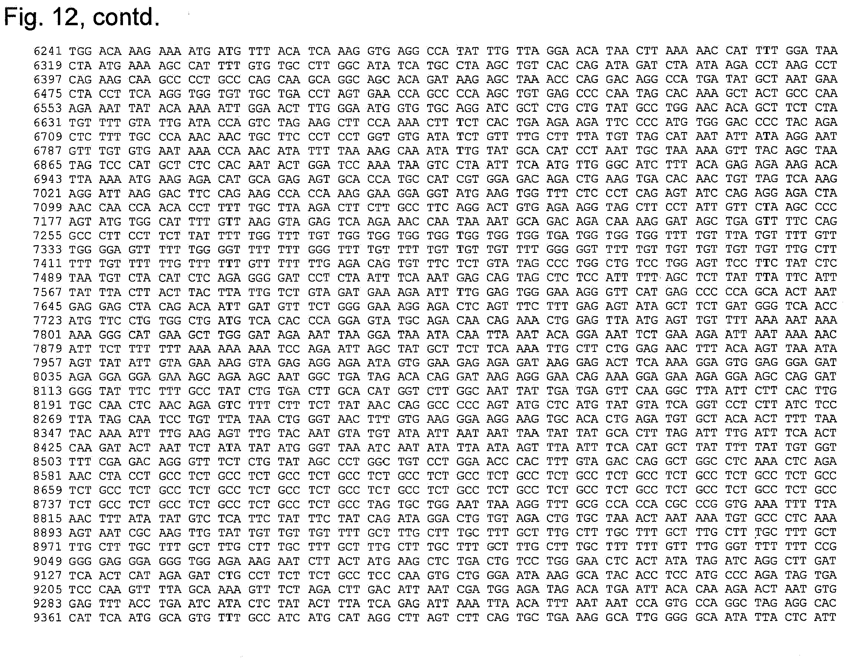

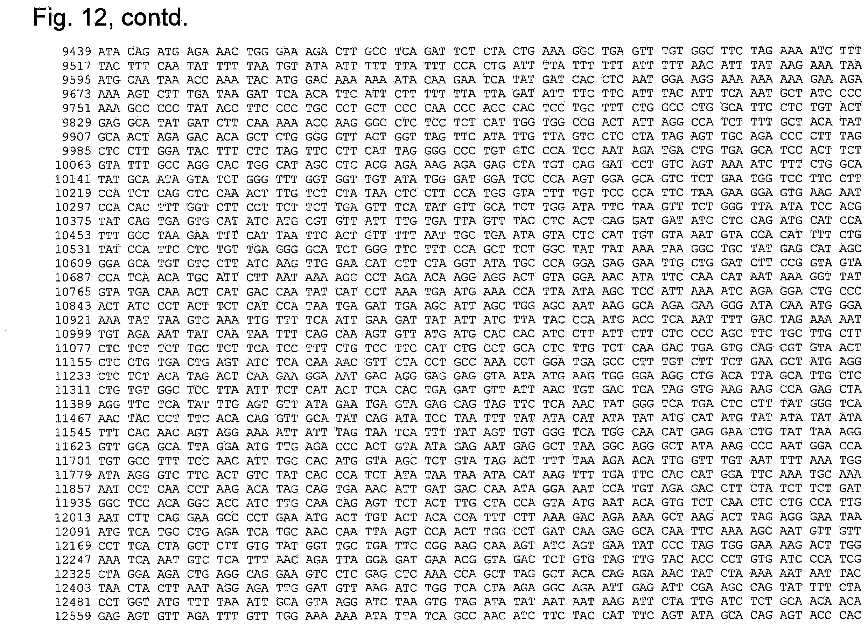

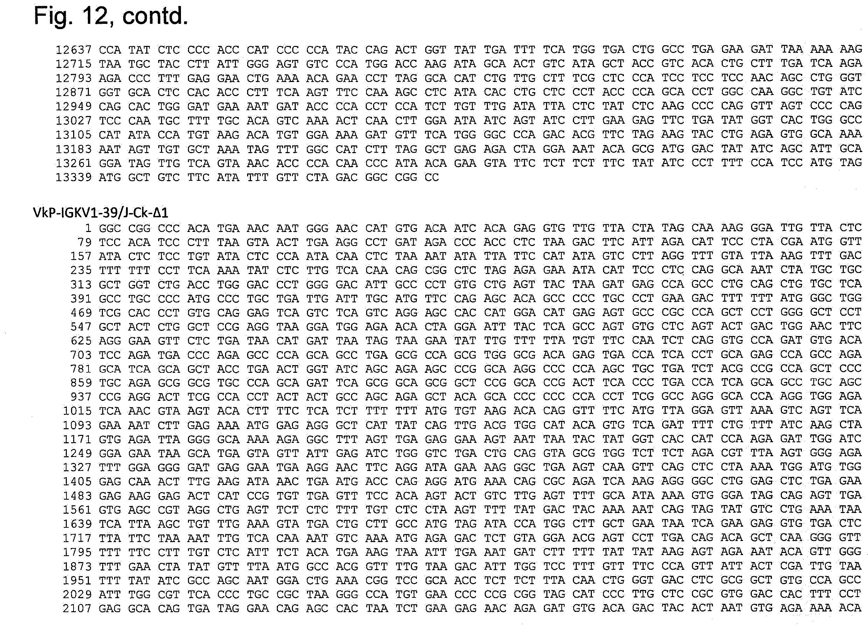

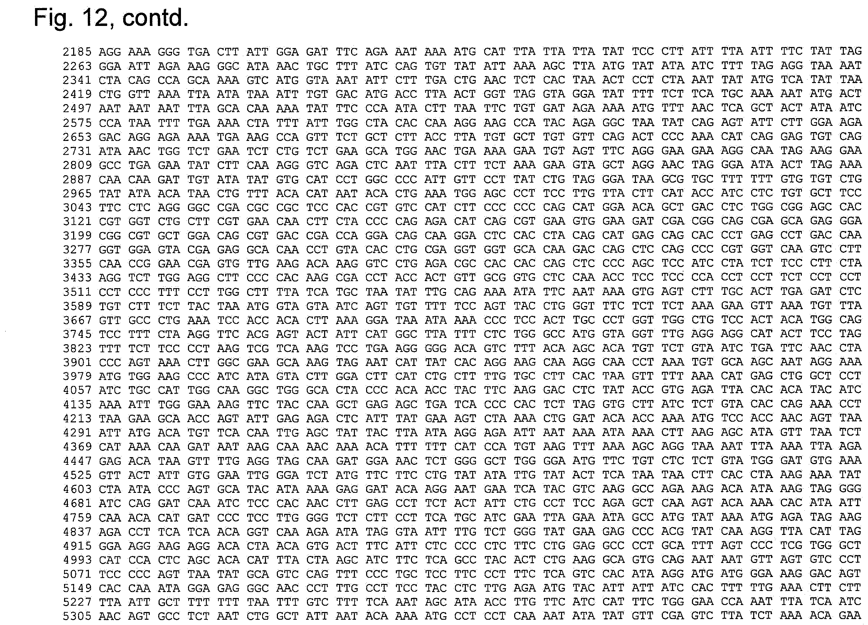

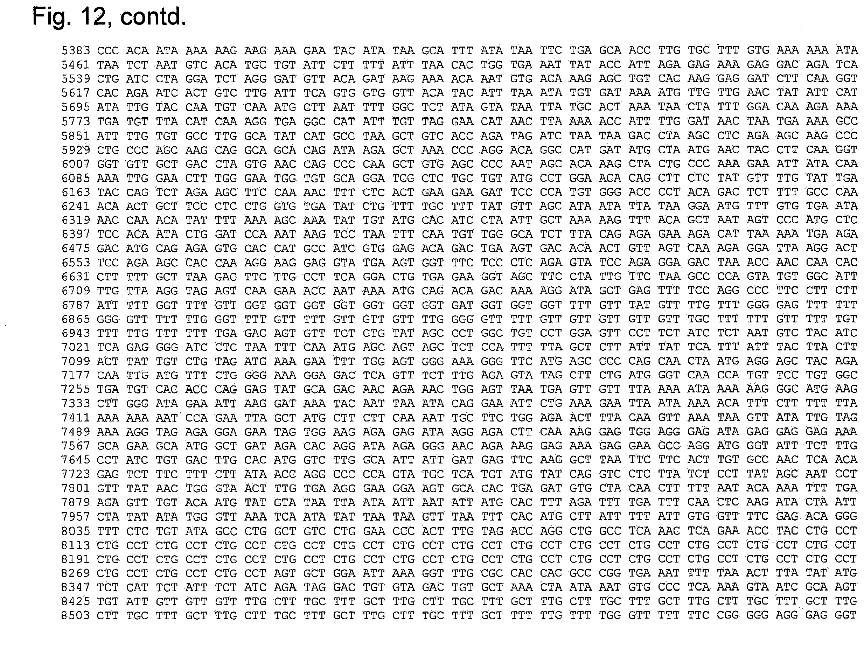

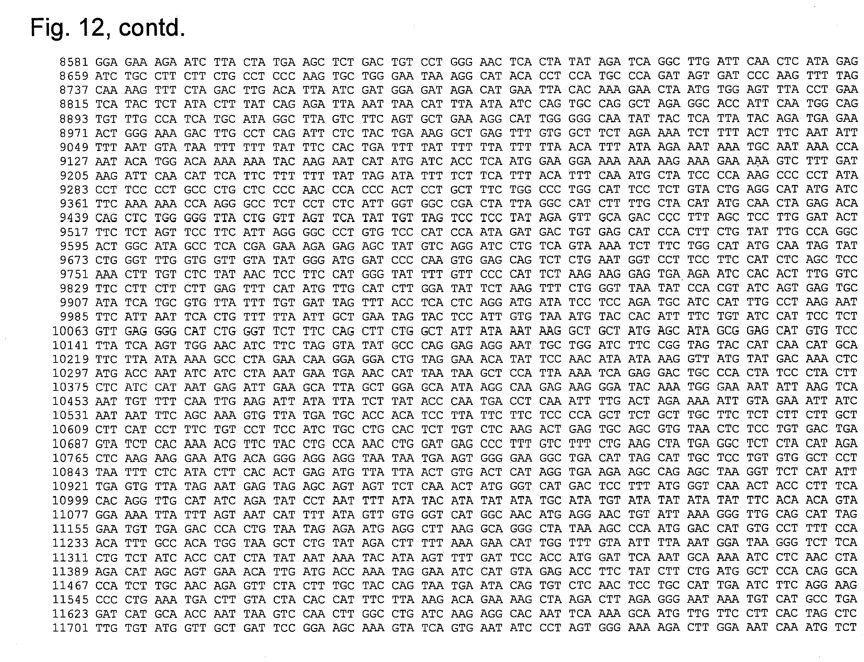

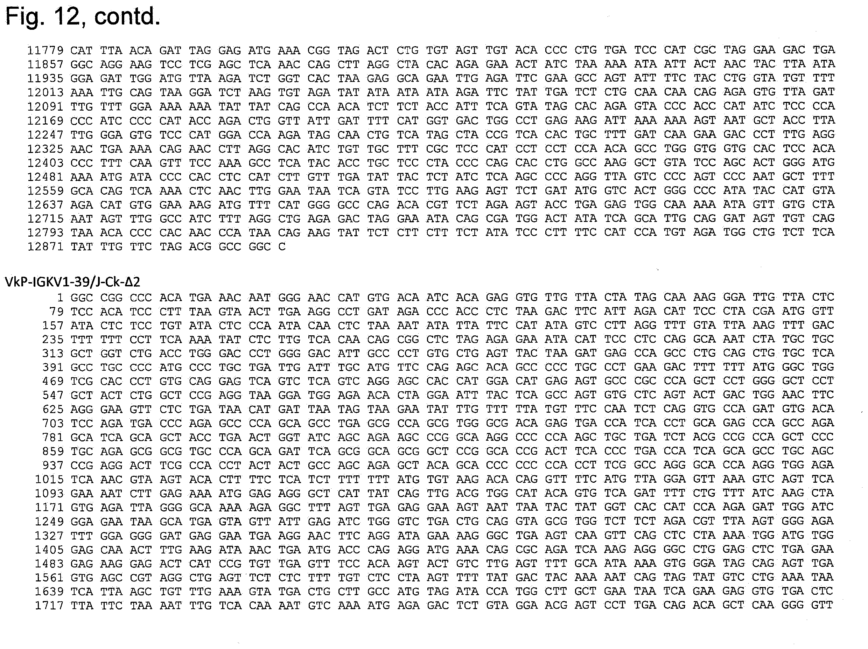

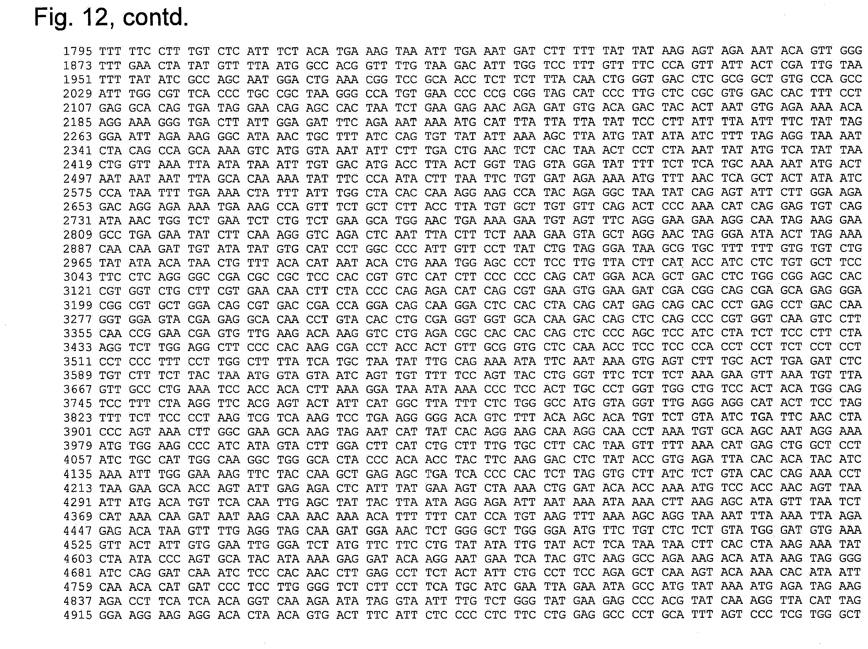

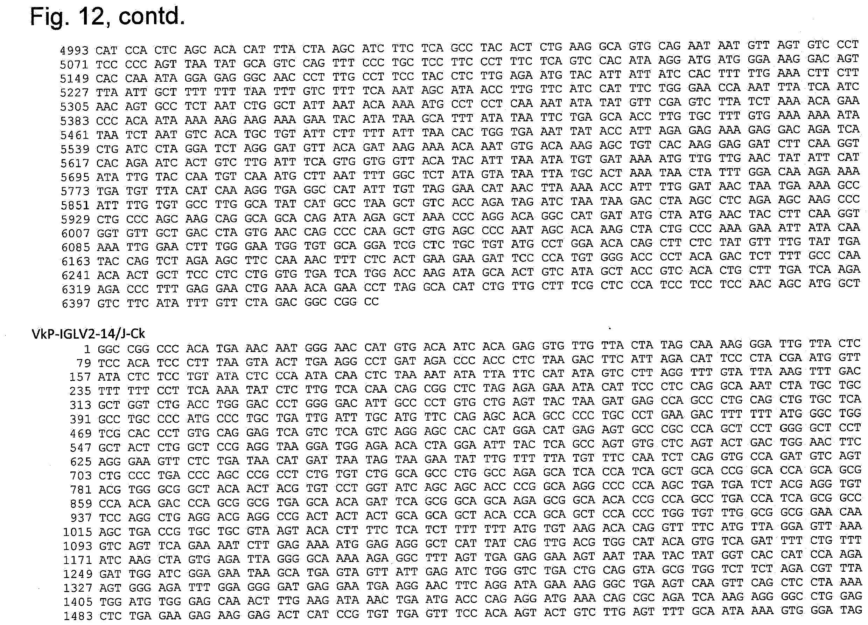

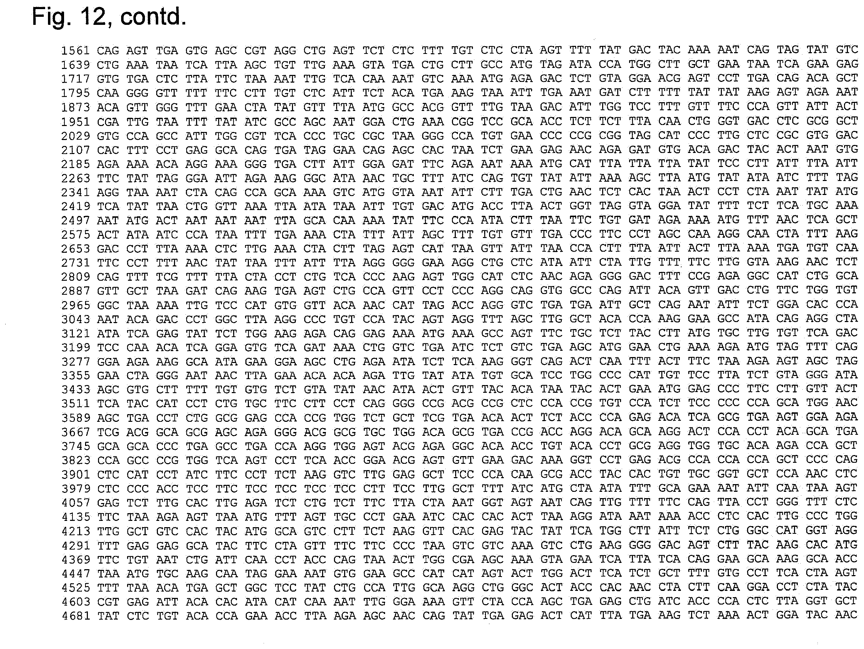

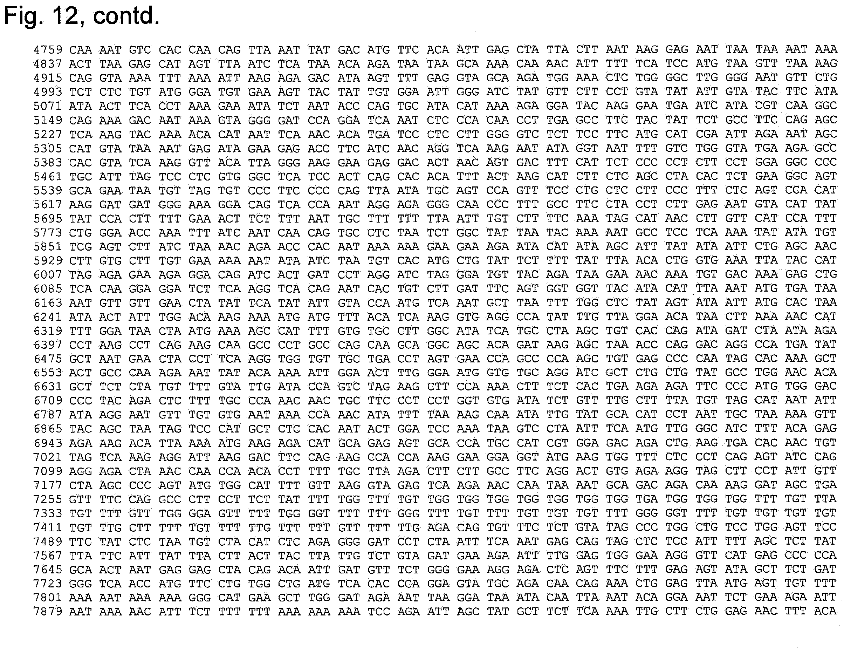

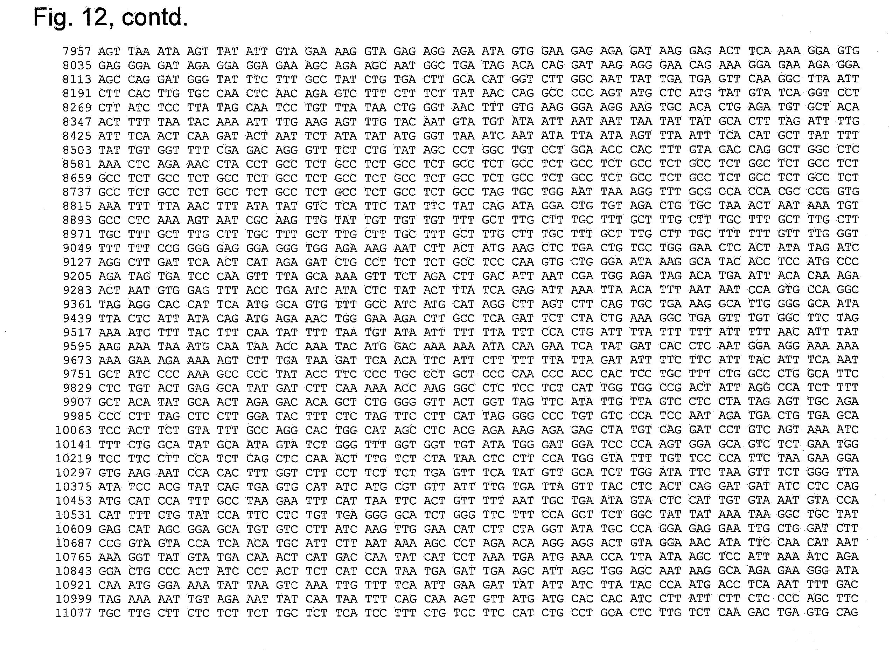

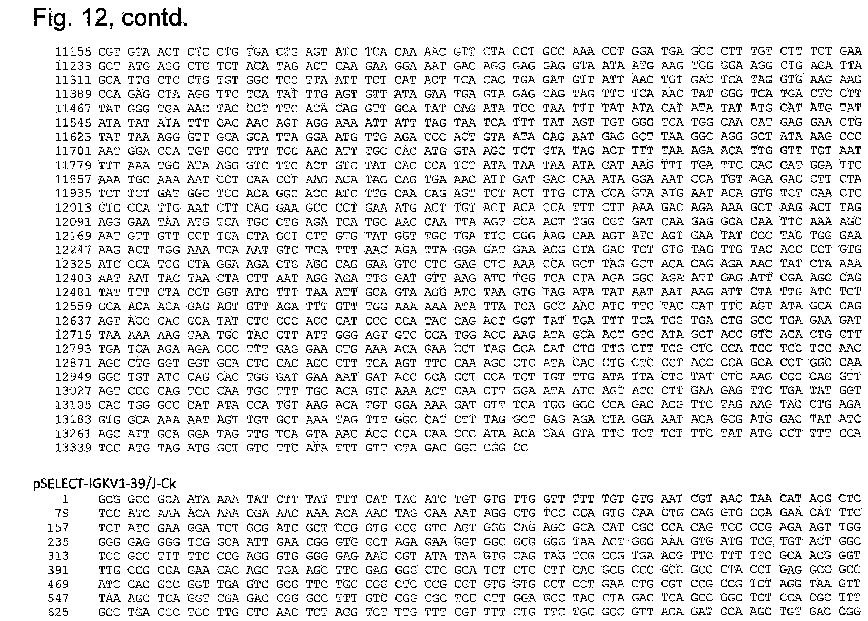

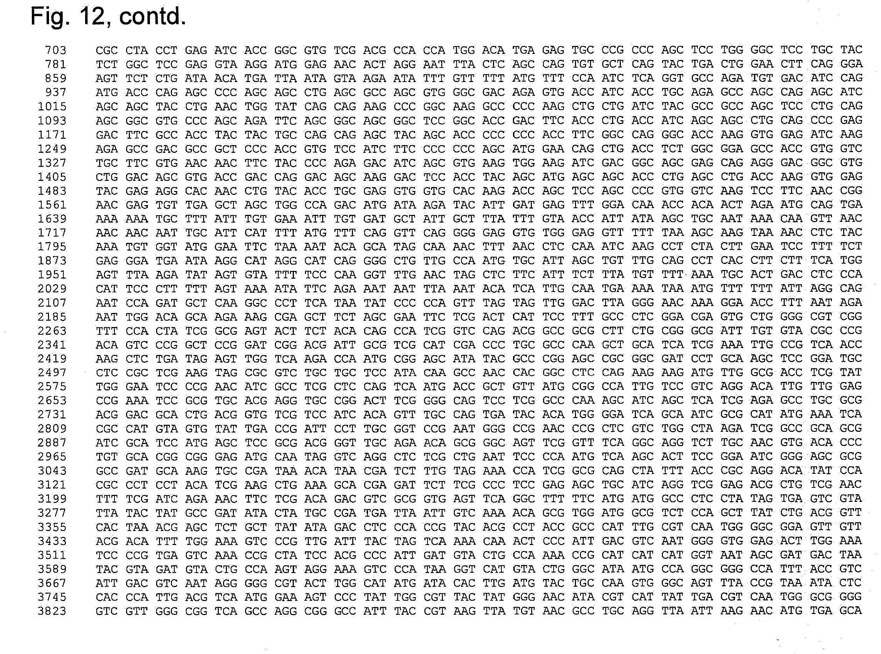

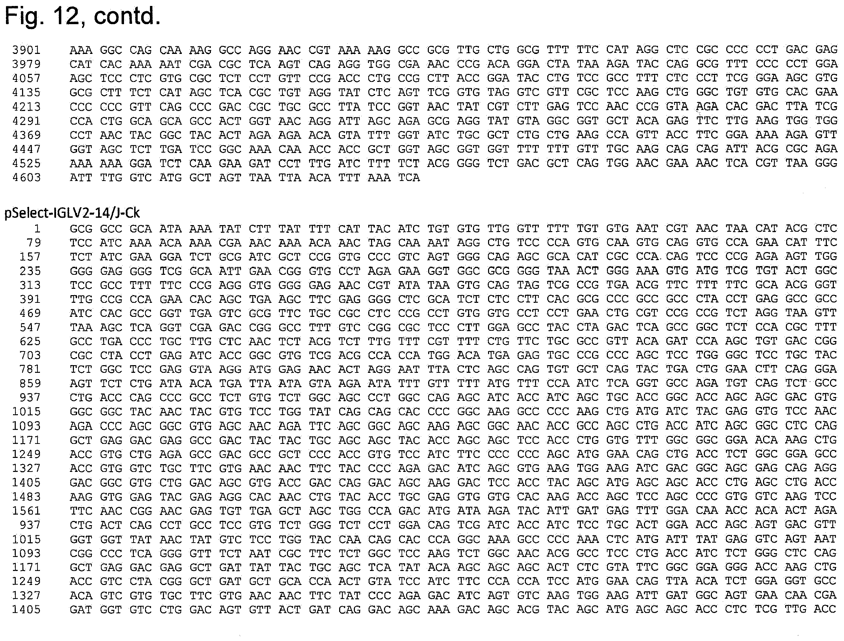

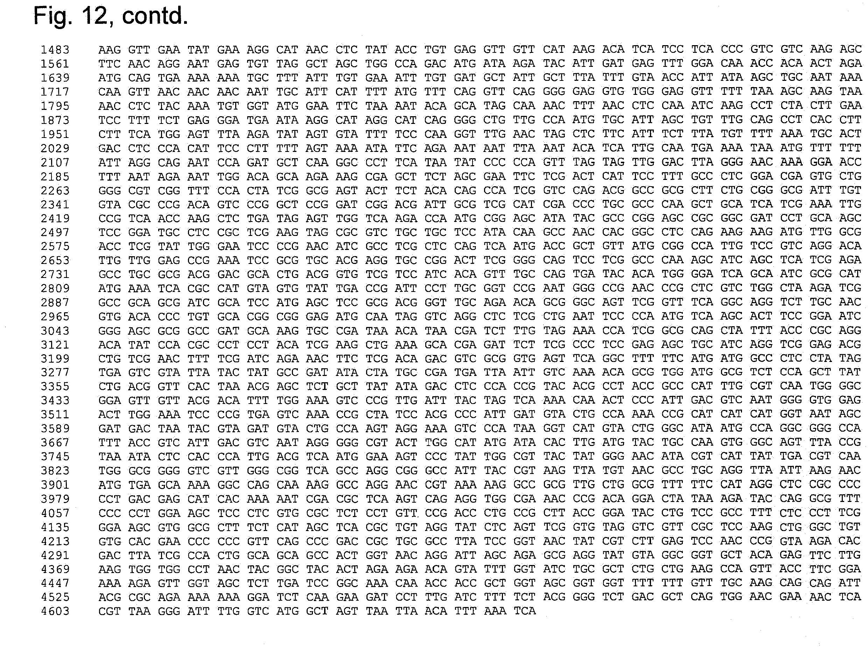

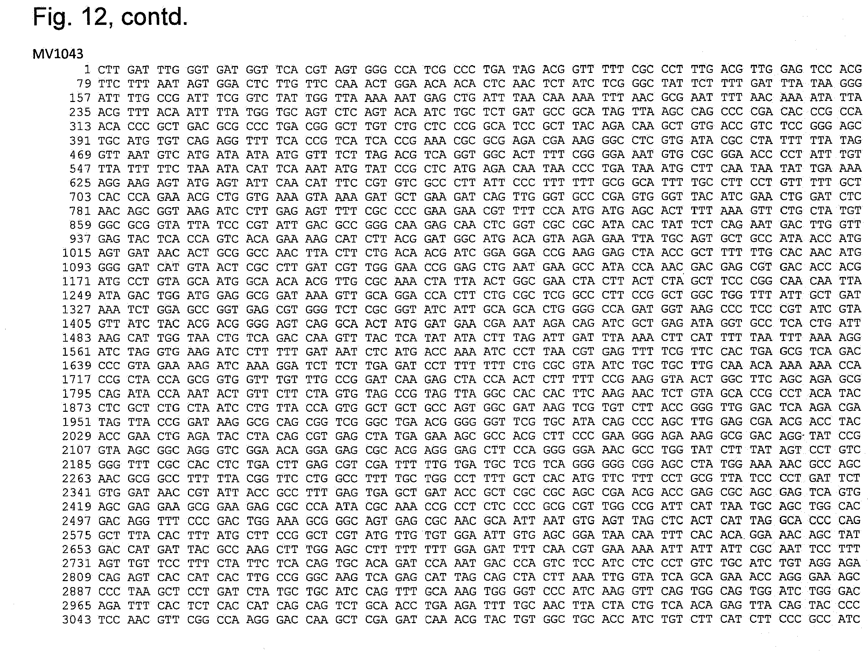

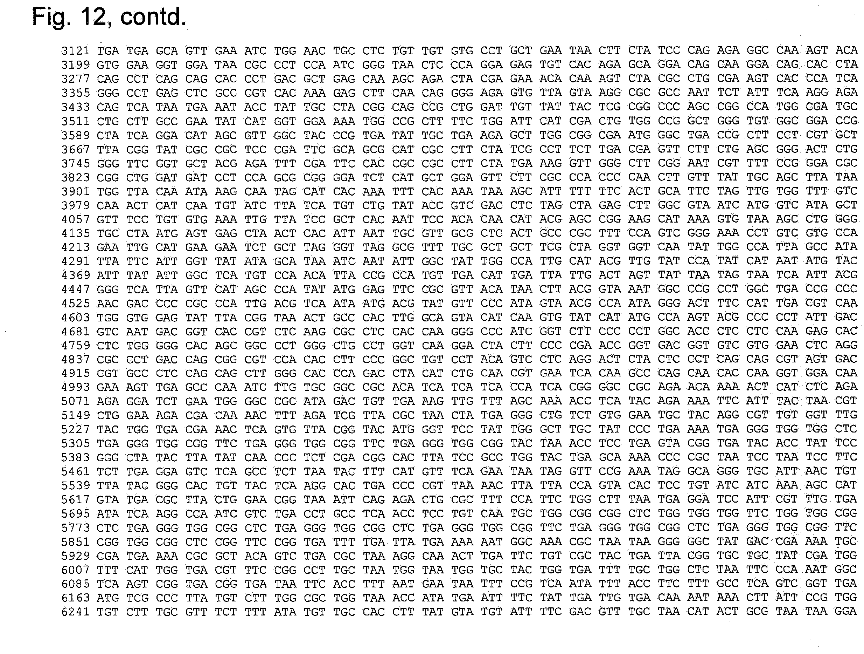

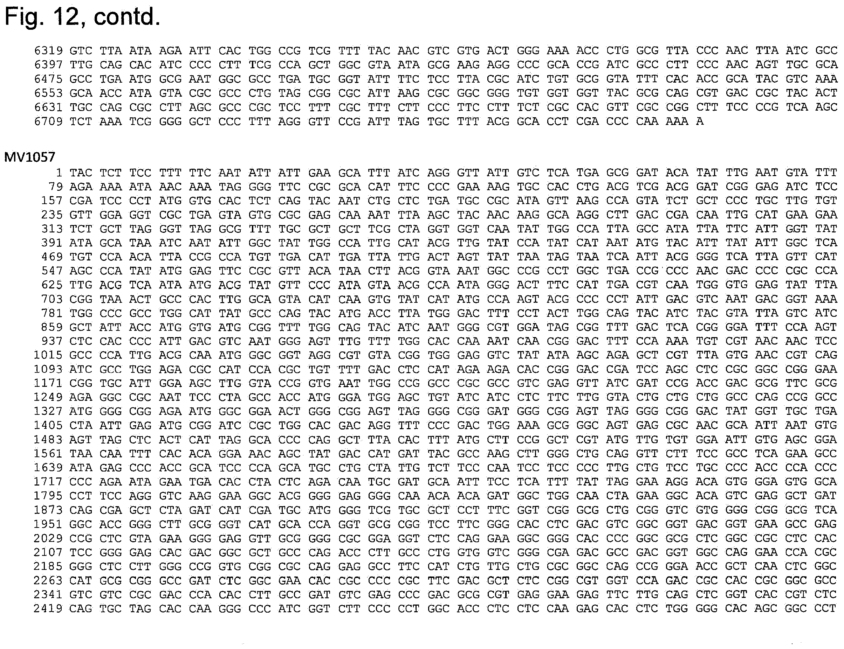

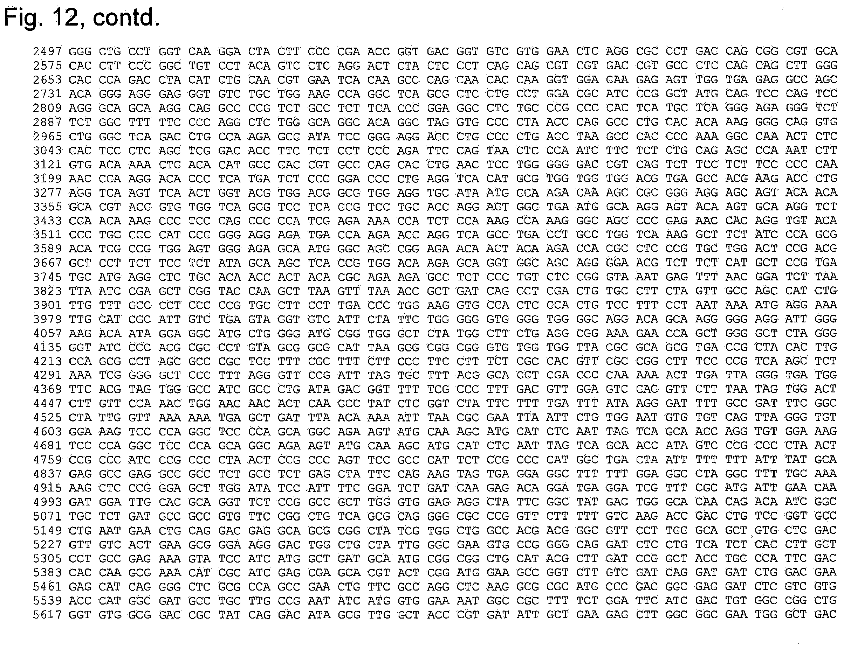

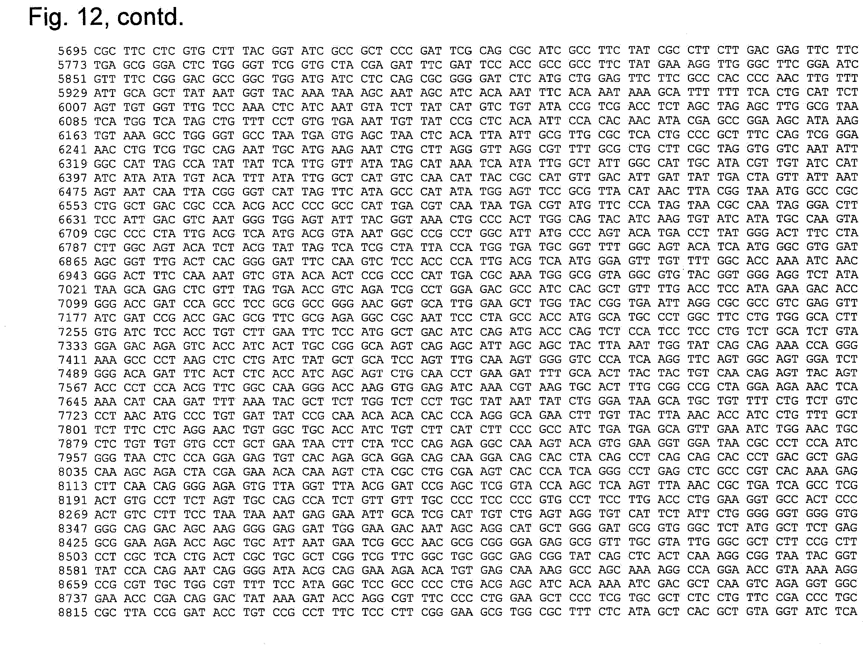

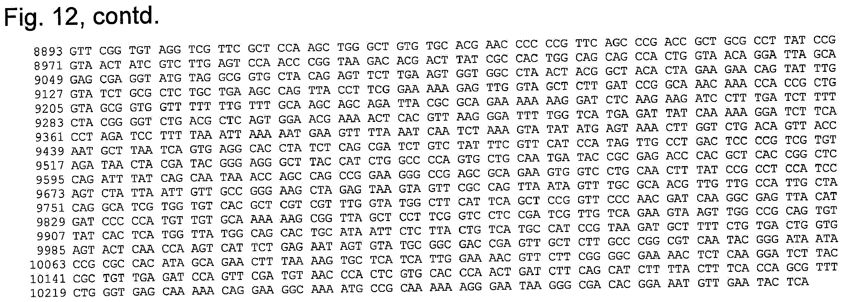

Purified products were digested with BstEII and SfiI in two steps. First 1 microgram of DNA was digested in 100 microliter reactions consisting of 10 microliters of 10* NEB buffer 3 (New England Biolabs), 1 microliter 100* BSA, 12,5 unit BstEII and sterile water for 6 hours at 60°C in a stove. The products were purified using Qiaquick PCR Purification kit from Qiagen according to the manual instructions and eluted in 40 microliter water. Next all products were further digested with SfiI in 100 microliter reactions consisting of 10 microliters of 10* NEB buffer 2 (New England Biolabs), 1 microliter 100* BSA, 12.5 unit SfiI and sterile water for 12 hours at 50°C in a stove. The digested fragments were purified by Qiaquick Gel Extraction kit following gel separation on a 20 cm 1.5% agarose TBE plus ethidium bromide gel at 80 V. 100 microgram of the acceptor vector (MV1043, figures 3 and 12) was digested with 50 units Eco91I in 600 microliter under standard conditions (Tango buffer) and next purified on a 0.9% agarose gel. After a second digestion step under prescribed conditions with 400 units SfiI in 500 microliter for 12 hours, 100 units BsrGI were added for 3 hours at 50°C.

### Ligations

Each PCR product was ligated separately according to the following scheme: 70 ng digested PCR products, 300 ng digested acceptor vector, 100 units T4 Ligase (NEB), 1* ligase buffer in 30 microliters for 16 hours at 12°C, The ligation reactions were purified with phenol/chloroform/isoamyl alcohol extractions followed by glycogen precipitations (Sigma-Aldrich #G1767) according to the manufacturer' s protocol and finally dissolved in 25 microliter sterile water.

### Transformations and library storage

The purified ligation products were transformed by electroporation using 1200 microliter TG1 electrocompetent bacteria (Stratagene #200123) per ligation batch and plated on LB carbenicillin plates containing 4% glucose. Libraries were harvested by scraping the bacteria in 50 ml LB carbenicillin. After centrifugation at 2000g for 20 minutes at 4°C, the bacterial pellets were resuspended carefully in 2 ml ice cold 2*TY/30% glycerol on ice water and frozen on dry-ice/ethanol before storage at -80°C.

### Library amplification

Libraries were grown and harvested according to procedures as described by Kramer et al. 2003 (Kramer et al. 2003. Nucleic Acids Res. 31(11): e59) using VCSM13 (Stratagene) as helper phage strain.

### Selection of phages on coated immunotubes

Tetanus toxoid was dissolved in PBS in a concentration of 2 µg/ml and coated to MaxiSorp Nunc-Immuno Tube (Nunc 444474) overnight at 4°C. After discarding the coating solution, the tubes were blocked with 2% skim milk (ELK) in PBS (blocking buffer) for 1 hour at RT. In parallel, 0.5 ml of the phage library was mixed with 1 ml blocking buffer and incubated for 20 minutes at room temperature. After blocking the phages, the phage solution was added to the tetanus toxoid coated tubes and incubated for 2 hours at RT on a slowly rotating platform to allow binding. Next, the tubes were washed 10 times with PBS/0.05% Tween-20 followed by phage elution by an incubation with 1 ml 50 mM glycine-HCl pH 2.2 10 min at RT on rotating wheel and directly followed by neutralization of the harvested eluent with 0.5 ml 1 M Tris-HCl pH 7.5.

Harvesting phage clones 5 ml XL1-Blue MRF (Stratagene) culture at O.D. 0.4 was added to the harvested phage solution and incubated for 30 minutes at 37°C without shaking to allow infection of the phages. Bacteria were plated on Carbenicillin/Tetracycline 4% glucose 2*TY plates and grown overnight at 37°C.

### Phage production

Phages were grown and processed as described by Kramer et al. 2003 (Kramer et al, 2003. Nucleic Acids Res. 31(11): e59) using VCSM13 as helper phage strain.

### Phage ELISA

ELISA plates were coated with 100 microliter tetanus toxoid per well at a concentration of 2 microgram/ml in PBS overnight at 4°C. Plates coated with 100 microliter thyroglobulin at a concentration of 2 microgram/ml in PBS were used as a negative control. Wells were emptied, dried by tapping on a paper towel, filled completely with PBS-4% skimmed milk (ELK) and incubated for 1 hour at room temperature to block the wells. After discarding the block solution, phage minipreps pre-mixed with 50 µl blocking solution were added and incubated for 1 hour at RT. Next 5 washing steps with PBS-0.05% Tween-20 removed unbound phages. Bound phages were detected by incubating the wells with 100 microliter anti-M13-HRP antibody conjugate (diluted 1/5000 in blocking buffer) for 1 hour at room temperature. Free antibody was removed by repeating the washing steps as described above, followed by TMB substrate incubation until color development was visible. The reaction was stopped by adding 100 microliter of 2 M H₂SO₄ per well and analyzed on an ELISA reader at 450 nm emission wavelength (Table 2). Higher numbers indicate stronger signals and thus higher incidence of specific binding of the phage-Fab complex.

### Sequencing

Clones that gave signals at least 3 times above the background signal (Table 2) were propagated, used for DNA miniprep procedures (see procedures Qiagen miniPrep manual) and subjected to nucleotide sequence analysis. Sequencing was performed according to the Big Dye 1.1 kit accompanying manual (Applied Biosystems) using a reverse primer (CH1_Rev1, table 1) recognizing a 5' sequence of the CH1 region of the human IgG1 heavy chain (present in the Fab display vector MV1043, figures 3 and 12). Mouse VH sequences of 28 tetanus toxoid binding clones are depicted in Table 3. The results show that the selected murine VH genes belong to different gene families, and different individual members from these gene families are able to pair with the rearranged human IGKV1-39/J VH region to form functional tetanus toxoid-specific antibody binding sites. From the sequence analyses, it was concluded that the murine VH regions utilize a diversity of DH and JH gene segments.

### Example 3

### Silencing of the mouse kappa light chain locus

This example describes the silencing of the mouse endogenous kappa light chain locus. The endogenous kappa locus is modified by homologous recombination in ES cells, followed by the introduction of genetically modified ES cells in mouse embryos to obtain genetically adapted offspring.

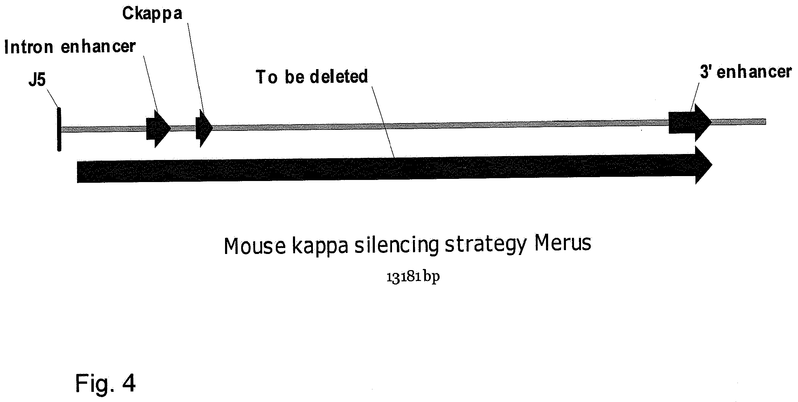

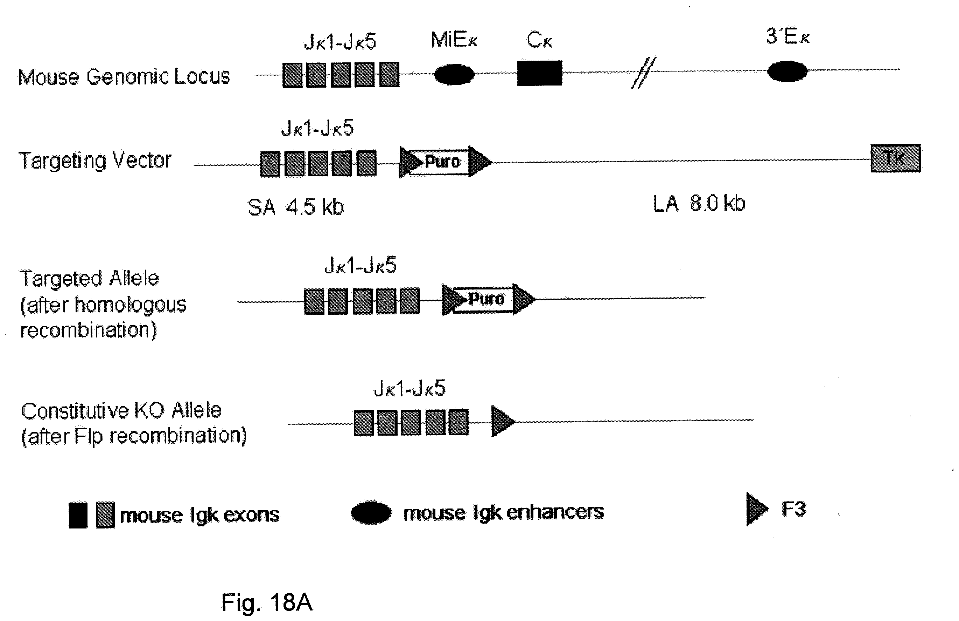

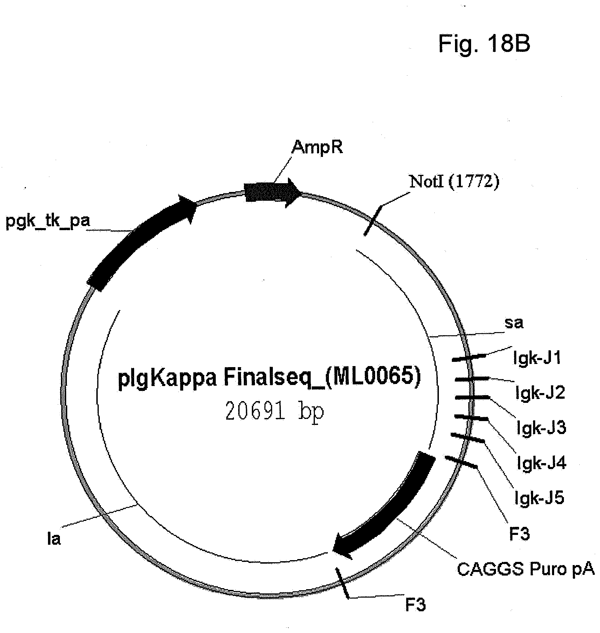

A vector that contains an assembled nucleotide sequence consisting of a part comprising the J-region to 338 bp downstream of the J5 gene segment fused to a sequence ending 3' of the 3' CK enhancer is used for homologous recombination in ES cells. The assembled sequence is used to delete a genomic DNA fragment spanning from 3' of the JK region to just 3' of the 3' CK enhancer. As a consequence of this procedure, the CK constant gene, the 3' enhancer and some intergenic regions are removed (see figures 4 and 18).

### Construction of the targeting vector

A vector that received 4.5-8 kb flanking arms on the 3' and 5' end fused to the deletion segment was used for targeted homologous recombination in an ES cell line. Both arms were obtained by PCR means ensuring maximum homology. The targeting strategy allows generation of constitutive KO allele. The mouse genomic sequence encompassing the Igk intronic enhancer, Igk constant region and the Igk 3' enhancer was replaced with a PuroR cassette, which was flanked by F3 sites and inserted downstream of the Jk elements. Flp-mediated removal of the selection marker resulted in a constitutive KO allele. The replacement of the Igk MiEk-Igk C-Igk 3'E genomic region (approx. 10 kb) with a F3-Puro cassette (approx. 3 kb) was likely to decrease the efficiency of homologous recombination. Therefore, the arms of homology were extended accordingly and more ES cell colonies were analysed after transfection in order to identify homologous recombinant clones.

### Generation of ES cells bearing the deleted kappa fragment

The generation of genetically modified ES cells was essentially performed as described (Seibler et al. Nucleic Acids Res. 2003 Feb 15;31(4):e12). See also example 14 for a detailed description.

Generation of ES mice by tetraploid embryo complementation.

The production of mice by tetraploid embryo complementation using genetically modified ES cells was essentially performed as described (Eggan et al., PNAS 98, 6209-6214; Seibler J, et al. Nucleic Acids Res. 2003 Feb 15;31(4):e12; Hogan et al., (Summary of mouse development. Manipulating the Mouse Embryo, (1994) Cold Spring Harbor Laboratory Press, Cold Spring Harbor NY.), pp. 253-289.)).

### Example 4

### Silencing of the mouse lambda light chain locus

This example describes the silencing of the mouse endogenous lambda light chain locus. The endogenous lambda locus is modified by homologous recombination in ES cells followed by the introduction of genetically modified ES cells in mouse embryos to obtain genetically adapted offspring.

Two regions of the murine lambda locus that together contain all functional lambda V regions are subject to deletion.

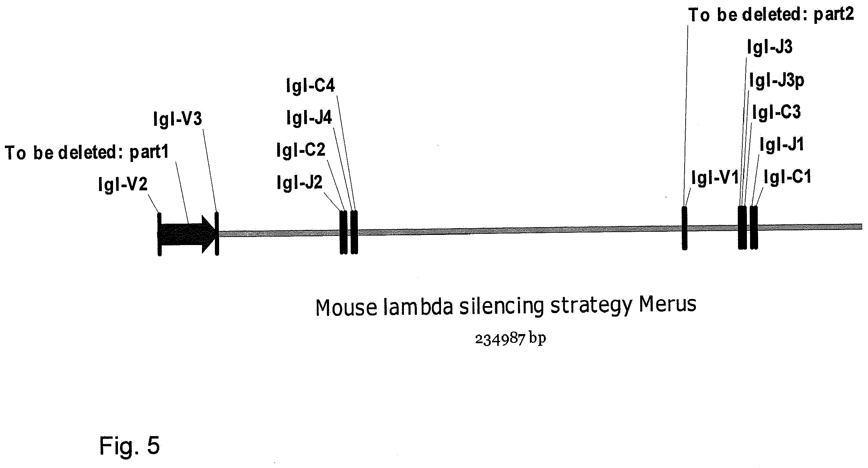

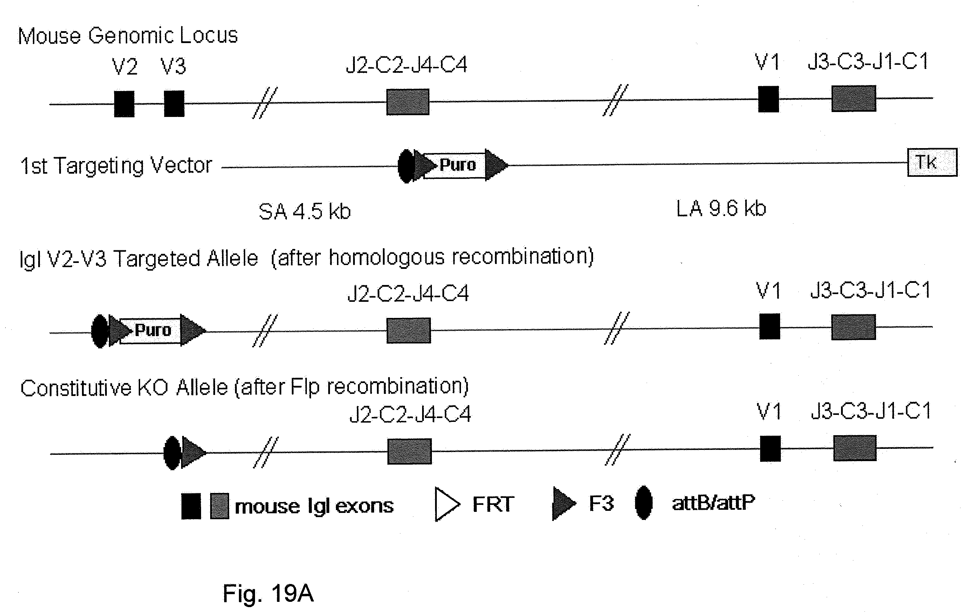

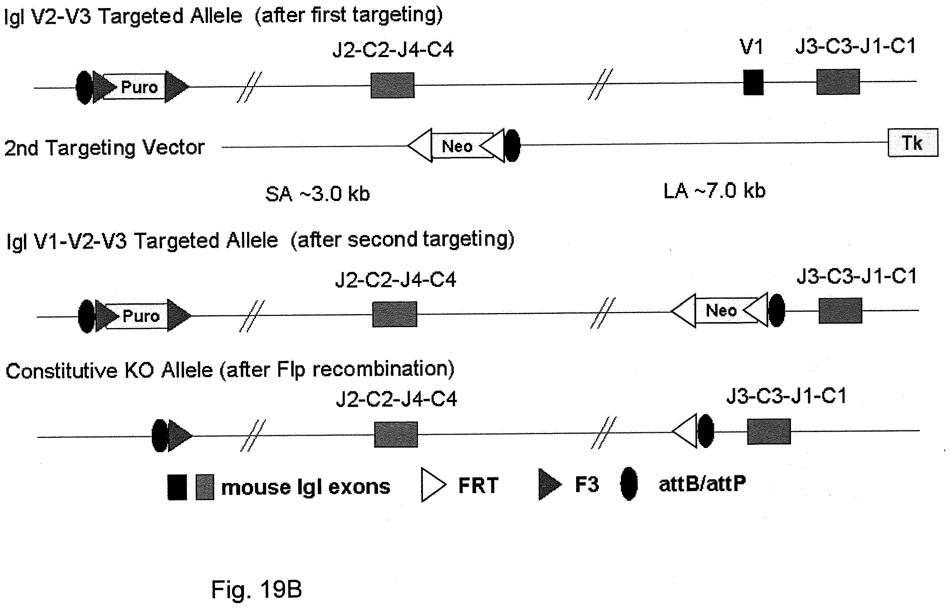

The first region targeted for homologous recombination-based deletion is a region that is located 408 bp upstream of the start site of the IGLV2 gene segment and ends 215 bp downstream of IGLV3 gene segment, including the intergenic sequence stretch between these IGLV gene segments. The second region that is subject to a deletion involves the IGLV1 gene segment consisting of a fragment spanning from 392 bp upstream to 171 bp downstream of the IGLV1 gene segment. As a consequence of these two deletion steps, all functional V-lambda genes segments are deleted, rendering the locus functionally inactive (figures 5 and 19).

### Construction of the targeting vectors

Vectors that received 3-9.6 kb flanking arms on the 3' and 5' end fused to the deletion segment were used for targeted homologous recombination in an ES cell line. Both arms were obtained by PCR means ensuring maximum homology. In a first step, the mouse genomic sequence encompassing the Igl V2-V3 regions were replaced with a PuroR cassette flanked by F3 sites, which yields a constitutive KO allele after Flp-mediated removal of selection marker (see figure 19A). In a second step, the mouse genomic sequence encompassing the Igl V1 region was replaced with a Neo cassette in ES cell clones which already carried a deletion of the Igl V2-V3 regions (see figure 19B). The selection marker (NeoR) was flanked by FRT sites. A constitutive KO allele was obtained after Flp-mediated removal of selection markers.

### Generation of ES cells bearing the deleted lambda fragment

The generation of genetically modified ES cells was essentially performed as described (Seibler J, Zevnik B, Küter-Luks B, Andreas S, Kern H, Hennek T, Rode A, Heimann C, Faust N, Kauselmann G, Schoor M, Jaenisch R, Rajewsky K, Kuhn R, Schwenk F. Nucleic Acids Res. 2003 Feb 15;31(4):e12). See also example 14 for a detailed description. To show that both targeting events occurred on the same chromosome several double targeted clones were selected for the in vitro deletion with pCMV C31deltaCpG. The clones were expanded under antibiotic pressure on a mitotically inactivated feeder layer comprised of mouse embryonic fibroblasts in DMEM High Glucose medium containing 20% FCS (PAN) and 1200 u/mL Leukemia Inhibitory Factor (Millipore ESG 1107). 1x10⁷ cells from each clone were electroporated with 20 µg of circular pCMV C31deltaCpG at 240 V and 500 µF and plated on four 10 cm dishes each. 2 - 3 days after electroporation cells were harvested and analysed by PCR. Primers used were:
2005_5: CCCTTTCCAATCTTTATGGG
2005_7: AGGTGGATTGGTGTCTTTTTCTC
2005_9: GTCATGTCGGCGACCCTACGCC
PCR reactions were performed in mixtures comprising 5 µl PCR Buffer 10x (Invitrogen), 2 µl MgCl₂ (50 mM), 1 µl dNTPs (10 mM), 1 µl first primer (5 µM), 1 µl second primer (5 pM), 0,4 µl Taq (5 U/ul, Invitrogen), 37,6µl HzO, and 2 µl DNA. The program used was 95°C 5'; followed by 35 cycles of 95°C 30"; 60°C 30"; 72°C 1'; followed by 72°C 10'

### Generation of ES mice by tetraploid embryo complementation.

The production of mice by tetraploid embryo complementation using genetically modified ES cells was essentially performed as described (Eggan et al., PNAS 98, 6209-6214; Seibler J, Zevnik B, Küter-Luks B, Andreas S, Kern H, Hennek T, Rode A, HeimannC, Faust N, Kauselmann G, Schoor M, Jaenisch R, Rajewsky K, Kuhn R, Schwenk F. Nucleic Acids Res. 2003 Feb 15;31(4):e12; Hogan et al., (Cold Spring Harbor Laboratory Press, Cold Spring Harbor NY.), pp. 253-289).

### Example 5

### Construction of the CAGGS expression insert based on a rearranged human germline IGKV1-39/J-Ck gene (IGKV1-39/J-Ck)

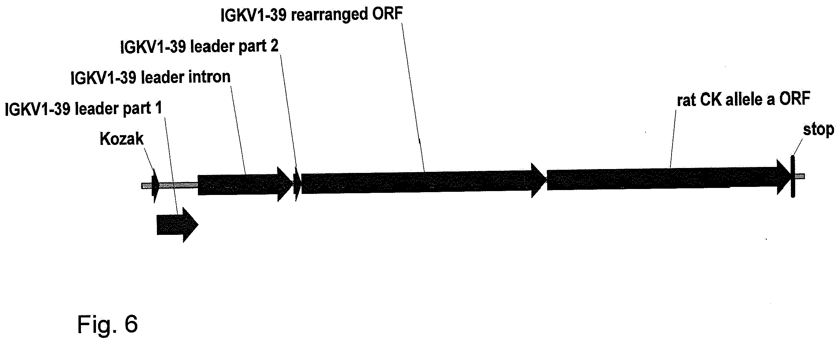

This example describes the construction of a CAGGS expression cassette incorporating the rearranged human germline IGKV1-39/J region. This insert expression cassette encompasses cloning sites, a Kozak sequence, a leader sequence containing an intron, an open reading frame of the rearranged IGKV1-39 region, a rat CK constant region from allele a and a translational stop sequence (IGKV1-39/J-Ck; figure 6). The primary construct consists of naturally occurring sequences and has been analyzed and optimized by removing undesired cis acting elements like internal TATA-boxes, poly adenylation signals, chi-sites, ribosomal entry sites, AT-rich or GC-rich sequence stretches, ARE-, INS- and CRS sequence elements, repeat sequences, RNA secondary structures, (cryptic) splice donor and acceptor sites and splice branch points (GeneArt GmbH). In addition, the codon usage in the open reading frame regions is optimized for expression in mice. The intron sequence is unchanged and thus represents the sequence identical to the coding part of the human IGKV1-39 leader intron.

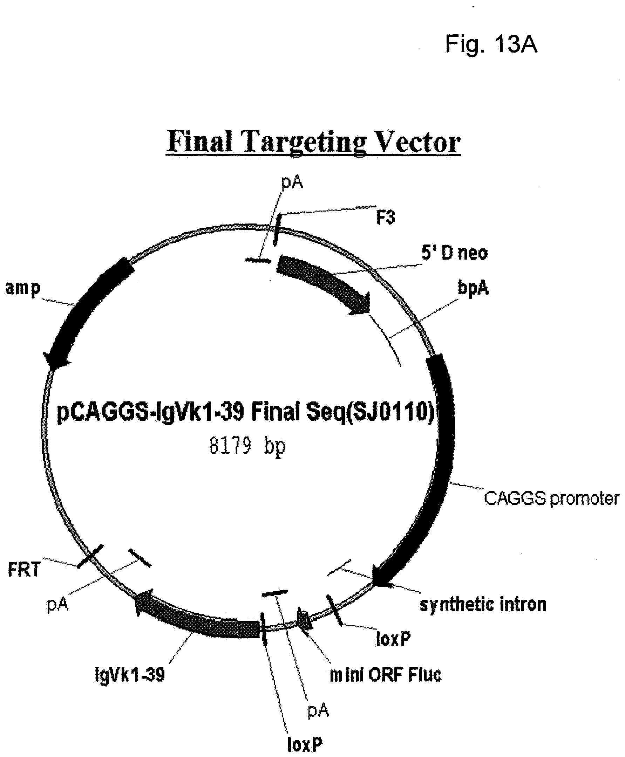

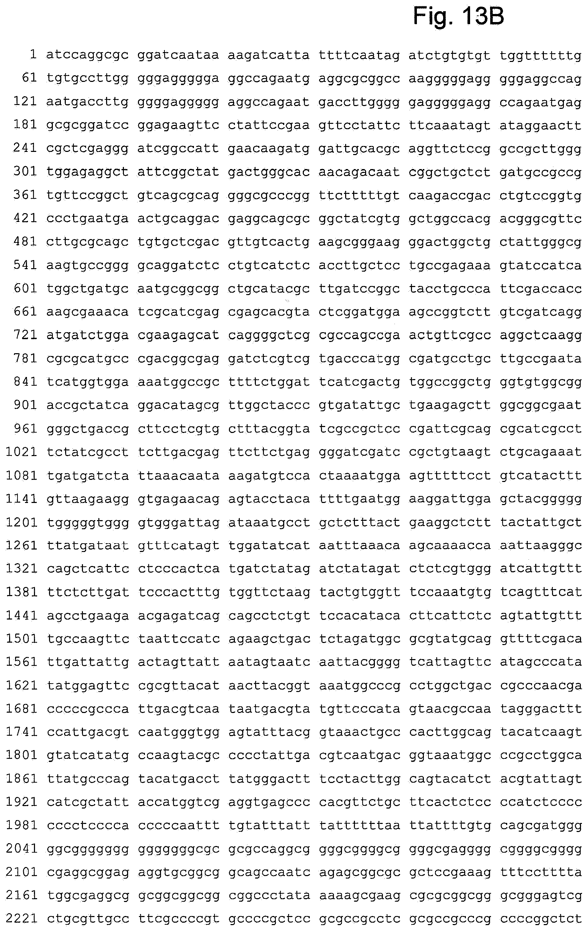

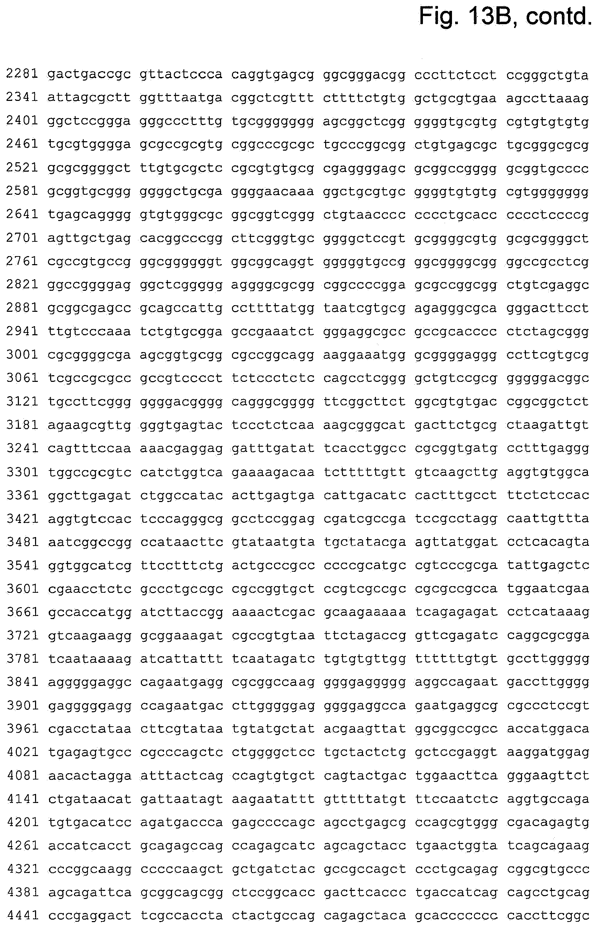

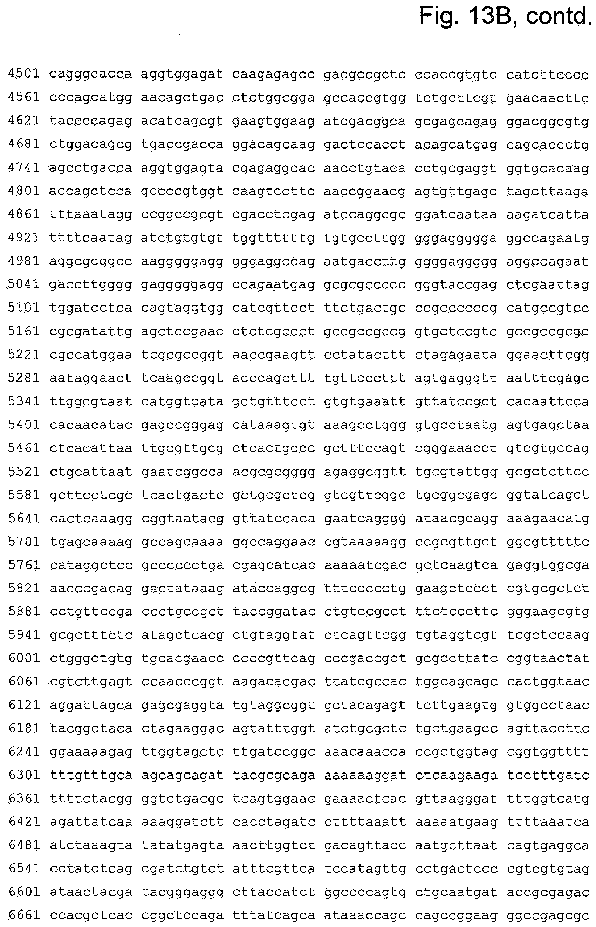

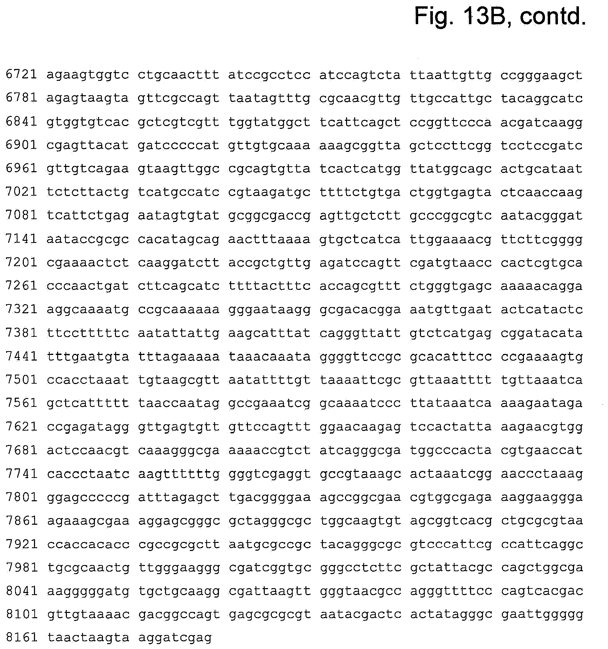

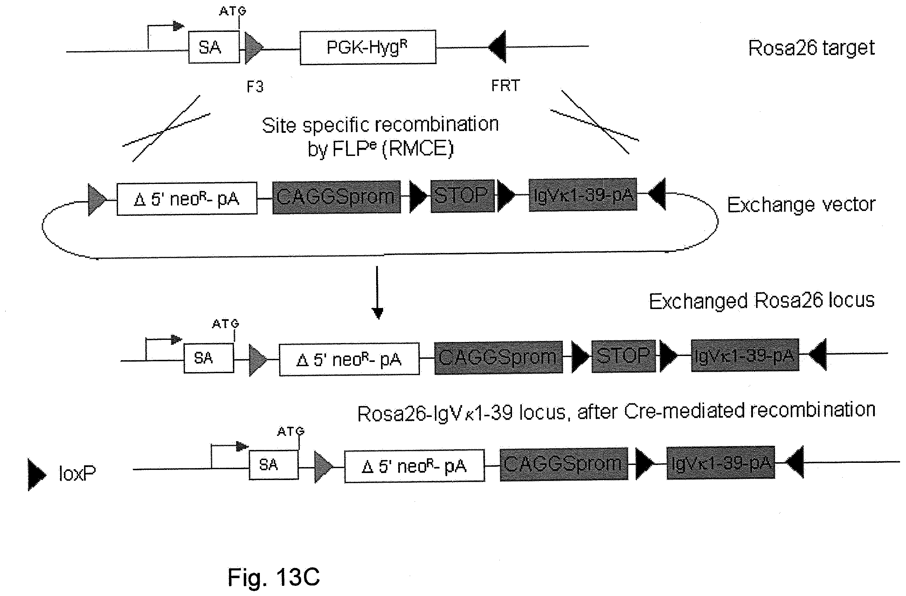

At the 5' end of the expression cassette, a NotI site was introduced and on the 3' site a NheI site. Both sites are used for cloning in the CAGGS expression module. After gene assembly according to methods used by GeneArt, the insert is digested with NotI-NheI and cloned into the expression module containing a CAGGS promoter, a stopper sequence flanked by LoxP sites ('floxed'), a polyadenylation signal sequence and, at the 5' and 3' end, sequences to facilitate homologous recombination into the Rosa26 locus of mouse ES cell lines. Promoter and/or cDNA fragments were amplified by PCR, confirmed by sequencing and/or cloned directly from delivered plasmids into an RMCE exchange vector harbouring the indicated features. A schematic drawing and the confirmed sequence of the final targeting vector pCAGGS-IgVK1-39 are shown in figure 13A and 13B. The targeting strategy is depicted in figure 13C.

### Example 6

### CAGGS expression insert based on the rearranged germline IGLV2-14/J V lambda region (IGLV2-14/J-Ck)

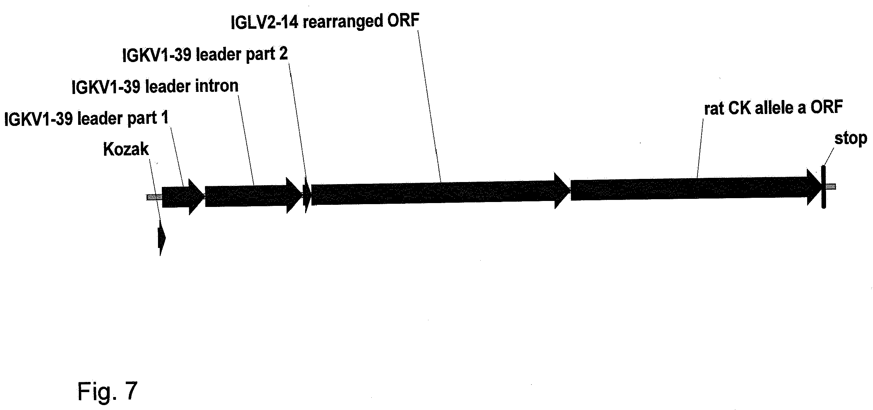

This example describes the sequence and insertion of an expression cassette incorporating the rearranged germline IGLV2-14/J V lambda region. This insert encompasses cloning sites, a Kozak sequence, a leader sequence containing an intron, an open reading frame of the rearranged IGLV2-14/J region, a rat CK constant region from allele a and a translational stop sequence (IGLV2-14/J-Ck; figure 7). The primary construct consists of naturally-occurring sequences and has been analyzed and optimized by removing undesired cis acting elements like: internal TATA-boxes, poly adenylation signals, chi-sites, ribosomal entry sites, AT-rich or GC-rich sequence stretches, ARE-, INS- and CRS sequence elements, repeat sequences, RNA secondary structures, (cryptic) splice donor and acceptor sites and splice branch points (GeneArt GmbH). In addition, the codon usage in the open reading frame regions was optimized for expression in mice. The intron sequence is unchanged and thus represents the sequence identical to the human IGKV1-39 leader intron.

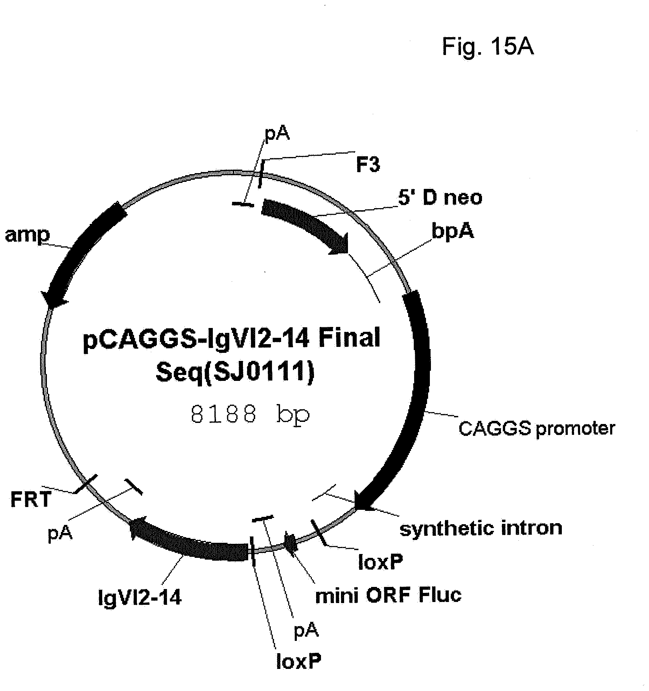

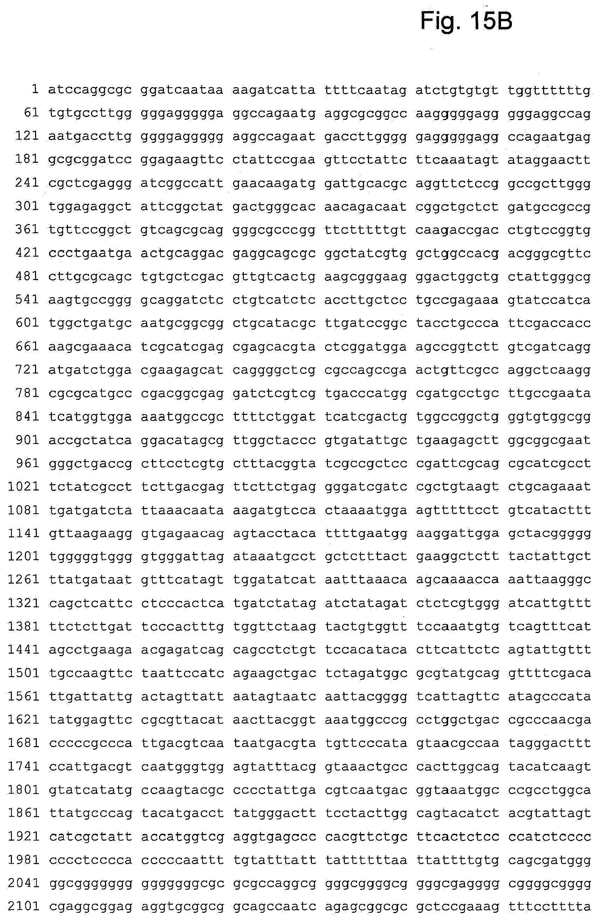

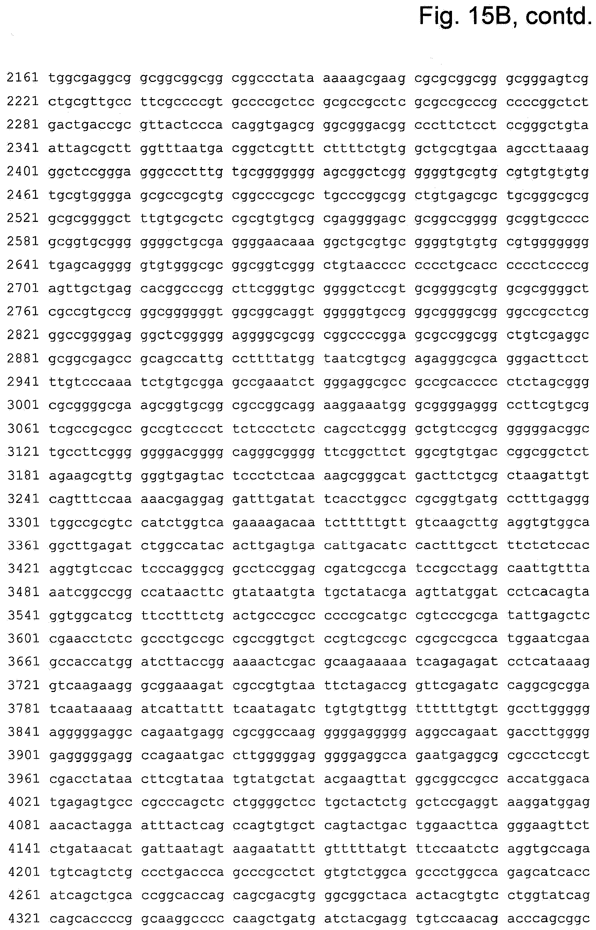

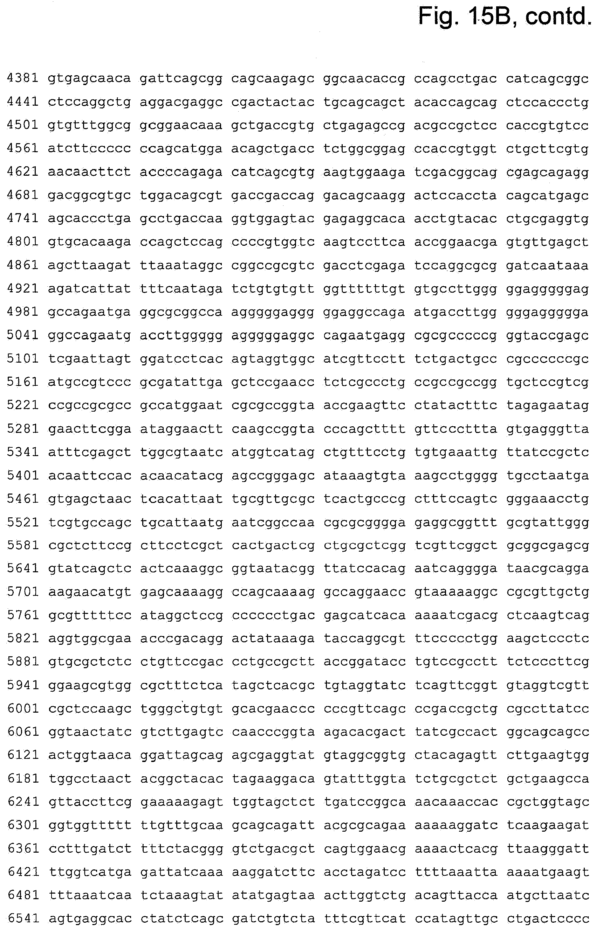

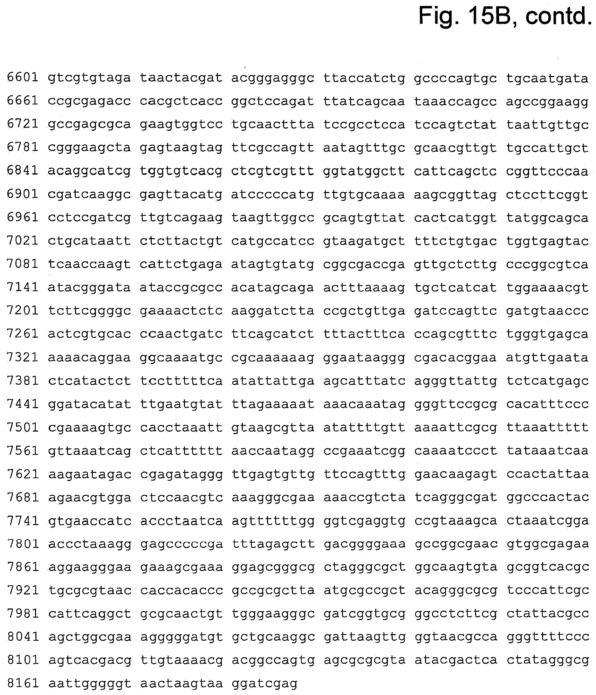

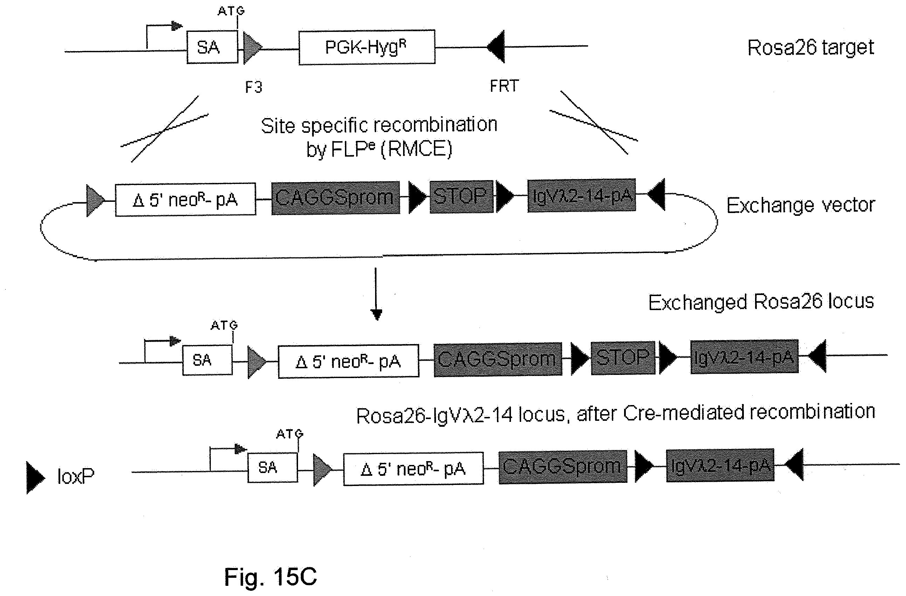

At the 5' end of the expression cassette, a NotI site was introduced and on the 3' site a NheI site. Both sites are used for cloning in the CAGGS expression module as described by TaconicArtemis. After gene assembly according to methods used by GeneArt, the insert was digested with NotI-NheI and cloned into the expression module containing a CAGGS promoter, a stopper sequence flanked by LoxP sites ('floxed'), a polyadenylation signal sequence and, at the 5' and 3' end, sequences to facilitate homologous recombination into the Rosa26 locus of mouse ES cell lines. To construct the final ROSA26 RMCE targeting vector, promoter and/or cDNA fragments were amplified by PCR. Amplified products were confirmed by sequencing and/or cloned directly from delivered plasmids into an RMCE exchange vector harbouring the indicated features. A schematic drawing and the confirmed sequence of the final targeting vector pCAGGS-IgVL2-14 is shown in figure 15A and 15B. The targeting strategy is depicted in figure 15C.

### Example 7

### Expression of IGKV1-391J-Ck in HEK293 cell lines (pSELECT-IGKV1-39/J-Ck)

This example describes a method to verify that the IGKV1-39/J-Ck constructs described in example 5 enable expression and detection of the IGKV1-39/J-Ck L chain in HEK293 cells. The IGKV1-39/J insert (figure 6) was modified at the 5' end by changing the NotI site into a SalI site. This change is required for cloning of the product into the expression cassette plasmid pSELECT-hygro (InvivoGen). The CAGGS expression insert IGKV1-39/J-Ck and pSELECT-hygro were digested with SalI and NheI, ligated and used to transform competent XL1-Blue cells using standard techniques. Colonies were picked and DNA purified using Qiagen Midi-prep columns according to the manufacturer's procedures. The resulting light chain (LC) expressing vector named 0817676_pSELECT_0815426 was used to transfect HEK293 cells with Fugene6 (Roche) according to the manufacturer's protocols. Supernatants were screened for the presence of IGKV1-39/J-Ck light chains by ELISA and western blot using anti-rat-Ck antibodies (Beckton Dickinson #550336 and 553871) and protocols used in the art.

The VH of anti-tetanus toxoid (TT) IgG MG 1494 was cloned into IgG expression vector MV1056 using restriction sites SfiI and BstEII. The resulting clone was sequence verified. HEK293T cells were transfected with five different vector combinations as shown in Table 4 (see example 8 for details of vector 0817678_pSELECT_0815427). Supernatants were harvested and IgG concentrations determined (see Table 4). No IgG could be detected for supernatants A and B containing light chain only as expected (detection antibody recognized Fc part of IgG). IgG concentration in supernatants C and D was comparable to that of positive control supernatant E, indicating correct expression of the light chain constructs.

Binding to TT was analyzed by ELISA to check functionality of the produced antibodies, using hemoglobin as negative control antigen. No TT-specific binding could be detected for supernatants A and B containing light chain only, as expected. TT-specific binding for supernatants C and D was at least as good as for positive control supernatant E, confirming correct expression of the light chain constructs and functional assembly with heavy chain. Antibodies were detected not only using an anti-human IgG secondary antibody, but also an anti-rat Ckappa light chain secondary antibody. The results confirm that the anti-rat Ckappa antibody (BD Pharmingen #553871, clone MRK-1) recognizes the light chain expressed by the pSELECT vectors.

Supernatants were analyzed by non-reducing SDS-PAGE and Western blot (not shown). Detection using an anti-human IgG heavy chain antibody did not show bands for supernatants A and B containing light chain only, as expected. Results for supernatants C and D were comparable to positive control supernatant E, with a band close to the 170 kD marker as expected for intact IgG. Additional lower molecular weight bands were observed as well for supernatants C, D and E, which might represent degradation products, IgG fragments resulting from (partial) reduction and/or irrelevant protein bands due to non-specific binding of the detection antibody.

Detection using an anti-rat Ckappa light chain antibody showed a band close to the 26 kD marker for supernatants A and B, as expected for light chain only. This band was much more intense for A compared to B, indicating that the free IGKV1-39 light chain may be better expressed and/or more stable than the free IGLV2-14 light chain. No bands were detected for control supernatant E as expected, since the expressed IgG contains a human Ckappa light chain. For supernatants C and D, expected bands close to the 170 kD marker were observed; lower molecular weight bands were also observed, but to a lesser extent than above using the anti-human IgG antibody.

In conclusion, transfection of the light chain expression constructs combined with the heavy chain of anti-tetanus toxoid (TT) IgG MG1494 resulted in IgG production comparable to the positive control construct for both the pSELECT kappa and lambda light chain constructs. Both IgG productions yielded ELISA signals in a TT ELISA that were better than or comparable to the control IgG. SDS-PAGE and Western blot analysis confirmed the presence of intact IgG. The tested anti-rat Ckappa antibody worked efficiently in both ELISA and Western blot. Culture supernatant from cells transfected with light chain constructs only did not result in detectable IgG production nor in detectable TT-specific binding, while free light chain was detected on Western blot.

### Example 8

### Expression of IGLV2-14/J-Ck in HEK293 cell lines (pSELECT-IGLV2-14/J-Ck)

This example describes a method to verify that the IGLV2-14/J constructs described in example 6 enable expression and detection of the IGLV2-14/J-Ck L chain in HEK293 cells. The IGLV2-14/J-Ck insert (figure 7) was modified at the 5' end by changing the NotI site into a SalI site. This change is required for cloning of the product into the expression cassette plasmid pSELECT-hygro (InvivoGen). The CAGGS expression insert IGLV2-14/J-Ck and pSELECT-hygro were digested with SalI and NheI ligated and used to transform competentXL1-Blue cells using standard techniques. Colonies were picked and DNA purified using Qiagen Midi-prep columns according to the manufacturer's procedures. The resulting light chain (LC) expressing vector named 0817678_pSELECT_0815427 was used to transfect HEK293 cells with Fugene6 (Roche) according to the manufacturer's protocols. Supernatants were screened for the presence of IGLV2-14/J-Ck light chains by ELISA and western blot using anti-rat-Ck antibodies (Becton Dickinson #550336 and 553871) and protocols used in the art. See example 7 for details and results.

### Example 9

### Construction of a VK promoter-driven expression construct containing an IGKV1-39/J insert and multiple enhancer elements derived from the murine CK locus (VkP-IGKV1-39/J-Ck; VkP-O12)

This example describes the construction of an expression cassette that contains relevant elements to enable B-cell and developmental/differentiation stage-specific expression of the rearranged human IGKV1-39 VK region, based on the IGKV1-39 VK promoter region, leader containing an intron, germline V-gene, CDR3, IGKJ segment, mouse intergenic region located between Jk and CK, rat Ck allele a open reading frame, and a mouse intergenic fragment from the 3' end of the mouse CK gene ending just 3' of the 3'CK enhancer.

Optimized open reading frames of the leader, IGKV1-39 rearranged gene, and rat CK allele a gene, as described in example 5, was used for the construction of the expression cassette. The VK promoter region was obtained by gene synthesis procedures (GeneArt, GmbH) and is almost identical to the sequence of the human IGKV1-39 region between -500 bp and the ATG (start site) of the gene. The only deviation from the natural sequence is the introduction of a GCCACCATGG Kozak sequence at the ATG (start) site in order to promote translation. A genomic fragment from a mouse BAC clone (TaconicArtemis) is used as the basis for the introduction of individual elements. This fragment is identical to the sequence of the mouse VK locus starting with the intron donor site located directly 3' of the JK5 region and ending just 3' of the 3' CK enhancer and covers approximately 12.5 kb.

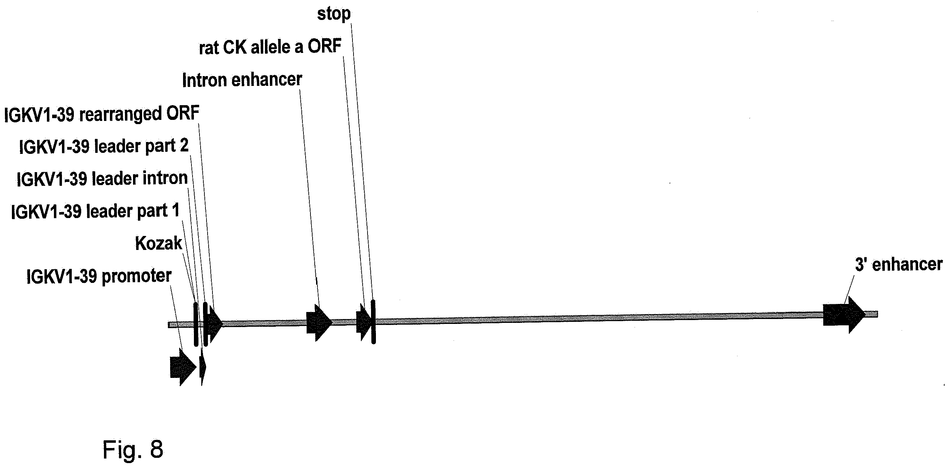

The final construct contains from 5' to 3' end the following elements: human genomic IGKV1-39 promoter (500 bp), a Kozak sequence, a human IGKV1-39 leader part 1 (optimized), a human IGKV1-39 leader intron, a human IGKV1-39 leader part 2 (optimized), a human IGKV1-39 germline gene (optimized), a human J-region (optimized), a mouse intergenic region including the intron enhancer element, a rat (Rattus norvegicus) kappa constant region (optimized), and a mouse intergenic region including the 3' kappa enhancer. The elements of this expression cassette are shown in figure 8 and named VkP-IGKV1-39/J-Ck (VkP-O12). An outline of the pVkP-O12 vector and the targeting strategy is depicted in figure 20A and 21A. The vector was introduced into ES cells following standard procedures (see example 14).

### Example 10

### Construction of a VK promoter-driven expression construct containing an IGLV2-14/J clone and multiple CK locus-derived enhancer elements (VkP-IGLVL2-14/J-Ck; VkP-2a2).

This example describes the same construct as described in example 9, except that the IGKV1-39 gene and J-region are replaced by the optimized human IGLV2-14 germline gene including a unique V-J region (VkP-IGLV2-14/J-Ck; VkP-2a2; figure 9).

### Example 11

### Construction of a VK promoter-driven expression construct containing an IGKV1-39 clone lacking the CK intron enhancer element (VkP-IGKV1-39/J-Ck-Δ1; VkP-O12-del1)

The construct described in example 9 was modified by removing the CK intron enhancer element, located in the intergenic region between the human J region and the rat CK region by standard PCR modification and DNA cloning methodologies (GeneArt, GmBH). The resulting expression cassette is shown in figure 10 and named VkP-IGKV1-39/J-Ck-Δ1 (VkP-O12-del1).

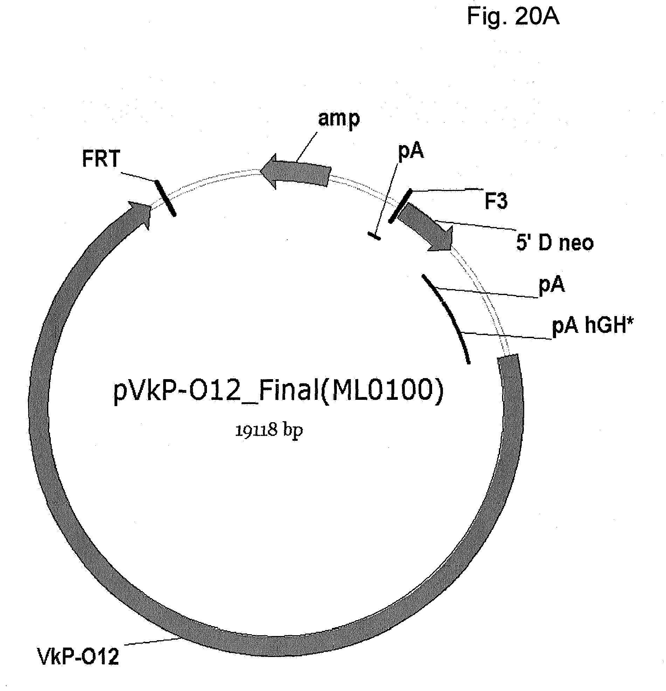

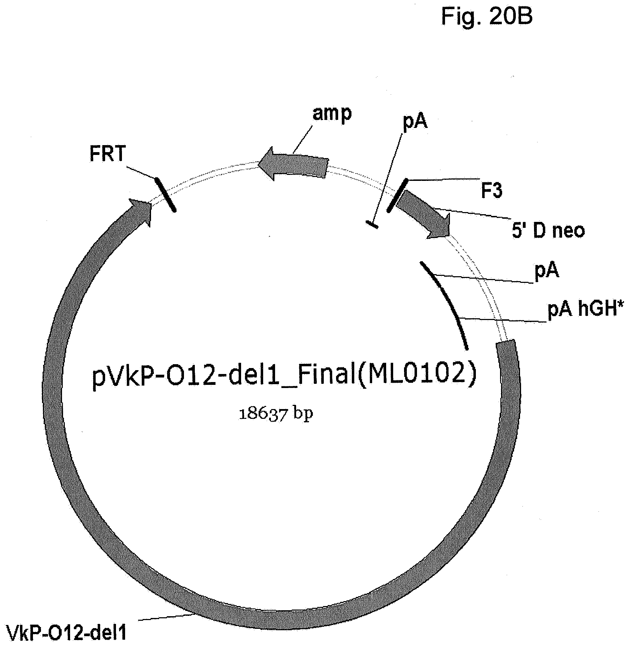

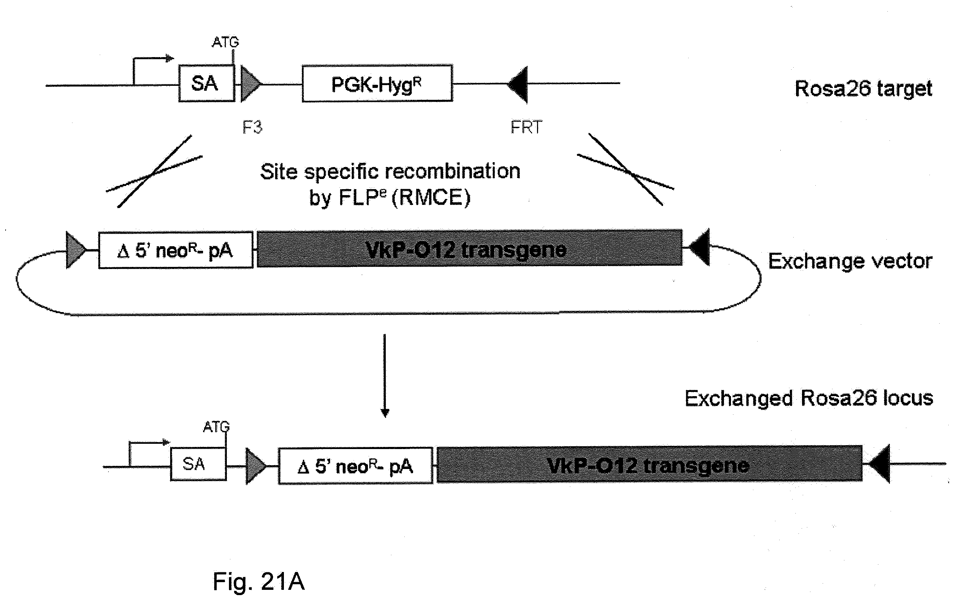

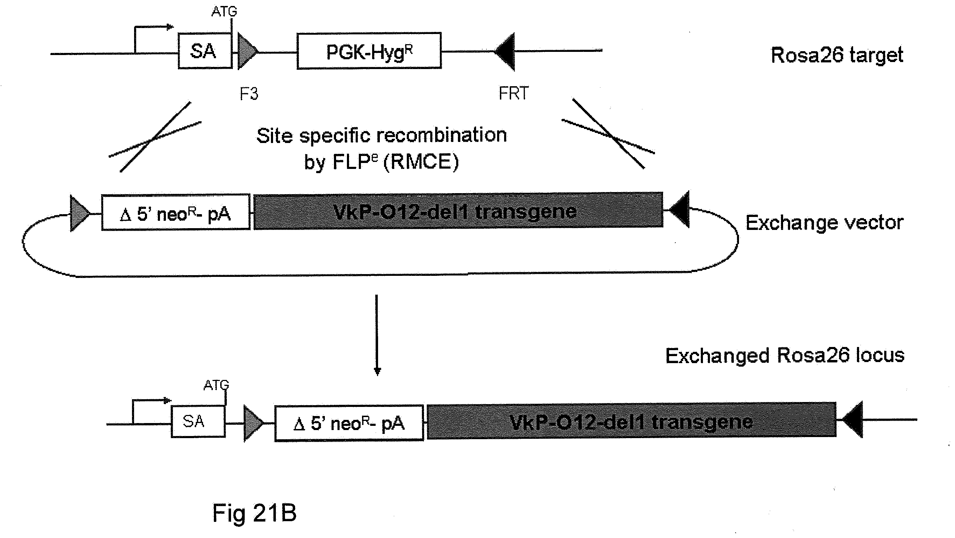

An outline of the pVkP-O12-del1 vector and the targeting strategy is depicted in figure 20B and 21B. The vector was introduced into ES cells following standard procedures (see example 14).

### Example 12

### Construction of a VK promoter-driven expression construct containing an IGKV1-39 clone lacking the CK intron enhancer element and a truncated 3' CK enhancer element (VkP-IGKV1-39/J-Ck-Δ2; VkP-O12-del2)

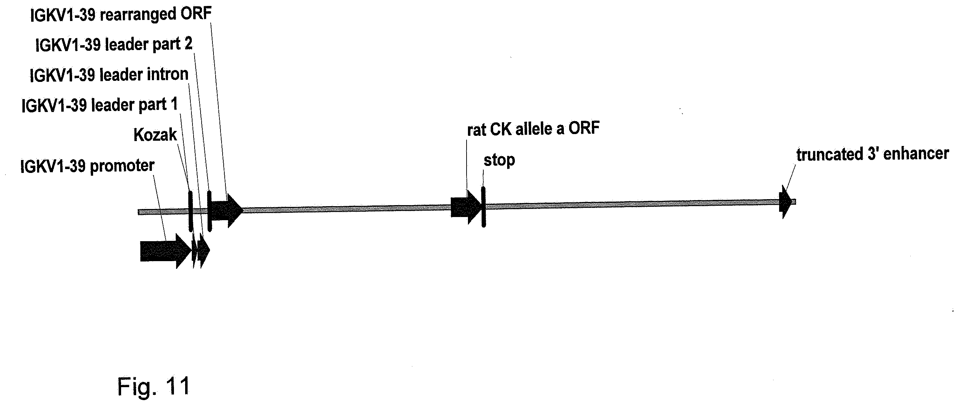

The construct described in example 11 was modified by truncating the 3' CK enhancer element and deleting part of the intergenic region 3' of the rat Ck gene, to remove potential inhibitory elements. This was achieved by removing the intergenic sequence between an EcoRV site (located 3' of the rat Ck gene) and the NcoI site present in the 3' enhancer (5993 bp) and further removing the sequence between the 3' enhancer BstXI site and the BstXI site 3' of the 3'enhancer (474 bp) using standard methods. The resulting expression cassette is shown in figure 11 and named VkP-IGKV1-39/J-Ck-Δ2 (VkP-O12-del2).

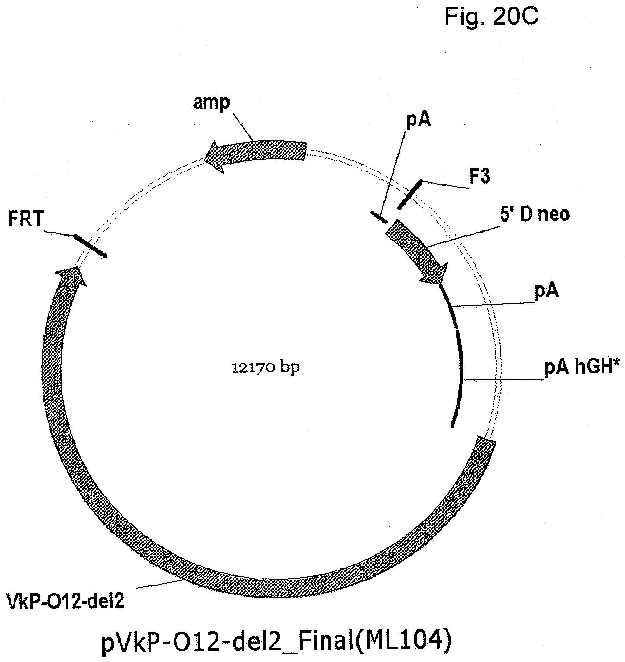

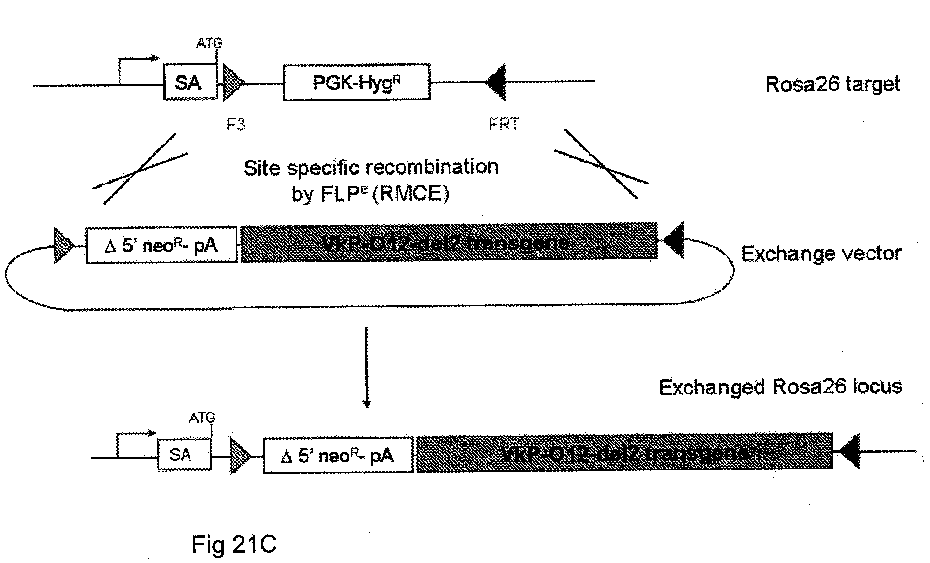

An outline of the pVkP-O12-del2 vector and the targeting strategy is depicted in figure 20C and 21C. The vector was introduced into ES cells following standard procedures (see example 14).

### Example 13

### Expression of Vk constructs in cell lines

The constructs described in example 9-12 are tested for their ability to produce light chain proteins in the myeloma cell lines MPC11 (ATCC CCL167), B-cell lymphoma WEHI231 (ATCC CRL-1702), the T-cell lymphoma EL4 (ATCC TIB-39) and in HEK293 (ATCC CRL1573). The enhancer and promoter elements in the construct enable expression in the B-cell lines but not in cell lines derived form other tissues. After transfection of the cell lines using purified linearized DNA and Fugene6 (Roche) cells are cultured for transient expression. Cells and supernatant are harvested and subjected to SDS-PAGE analysis followed by western blotting using a specific anti-rat-C-kappa antibody. Supernatants are analyzed in ELISA for secreted L chains using the anti-rat CK antibody (Beckton Dickinson #550336).

### Example 14

### Generation of transgenic ES lines

All constructs as described in examples 3, 4, 5, 6, 9, 10, 11 and 12 were used to generate individual stable transgenic ES lines by means of homologous recombination. The methods for generation of transgenic ES lines via homologous recombination are known in the field (e.g. Eggan et al., PNAS 98, 6209-6214; Seibler J, Zevnik B, Küter-Luks B, Andreas S, Kern H, Hennek T, Rode A, HeimannC, Faust N, Kauselmann G, Schoor M, Jaenisch R, Rajewsky K, Kühn R, Schwenk F. Nucleic Acids Res. 2003 Feb 15;31(4):e12; Hogan et al. (Cold Spring Harbor Laboratory Press, Cold Spring Harbor NY.), pp. 253-289.).

For all constructs described in examples 5-6, and examples 9-12, the RMCE ES cell line (derived from mouse strain 129S6B6F1-Gt(ROSA)26Sortm10Arte) was grown on a mitotically inactivated feeder layer comprised of mouse embryonic fibroblasts (MEF) in DMEM High Glucose medium containing 15% FBS (PAN 1302-P220821). Leukemia Inhibitory Factor (Chemicon ESG 1107) was added to the medium at a concentration of 900 U/mL. For manipulation, 2x10⁵ ES-cells were plated on 3,5 cm dishes in 2 ml medium. Directly before transfection, 2 ml fresh medium was added to the cells. 3 ul Fugene6 Reagent (Roche; Catalog No. 1 814 443) was mixed with 100 ul serum free medium (OptiMEM I with Glutamax I; Invitrogen; Catalog No. 51985-035) and incubated for 5 min. 100 ul of the Fugene/OptiMEM solution was added to 2 ug circular vector and 2 ug CAGGS-Flp and incubated for 20 min. This transfection complex was added dropwise to the cells and mixed. Fresh medium was added to the cells the following day. From day 2 onwards the medium was replaced daily with medium containing 250 ug/mL G418 (Geneticin; Invitrogen; Catalog No. 10131-019). Seven days after transfection, single clones were isolated, expanded, and molecular analyzed by Southern blotting according to standard procedures.

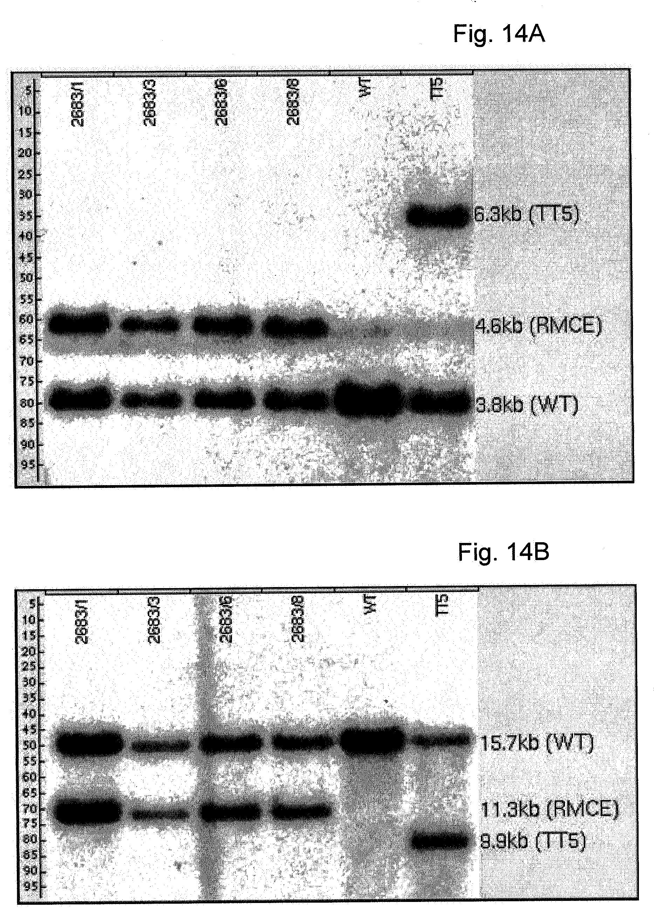

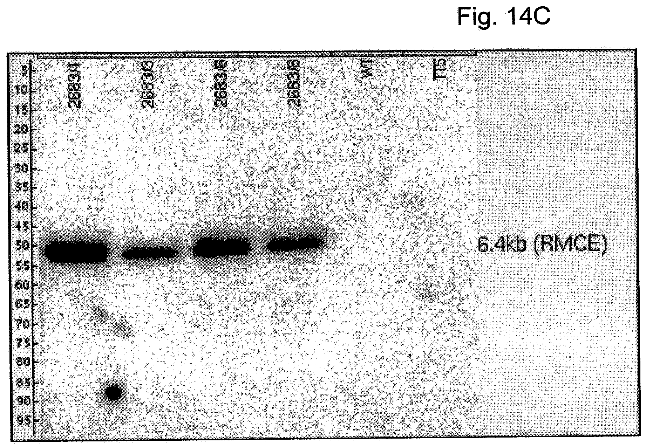

For each construct, analysis of multiple clones by restriction enzyme digestion of genomic DNA of single clones followed by hybridization with 5' probes, 3' probes, and internal probes resulted in clones that comprised a correct, single insertion at the correct position in the Rosa26 locus. An example is provided in figure 14.

### Example 15

### Generation of transgenic mouse strains

All ES cell lines that were generated and verified for their modifications as described in example 14 were used to generate stable transgenic mice by means of tetraploid recombination. The methods are known in the field. In general, after administration of hormones, superovulated Balb/c females were mated with Balb/c males. Blastocysts were isolated from the uterus at dpc 3.5. For microinjection, blastocysts were placed in a drop of DMEM with 15% FCS under mineral oil. A flat tip, piezo actuated microinjection-pipette with an internal diameter of 12 - 15 micrometer was used to inject 10-15 targeted C57BL/6 N.tac ES cells into each blastocyst. After recovery, injected blastocysts were transferred to each uterine horn of 2.5 days post coitum, pseudopregnant NMRI females. Chimerism was measured in chimeras (G0) by coat colour contribution of ES cells to the Balb/c host (black/white). Highly chimeric mice were bred to strain C57BL/6 females. Depending on the project requirements, the C57BL/6 mating partners are non-mutant (W) or mutant for the presence of a recombinase gene (Flp-Deleter or Cre-deleter or CreER inducible deleter or combination of Flp-deleter/CreER). Germline transmission was identified by the presence of black, strain C57BL/6, offspring (G1).

For example, ESC clone IgVK1-39 2683 8 (see examples 5 and 14) was injected in a total of 62 blastocysts in 3 independent experiments. 3 litters were obtained with a total of 6 pups. All pups were chimaeric. 3 heterozygous offspring pups were obtained that were used for further crossing.

ESC Clone Kappa 2692 A-C10 (see examples 3 and 14) was injected in a total of 54 blastocysts in 3 independent experiments. 3 litters were obtained with a total of 11 pups, of which 10 were chimaeric. 8 heterozygous offspring pups were obtained that were used for further crossing.

ESC Clone Kappa 2692 B-C1 (see examples 3 and 14) was injected in a total of 51 blastocysts in 3 independent experiments. 2 litters were obtained with a total of 6 pups, of which 4 were chimaeric. 3 heterozygous offspring pups were obtained that were used for further crossing.

### Example 16

### Breeding

This example describes the breeding for obtaining mice that contain transgenic expression cassettes as described example 14 and knock out mice in which the endogenous lambda and kappa loci have been silenced. The localization of V-lambda on chromosome 16 and CD19 on chromosome 7 allow standard breeding procedures. The breeding of the co-localized Vk locus and Rosa26 locus on chromosome 6 with a distance of about 24 cM requires special attention during the screening as only a percentage of the offspring shows crossover in a way that both modifications are brought together on one chromosome.

All four loci have to be combined in a single mouse strain that is homo- or heterozygous for CD19-cre (not described) and modified Rosa26 transgene and homozygous for the other loci. Breeding is performed by standard breeding and screening techniques as appropriate and offered by commercial breeding companies (e.g. TaconicArtemis).

### Example 17

### Immunizations of mice

Primary and booster immunization of mice are performed using standard protocols.

To validate the transgenic expression of human rearranged Vκ 012 (IGKV1-39) - rat Cκ light chains (see example 5, 14-16) in B cells from CD19-HuVκ1 mice and to assess its impact on VH repertoire size, diversity of VH family usage and V(D)J recombination after immunization, the CD19-HuVκ1 transgenic mice are immunized with tetanus toxin vaccine (TT vaccine) and VH sequence diversity of randomly-picked clones from CD19-HuVκ1 mice are compared with TT-immunized wt mice and CD19-Cre HuVk1 negative littermates. Data on the SHM frequency of the human Vκ 012 transgene in the immunized mice are obtained. A diverse collection of at least 40 TT-specific, clonally-unrelated mAbs containing the human Vκ 012 are recovered from CD19-HuVκ1 mice by phage display.

For this, three adult CD19-HuVκ1 mice are vaccinated with TT vaccine using standard immunization procedures. After immunization, serum titers are measured using TT specific ELISA (TT: Statens Serum Institute, Art. no. 2674) and spleen suspensions subjected to cell sorting by the FACS procedure after staining with a rat Cκ-specific monoclonal antibody to isolate transgenic B cells (clone RG7/9.1; BD Pharmingen# 553901, Lot# 06548). RNA from rat Cκ-positive B cells are extracted and the resulting cDNA material used for library building and SHM analysis.

The standard monoclonal mouse anti-rat Cκ antibody (clone RG7/9.1; BD Pharmingen# 553901, Lot# 06548) is used in FACS analysis of transgene expressing B cells (Meyer et al., 1996, Int. Immunol., 8: 1561). The clone RG7/9.1 antibody reacts with a monotypic (common) kappa chain determinant. This anti-rat Cκ antibody (clone RG7/9.1 (BD Pharmingen# 553901, Lot# 06548) is labeled with R-phycoerythrin (PE) using the LYNX rapid conjugation kit according to the manufacturer's instructions for FACS analysis and sorting. The labeled antibody is firstly tested by flow cytometry for binding to rat Cκ-containing functional light chain proteins produced into transiently transfected HEK-293T cells; the un-conjugated antibody serves as a positive control. Two other antibodies shown to bind to rat Cκ by ELISA and Western-blot (see example 7) are tested as well by flow cytometry.

Fab-phage display library building is carried out with a set of optimized degenerate PCR primers designed to amplify C57BL/6 VH genes; the minimal library size is 10⁶ clones, and minimal insert frequency is 80%. The vector used, MV1043 (figures 3 and 12), contains the human Vκ 012 fused to a human Cκ region. The rat Cx is therefore exchanged for the human counterpart in the library generation process.

Before selection, VH sequencing of 96 randomly picked clones is performed to validate VH repertoire diversity that is compared to diversity obtained from an unselected library previously generated using the same procedures from BALB/c mice immunized with TT. A library from C57B1/6 wt mice that are immunized in the same way allows diversity comparison between two preselected libraries sharing the same vaccine and the same genetic background.

Several independent selections are performed on TT coated in immunotubes. Variables that may be included are selections using biotinylated antigens in solution or selections on captured TT. Based on the number and diversity of ELISA-positive clones obtained in the first selections, decisions on additional rounds of selection are made. Clones are considered positive when > 3x positive over a negative control clone. Positive clones are analyzed by ELISA against a panel of negative control antigens to verify antigen specificity. The aim is to identify at least 40 unique VH regions, as based on unique CDR3 sequences and V_{H}DJ_{H} rearrangements.

Amplification of the cDNA material from rat Cκ-positive sorted B cells is performed with a PCR forward primer specific to the human leader sequence and a PCR reverse primer specific to the rat Cκ sequence, in a region not redundant with the mouse Cκ sequence, as reported in a recent study (Brady et al., 2006, JIM, 315: 61). Primer combinations and annealing temperatures are firstly tested on cDNA from HEK-293T cells transfected with 0817676_pSELECT_0815426 = pSELECT vector with IGKV1-39 DNA cassette (see example 7).

The amplification products is cloned in pJET-1 vector and after XL1-blue transformation, 96 colonies are sequenced for assessing VL SHM frequency by direct comparison to the Vκ 012 (IGKV1-39) germline sequence. The R/S ratio method, as described in our study on human TT-specific antibodies (de Kruif et al., 2009, J. Mol. Biol., 387: 548) allows discrimination between random mutations and antigen-driven mutations that occurred on VL sequences.

### Example 18

Immunofluorescent analysis of B cell populations in transgenic mouse lines.

This example describes the use of antibodies and flow cytometry to analyze B cell populations in primary (bone marrow) and secondary (spleen, peritoneal) lymphoid organs and blood. Methods and reagents are described in Middendorp et al. (2002) J. Immunol. 168:2695 and Middendorp et al. (2004) J. Immunol. 172:1371. For analysis of early B cell development in bone marrow, cells were surface stained with combinations of antibodies (Becton Dickinson) specific for B220, CD19, CD25, IgM, IgD, mouse Ckappa, mouse Clambda and rat Ckappa to detect pro-B cells, pre-B cells, large pre-B cells, early and late immature B cells and recirculating B cell populations expressing the transgene on their surface. DAPI staining (Invitrogen) was included to exclude dead cells from the analysis and FC block (Becton Dickinson) to inhibit antibody interaction with Fc receptors on myeloid cells. For analysis of surface transgene expression on B cell populations in peripheral lymphoid organs and blood, cells were stained with combinations of antibodies (Becton Dickinson) specific for B220, CD5, CD19, CD21, CD23, IgM, IgD, mouse Ckappa, mouse Clambda and rat Ckappa. DAPI staining was included to exclude dead cells from the analysis and FC block to inhibit antibody interaction with Fc receptors on myeloid cells. In addition, combinations of antibodies (Becton Dickinson) specific for CD3, CD4, CD11b, CD11c and NK1.1 were included to determine if transgene expression occurred in cell types outside of the B cell compartment.

Three mice heterozygous for the human IGKV1-39/rat Ckappa transgene and heterozygous for the CD19-Cre transgene on a C57BL6 background (HuVk1/CD19-Cre) were analysed. As controls for the FACS analysis, three littermate mice wild type for the human IGKV1-39/rat Ckappa transgene and heterozygous for the CD19-Cre transgene on a C57BL6 background (CD19-Cre) and two C57BL6/NTac mice (Wt) were included. All animals were allowed to acclimatize in the animal facility for 1 week before analysis and all mice were male and 6 weeks of age. Lymphocytes were isolated from the femurs, spleens, peritoneal cavity and blood of mice using conventional techniques as previously described (Middendorp et al. (2002) J. Immunol. 168:2695 and Middendorp et al. (2004) J. Immunol. 172:1371). Antibodies were pre-combined as shown in Table 10 and staining was carried out in 96 well plates. Incubation with the PE-conjugated anti-rat C kappa (described above) was carried out before staining with the rat anti-murine antibodies to avoid non-specific binding. After completion of cell staining, labeled cells were analysed on a Becton Dickinson LSR II FACS machine and the acquired data analysed with FlowJo software (v6.4.7).

Transgenic mice were similar in weight, appearance and activity to wild type mice. No gross anatomical alterations were observed during the harvesting of tissues. No difference was observed in the numbers of B cells in the bone marrow (BM) and spleen (Table 11) or in the numbers of B cells, T cells and myeloid cells in peripheral organs between transgenic and wild type mice. In addition, the frequency or proportion of the cells in the different lymphocyte developmental pathways was not altered in transgenic mice when compared to wild type mice. Thus in the double transgenic (HuVk1/CD19-Cre) and transgenic (CD19-Cre) mice lymphoid and most importantly B cell development was indistinguishable from wild type mice.

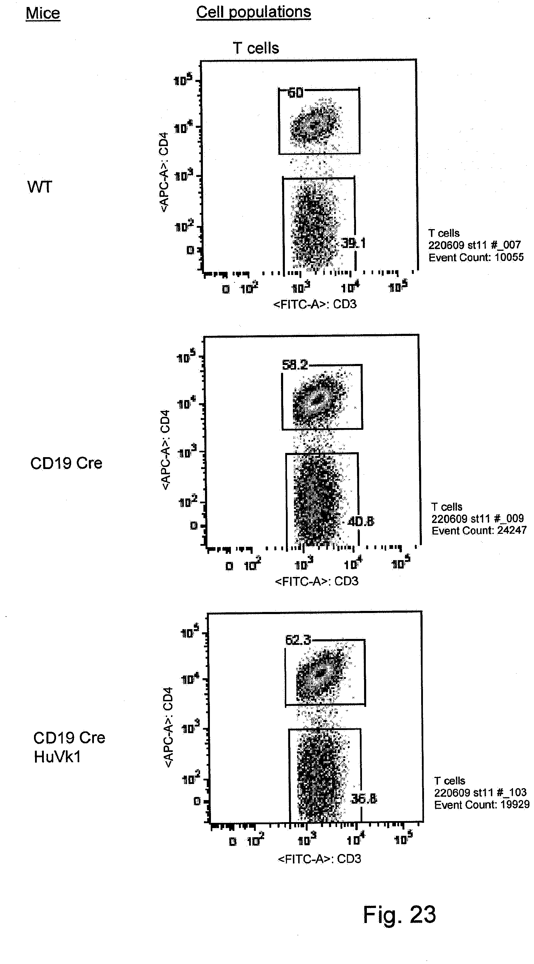

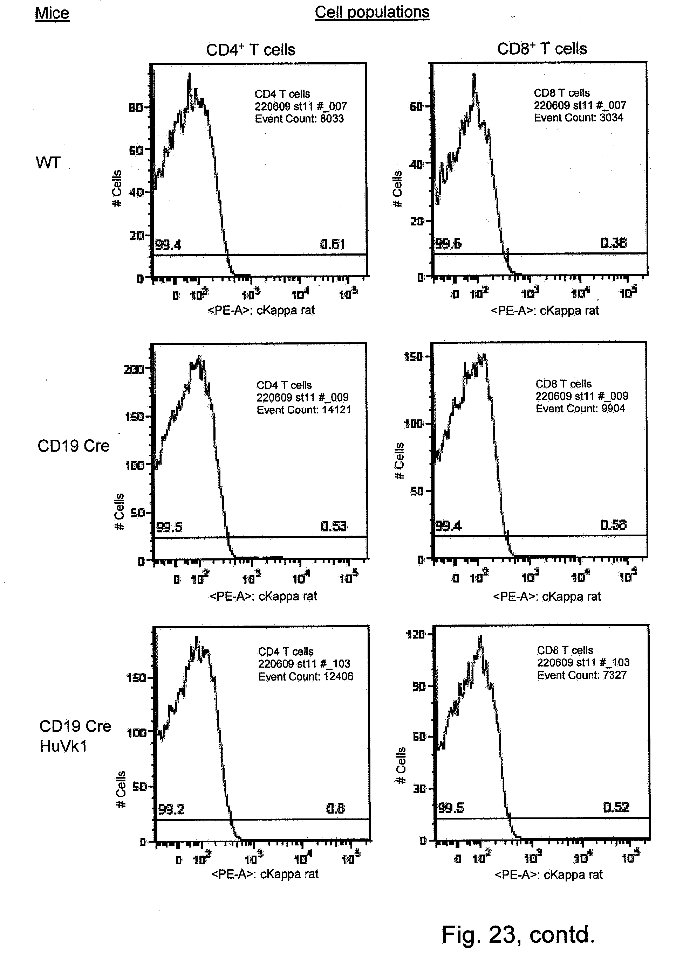

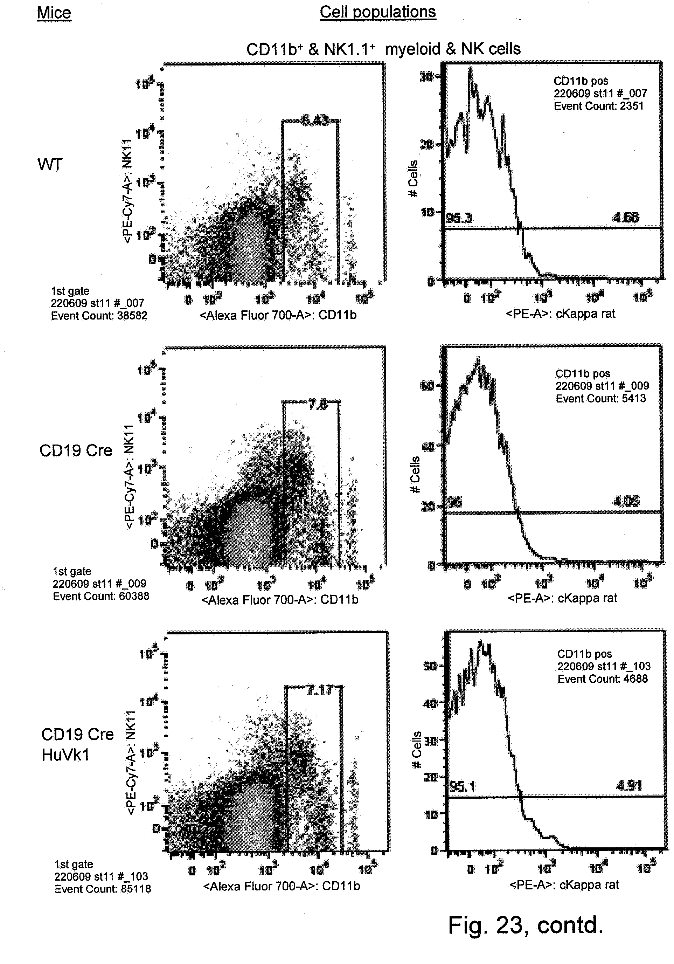

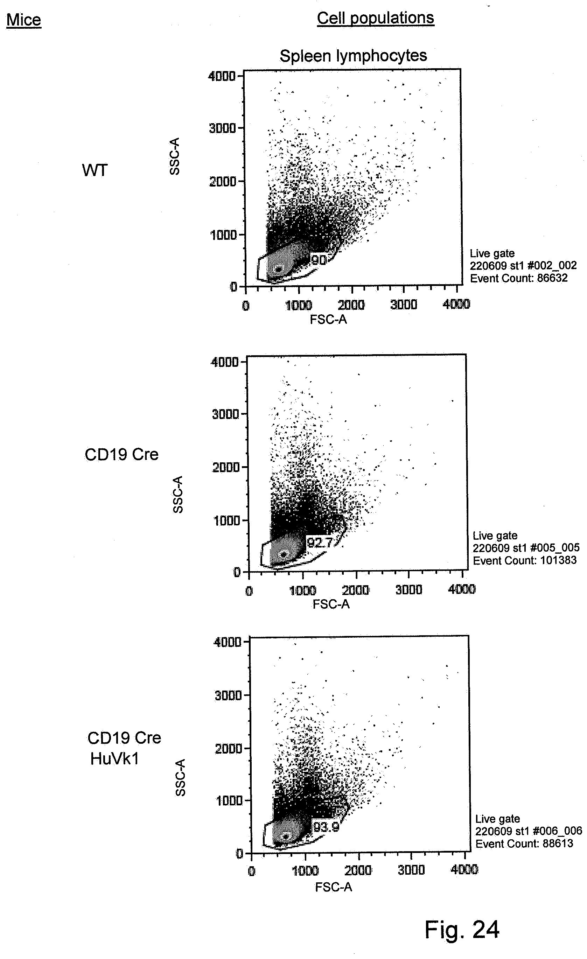

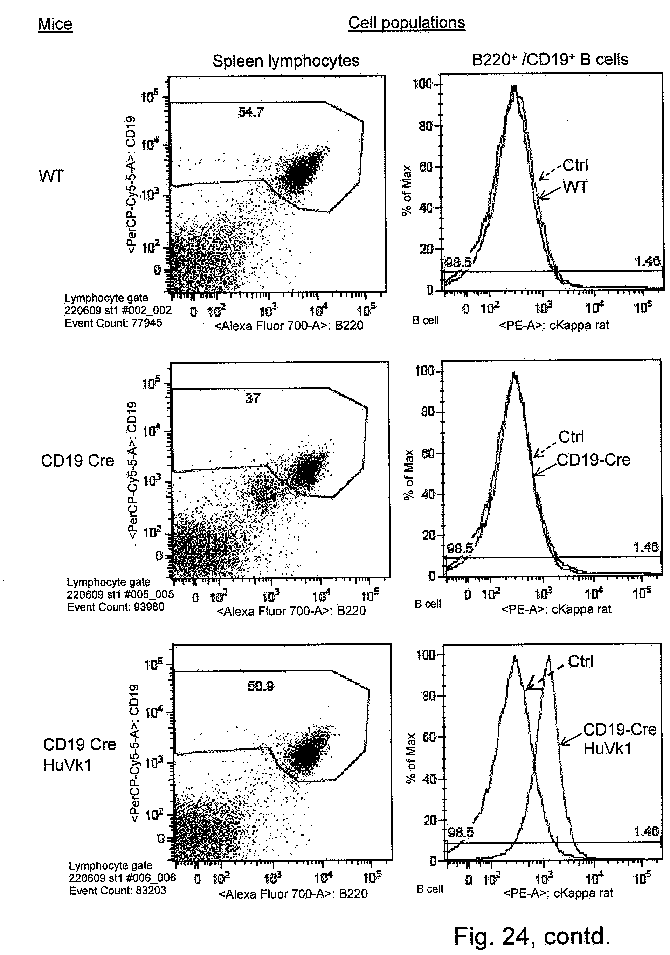

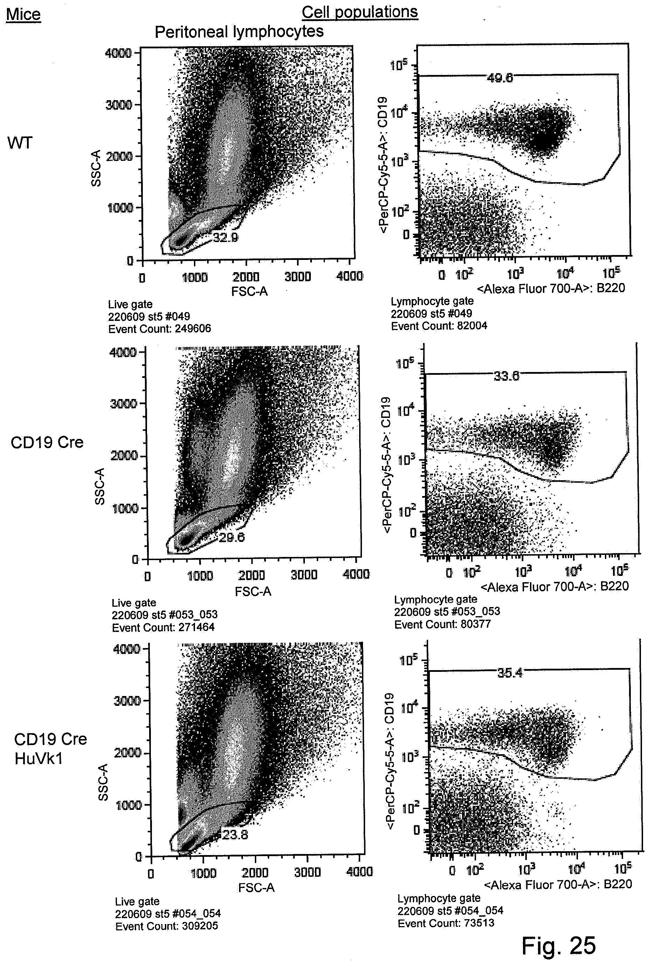

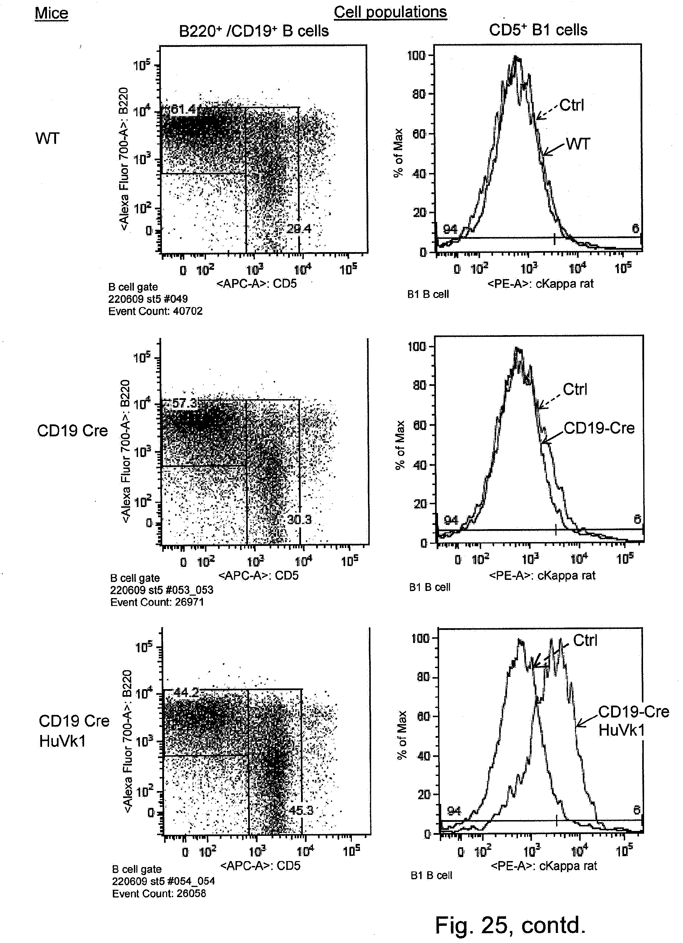

In the peripheral lymphoid organs, staining with the transgene specific antibody (anti-ratCkappa-PE) was only observed in the B cell populations. T cell, myeloid cell and NK cell populations were all negative for surface expression of the transgene in the spleen (Figure 23). In contrast, in cells stained with the pan B cell markers B220 and CD19 all cells were shifted to the right in the FACS plot indicating cell surface expression of the transgene (Figure 24). A similar transgene-specific staining was measured in CD5⁺ B1 cells of the peritoneum, a developmentally distinct population of B cells (Figure 25).

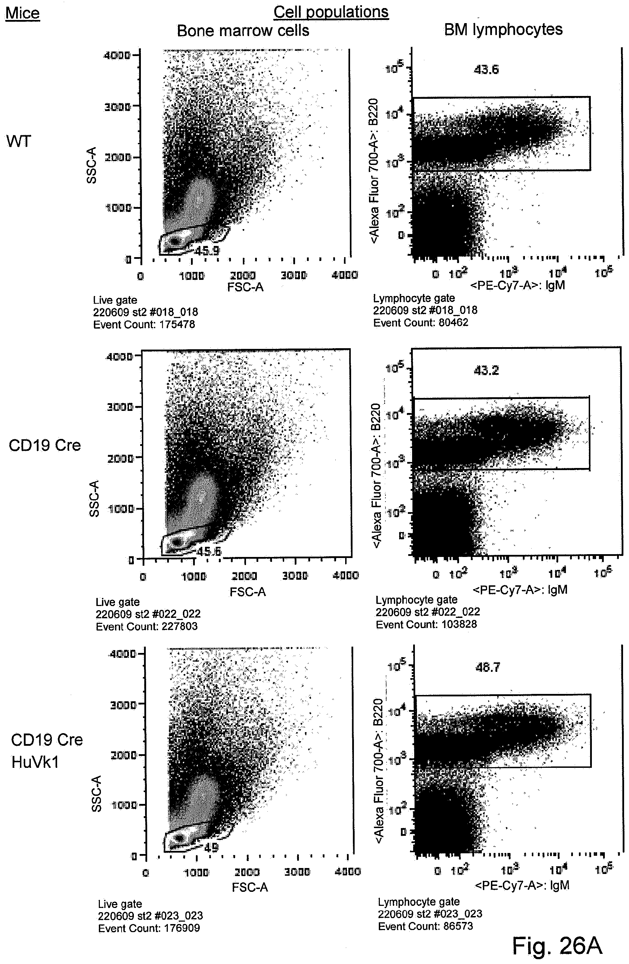

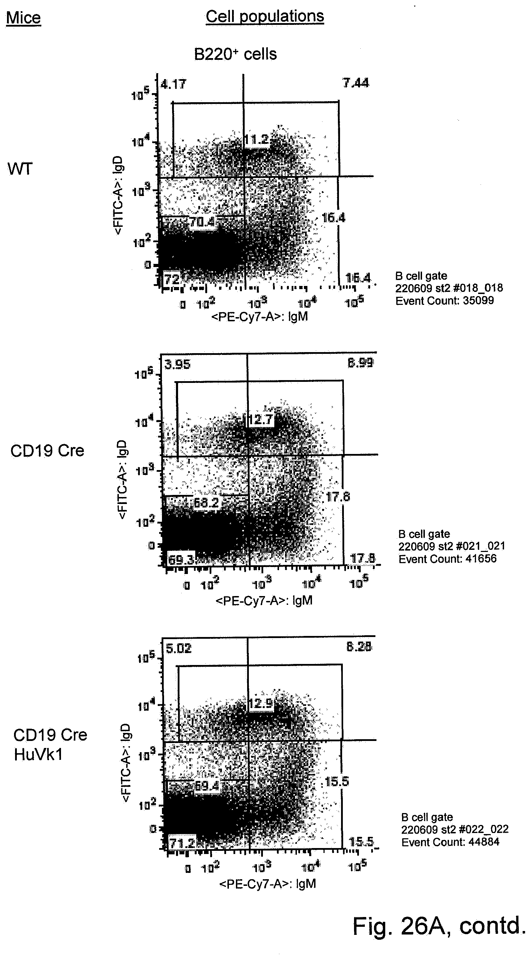

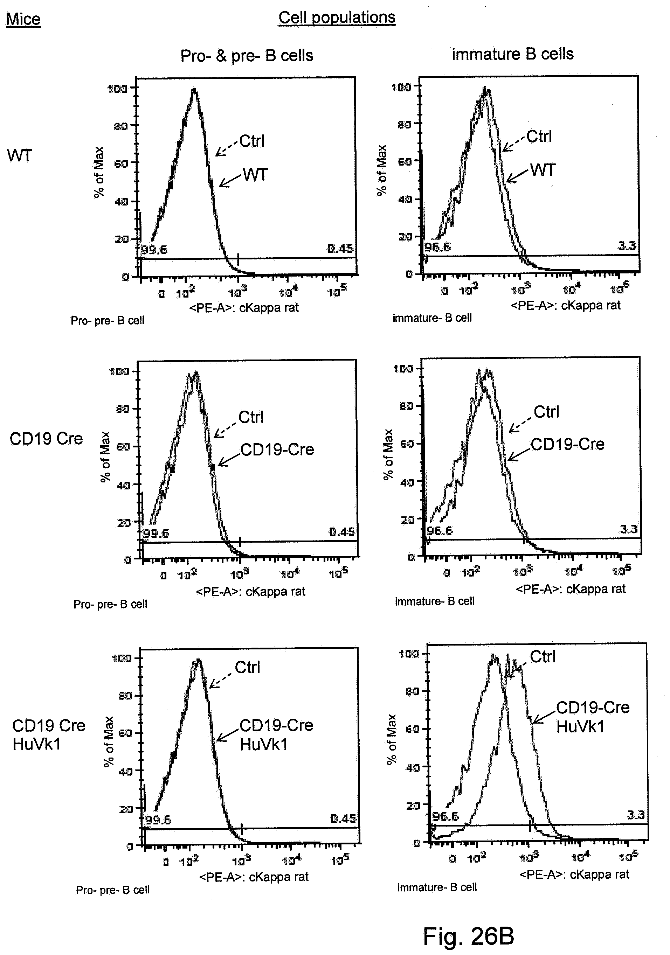

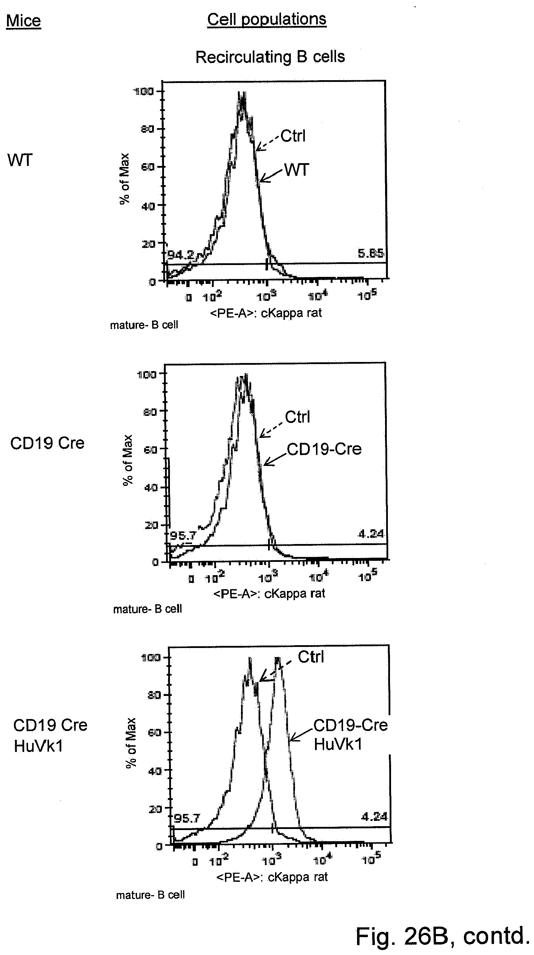

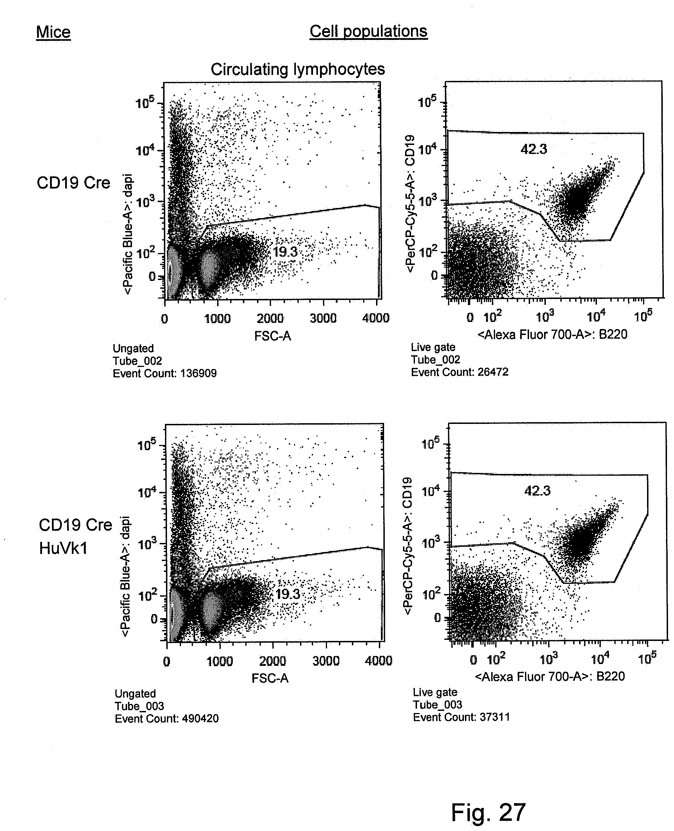

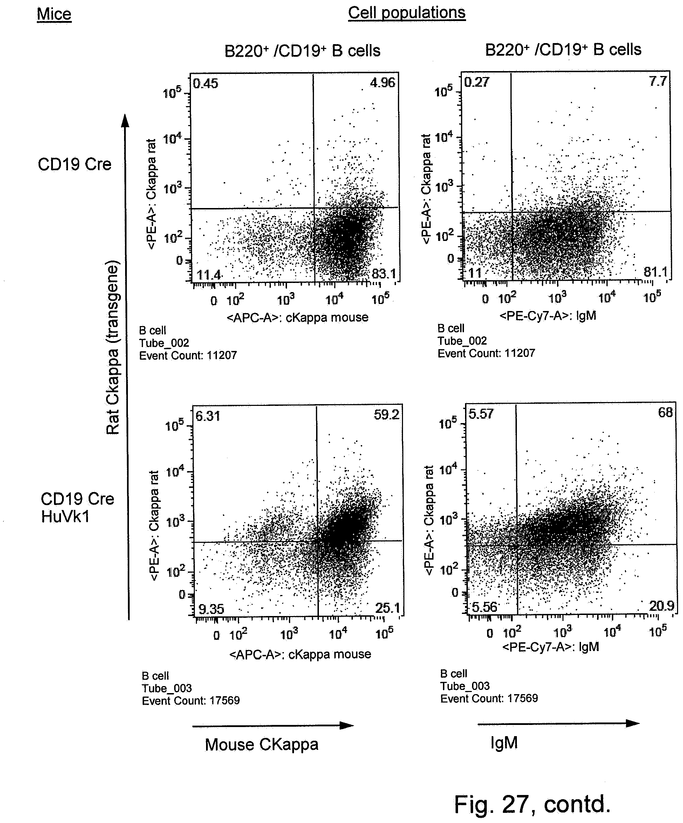

Differentiation of B cells from multilineage precursors to mature B cells occurs in the bone marrow. In the lymphocytes analyzed from the bone marrow, extracellular and transgene expression was not detectable in the earliest B cell progenitors the pro- and pre-B cell consistent with the pattern of normal light chain expression (Figure 26). Transgene expression first becomes detectable in immature B cells, the developmental stage at which the germline murine light chain undergoes rearrangement and is expressed at the cell surface in the context of the preselected heavy chain (Figure 26). Consistent with the staining in the spleen transgenic light chain expression is also detected on mature recirculating B cells (Figure 26). Thus the CD19-Cre driven expression of the transgene is consistent with the normal pattern of light chain expression. The staining with the endogenous light chain- specific antibody is more intense than that of the transgene-specific light chain antibody. This may indicate a higher expression level of the endogenous light chain, a more sensitive staining with the endogenous light chain-specific antibody or a combination of both. Importantly, the intensity of the surface expression of the transgenic light chain is correlated with both endogenous light chain and IgM surface expression as observed in staining of circulating B cells in the blood (Figure 27).

Thus overall this analysis demonstrates that expression of the human IGKV1-39/Ckappa transgene is restricted to the B cell compartment and the temporal regulation of its expression is similar to the endogenous kappa and lambda light chains resulting in normal development of all B cell populations. The apparent lower level of expression of the transgene could be explained by the strength of the promoter in comparison to the promoter and enhancers present on endogenous light chain genes or by a delay in transgene expression that gives the endogenous light chains a competitive advantage in pairing with the rearranged heavy chain. This is consistent with the observation that as B cells mature the relative intensity of transgene staining increases compared to the endogenous light chains. In addition, the observation that B cells numbers are normal and that every surface Ig+ B cell co-expresses an endogenous and transgenic light chain supports the conclusion that the IGKV1-39 variable region is capable of pairing with a normal repertoire of different murine heavy chain variable regions. We conclude from this analysis that insertion of the IGKV1-39/rat Ckappa transgene driven by the CD19-Cre activated CAGGS promoter in the Rosa locus facilitates timely and B cell-specific expression of the transgene and that the transgene is capable of pairing with a normal repertoire of murine heavy chains.

### Example 19

### Epibase® T-cell epitope profile for IGKV1-39.

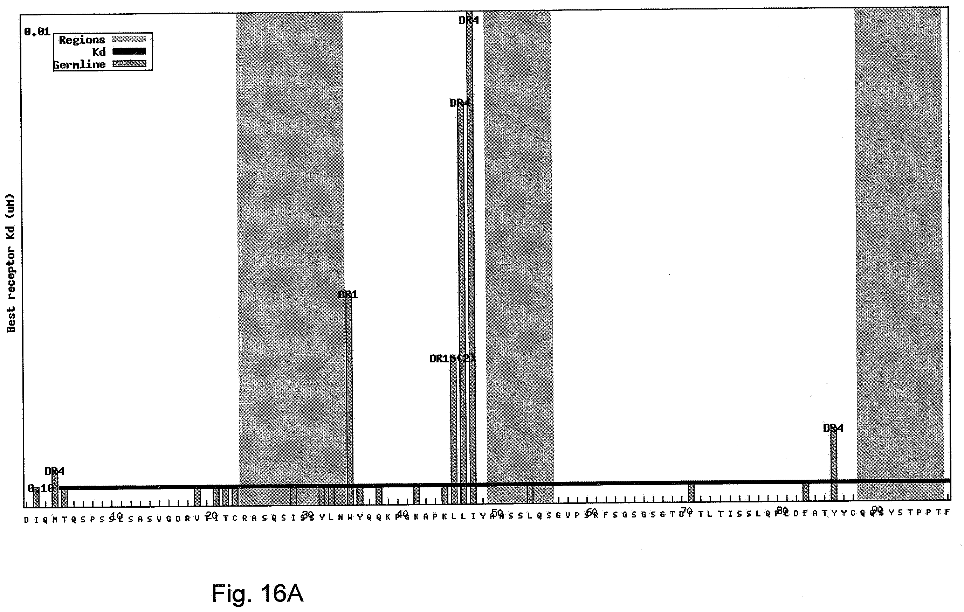

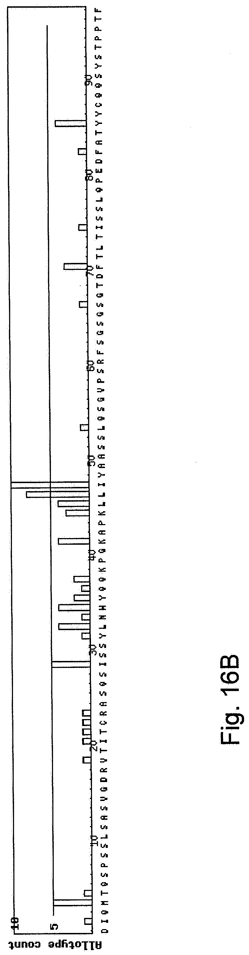

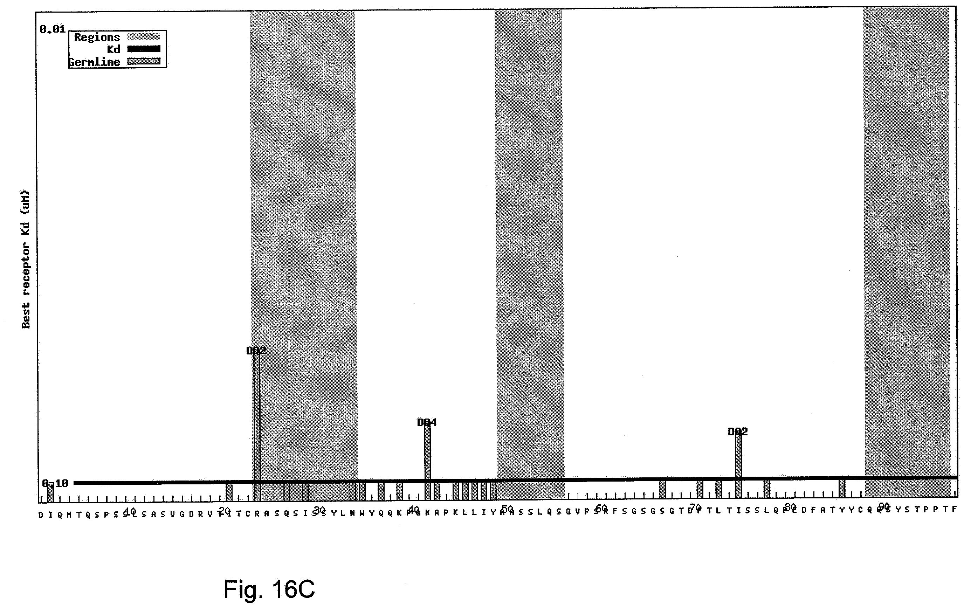

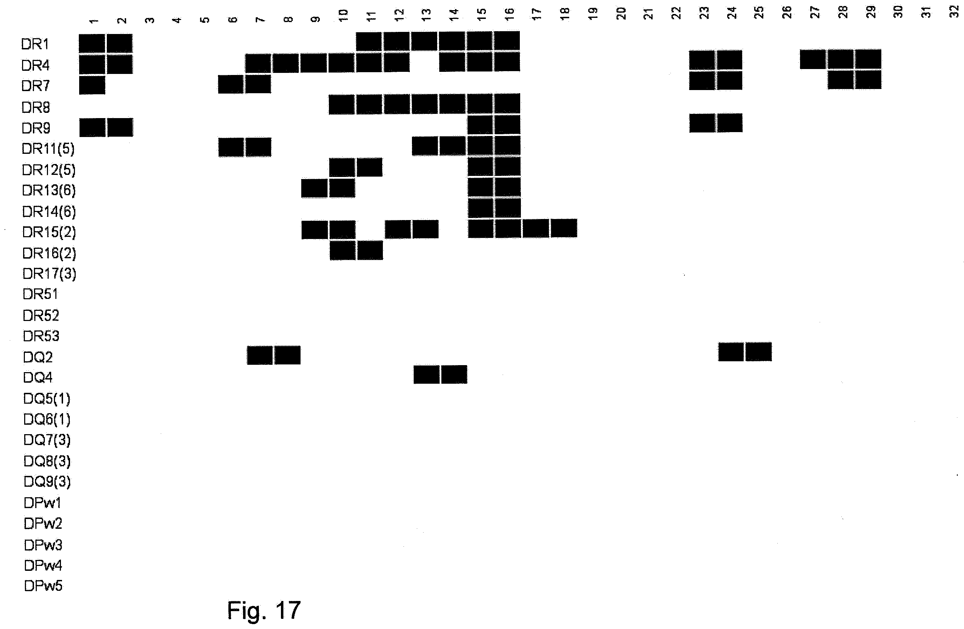

The protein sequence of IGKV1-39 (figure 12, human germline IGKV1-39/J Protein) was scanned for the presence of putative HLA class II restricted epitopes, also known as T_{H}-epitopes. For this, Algonomics' Epibase® platform was applied to IGKV1-39. In short, the platform analyzes the HLA binding specificities of all possible 10-mer peptides derived from a target sequence (Desmet et al. Nature 1992, 356:539-542; Desmet et al. FASEB J. 1997, 11:164-172; Desmet et al. Proteins 2002, 48:31-43; Desmet et al. Proteins 2005, 58:53-69). Profiling is done at the allotype level for 20 DRB1, 7 DRB3/4/5, 13 DQ and 7 DP, i.e. 47 HLA class II receptors in total (see Table 5). Epibase® calculates a quantitative estimate of the free energy of binding ΔG_{bind} of a peptide for each of the 47 HLA class II receptors. These data were then further processed as follows:
- Free energies were converted into Kd-values through ΔG_{bind} = RT ln(Kd).
- Peptides were classified as strong (S), medium (M), weak and non (N) binders. The following cutoffs were applied;
   S: strong binder: Kd < 0.1 µM.
   M: medium binder: 0.1 µM ≤ Kd < 0.8 µM.
   N: weak and non-binder: 0.8 µM ≤ Kd.
- Peptides corresponding to self-peptides were treated separately. The list of self-peptides was taken from 293 antibody germline sequences. They are referred to as 'germline-filtered' peptides.
S- and M-peptides are mapped onto the target sequence in so-called epitope maps; S-affinities are plotted quantitatively; M-values are presented qualitatively. As a general overview of the results, Table 6 lists the number of strong and medium binders in the analyzed proteins, for the groups of HLA class II receptors corresponding to the DRB1, DQ, DP and DRB3/4/5 genes. Counting was done separately for strong and medium affinity binders. Peptides binding to multiple allotypes of the same group were counted as one. Values between brackets refer to germline-filtered peptides. In Table 7, the sequence is shown in a format suitable for experimental work. The sequence is broken down in consecutive 15-mers overlapping by 12 residues. For each 15mer, the promiscuity is listed (the number of allotypes out of a total of 47 for which the 15-mer contains a critical binder), as well as the implied serotypes. The Epibase® profile and epitope maps are shown in figure 16 and 17.

It was concluded that IGKV1-39 contains no strong non-self DRB1 binders. Typically, significantly more binders were found for DRB1 than for other HLA genes. This is in agreement with experimental evidence that allotypes belonging to the DRB1 group are more potent peptide binders. Medium strength epitopes for DRB1 allotypes are expected to contribute to the population response, and cannot be disregarded. Again, no non-self DRB1 binders were found in IGKV1·39.
In the humoral response raised against an antigen, the observed T_{H} cell activation/proliferation is generally interpreted in terms of the DRB1 specificity. However, one cannot ignore the possible contribution of the DRB3/4/5, DQ and DP genes. Given the lower expression levels of these genes as compared to DRB1, the focus was on the class of strong epitopes for DRB3/4/5, DQ and DP. 'Critical epitopes' are those epitopes that are strong binders for any DRB1, DRB3/4/5, DQ or DP allotype or are medium binders for DRB1. IGKV1-39 contains no strong or medium non-self binders for DRB3/4/5, DQ, or DP.

A number of peptides are also present in germline sequences (values between brackets in Table 6). Such peptides may very well bind to HLA but they are assumed to be self and, hence, non-immunogenic. In total, 6 strong and 16 medium germline-filtered DRB1 binders were found in IGKV1-39. Framework region 1 up to framework region 3 is an exact match for germline V-segment VKI 2-1-(1) 012 (VBase), a.k.a. IGKV1-39*01 (IMGT). Framework region 4 is an exact match for germline J-segment JK1 (V-base) a.k.a. IGKJ1*01(IMGT). It is hardly surprising that these segments do not contain any non-self epitopes.

### Example 20

### Production characteristics of IGKV1-39

There is a great demand for antibody discovery platforms that yield therapeutic antibodies that are thermodynamically stable and give good expression yields. These characteristics are important in ensuring the stability of the drug substance during production and after injection of the drug product into the patient. In addition good expression yields impact directly on the cost of drug manufacture and thus pricing, patient access and profitability. Virtually all therapeutic antibodies in clinical use today are composed of human IgG1 and kappa constant regions but use different heavy and light chain variable regions that confer specificity. Human variable heavy and light chain domains can be divided into families that have greater than 80% sequence divergence. When rearranged examples of these families in germline configuration are combined and compared for stability and yield it is clear that the gene families are not equal in terms of biophysical properties. In particular V_{H}3, V_{H}1 and V_{H}5 have favourable stability for the heavy chains and Vk1 and Vk3 have the best stability and yield of light chains. In addition when mutations are introduced as part of the somatic hypermutation process they can interfere with V_{H}/V_{L} pairing. To assess the effect that different light chain genes with different rates of mutation have on the production characteristics of a fixed VH chain, a Fab phage display library was built of light chains (kappa and lambda) from six naive healthy donors combined with a panel of 44 TT binding heavy chains from immunized donors. After one round of selection TT binding Fab clones were isolated. Several of these shared the same V_{H} gene as the TT clone PG1433 in combination with different light chains. The Fab light chain fragments were recloned into a kappa expression vector and transfected in combination with DNA encoding the heavy chain of PG1433 into 293 cells and specific IgG production measured by ELISA. As demonstrated in table 8 the selected clones containing PG1433 V_{H} combined with different light chains had between 5 and 10 fold lower protein expression PG1433 V_{H} combined with IGKV1-39. Note that all of the light chains contained amino acid mutations within their coding regions that might disrupt V_{H} paring and reduce production stability. Thus, in addition to reducing the chances of unwanted immunogenicity, it is expected that the use of the light chain IGKV1-39 without mutations contributes to improved production stability and yields of various specificity-contributing V_{H} genes. Indeed stable clones generated by the transfection of different V_{H} genes all paired with IGKV1-39 are able to be passaged extensively and still retain robust production characteristics as shown in table 9.

### Example 21

### Generation of mice expressing fully human VH and VL regions.

Transgenic mice according to the invention are crossed with mice that already contain a human VH locus. Examples of appropriate mice comprising a human VH locus are disclosed in Taylor et al. (1992). Nucleic Acids Res 20: 6287-95; Lonberg et al. (1994). Nature 368: 856-9; Green et al. (1994). Nat Genet 7: 13-21; Dechiara et al. (2009). Methods Mol Biol 530: 311-24.).

After crossing and selecting for mice that are at least heterozygous for the IGKV1-39 transgene and the human VH locus, selected mice are immunized with a target. VH genes are harvested as described hereinabove. This method has the advantage that the VH genes are already fully human and thus do not require humanization.

### Example 22

### Isolation, characterization, Oligoclonics formatting and production of antibodies targeting human IL6 for treatment of chronic inflammatory diseases such as rheumatoid arthritis

A spleen VH repertoire from transgenic mice that are immunized with human recombinant IL6 is cloned in a phage display Fab vector with a single human IGKV1-39-C kappa light chain (identical to the mouse transgene) and subjected to panning against the immunogen human IL6. Clones that are obtained after two to four rounds of panning are analyzed for their binding specificity. VH genes encoding IL6-specific Fab fragments are subjected to sequence analysis to identify unique clones and assign VH, DH and JH utilization. The Fab fragments are reformatted as IgG1 molecules and transiently expressed. Unique clones are then grouped based on non-competition in binding assays and subjected to affinity and functional analysis. The most potent anti-IL6 IgG1 mAbs are subsequently expressed as combinations of two, three, four or five heavy chains comprising different VH-regions in the Oligoclonics format, together with one IGKV1-39-C-based kappa light chain and tested in vitro for complex formation with IL-6. The Oligoclonics are also tested *in vivo* for clearance of human IL-6 from mice. An Oligoclonic with the most potent clearance activity is chosen and the murine VH genes humanized according to conventional methods. The humanized IgG1 are transfected into a mammalian cell line to generate a stable clone. An optimal subclone is selected for the generation of a master cell bank and the generation of clinical trial material.

Many of the protocols described here are standard protocols for the construction of phage display libraries and the panning of phages for binding to an antigen of interest and are described, for example, in Antibody Phage Display: Methods and Protocols. 2002. Editor(s): Philippa M. O'Brien, Robert Aitken. Humana Press, Totowa, New Jersey, USA.

### Immunizations

Transgenic mice receive three immunizations with human IL6 every two weeks using the adjuvant Sigma titerMax according to manufacturer's instructions.

### RNA isolation and cDNA synthesis

Three days after the last immunization, spleens and lymphnodes from the mice are removed and passed through a 70 micron filter into a tube containing PBS pH 7.4 to generate a single cell suspension. After washing and pelleting of lymphocytes, cells are suspended in TRIzol LS Reagent (Invitrogen) for the isolation of total RNA according to the manufacturer's protocol and subjected to reverse transcription reaction using 1 microgram of RNA, Superscript III RT in combination with dT20 according to manufacturer' s procedures (Invitrogen).

The generation of Fab phage display libraries is carried out as described in Example 2.

### Selection of phages on coated immunotubes

Human recombinant IL6 is dissolved in PBS in a concentration of 5 µg/ml and coated to MaxiSorp Nunc-Immuno Tube (Nunc 444474) overnight at 4°C. After discarding the coating solution, the tubes are blocked with 2% skim milk (ELK) in PBS (blocking buffer) for 1 hour at Room Temperature (RT). In parallel, 0.5 ml of the phage library is mixed with 1 ml blocking buffer and incubated for 20 minutes at room temperature. After blocking the phages, the phage solution is added to the IL6 coated tubes and incubated for 2 hours at RT on a slowly rotating platform to allow binding. Next, the tubes are washed 10 times with PBS/0.05% Tween-20 followed by phage elution by incubating with 1 ml 50 mM glycine-HCl pH 2.2 10 min at RT on rotating wheel and directly followed by neutralization of the harvested eluent with 0.5 ml 1 M Tris-HCl pH 7.5.

### Harvesting phage clones

A 5 ml XL1-Blue MRF (Stratagene) culture at O.D. 0.4 is added to the harvested phage solution and incubated for 30 minutes at 37°C without shaking to allow infection of the phages. Bacteria are plated on Carbenicillin/Tetracycline 4% glucose 2*TY plates and grown overnight at 37°C.

### Phage production

Phages are grown and processed as described by Kramer et al. 2003 (Kramer et al. 2003. Nucleic Acids Res. 31(11): e59) using VCSM13 as helper phage strain.

### Phage ELISA

ELISA plates are coated with 100 microliter human recombinant IL6 per well at a concentration of 2.5 microgram/ml in PBS overnight at 4°C. Plates coated with 100 microliter thyroglobulin at a concentration of 2 microgram/ml in PBS are used as a negative control. Wells are emptied, dried by tapping on a paper towel, filled completely with PBS-4% skimmed milk (ELK) and incubated for 1 hour at room temperature to block the wells. After discarding the block solution, phage minipreps pre-mixed with 50 µl blocking solution are added and incubated for 1 hour at RT. Unbound phages are subsequently removed by 5 washing steps with PBS-0.05% Tween-20, Bound phages are detected by incubating the wells with 100 microliter anti-M13-HRP antibody conjugate (diluted 1/5000 in blocking buffer) for 1 hour at room temperature. Free antibody is removed by repeating the washing steps as described above, followed by TMB substrate incubation until color development was visible. The reaction is stopped by adding 100 microliter of 2 M H2SO4 per well and analyzed on an ELISA reader at 450 nm emission wavelength.

### Sequencing

Clones that give signals at least 3 times above the background signal are propagated, used for DNA miniprep procedures (see procedures Qiagen miniPrep manual) and subjected to nucleotide sequence analysis. Sequencing is performed according to the Big Dye 1.1 kit accompanying manual (Applied Biosystems) using a reverse primer (CH1_Rev1, table 1) recognizing a 5' sequence of the CH1 region of the human IgG1 heavy chain (present in the Fab display vector MV1043, figures 3 and 12). The sequences of the murine VH regions are analyzed for diversity of DH and JH gene segments.

### Construction and expression of chimeric IgG1

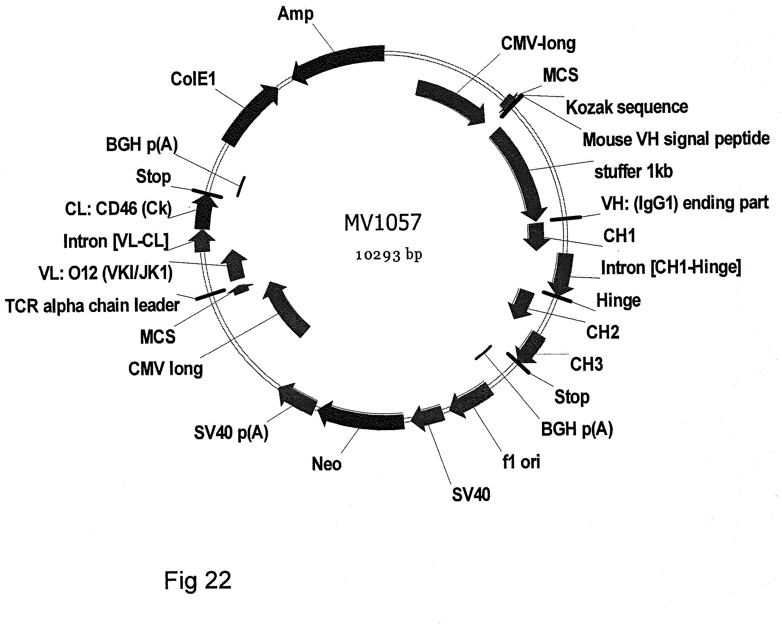

Vector MV1057 (figures 12 and 22) was generated by cloning the transgene (IGKV1-39) L chain fragment into a derivative of vector pcDNA3000Neo (Crucell, Leiden, The Netherlands) that contains the human IgG1- and kappa constant regions. VH regions are cloned into MV1057 and nucleotide sequences for all constructs are verified according to standard techniques. The resulting constructs are transiently expressed in HEK293T cells and supernatants containing chimeric IgG1 are obtained and purified using standard procedures as described before (Throsby, M. 2006. J Virol 80: 6982-92).

### IgG 1 binding and competition analysis

IgG1 antibodies are titrated in ELISA using IL6 coated plates as described above and an anti-human IgG peroxidase conjugate. Competition ELISAs to group antibodies based on epitope recognition are performed by incubating Fab phages together with IgG1 or with commercial antibodies against IL6 (e.g. Abcam cat. no. ab9324) in IL6 coated plates, followed by detection of bound Fab phage using an anti-M13 peroxidase conjugate.

### IgG 1 affinity measurements

The affinities of the antibodies to IL6 are determined with the Quantitative kinetic protocol on the Octet (ForteBio). Antibodies are captured onto an Anti-Human IgG Fc Capture biosensor and exposed to free IL6 and analyzed using proprietary software to calculate the Kd of each antibody.

### Functional activity of IL6 antibodies

To test the ability of the selected antibodies to inhibit binding between IL6 and IL6 receptor (IL6R), an ELISA based assay is used. Various concentrations of antibody are mixed with a fixed concentration (10 ng/ml) of biotinylated IL6 as described by Naoko et al. 2007, Can. Res. 67: 817-875. The IL6-antibody immune complex is added to immobilized IL6R. The binding of biotinylated IL6 to IL6R is detected with horseradish peroxidase-conjugated streptavidin. The reduction of ELISA signal is a measurement of inhibition. As positive control for inhibition of binding between IL6 and IL6R either anti-IL6R antibody (Abeam cat. no. ab34351; clone B-R6) or anti IL6 antibody (Abeam cat. no. ab9324) is used. *In vitro* blocking activity of the selected anti-IL6 antibodies is measured in a proliferation assay using the IL6 dependent cell line 7TD1. Briefly, cells are incubated with different concentrations of human IL6 with or without the anti-IL6 antibody. The available amount of IL6 determines the degree of proliferation. Thus if an added antibody blocks IL6 binding the proliferation readout is reduced compared to a non binding antibody control. Proliferation is measured by the incorporation of 5-bromo-2*'*-deoxy-uridine (BrdU) into the DNA using the BrdU proliferation kit (Roche cat. no. 11444611001) according to the manufacturer's instructions.

### Generation of anti-IL6 Oligoclonics

The most potent anti-IL6 antibodies are selected from each epitope group. The expression constructs expressing these antibodies are transfected into HEK293T cells in non-competing groups of three in different ratios (1:1:1; 3:1:1; 1:3:1; 1:1:3; 3:3:1; 1:3:3; 3:1:3; 10:1:1; 1:10:1; 1:1:10; 10:10:1; 1:10:10; 10:1:10; 3:10:1; 10:3:1; 1:10:3; 3:1:10; 10:1:3; 1:3:10). Antibody containing supernatants are harvested and purified and characterized as above.

### Complex formation and in vivo clearance of anti-IL6 Oligoclonics

To measure the ability of anti-IL6 Oligoclonics to form immune complexes and to analyze these complexes Size Exclusion Chromatography (SEC) is used according to the approach disclosed by Min-Soo Kim et al. (2007) JMB 374: 1374-1388 to characterize the immune-complexes formed with different antibodies to TNFα. Different molar ratios of the anti-IL6 Oligoclonics are mixed with human IL6 and incubated for 20 hours at 4°C or 25°C. The mixture is analyzed on an HPLC system fitted with a size exclusion column; different elution times are correlated to molecular weight using a molecular weight standards.
The ability of antibodies to form complexes with IL6 is correlated with their ability to rapidly clear the cytokine from the circulation in vivo. This is confirmed by measuring the clearance of radiolabelled IL6 from mice. Briefly, female, 6- to 8-week-old Balb/c mice are obtained and 18 hrs before the experiment, the animals are injected intravenously (IV) via the lateral tail vein with different doses of purified anti-IL6 Oligoclonics. On day 0 the mice are
injected IV with 50 microliters of radiolabeled IL-6 (1x10E7 cpm/mL) under the same conditions. Blood samples (approximately 50 microliters) are collected at several time intervals and stored at 4°C. The samples are centrifuged for 5 minutes at 4000 xg and the radioactivity of the serum determined. All pharmacokinetic experiments are performed simultaneously with three animals for each treatment.

### Generation of anti-IL6 Oligoclonics stable clones and preclinical development

A lead anti-IL6 Oligoclonic is selected based on the *in vitro* and *in vivo* potency as determined above. The murine VH genes are humanized according to standard methods and combined with the fully human IGKV1-39 light chain in an expression vector as described above. Examples of humanization methods include those based on paradigms such as resurfacing (Padlan, E. A., et al., (1991). Mol. Immunol., 28, 489), superhumanization (Tan, P., D. A., et al., (2002) J. Immunol., 169, 1119) and human string content optimization (Lazar, G. A., et al., (2007). Mol. Immunol., 44, 1986). The three constructs are transfected into PER.C6 cells at the predetermined optimal ratio (described above) under the selective pressure of G418 according to standard methods. A stable high producing anti-IL6 Oligoclonic clone is selected and a working and qualified master cell bank generated.

**Table 1 List of primers**

| DO- | **Primer** | **Sequence** |
|---|---|---|
| 0012 | **CH1_Rev1** | TGCCAGGGGGAAGACCGATG |
| 0656 | **MVH-1** | GCCGGCCATGGCC**GAGGTRMAGCTTCAGGAGTCAGGAC** |
| 0657 | **MVH-2** | GCCGGCCATGGCC**GAGGTSCAGCTKCAGCAGTCAGGAC** |
| 0658 | **MVH-3** | GCCGGCCATGGCCC**AGGTGCAGCTGAAGSASTCAGG** |
| 0659 | **MVH-4** | GCCGGCCATGGCC**GAGGTGCAGCTTCAGGAGTCSGGAC** |
| 0660 | **MVH-5** | GCCGGCCATGGCC**GARGTCCAGCTGCAACAGTCYGGAC** |
| 0661 | **MVH-6** | GCCGGCCATGGCC**CAGGTCCAGCTKCAGCAATCTGG** |
| 0662 | **MVH-7** | GCCGGCCATGGCCC**AGSTBCAGCTGCAGCAGTCTGG** |
| 0663 | **MVH-8** | GCCGGCCATGGCC**CAGGTYCAGCTGCAGCAGTCTGGRC** |
| 0664 | **MVH-9** | GCCGGCCATGGCC**CAGGTYCAGCTYCAGCAGTCTGG** |
| 0665 | **MVH-10** | GCCGGCCATGGCC**GAGGTCCARCTGCAACAATCTGGACC** |
| 0666 | **MVH-11** | GCCGGCCATGGCC**CAGGTCCACGTGAAGCAGTCTGGG** |
| 0667 | **MVH-12** | GCCGGCCATGGCC**GAGGTGAASSTGGTGGAATCTG** |
| 0668 | **MVH-13** | GCCGGCCATGGCC**GAVGTGAAGYTGGTGGAGTCTG** |
| 0669 | **MVH-14** | GCCGGCCATGGCC**GAGGTGCAGSKGGTGGAGTCTGGGG** |
| 0670 | **MVH-15** | GCCGGCCATGGCC**GAKGTGCAMCTGGTGGAGTCTGGG** |
| 0671 | **MVK-1G** | GCCGGCCATGGCC**GAGGTGAAGCTGATGGARTCTGG** |
| 0672 | **MVH-17** | GCCGGCCATGGCC**GAGGTGCARCTTGTTGAGTCTGGTG** |
| 0673 | **MVH-18** | GCCGGCCATGGCC**GARGTRAAGCTTCTCGAGTCTGGA** |
| 0674 | **MVH-19** | GCCGGCCATGGCC**GAAGTGAARSTTGAGGAGTCTGG** |
| 0675 | **MVH-20** | GCCGGCCATGGCC**GAAGTGATGCTGGTGGAGTCTGGG** |
| 0676 | **MVH-21** | GCCGGCCATGGCC**CAGGTTACTCTRAAAGWGTSTGGCC** |
| 0677 | **MVH-22** | GCCGGCCATGGCC**CAGGTCCAACTVCAGCARCCTGG** |
| 0678 | **MVH-23** | GCCGGCCATGGCC**CAGGTYCARCTGCAGCAGTCTG** |
| 0679 | **MVH-24** | GCCGGCCATGGCC**GATGTGAACTTGGAAGTGTCTGG** |
| 0680 | **MVH-25** | GCCGGCCATGGCC**GAGGTGAAGGTCATCGAGTCTGG** |
| 0681 | **ExtMVH-1** | CAGTCACAGATCCTCGC**G**AATT*GGCCC****AGCCGGCC*ATGGCCSANG** |
| 0682 | **ExtMVH-2** | CAGTCACAGATCCTCGCGAATT*GGCCC****AGCCGGCC*ATGGCCSANC** |
| 0683 | **MJH-Rev1** | GGGGGTGTCG**TTTTGGCTGAGGAGAC *GGTGACC* GTGG** |
| 0684 | **MJH-Rew2** | GGGGGTGTCGT**TTTGGCTGAGGAGAC *TGTGAGA* GTGG** |
| 0685 | **MJH-Rev3** | GGGGGTGTCGT**TTTGGCTGCAGAGAC *AGTGACC* AGAG** |
| 0686 | **MJH-Rev4** | GGGGGTGTCGT**TTTGGCTGAGGAGAC *GGTGACT* GAGG** |
| 0687 | **ExtMJH-** | GGGGGTGTCGT**TTTGGCTGAGGAGAC *GGTGACC* GTGG** |
| 0688 | **ExtMJH-** | GGGGGTGTCGT**TTTGGCTGAGGAGAC *GGTGACA* GTGG** |
| 0690 | **ExtMJH-** | GGGGGTGTCGT**TTTGGCTGAGGAGAC *GGTGACC AGAG*** |
| 0691 | **ExtMJH-** | GGGGGTGTCGT**TTTGGCTGAGGAGAC *GGTGACC* GAGG** |

**Table 2**

| Phage ELISA signal levels as measured at 450nm. TT-coated plates represent plates that were coated with tetanus toxoid. Thyroglobulin coated plates are used as negative controls. 10/10 and 15/15 indicate the number of wash steps with PBS-Tween during panning procedures. The 10/10 tetanus toxoid and 10/10 thyroglobulin plates and the 15/15 tetanus toxoid and 15/15 thyroglobulin plates are duplicates from each other except for the coating agent. OD values higher than 3 times the background are assumed specific. | | | | | | | | | | | | |
|---|---|---|---|---|---|---|---|---|---|---|---|---|
| **TT-coated plate 10/10 washings** | | | | | | | | | | | | |
| | **1** | **2** | **3** | **4** | **5** | **6** | **7** | **8** | **9** | **10** | **11** | **12** |
| **A** | 0.139 | 0.093 | 0.089 | 0.121 | 0.117 | 0.598 | 0.146 | 0.115 | 0.18 | 0.155 | 0.543 | **0.601** |
| **B** | 0.136 | 0.404 | 0.159 | 0.187 | 0.489 | 0.134 | 0.216 | 0.092 | 0.222 | 0.108 | 0.181 | **0.484** |
| **C** | 0.197 | 0.526 | 0.09 | 0.213 | 0.395 | 0.155 | 0.108 | 0.12 | 0.183 | 0.136 | 0.092 | **0.866** |
| **D** | 0.143 | 0.258 | 0.101 | 0.422 | 0.088 | 0.243 | 0.485 | 0.251 | 0.304 | 0.198 | 0.478 | **0.091** |
| **E** | 0.445 | 0.169 | 0.526 | 0.481 | 0.206 | 0.285 | 0.111 | 0.119 | 0.128 | 0.2 | 0.118 | **0.098** |
| **F** | 0.237 | 0.291 | 0.594 | 0.139 | 0.206 | 0.565 | 0.543 | 0.091 | 0.136 | 0.227 | 0.228 | **0.099** |
| **G** | 0.459 | 0.102 | 0.152 | 0.659 | 0.203 | 0.452 | 0.152 | 0.133 | 0.094 | 0.102 | 0.375 | 0.098 |
| **H** | 0.341 | 0.623 | 0.745 | 0.415 | 0.682 | 0.527 | 0.655 | 0.114 | 0.258 | 0.284 | 0.685 | 0.113 |
| | | | | | | | | | | | | |

| **TT-coated plate 15/15 washings** | | | | | | | | | | | | |
|---|---|---|---|---|---|---|---|---|---|---|---|---|
| | **1** | **2** | **3** | **4** | **5** | **6** | **7** | **8** | **9** | **10** | **11** | **12** |
| **A** | 0.247 | 0.582 | 0.421 | 0.428 | 0.133 | 0.082 | 0.262 | 0.079 | 0.343 | 0.414 | 0.095 | **0.292** |
| **B** | 0.065 | 0.364 | 0.073 | 0.042 | 0.049 | 0.071 | 0.046 | 0.103 | 0.078 | 0.057 | 0.048 | **0.155** |
| **C** | 0.081 | 0.044 | 0.066 | 0.082 | 0.225 | 0.444 | 0.203 | 0.362 | 0.122 | 0.047 | 0.052 | **0.309** |
| **D** | 0.092 | 0.11 | 0.59 | 0.22 | 0.33 | 0.544 | 0.058 | 0.159 | 0.047 | 0.174 | 0.086 | **0.05** |
| **E** | 0.469 | 0.577 | 0.206 | 0.304 | 0.13 | 0.749 | 0.431 | 0.062 | 0.167 | 0.049 | 0.056 | **0.049** |
| **F** | 0.846 | 0.07 | 0.561 | 0.656 | 0.882 | 0.094 | 0.383 | 0.13 | 0.152 | 0.098 | 0.134 | **0.048** |
| **G** | 0.537 | 0.052 | 0.49 | 0.105 | 0.337 | 0.193 | 0.514 | 0.294 | 0.068 | 0.35 | 0.525 | 0.05 |
| **H** | 0.061 | 0.306 | 0.157 | 0.853 | 0.054 | 0.534 | 0.102 | 0.235 | 0.441 | 0.412 | 0.565 | 0.061 |

| **Thyroglobulin-coated plate 10/10 washings** | | | | | | | | | | | | |
|---|---|---|---|---|---|---|---|---|---|---|---|---|
| | **1** | **2** | **3** | **4** | **5** | **6** | **7** | **8** | **9** | **10** | **11** | **12** |
| **A** | 0.047 | 0.051 | 0.045 | 0.043 | 0.051 | 0.044 | 0.046 | 0.042 | 0.047 | 0.048 | 0.049 | **0.05** |
| **B** | 0.042 | 0.042 | 0.042 | 0.042 | 0.043 | 0.041 | 0.041 | 0.042 | 0.043 | 0.045 | 0.042 | **0.046** |
| **C** | 0.044 | 0.043 | 0.043 | 0.044 | 0.043 | 0.044 | 0.043 | 0.042 | 0.043 | 0.041 | 0.044 | **0.046** |
| **D** | 0.045 | 0.044 | 0.044 | 0.044 | 0.045 | 0.046 | 0.045 | 0.056 | 0.045 | 0.049 | 0.048 | **0.73** |
| **E** | 0.046 | 0.045 | 0.046 | 0.044 | 0.045 | 0.044 | 0.044 | 0.044 | 0.047 | 0.046 | 0.047 | **0.926** |
| **F** | 0.048 | 0.045 | 0.044 | 0.046 | 0.044 | 0.043 | 0.044 | 0.046 | 0.046 | 0.046 | 0.046 | 0.792 |
| **G** | 0.051 | 0.048 | 0.045 | 0.045 | 0.044 | 0.043 | 0.048 | 0.045 | 0.048 | 0.051 | 0.045 | 0.053 |
| **H** | 0.064 | 0.05 | 0.049 | 0.047 | 0.05 | 0.051 | 0.047 | 0.046 | 0.047 | 0.047 | 0.047 | 0.056 |

| **Thyroglobulin-coated plate 15/15 washings** | | | | | | | | | | | | |
|---|---|---|---|---|---|---|---|---|---|---|---|---|
| | **1** | **2** | **3** | **4** | **5** | **6** | **7** | **8** | **9** | **10** | **11** | **12** |
| **A** | 0.036 | 0.049 | 0.045 | 0.044 | 0.046 | 0.047 | 0.046 | 0.042 | 0.042 | 0.043 | 0.042 | **0.041** |
| **B** | 0.045 | 0.042 | 0.041 | 0.043 | 0.043 | 0.043 | 0.045 | 0.045 | 0.047 | 0.048 | 0.044 | **0.045** |
| **C** | 0.049 | 0.047 | 0.047 | 0.046 | 0.046 | 0.046 | 0.045 | 0.047 | 0.046 | 0.045 | 0.045 | **0.052** |
| **D** | 0.047 | 0.049 | 0.048 | 0.048 | 0.048 | 0.048 | 0.047 | 0.052 | 0.048 | 0.046 | 0.048 | **0.456** |
| **E** | 0.049 | 0.047 | 0.047 | 0.047 | 0.047 | 0.049 | 0.047 | 0.048 | 0.047 | 0.046 | 0.048 | **0.412** |
| **F** | 0.05 | 0.047 | 0.046 | 0.046 | 0.046 | 0.046 | 0.046 | 0.046 | 0.046 | 0.047 | 0.048 | **0.528** |
| **G** | 0.05 | 0.048 | 0.045 | 0.045 | 0.046 | 0.049 | 0.048 | 0.046 | 0.053 | 0.049 | 0.05 | 0.057 |
| **H** | 0.057 | 0.05 | 0.046 | 0.045 | 0.047 | 0.049 | 0.047 | 0.047 | 0.046 | 0.047 | 0.053 | 0.048 |

**Table 3**

| Protein sequence analysis of ELISA positive tetanus toxoid binders. CDR3 sequence, CDR3 length, VH family members and specific name, JH origin and DH origin of the clones is indicated. | | | | | |
|---|---|---|---|---|---|
| **CDR3** | **CDR3 length** | **VH** | **DH** | **JH** | **V Gene family** |
| HGAYYTYDEKAWFAY | 15 | musIGHV192 | DSP2.11 | JH3 mouse | VH7183 |
| HGAYYTYDEKAWFAY | 15 | musIGHV192 | DSP2.11 | JH3 mouse | VH7183 |
| HGAYYTYDEKAWFAY | 15 | musIGHV192 | DSP2.11 | JH3 mouse | VH7183 |
| HGAYYTYDEKAWFAY | 15 | musIGHV192 | DSP2.11 | JH3 mouse | VH7183 |
| HGAYYTYDEKAWFAY | 15 | musIGHV192 | DSP2.11 | JH3 mouse | VH7183 |
| HGAYYTYDEKAWFAY | 15 | musIGHV192 | DSP2.11 | JH3 mouse | VH7183 |
| HGAYYTYDEKAWFAY | 15 | musIGHV192 | DSP2.11 | JH3 mouse | VH7183 |
| HGAYYTYDEKAWFAY | 15 | musIGHV192 | DSP2.11 | JH3 mouse | VH7183 |
| HGAYYTYDEKAWFAY | 15 | musIGHV192 | DSP2.11 | JH3 mouse | VH7183 |
| HGAFYTYDEKPWFAY | 15 | musIGHV192 | IGHD2-14*01 | JH3 mouse | VH7183 |
| HISYYRYDEEVSFAY | 15 | musIGHV192 | IGHD2-14*01 | JH3 mouse | VH7183 |
| HISYYRYDEEVSFAY | 15 | musIGHV192 | IGHD2-14*01 | JH3 mouse | VH7183 |
| GWRAFAY | 7 | musIGHV131 | DSP2.9 | JH3 mouse | VH7183 |
| GWRAFAY | 7 | musIGHV131 | DSP2.9 | JH3 mouse | VH7183 |
| GWRAFAY | 7 | musIGHV131 | DSP2.9 | JH3 mouse | VH7183 |
| DRGNYYGMDY | 10 | musIGHV178 | DSP2.1 | JH4 mouse | VH7183 |
| LGDYYVDWFFAV | 12 | muaIGHV165 | DFL16.1 | JH1 mouse | VH7183 |
| NFPAWFAF | 8 | musIGHV547 | DST4.3inv | JH3 mouse | VJH558 |
| NFPAWFAY | 8 | musIGHV547 | DSP2.1 | JH3 mouse | VJH558 |
| NFPAWFVY | 8 | musIGHV547 | DSP2.1 | JH3 mouse | VJH558 |
| SFTPVPFYYGYDWYFDV | 17 | musIGHV532 | DSP2.3 | JH1 mouse | VJH558 |
| SFTPVPFYYGYDWYFDV | 17 | musIGHV532 | DSP2.3 | JH1 mouse | VJH558 |
| SDYDWYFDV | 9 | musIGHV286 | DSP2.2 | JH1 mouse | VJH556 |
| SDYDWYFDV | 9 | musIGNV286 | DSP2.2 | JH1 mouse | VJH558 |
| DSKWAYYFDY | 10 | musIGHV532 | DST4.3 | JH2 mouse | VJH558 |
| GDYTGYGMDY | 10 | musIGHV125 | DSP2.13 | JH4 mouse | VHSM7 |
| GDYTGYGMDY | 10 | musIGHV125 | DSP2.13 | JH4 mouse | VHSM7 |
| GGYDGYWFPY | 10 | mus1GHV125 | OSP2.9 | JH3 mouse | VHSM7 |

**Table 4**

| Vector combinations that were transfected to HEK293T. | | | | | |
|---|---|---|---|---|---|
| **Code** | **HC vector** | **LC vector** | **Combined vector** | **Prep name** | **Conc. (µg/ml)** |
| A | x | 0817676_pSELECT_ 0815426 IGKV1-39 | x | PIGKV1-39/ P1 | - |
| B | x | 0817678_pSELECT_ 0815427 (IGLV2-14) | x | PIGLV2-14/ P1 | - |
| C | MV1110 | 0817676_pSELECT_ 0815426 (IGKV1-39) | x | PMV1110/ IGKV1-39/P1 | 11.0 |
| D | MV1110 | 0817678_pSELECT_ 0815427 IGLV2-14 | x | PMV1110/ IGLV2-14/P1 | 15.4 |
| E | x | x | MG1494 | MG1494/P2 | 16.1 |

**Table 5. HLA allotypes considered in T_{H}-epitope profiling. The corresponding serotypes are shown, as well as allotype frequencies in the Caucasian population (Klitz et al. Tissue Antigens 2003, 62:296-307; Gjertson and Terasake (eds) in: HLA 1997; Gjertson and Terasake (eds) in: HLA 1998; Castelli et al. J. Immunol. 2002, 169:6928-6934). Frequencies can add up to more than 100% since each individual has 2 alleles for each gene. If all allele frequencies of a single gene were known, they would add up to slightly less than 200% due to homozygous individuals.**

| HLA type | Serotype | Population % | HLA type | Serotype | Population % |
|---|---|---|---|---|---|
| DRB1*0101 | DR1 | 17.4 | DRB4*0103 | DR53 | 21 |
| DRB1*0102 | DR1 | 49 | DRB5*0101 | DR51 | 15.8 |
| DRB1*0301 | DR17(3) | 21.2 | DRB5*0202 | DR51 | 5.7 |
| DRB1*0401 | DR4 | 115 | DQA1*0101/DQB1*0501 | DQ5(1) | 205 |
| DRB1*0402 | DR4 | 3.1 | DQA1*0102/DQB1*C002 | DQ5(1) | 2.6 |
| DRB1*0404 | DR4 | 5.5 | DQA1*0102/DQB1*0602 | DQ6(1) | 26.5 |
| DRB1*0405 | DR4 | 2.2 | DQA1*0102/DQB1*0604 | DQ6(1) | 67 |
| DRB1*0407 | DR4 | <2 | DQA1*0103DQB1*0603 | DQ6(1) | 11 |
| DRB1*0701 | DR7 | 23.4 | DQA1*0104/DQB1*0503 | DQ5(1) | 4 |
| DRB1*0801 | DR8 | 3.3 | DQA1*0201DQB1*0202 | DQ2 | 20.9 |
| DRB1*0802 | DR8 | <2 | DQA1*0201/DQB1*0303 | DQ9(3) | 7.2 |
| DRB1*0901 | DR9 | <2 | DQA1*03010QB1*0301 | DQ7(3) | 12.5 |
| DRB1*1101 | DR11(5) | 17 | DQA1*0301/DQB1*0302 | DQ8(3) | 18.3 |
| DRB1*1104 | DR11(5) | 5.7 | DQA1*0401/DQB1*0402 | DQ4 | 4.5 |
| DRB1*1201 | DR12(5) | 31 | DQA1*0501/DQB1*0201 | DQ2 | 24.6 |
| DRB1*1301 | DR13(6) | 15.4 | DQA1*0501DQB1*0301 | DQ7(3) | 209 |
| DRB1*1302 | DR13(6) | 10.8 | DPA1*0103/DPB1*0201 | DPw2 | 199 |
| DRB1*1401 | DR14(6) | 4.2 | DPA1*0103/DPB1*0401 | DPw4 | 65.1 |
| DRB1*1501 | DR15(2) | 13.2 | DPA1*0103/DPB1*0402 | DPw4 | 24.3 |
| DRB1*1601 | DR16(2) | 5.5 | DPA1*0201/DPB1*0101 | DPw1 | 6.3 |
| DRB3*0101 | DR52 | 24.6 | DPA1*0201/DPB1*0301 | DPw3 | <2 |
| DRB3*0202 | DR52 | 43 | DPA1*0201/DPB1*0501 | DPw5 | <2 |
| DRB3*0301 | DR52 | 10 | DPA1*0201/DPB1*0901 | | 2 4 |
| DRB4*0101 | DR53 | 25.5 | | | |

**Table 6. T_{H} epitope counts for IGKV1-39. Peptides binding to multiple HLAs of the same group (DRB1, DRB3/4/5, DP, DQ) are counted as one. Values between brackets refer to germline-filtered peptides.**

| | DRB1 | | DRB3/4/5 | | DQ | | DP | |
|---|---|---|---|---|---|---|---|---|
| | Strong | Medium | Strong | Medium | Strong | Medium | Strong | Medium |
| Merus IGKV1-39 | 0 (+ 6) | 0 (+ 16) | 0 (+ 0) | 0(+ 5) | 0 (+ 3) | 0 (+ 9) | 0 (+ 0) | 0 (+ 9) |

**Table 7. Mapping of Epibase® predictions for Merus IGKV1-39 in the classical 15-mer peptide format. This table shows the allotype count of critical epitopes and implicated serotypes for each of the 15-mers spanning the Merus IGKV1-39 sequence.**

| 15mer | Start position | 15-mer sequence | Allotype count | Implicated serotypes |
|---|---|---|---|---|
| 1 | 1 | DIQMTQSPSSLSAFV | 6 | DR1, DR4, DR7, DR9 |
| 2 | 4 | MTQSPSSLSASVGDR | 5 | DR1, DR4, DR9 |
| 3 | 7 | SPSSLSASVGDRVTI | 0 | |
| 4 | 10 | SLSASVGDRVTITCR | 0 | |
| 5 | 13 | ASVGDRVT ITCRASQ | 0 | |
| 6 | 16 | GDRVT ITCRASQSIS GDRVTTTCRASQSIS | 2 | DR11(5), DR7 |
| 7 | 19 | VTITCARASQSISSYL | 4 | DQ2 DR11(5), DR4, DR7 |
| 8 | 22 | TCRASQSISSYILNWY | 2 | DQ2, DR4 |
| 9 | 25 | ASQSISSYLNWYQQK | 5 | DR13(6), DR15(2), DR4 |
| 10 | 28 | SISSYLNWYQQKPGK | 8 | DR12(5), DR13(6), DR15(2), DR16(2), DR4, DR8 |
| 11 | 31 | SYLNWYQQKPGKAPK | 10 | DR1, DR12(5) DR16(2), DR4, DR51, DR8 |
| 12 | 34 | NWYQ QKPGKAPKLLI | 9 | DR1, DR15(2), DR4, DR51, DR8 |
| 13 | 37 | QQKPGKAPKLLIYAA | 7 | DQ4, DR1, DR11(5), DR15(2), DR51, DR8 |
| 14 | 40 | PGKAPKLLIAASSL | 7 | DQ4, DR1, DR11(5), DR4,DR8 |
| 15 | 43 | APKLLIYAASSLQSG | 15 | DR1, DR11(5), DR12(5), DR13(6), DR14(6), DR15(2), DR4 DR51, DR8, DR9 |
| 16 | 46 | LLIYAASSLQSGVPS | 15 | DR1, DR11(5), DR12(5), DR13(6), DR14(6), DR15(2), DR4 DR51, DR8, DR9 |
| 17 | 49 | YAASSLQSGVPSRFS | 1 | DR15(2) |
| 18 | 52 | SSLQSGVPSRFSGSG | 1 | DR15(2) |
| 19 | 55 | QSGVPSRFSGSGSGT | 0 | |
| 20 | 58 | VPSRFSGSGSGTDFT | 0 | |
| 21 | 61 | RFSGSGSGTDFTLTI | 0 | |
| 22 | 64 | GSGSGTDFTLTISSL | 1 | DR52 |
| 23 | 67 | SGTDFTLTISSLQPE | 4 | DR4, DR52, DR7, DR9 |
| 24 | 70 | DFTLTISSLQPEDFA | 4 | DQ2, DR4, DR7, DR9 |
| 25 | 73 | LTISSLQPEDFATYY | 1 | DQ2 |
| 26 | 76 | SSLQPEDFATYYCQQ | 0 | |
| 27 | 79 | QPEDFATYYCQQSYS | 1 | DR4 |
| 28 | 82 | DFATYYCQQSYSTPP | 5 | DR4, DR51, DR7 |
| 29 | 85 | TYYCQQSY STPPTFG | 4 | DR4, DR51, DR7 |
| 30 | 88 | CQQSYSTPPTFGQGT | 0 | |
| 31 | 91 | SYSTPPTFGQGTKVE | 0 | |
| 32 | 94 | TPPTFGQGTKVEIK | 0 | |

**Table 8: The V_{H} gene from PG1433 paired with various light chain genes with differing rates of amino acid mutation were compared for production levels with the original clone containing the IGKV1-39 gene.**

| **IgG name** | **Light chain gene** | **Number of amino acid mutations** | **concentration (µg/ml)** |
|---|---|---|---|
| *PG1433* | ***1*-*39*** | ***0*** | *63,45.5,38.6 (avg* = ***49**)* |
| PG1631 | 1-12 | **4** | 10.5 |
| PG1632 | 1-27 | 7 | 9.3 |
| PG1634 | 1D-12 | 10 | 10.8 |
| PG1635 | 1D-33 | 6 | 10.2 |
| PG1642 | 1-5 | 8 | 7.1 |
| PG1644 | 1-9 | 3 | 7.8 |
| PG1650 | 1D-39 | 3 | 9.1 |
| PG1652 | 2D-28 | 3 | 7.1 |
| PG1653 | 3-15 | 14 | 7 |
| PG1654 | 3-20 | 2 | 5.2 |
| PG1674 | 1-40 | 7 | 8.2 |
| PG1678 | 2-11 | 2 | 8.1 |
| PG1680 | 2-14 | 15 | 10.8 |
| PG1682 | 3-1 | 13 | 9.9 |
| PG1683 | 6-57 | 6 | 13.9 |

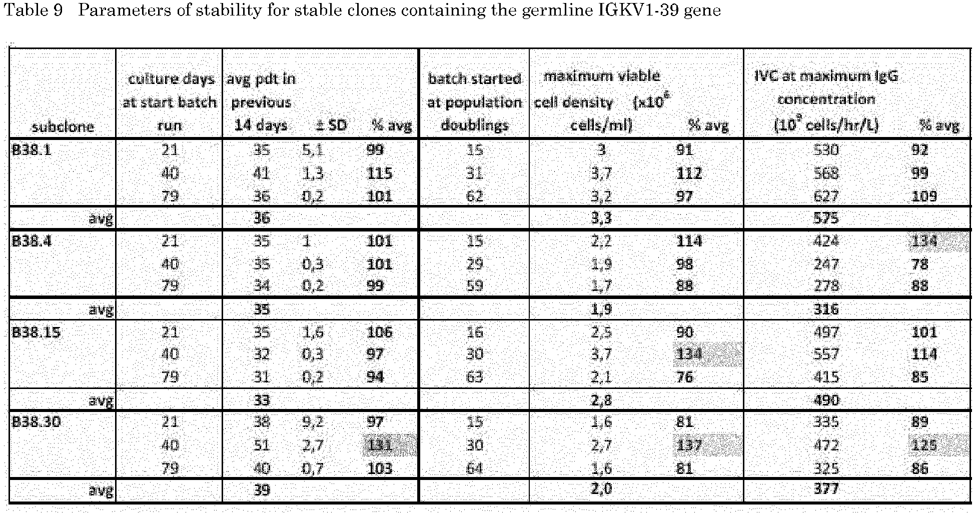

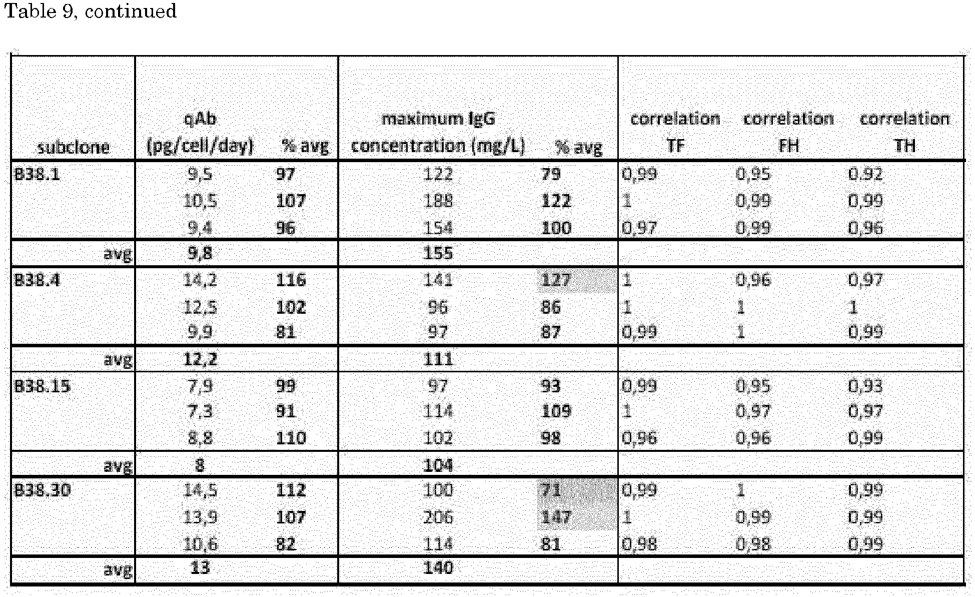

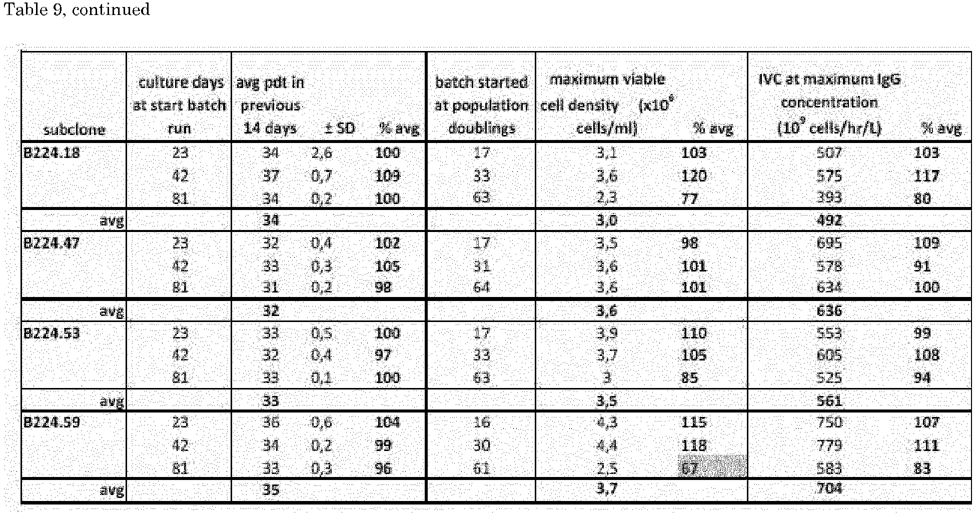

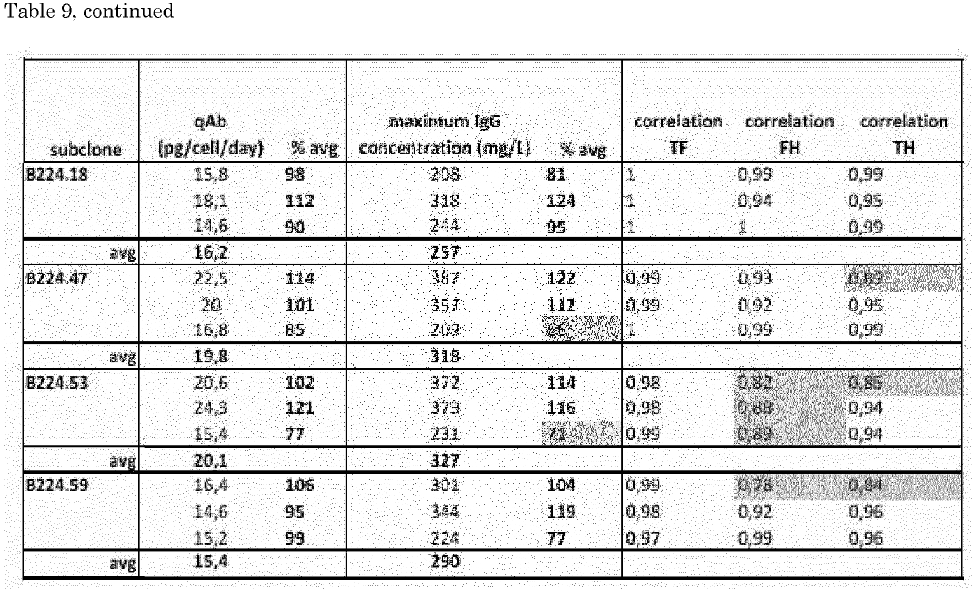

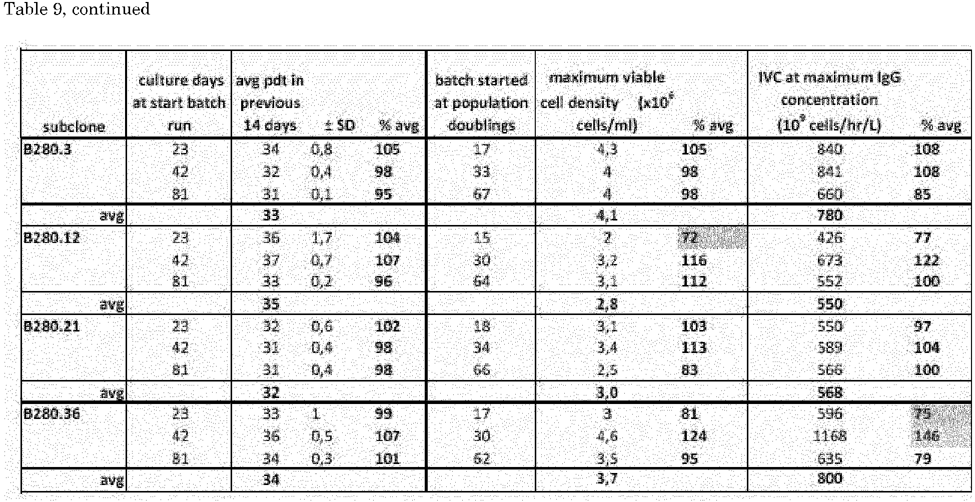

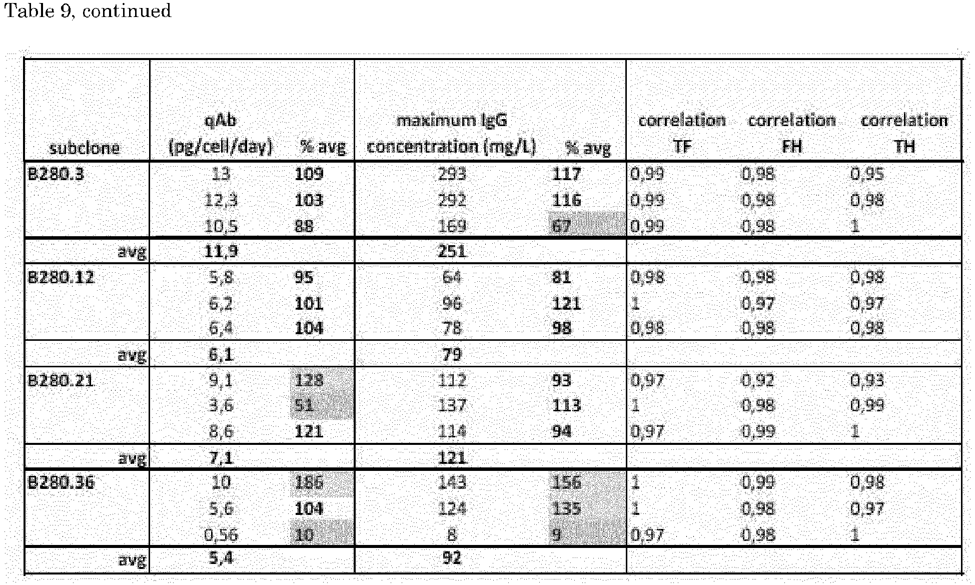

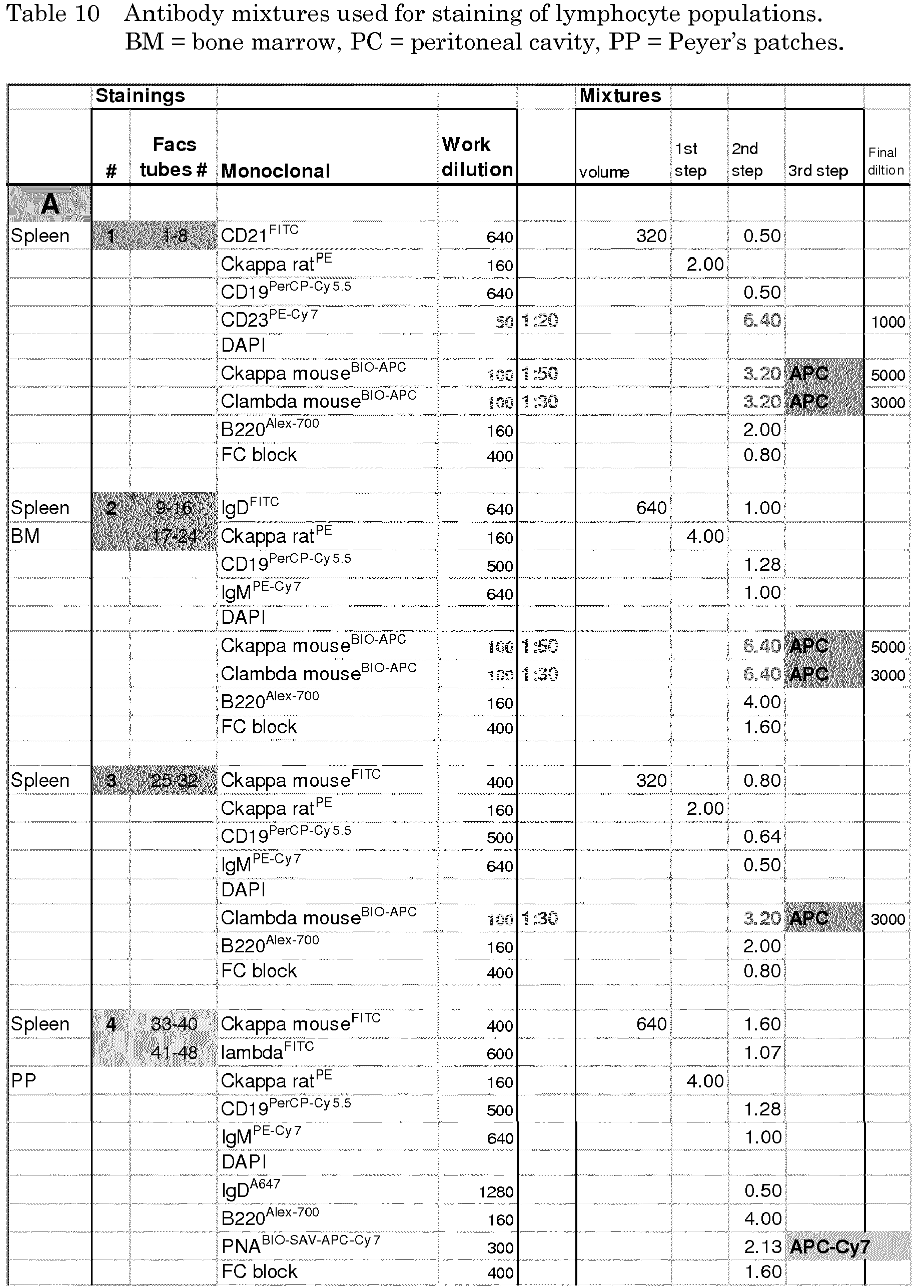

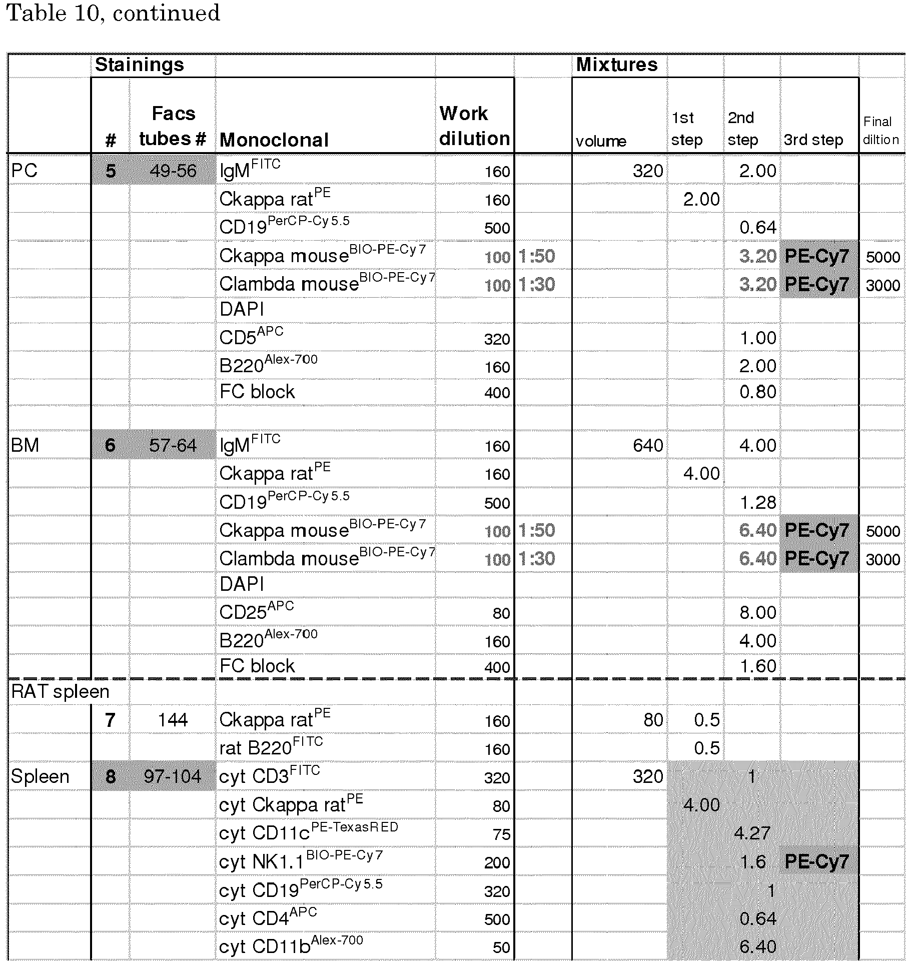

**Table 11 Numbers of lymphocytes harvested from the bone marrow and spleen of wild type and transgenic mice**

| **Bone Marrow** | | | |
|---|---|---|---|
| | *10a6/ml | total vol | total cells |
| | cells | (ml) | *10⁶ |
| **Wt** | 18.82 | 5.05 | 95.0 |
| **Wt** | 19.24 | 4.96 | 95.4 |
| **CD19-Cre** | 23.42 | 5.08 | 119.0 |
| **CD19-Cre** | 20.58 | 4.82 | 99.2 |
| **CD19-Cre** | 25.77 | 5.15 | 132.7 |
| **CD19-Cre / HuVk1** | 17.71 | 5.06 | 89.6 |
| **CD19-Cre / HuVk1** | 12.60 | 5.33 | 67.2 |
| **CD19-Cre / HuVk1** | 18.13 | 5.27 | 95.5 |

| **Spleen** | | | |
|---|---|---|---|
| | *10a6/ml | total vol | total cells |
| | cells | (ml) | *10⁶ |
| **Wt** | 41.70 | 5.36 | 223.5 |
| **Wt** | 37.85 | 4.71 | 178.3 |
| **CD19-Cre** | 60.19 | 3.77 | 226.9 |
| **CD19-Cre** | 35.06 | 3.66 | 128.3 |
| **CD19-Cre** | 80.69 | 4.60 | 371.2 |
| **CD19-Cre / HuVk1** | 51.67 | 4.48 | 231.5 |
| **CM9-Cre / HuVk1** | 58.80 | 6.24 | 366.9 |
| **CD19-Cre / HuVk1** | 24.37 | 6.25 | 152.3 |

The following pages 88 to 93 contain specific embodiments.
1. A transgenic non-human mammal comprising, at least in its B cell lineage, a nucleic acid encoding at least an immunoglobulin light chain or heavy chain, wherein the heavy- or light chain encoding sequence is provided with a means that renders it resistant to DNA rearrangements and/or somatic hypermutations.
2. A transgenic non-human mammal according to 1, which is a rodent, preferably a mouse.
3. A transgenic non-human mammal according to any one of
   1-2, wherein the light/heavy chain encoding sequence is integrated in the genome of the non-human mammal.
4. A transgenic non-human mammal according to 3, wherein the integration is in a locus that is resistant to silencing.
5. A transgenic non-human mammal according to 4, wherein the integration is in the Rosa-locus.
6. A transgenic non-human mammal according to any one of
   1-5, wherein the light chain encoding nucleic acid is provided with a means that allows expression of said nucleic acid essentially limited to cells of B cell lineage.
7. A transgenic non-human mammal according to 6, wherein the light chain encoding nucleic acid is provided with a means that allows expression of the light chain encoding nucleic acid predominantly during a certain stage of the development of B cells.
8. A transgenic non-human mammal according to 7, wherein said means comprises a promoter selected from the group of CD19, CD20, (µHC, VpreB1, VpreB2, VpreB3, λ5, Igα, Igβ, κLC, λLC, and BSAP (Pax5).
9. A transgenic non-human mammal according to 7 or 8, wherein said means comprises a cre-lox system or the like.
10. A transgenic non-human mammal according to any one of
   1-9, wherein the light chain encoding sequence encodes a light chain capable of pairing with at least two different heavy chains encoded by the non-human mammal.
11. A transgenic non-human mammal according to any one of
   1-10, wherein at least one of the endogenous loci encoding an endogenous light chain is functionally silenced.
12. A transgenic non-human mammal according to anyone of
   1-11, wherein the endogenous κ light chain locus is functionally silenced.
13. A transgenic non-human mammal according to any one of
   1-12, wherein the sequence of the light chain encoding sequence is a human vκ sequence.
14. A transgenic non-human mammal according to 13 wherein the light chain encoding sequence is a germline-like sequence.
15. A transgenic non-human mammal according to 14, wherein the light chain encoding sequence is a germline sequence.
16. A transgenic non-human mammal according to 15, wherein the germline sequence is based on 012.
17. A transgenic non-human mammal according to 1 wherein the light chain encoding nucleic acid comprises in 5'-3' direction: a vκ promoter, a human leader, a human V gene, optionally a MοEκi enhancer, a rat constant region (κ) and optionally a (truncated) MoEκ3' enhancer.
18. A transgenic non-human animal which has been provided with an expression cassette for the expression of a desired proteinaceous molecule in cells during a certain stage of development in cells developing into mature B cells, said cassette comprising:
   means for preventing silencing of expression of the desired proteinaceous molecule after introduction into a host cell, and
   means for timing expression of the desired proteinaceous molecule with the desired developmental stage of the host cell.
19. A transgenic non-human animal which has been provided with an expression cassette according to 18, wherein said means for timing expression is a promoter of which the activity is essentially limited to the certain stage of development.
20. A transgenic non-human animal which has been provided with an expression cassette according to 18 or 19 wherein said certain stage starts at a stage immediately preceding or coinciding with the onset of the expression of light chain molecules by said cells at a certain stage of development into a mature B cell.
21. A transgenic non-human animal which has been provided with an expression cassette according to 19 or 20, wherein said promoter is selected from the group consisting of the CD 19 promoter, the Ig-a promoter, the Ig-β promoter, the µhc promoter, the vk promoter, or analogues and/or homologues thereof.
22. A transgenic non-human animal which has been provided with an expression cassette according to any one of 19-21, wherein said promoter drives the expression of a cre-like protein.
23. A transgenic non-human animal which has been provided with an expression cassette according to 22 whereby the sequence encoding the desired proteinaceous molecule is activated by the action of said cre-like protein.
24. A transgenic non-human animal which has been provided with a set of nucleic acids that are expression cassettes, wherein one nucleic acid comprises an expression cassette according to 22 and the second nucleic acid comprises a sequence encoding a desired proteinaceous molecule under control of a constitutive promoter which can be activated by the action of a cre-like protein.
25. A transgenic non-human animal which has been provided with an expression cassette according to 23 or 24, wherein said activation is achieved by removal of a "stop".
26. A transgenic non-human animal which has been provided with an expression cassette according to anyone of 18-25, wherein said desired proteinaceous molecule is a polypeptide chain of an immunoglobulin.
27. A transgenic non-human animal which has been provided with an expression cassette according to 26, wherein said polypeptide chain is a light chain.
28. A transgenic non-human animal which has been provided with an expression cassette according to 27, wherein the sequence encoding said polypeptide chain is rendered essentially incapable of rearrangement.
29. A transgenic non-human animal which has been provided with an expression cassette according to 27 or 28, wherein said light chain is a germ line like light chain.
30. A transgenic non-human animal which has been provided with an expression cassette according to 29, wherein said germ line like light chain is 012.
31. A transgenic non-human animal which has been provided with an expression cassette according to 28-30, wherein said rearrangement is prevented by the absence of elements at least partially responsible for somatic hypermutation such as the MοEκi enhancer.
32. A transgenic non-human animal which has been provided with an expression cassette according to anyone of 18-31, wherein said means for prevention of silencing are means for insertion into a locus in the genome of the host cell that is resistant to silencing.
33. A transgenic non-human animal which has been provided with an expression cassette according to 32, wherein said means for insertion are means for homologous recombination into said site resistant to silencing.
34. A transgenic non-human animal which has been provided with an expression cassette according to 32 or 33 wherein said locus is the rosa-locus.
35. A transgenic non-human animal which has been provided with an expression cassette according to any one of 1-16, which comprises in 5'-3' direction: a vκ promoter, a human leader, a human V gene, optionally a MοEκi enhancer, a rat constant region (κ) and optionally a (truncated) MoEκ3' enhancer.
36. A method for producing a desired antibody comprising exposing a non-human mammal according to any one of 1-35 to an antigen such that an antibody response is induced and isolating the antibodies specific for the antigen
37. A method for producing a desired antibody comprising exposing a non-human mammal according to any one of 1-36 to an antigen such that an antibody response is induced and isolating cells producing such antibodies, culturing and optionally immortalizing said cells and harvesting said antibodies.
38. A method for producing a desired antibody comprising exposing a non-human mammal according to any one of 1-37 to an antigen such that an antibody response is induced and isolating a nucleic acid encoding at least part of such an antibody, inserting said nucleic acid or a copy or a derivative thereof in an expression cassette and expressing said antibody in a host cell.
39. Progeny of a transgenic non-human animal according to any one of 1-17, the progeny comprising, at least in its B-cell lineage, a heavy- or light chain encoding sequence together with a means that renders the sequence resistant to DNA rearrangements and/or somatic hypermutations.
40. Progeny of a transgenic non-human animal according to any one of 18-35, the progeny comprising an expression cassette for the expression of a desired proteinaceous molecule in cells during a certain stage of development in cells developing into mature B cells.
41. A cell that is isolated from a transgenic non-human animal according to any one of 1-17, the cell comprising a heavy- or light chain encoding sequence together with a means that renders the sequence resistant to DNA rearrangements and/or somatic hypermutations.
42. A cell that is isolated from a transgenic non-human animal according to any one of 18-35, the cell comprising an expression cassette for the expression of a desired proteinaceous molecule in cells during a certain stage of development in cells developing into mature B cells.

## Claims

1. A method for the generation of monoclonal antibodies (mAbs), wherein the antibodies constitute the basis for mixtures of antibodies produced by clonal cells the method comprising immunizing a transgenic murine mammal comprising integrated in its genome a nucleic acid molecule encoding a rearranged human immunoglobulin light chain variable region, wherein said light chain is capable of pairing with at least two different heavy chains encoded by the murine mammal, such that variety in specificity of antibodies is retained through rearrangements and hypermutations in the heavy chains, and wherein said light chain further comprises a murine light chain constant region, and isolating a nucleic acid molecule encoding at least part of such an antibody, inserting said nucleic acid molecule or a copy or a derivative thereof in an expression cassette and expressing said antibody in a host cell.

2. The method according to claim 1 , wherein said mAbs comprise mAbs that are directed against different epitopes on at least one of the targets.

3. The method according to claim 1 , wherein said mAbs comprise mAbs that are directed against different targets,

4. The method according to claim 3 , wherein said mAbs are directed against one or more members of the EGF-receptor family.

5. A method according to any one of claim 1-4, wherein the transgenic murine mammal is a mouse.

6. A method according to any one of claim 1-5, wherein said antibodies are directed against cellular targets..

7. A method according to claim 6, wherein said cellular targets are human surface-expressed or soluble proteins or carbohydrate molecules.

8. A method according to claim 6, wherein said cellular targets are surface-expressed proteins or carbohydrate molecules that are expressed on the surface of bacteria, viruses, and other pathogens, especially of humans.

9. A method according to claim 6 or 7, wherein said targets include cytokines and chemokines, receptor molecules for cytokines and chemokines, immunoglobulin (Ig) superfamily receptors, tumor necrosis factor family receptors, serine/threonine-protein kinase receptors, tyrosine kinase receptors, EGF receptor family members, insulin receptor family members, PDGF receptor family members, Hepatocyte growth factor receptor family members, Trk receptor family members, AXL receptor family members, LTK receptor family members, TIE receptor family members, ROR receptor family members, DDR receptor family members, KLG receptor family members, RYK receptor family members, MuSK receptor family members, and vascular endothelial growth factor receptor (VEGFR) family members.

10. A method according to claim 9, wherein the antibodies are directed against targets that are over-expressed or selectively expressed in tumors.

11. A method according to claim 10, wherein the targets are VEGF, CD20, CD38, CD33, CEA, EpCAM, PSMA, CD54, Lewis Y, CD52, CD40, CD22, CD51/CD61, CD74, MUC-1, CD38, CD19, CD262 (TRAIL-R2), RANKL, CTLA4, and CD30.

12. A method according to claim 9, wherein the antibodies are directed against targets that are involved in chronic inflammation.

13. A method according to claim 12, wherein the targets are CD25, CD11a, TNF, CD4, CD80, CD23, CD3, CD14, IFNgamma, CD40L, CD50, CD122, TGFbeta and TGFalpha.

14. A method according to claim 6 or 8, wherein said targets include surface markers of influenza A and B viruses such as hemagglutinin (HA) and neuraminidase (NA), filoviruses such as Ebola virus, rabies, measles, rubella, mumps, flaviviruses such as Dengue virus types 1-4, tick-borne encephalitis virus, West Nile virus, Japanese encephalitis virus, and Yellow fever virus, Paramyxoviruses including Paramyxovirus such as Parainfluenza 1, 3, Rubulavirus such as Mumpsvirus and Parainfluenza 2, 4, Morbillivirus, and Pneumovirus such as Respiratory syncytial virus, Vaccinia, small pox, coronaviruses, including Severe Acute Respiratory Syndrome (SARS) virus, hepatitis virus A, B and C, Human Immunodeficiency Virus, Herpes viruses, including cytomegalovirus, Epstein Barr virus, Herpes simplex virus, and Varicella zoster virus, parvoviruses such as, for example, B19; Legionella pneumophila; Listeria monocytogenes; Campylobacter jejuni; Staphylococcus aureus; E. coli 0157:H7; Borrelia burgdorferi; Helicobacter pylori; Ehrlichia chaffeensis; Clostridium difficile; Vibrio cholera; Salmonella enterica Serotype Typhimurium; Bartonella henselae; Streptococcus pyogenes (Group A Strep); Streptococcus agalactiae (Group B Strep); Multiple drug resistant S. aureus (e.g. MRSA); Chlamydia pneumoniae; Clostridium botulinum; Vibrio vulnificus; Parachlamydia pneumonia; Corynebacterium amycolatum; Klebsiella pneumonia; Linezolid-resistant enterococci (E. faecalis and E. faecium); and Multiple drug resistant Acinetobacter baumannii.

15. A method according to any one of claims 1-14, further comprising the step of isolating the antibodies specific for the antigen from the immunized transgenic murine mammal.

16. A method according to any one of claims 1-14, further comprising the step of isolating cells producing such antibodies, culturing and harvesting said antibodies.

17. A method according to any one of claims 1-14, wherein said isolated nucleic acid molecule encoding at least part of such an antibody encodes at least a heavy chain variable region.

18. A method according to any one of claims 1-17, wherein the antibodies constitute the basis for mixtures of antibodies produced by clonal cells.

19. A method according to claim 18, wherein the antibodies comprise antibodies that are directed against different epitopes on at least one target.

20. A method according to claim 18, wherein the antibodies comprise antibodies that are directed against different targets.
